# EUROPEAN PATENT SPECIFICATION

(11) **EP 2 885 404 B1**
(45) Date of publication and mention of the grant of the patent: **25.04.2018**
(21) Application number: 13829529.0
(22) Date of filing: 15.08.2013
(51) Int. Cl.: C12N 9/08, C12N 15/53, C07H 21/04, C07K 14/37

(54) **LIGNIN DEGRADING ENZYMES FROM MACROPHOMINA PHASEOLINA AND USES THEREOF**
LIGNINABBAUENDE ENZYME AUS MACROPHOMINA PHASEOLINA UND VERWENDUNGEN DAVON
ENZYMES D'ALTÉRATION DE LA LIGNINE PROVENANT DE MACROPHOMIA PHASEOLINA ET UTILISATIONS DE CELLES-CI

(30) Priority: 16.08.2012 US 201261683913 P
(43) Date of publication of application: 24.06.2015
(73) Proprietor: Bangladesh Jute Research Institute, Dhaka 1207 (BD)
(72) Inventor: ALAM, Maqsudul, Honolulu, HI 96816 (US); ISLAM, Mohammed, Shahidul, Dhaka 1207 (BD); HOSSEN, Mohammed, Mosaddeque, Dhaka 1207 (BD); HAQUE, Mohammed, Samiul, Dhaka 1207 (BD); ALAM, Mohammed, Monjurul, Dhaka 1207 (BD)
(74) Representative: HGF Limited
(86) International application number: PCT/US2013/055199
(87) International publication number: WO 2014/028773

(56) References cited:
- WO-A2-2009/005564
- WO-A2-2009/108941
- WO-A2-2012/027282
- US-B2- 7 423 195
- SCHALCH H ET AL: "MOLECULAR CLONING AND SEQUENCES OF LIGNIN PEROXIDASE GENES OF PHANEROCHAETE-CHRYSOSPORIUM", 1989, MOLECULAR AND CELLULAR BIOLOGY, VOL. 9, NR. 6, PAGE(S) 2743-2747, XP002751442, ISSN: 0270-7306 * figure 2 *
- BARNETT PHILIP ET AL: "Isolation, characterization, and primary structure of the vanadium chloroperoxidase from the fungus Embellisia didymospora", 4 September 1998 (1998-09-04), JOURNAL OF BIOLOGICAL CHEMISTRY, VOL. 273, NR. 36, PAGE(S) 23381-23387, XP002751443, ISSN: 0021-9258 * figure 4 *
- RUIZ-DUENAS F J ET AL: "MOLECULAR CHARACTERIZATION OF A NOVEL PEROXIDASE ISOLATED FROM THE LIGNINOLYTIC FUNGUS PLEUROTUS ERYNGII", 1 January 1999 (1999-01-01), MOLECULAR MICROBIOLOGY, WILEY-BLACKWELL PUBLISHING LTD, GB, PAGE(S) 223 - 235, XP008013345, ISSN: 0950-382X * figure 3 *
- LARRONDO LUIS F ET AL: "Characterization of a multicopper oxidase gene cluster in Phanerochaete chrysosporium and evidence of altered splicing of the mco transcripts", August 2004 (2004-08), MICROBIOLOGY (READING), VOL. 150, NR. PART 8, PAGE(S) 2775-2783, XP002751444, ISSN: 1350-0872 * figures 1-3 *
- MD SHAHIDUL ISLAM ET AL: "Tools to kill: Genome of one of the most destructive plant pathogenic fungi Macrophomina phaseolina", 19 September 2012 (2012-09-19), BMC GENOMICS, BIOMED CENTRAL LTD, LONDON, UK, PAGE(S) 493, XP021106747, ISSN: 1471-2164 * page 6, left-hand column, paragraph 1 * -& DATABASE UniProt [Online] 29 May 2013 (2013-05-29), XP002751445, Database accession no. K2RG38 -& DATABASE UniProt [Online] 29 May 2013 (2013-05-29), XP002751446, Database accession no. K2SCB4 -& DATABASE UniProt [Online] 29 May 2013 (2013-05-29), XP002751447, Database accession no. K2SCI0
- AKBAR MOHAMMED TOUAHA ET AL: "An insight into the lignin peroxidase of Macrophomina phaseolina.", 2013, BIOINFORMATION 2013, VOL. 9, NR. 14, PAGE(S) 730 - 735, XP002751448, ISSN: 0973-2063 * page 732, left-hand column, paragraph 2 - page 733, left-hand column, paragraph 2 *

## Description

### RELATED APPLICATIONS

This application claims the benefit of priority to United States Provisional Patent Application serial number 61/683913, filed August 16, 2012; the contents of which are hereby incorporated by reference.

### FIELD OF INVENTION

This invention relates to the lignin degrading proteins/enzymes and genes of *M*. *phaseolina.* More specifically, the present invention discloses nucleotide acid sequences of these proteins and facilitates the reduction lignin content of lignocellulosic materials. The present invention also relates to methods for selecting and isolating enzymes from *M*. *phaseolina* that are capable of degrading lignin, processes for cloning a gene segment from *M. phaseolina,* and methods of using the enzyme product of the gene segment. Particularly it can be used in the production or processing of pulps, woodpulps, methanol and textile.

### BACKGROUND OF THE INVENTION

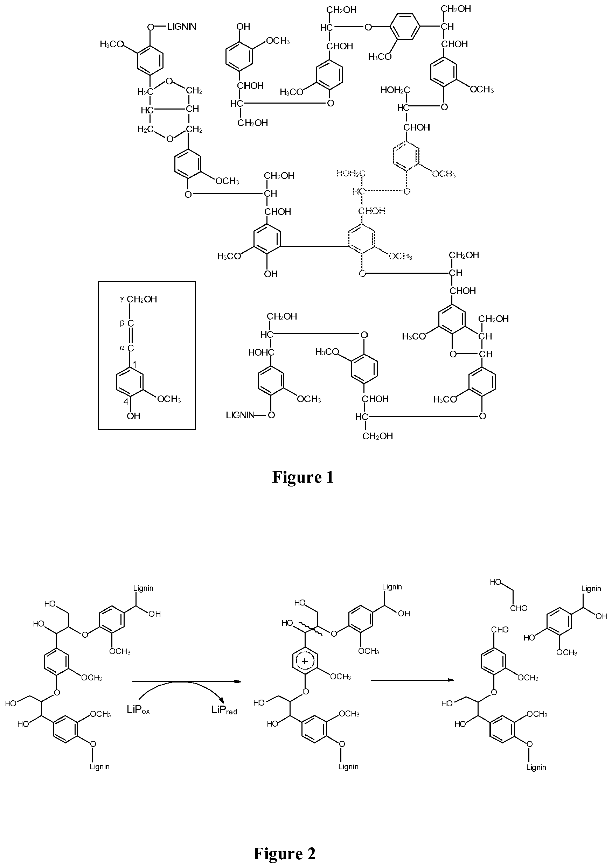

Lignin is the the second most abundant organic polymer, exceeded only by cellulose. It binds with cellulose fibres which make up approximately one-fourth of the weight of dry wood. This aromatic polymer is recalcitrant to degradation. Lignin is covalently associated with hemicelluloses in the cell wall via numerous types of linkage. Among the linkage, the most abundant lignin substructure is the β-aryl ether, which accounts for approximately 40% of the inter phenylpropane linkages (Higuchi T 1990. Lignin biochemistry: biosynthesis and biodegradation. Wood Sci. Technol. 24: 23-63). In addition lignin does not possess any repeating units like other biopolymer such as cellulose, protein, starch etc. Ether bonds are often prevalent, providing a variety of linkages to the numerous aromatic residues. These structural features dictate constraints on the degradation of the lignin. A portion of a typical structure is illustrated in Figure 1 and that the arylglycerol β-aryl ether structure red color in the figure is quantitatively the most important linkage, constituting at least 40% of the polymer. But, biodegradation of lignin is a prerequisite for the processing of biofuel, and wood pulp from plant raw materials. The improving of lignin degradation would drive the output from biofuel processing to better gain or better efficiency factor.

Lignin is a component of lignocellulose, which must be degraded to allow efficient use of cellulosic material for saccharification, paper production, biofuel production or upgradation of fodder. Ligincellulose degradation is a multienzymatic process involving both hydrolytic and oxidative enzymes. In general lignin composed of three principal building blocks namely, p-coumaryl alcohol, coniferyl alcohol and sinapyl alcohol. In addition, grass and dicot lignin also contain large amounts of phenolic acids such as p-coumaric and ferulic acid, which are esterified to alcohol groups.

Lignin peroxidases (LiP), manganese peroxidases (MnPs) and laccases are three families of enzymes that are involved in the biological degradation of lignin. LiP oxidizes nonphenolic lignin substructures by abstracting one electron and generating cation radicals (Kirk TK, Tien M, Kersten PJ, Mozuch MD and Kalyanaraman B 1986. Ligninase of Phanerochaete chrysosporium. Mechanism of its degradation of the non-phenolic arylglycerol β-aryl ether substructure of lignin. Biochem J 236: 279-287; Kirk TK and Farrell RL 1987. Enzymatic "combustion": The microbial degradation of lignin. Annu Rev Microbiol 41: 465-505) and MnP oxidizes phenolic rings to phenoxyl radicals which lead to decomposition of Lignin (Gold MH, Wariishi H and Valli K 1989. Extracellular peroxidases involved in lignin degradation by the white-rot basidiomycete Phanerochaete chrysosporium. In: Biocatalysis in Agricultural Biotechnology, pp. 127-140. Edited by Whitaker JR and Sonnet PE. American Chemical Society, Washington, DC). Whereas laccase is capable of oxidizing both phenolic and non-phenolic moieties of lignin but that the latter is dependent on the co-presence of primary laccase substrates (Bourbonnais R and Paice MG 1990. Oxidation of non-phenolic substrates. An expanded role for laccase in lignin biodegradation. FEBS Lett 267(1): 99-102). Figure 2 showed a dimeric model compounds that represent the major arylglycerol β-aryl ether lignin structure undergo Cα -Cβ cleavage upon oxidation by LiP (Kirk et al. 1986. Ligninase of Phanerochaete chrysosporium. Mechanism of its degradation of the non-phenolic arylglycerol β-aryl ether substructure of lignin. Biochem J 236: 279-287).

Research on lignin biodegradation has increased enormously in recent years. Especially since the discovery of the first lignocellulose degrading fungus *Phanerochaete chrysosporium* genome was sequenced due to the interest on biological degradation of lignin (Martinez et al. 2004. Genome sequence of the lignocellulose degrading fungus Phanerochaete chrysosporium strain RP78. Nature Biotehnol 22(6): 695-700). Perhaps the best studied, is the degradation of lignin in lignocellulose by white rot fungi, *Phanerochaete chrysosporium* (Aust SD 1995. Mechanisms of Degradation by White Rot Fungi. Environ Health Perspect 103:59-61; Leisola MSA, Ulmer D and Fiechter A 1984. Factors affecting lignin degradation in lignocellulose by Phanerochaete chrysosporium. Arch Microbiol 137: 171-175; Ulmer D, Leisola M, Puhakka J and Fiechter A 1983. Phanerochaete chrysosporium: Growth pattern and lignin degradation. Appl Microbiol Biotechnol 18: 153-157; Chua MGS, Chen CL, Chang HM and Kirk TK 1982. 13CNMR spectroscopic study of lignin degraded by Phanerochaete chrysosporium. I. New structures. Holzforschung 36: 165-172). White-rot fungi produce a range of extracellular lignolytic enzymes, including heme-dependent lignin peroxidases, manganese peroxidases, and versatile peroxidases and copper-dependent laccases (Sanchez C 2009. Lignocellulosic residues: biodegradation and bioconversion by fungi. Biotechnol Adv 27: 185-194; Ten Have R, Teunissen PJM 2001. Oxidative mechanisms involved in lignin degradation by white-rot fungi. Chem Rev 101: 3397-3413). With the aid of these extracellular peroxidase and laccase enzyme, white-rot fungi degrade lignin (Timothy DHB , Ahmad M, Hardiman EM and Rahmanpour R 2001. Pathways for degradation of lignin in bacteria and fungi. Nat. Prod. Rep 28: 1883-1896).

Increasing interest in the exploitation of plant biomass as a renewable resource has provided an impetus for research on microbial degradation of lignocellulose. Large quantity of biomass (mainly lignocellulosic materials) is generated from forestry, agriculture and food industry which are not used in byproduct processes. To effectively utilize this plant biomass as renewable resources, alternative means of degradation of lignocellulose need to be explored. It is desirable to identify new fungi capable of degrading lignin for use in the manufacture of cellulosic products from lignocellulosic materials.

In order to be efficient, the degradation of lignin requires several types of enzymes acting cooperatively. At least two categories of enzymes are necessary to degrade lignin: Lignin peroxidase that oxidizes nonphenolic lignin substructures and laccase that oxidize both phenolic and nonphenolic lignin substructure thus they can degrade cooperatively. To realize and commercialize the mentioned enzymes, stable supply of various lignin degrading proteins are needed. Therefore, it is desirable for the industry to completely identify these lignin degrading genes and their encoded proteins, thus utilizing the genetic information for degradation of lignin from lignocellulosic materials.

### SUMMARY OF THE INVENTION

It has surprisingly been found that the fungus *M*. *phaseolina,* which are not closely related to either *Trichoderma reesi* or *Phanerochaete chrysoporium,* are capable of producing lignin degrading enzymes.

Accordingly, among other things, the present invention relates to the identification of and its corresponding use of lignin degrading enzyme which is derivable from *M. phaseolina.* The present invention also relates to the use of *M*. *phaseolina* fungi in the degradation of lignin content of cellulosic materials.

The primary object of the present invention is to disclose sets of nucleotides sequences encoding lignin peroxidase (SEQ ID Nos. 1, 2, 4, 5, 7, 8, and/or any mixtures/combinations thereof, of the fungi *M. phaseolina.* Herein disclosed are also sets of nucleotides sequences encoding multicopper oxidase (SEQ ID Nos. 46, 47, 49, 50, 52, 53, 55, 56, 58, 59, 61, 62, 64, 65, 67, 68, 70, 71, 73, 74, 76, 77, 79, 80, 82, 83, 85, 86, 88, 89, 91, 92, 94, 95, 97, 98, 100, 101, 103, 104, 106, 107, 109, 110, and/or any mixtures/combinations thereof) of the fungi *M. phaseolina.*

It is preferred that the isolated polynucleotide of the present disclosure consists and/or comprises of a nucleic acid sequence selected from the group comprising and/or consisting of SEQ ID Nos. 2, 5, 8, 11, 14, 17, 20, 23, 26, 29, 32, 35, 38, 41, 44, 47, 50, 53, 56, 59, 62, 65, 68, 71, 74, 77, 80, 83, 86, 89, 92, 95, 98, 101, 104, 107, 110, 1, 4, 7, 10, 13, 16, 19, 22, 25, 28, 31, 34, 37, 40, 43, 46, 49, 52, 55, 58, 61, 64, 67, 70, 73, 76, 79, 82, 85, 88, 91, 94, 97, 100, 103, 106 and 109, and/or any mixtures/combinations thereof, that codes for the polypeptide selected from the group comprising and/or consisting of SEQ ID Nos. 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108 and 111, and/or any mixtures/combinations thereof. The present disclosure also relates to an isolated polynucleotide consisting and/or comprising the complement of the nucleotide sequences described above.

Another object of the present invention is to provide the molecular biology and genetic information of the genes and enzymes set forth in the primary object to be utilized for the regulation and conversion of lignin degradation for the production of valuable products from lignocellulosic materials.

In another aspect, to facilitate *in vitro* production of the lignin degrading polypeptide, the present disclosure include an expression construct capable of expressing polypeptide containing at least 70% sequential amino acids as set forth in SEQ ID Nos. 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108 and 111. Preferably, the expression construct has inserted DNA or cDNA with sequential nucleotide as set forth in SEQ ID Nos. 2, 5, 8, 11, 14, 17, 20, 23, 26, 29, 32, 35, 38, 41, 44, 47, 50, 53, 56, 59, 62, 65, 68, 71, 74, 77, 80, 83, 86, 89, 92, 95, 98, 101, 104, 107, 110, 1, 4, 7, 10, 13, 16, 19, 22, 25, 28, 31, 34, 37, 40, 43, 46, 49, 52, 55, 58, 61, 64, 67, 70, 73, 76, 79, 82, 85, 88, 91, 94, 97, 100, 103, 106 and 109. Herein described is also a recombinant gene construct comprising a polynucleotide template having nucleotide sequence set forth in SEQ ID Nos. 2, 5, 8, 11, 14, 17, 20, 23, 26, 29, 32, 35, 38, 41, 44, 47, 50, 53, 56, 59, 62, 65, 68, 71, 74, 77, 80, 83, 86, 89, 92, 95, 98, 101, 104, 107, 110, 1, 4, 7, 10, 13, 16, 19, 22, 25, 28, 31, 34, 37, 40, 43, 46, 49, 52, 55, 58, 61, 64, 67, 70, 73, 76, 79, 82, 85, 88, 91, 94, 97, 100, 103, 106 and 109, wherein the polynucleotide template is expressible in a host cell to produce an enzyme which oxidize lignin from lignocellulosic materials. Preferably, the recombinant gene construct further comprises a promoter region operably-linked to enhance expression of the polynucleotide template.

In accordance with one of the preferred embodiments of the present disclosure, the fungus *M. phaseolina* strain ms6 isolated from infected jute plant. The isolated polypeptide is also preferably derived from this strain.

Still another object of the present disclosure is to provide a potential commercially feasible way to isolate lignin degrading enzyme from *M. phaseolina* in order to keep up with the increasing global demand on exploitation/utilization of plant biomass as a renewable resource for the manufacture of cellulosic products.

Further object of the disclosure is directed to utilization of the lignin degrading substances in animal feeds, biofuel, woodpulp, textitle and paper industry.

One skilled in the art will readily appreciate that the present invention is well adapted to carry out the objects and obtain the ends and advantages mentioned, as well as those inherent therein. The embodiments described herein are not intended as limitations on the scope of the present invention.

### BRIEF DESCRIPTION OF THE DRAWINGS

The accompanying drawings, which are incorporated in and form a part of the specification, illustrate the embodiments of the present invention and, together with the description, serve to explain the principles of the invention.
- Figure 1: Displays a common structure of softwood lignin. Major arylglyserol-β-aryl ether structure is shown in red color. The inset shows coniferyl alcohol, the phenylpropanoid building block of softwood lignin.
- Figure 2: Displays a cleavage of internal an non-phenolic arylglyserol-β-aryl ether lignin structure by oxidized lignin peroxidase.

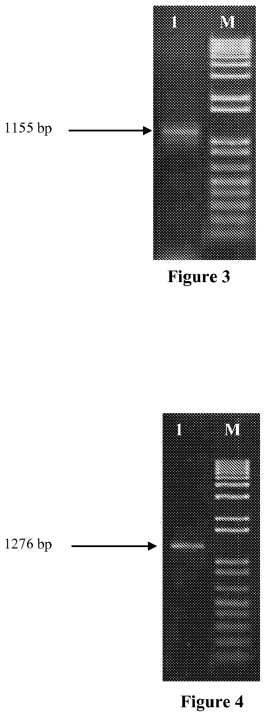

- Figure 3: is the electrophoresed agarose gel image showing the PCR amplification result in which lane 1 is polynucleotides of lignin peroxidase of SEQ ID NO. 1 and lane M is DNA molecular weight ladder.
- Figure 4: is the electrophoresed agarose gel image showing the PCR amplification result in which lane 1 is polynucleotides of lignin peroxidase of SEQ ID NO. 4 and lane M is DNA molecular weight ladder.

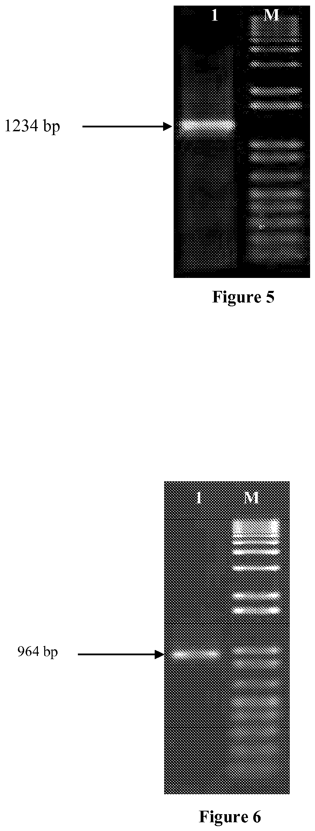

- Figure 5: is the electrophoresed agarose gel image showing the PCR amplification result in which lane 1 is polynucleotides of lignin peroxidase of SEQ ID NO. 7 and lane M is DNA molecular weight ladder.
- Figure 6: is the electrophoresed agarose gel image showing the PCR amplification result in which lane 1 is polynucleotides of chloroperoxidase of SEQ ID NO. 13 and lane M is DNA molecular weight ladder.

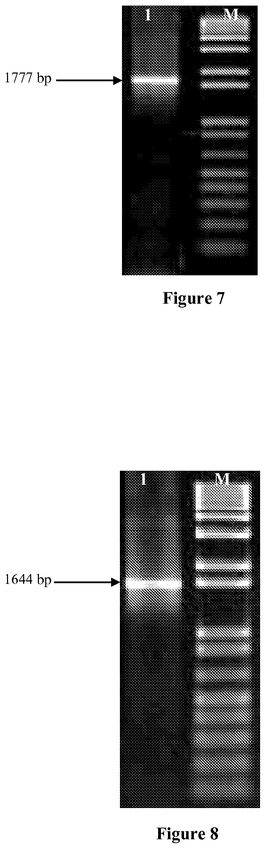

- Figure 7: is the electrophoresed agarose gel image showing the PCR amplification result in which lane 1 is polynucleotides of chloroperoxidase of SEQ ID NO. 16 and lane M is DNA molecular weight ladder.
- Figure 8: is the electrophoresed agarose gel image showing the PCR amplification result in which lane 1 is polynucleotides of chloroperoxidase of SEQ ID NO. 19 and lane M is DNA molecular weight ladder.

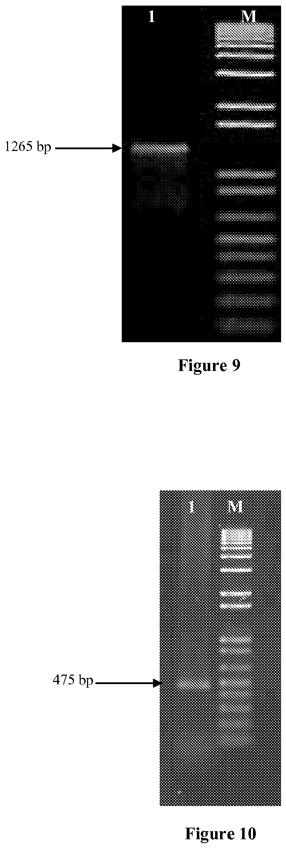

- Figure 9: is the electrophoresed agarose gel image showing the PCR amplification result in which lane 1 is polynucleotides of chloroperoxidase of SEQ ID NO. 22 and lane M is DNA molecular weight ladder.
- Figure 10: is the electrophoresed agarose gel image showing the PCR amplification result in which lane 1 is polynucleotides of chloroperoxidase of SEQ ID NO. 25 and lane M is DNA molecular weight ladder.

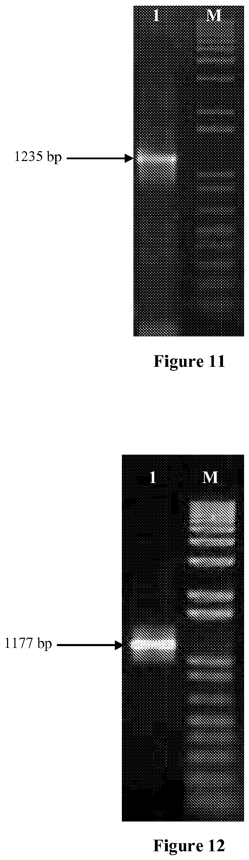

- Figure 11: is the electrophoresed agarose gel image showing the PCR amplification result in which lane 1 is polynucleotides of haemperoxidase of SEQ ID NO. 28 and lane M is DNA molecular weight ladder.
- Figure 12: is the electrophoresed agarose gel image showing the PCR amplification result in which lane 1 is polynucleotides of haemperoxidase of SEQ ID NO. 31 and lane M is DNA molecular weight ladder.

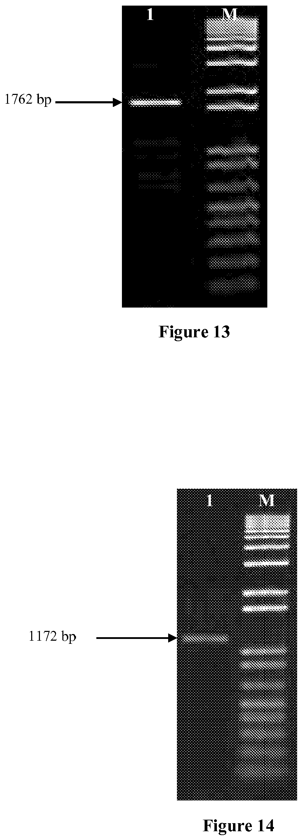

- Figure 13: is the electrophoresed agarose gel image showing the PCR amplification result in which lane 1 is polynucleotides of haemperoxidase of SEQ ID NO. 34 and lane M is DNA molecular weight ladder.
- Figure 14: is the electrophoresed agarose gel image showing the PCR amplification result in which lane 1 is polynucleotides of haemperoxidase of SEQ ID NO. 37 and lane M is DNA molecular weight ladder.

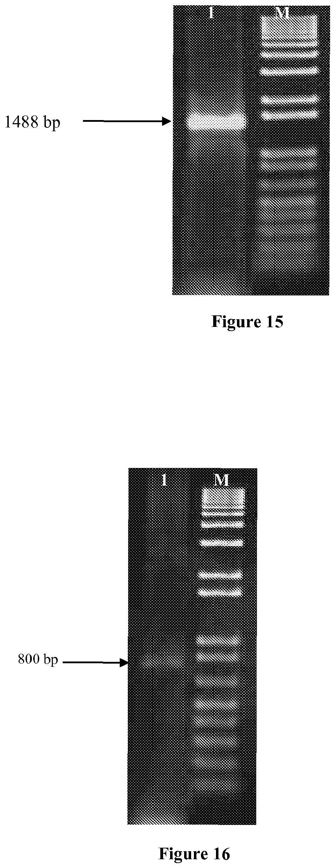

- Figure 15: is the electrophoresed agarose gel image showing the PCR amplification result in which lane 1 is polynucleotides of haemperoxidase of SEQ ID NO. 40 and lane M is DNA molecular weight ladder.
- Figure 16: is the electrophoresed agarose gel image showing the PCR amplification result in which lane 1 is polynucleotides of haemperoxidase of SEQ ID NO. 43 and lane M is DNA molecular weight ladder.

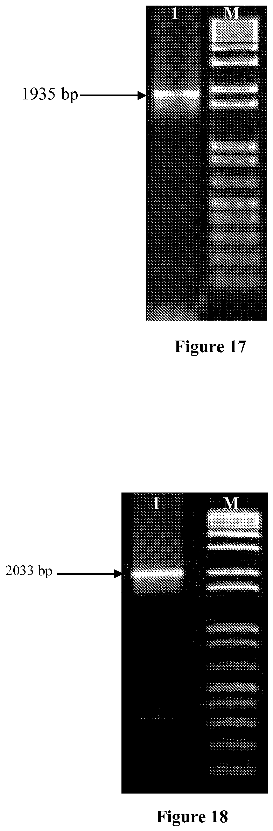

- Figure 17: is the electrophoresed agarose gel image showing the PCR amplification result in which lane 1 is polynucleotides of multicopper oxidase of SEQ ID NO. 46 and lane M is DNA molecular weight ladder.
- Figure 18: is the electrophoresed agarose gel image showing the PCR amplification result in which lane 1 is polynucleotides of multicopper oxidase of SEQ ID NO. 49 and lane M is DNA molecular weight ladder.

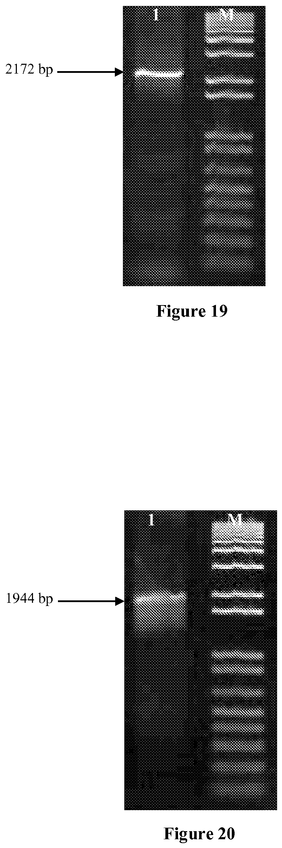

- Figure 19: is the electrophoresed agarose gel image showing the PCR amplification result in which lane 1 is polynucleotides of multicopper oxidase of SEQ ID NO. 52 and lane M is DNA molecular weight ladder.
- Figure 20: is the electrophoresed agarose gel image showing the PCR amplification result in which lane 1 is polynucleotides of multicopper oxidase of SEQ ID NO. 55 and lane M is DNA molecular weight ladder.

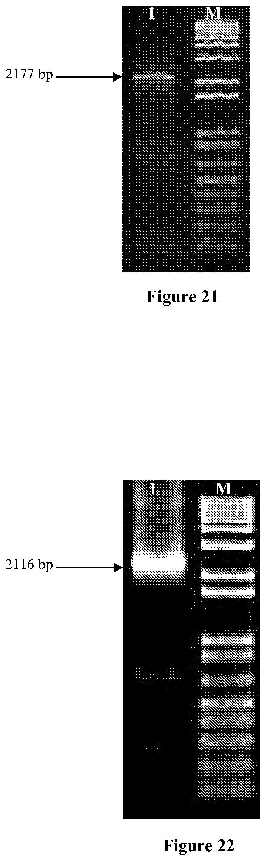

- Figure 21: is the electrophoresed agarose gel image showing the PCR amplification result in which lane 1 is polynucleotides of multicopper oxidase of SEQ ID NO. 58 and lane M is DNA molecular weight ladder.
- Figure 22: is the electrophoresed agarose gel image showing the PCR amplification result in which lane 1 is polynucleotides of multicopper oxidase of SEQ ID NO. 64 and lane M is DNA molecular weight ladder.

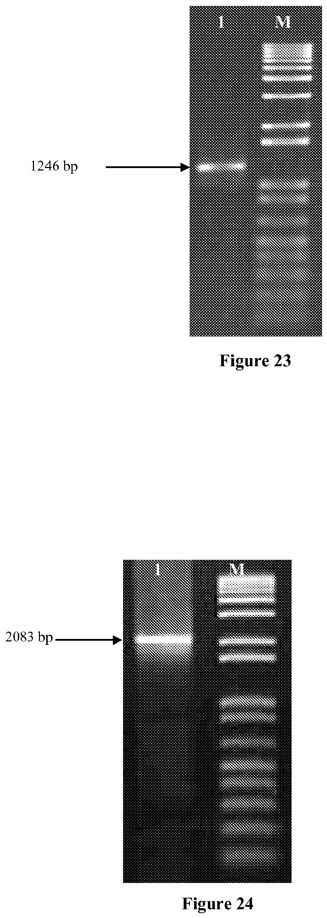

- Figure 23: is the electrophoresed agarose gel image showing the PCR amplification result in which lane 1 is polynucleotides of multicopper oxidase of SEQ ID NO. 67 and lane M is DNA molecular weight ladder.
- Figure 24: is the electrophoresed agarose gel image showing the PCR amplification result in which lane 1 is polynucleotides of multicopper oxidase of SEQ ID NO. 70 and lane M is DNA molecular weight ladder.

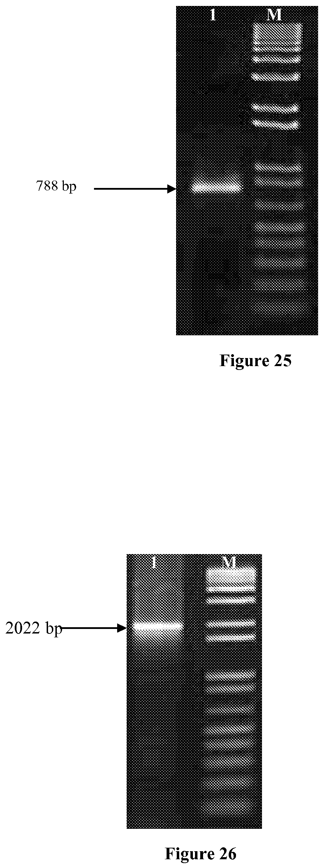

- Figure 25: is the electrophoresed agarose gel image showing the PCR amplification result in which lane 1 is polynucleotides of multicopper oxidase of SEQ ID NO. 73 and lane M is DNA molecular weight ladder.
- Figure 26: is the electrophoresed agarose gel image showing the PCR amplification result in which lane 1 is polynucleotides of multicopper oxidase of SEQ ID NO. 76 and lane M is DNA molecular weight ladder.

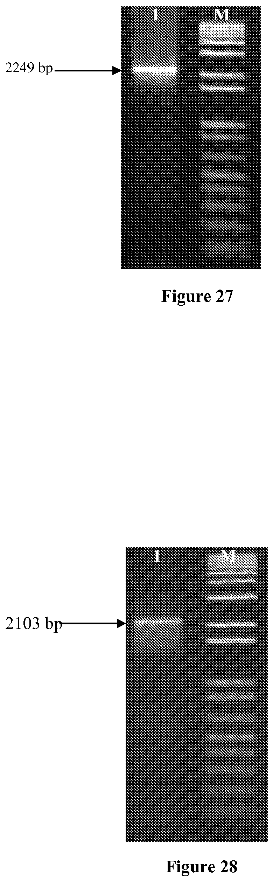

- Figure 27: is the electrophoresed agarose gel image showing the PCR amplification result in which lane 1 is polynucleotides of multicopper oxidase of SEQ ID NO. 79 and lane M is DNA molecular weight ladder.
- Figure 28: is the electrophoresed agarose gel image showing the PCR amplification result in which lane 1 is polynucleotides of multicopper oxidase of SEQ ID NO. 82 and lane M is DNA molecular weight ladder.

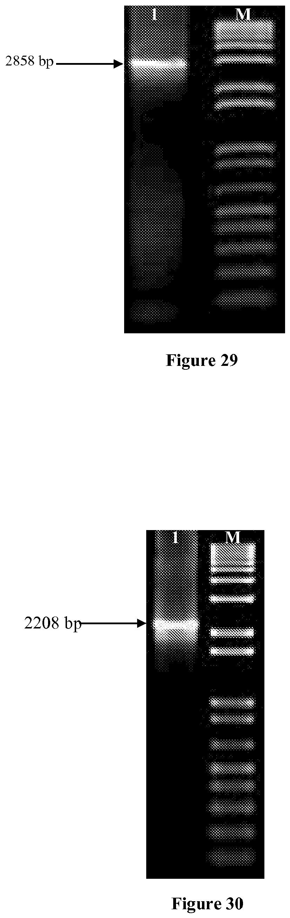

- Figure 29: is the electrophoresed agarose gel image showing the PCR amplification result in which lane 1 is polynucleotides of multicopper oxidase of SEQ ID NO. 85 and lane M is DNA molecular weight ladder.
- Figure 30: is the electrophoresed agarose gel image showing the PCR amplification result in which lane 1 is polynucleotides of multicopper oxidase of SEQ ID NO. 88 and lane M is DNA molecular weight ladder.

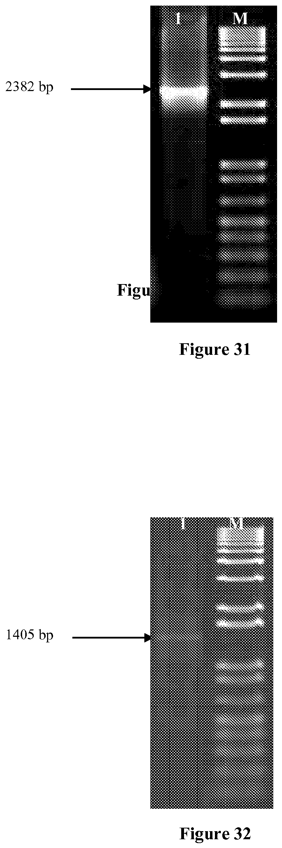

- Figure 31: is the electrophoresed agarose gel image showing the PCR amplification result in which lane 1 is polynucleotides of multicopper oxidase of SEQ ID NO. 91 and lane M is DNA molecular weight ladder.
- Figure 32: is the electrophoresed agarose gel image showing the PCR amplification result in which lane 1 is polynucleotides of multicopper oxidase of SEQ ID NO. 97 and lane M is DNA molecular weight ladder.

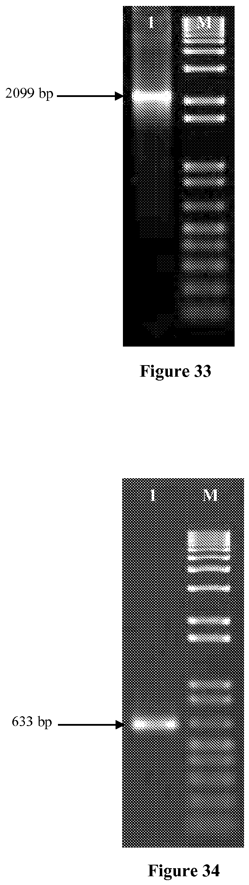

- Figure 33: is the electrophoresed agarose gel image showing the PCR amplification result in which lane 1 is polynucleotides of multicopper oxidase of SEQ ID NO. 100 and lane M is DNA molecular weight ladder.
- Figure 34: is the electrophoresed agarose gel image showing the PCR amplification result in which lane 1 is polynucleotides of multicopper oxidase of SEQ ID NO. 103 and lane M is DNA molecular weight ladder.

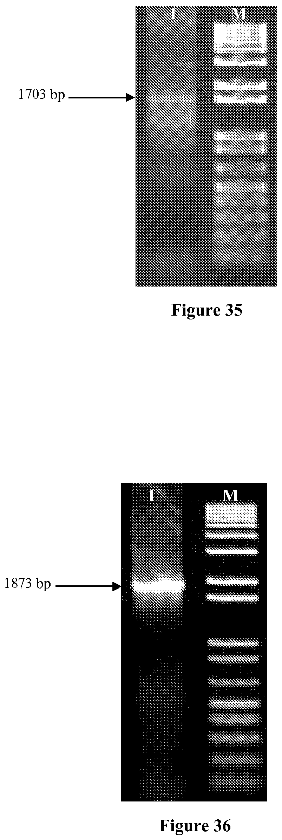

- Figure 35: is the electrophoresed agarose gel image showing the PCR amplification result in which lane 1 is polynucleotides of multicopper oxidase of SEQ ID NO. 106 and lane M is DNA molecular weight ladder.
- Figure 36: is the electrophoresed agarose gel image showing the PCR amplification result in which lane 1 is polynucleotides of multicopper oxidase of SEQ ID NO. 109 and lane M is DNA molecular weight ladder.

### DETAILED DESCRIPTION OF THE INVENTION

The definitions and/or methods provided herein define the present invention and guide those of ordinary skill in the art in the practice of the present invention. Except where otherwise stated, terms are to be understood according to conventional usage by those of ordinary skill in the relevant art. To the extent to which any of the definitions and/or methods is found to be inconsistent with any of the definitions and/or methods provided in any patent or non-patent reference incorporated herein or in any reference found elsewhere, it is understood that the said definition and/or method which has been expressly provided/adopted in this application will be used herein. The singular terms "a," "an," and "the" include plural referents unless context clearly indicates otherwise. Similarly, the word "or" is intended to include "and" unless the context clearly indicates otherwise. Hence, "comprising A or B" means including A, or B, or A and B. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present disclosure, suitable methods and materials are described below.

The present invention provides the nucleotide sequences of *M*. *phaseolina* encoded protein/enzyme involved in lignin degradation. The genes encode proteins with an enzyme activity that is either in use in an industry or of interest to an industry. The genomic sequences of the invention that encode the enzymes are identified primarily by comparison of nucleotide sequences of *M*. *phaseolina* genomic DNA and the nucleotide sequences of known enzyme genes of other microorganisms. Prior to this invention, the nucleotide sequences of these *M. phaseolina* genes, the reading frames, the positions of exons and introns, the structure of the enzymes, and their potential usefulness in various industries, such as those involved in the making of food and feed, beverages, textiles and detergents, were not known.
Analysis of the genome sequence of *M*. *phaseolina* reveals an abundance of genes coding for enzymes which degrade lignin in plant. The nucleotide sequences were initially annotated by software programs, such as Genescan and Glimmer M (The Institute of Genome Research, Rockville, NM), which can identify putative coding regions, introns, and splice junctions. Further automated and manual curation of the nucleotide sequences was performed to refine and establish precise characterization of the coding regions and other gene features.
Over 14000 cDNAs from *M. phaseolina* were partially or fully sequenced. Among them thirty-seven cDNAs encoding new enzymes with putative roles in lignin degradation were discovered.
Open reading frames (ORFs) are analyzed following full or partial sequencing of clones of cDNA libraries derived from *M. phaseolina* mRNA and are further analyzed using sequence analysis software, and by determining homology to known sequences in databases (public/private).
In the context of this disclosure, a number of terms used throughout the specification have the indicated meanings unless expressly indicated to have a different meaning.
As used herein, a "polynucleotide" is a nucleotide sequence such as a nucleic acid fragment. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. An isolated polynucleotide of the present disclosure may include at least one of 150 contiguous nucleotides (both upstream and downstream) derived from SEQ ID No. 1, 4, 7, 10, 13, 16, 19, 22, 25, 28, 31, 34, 37, 40, 43, 46, 49, 52, 55, 58, 61, 64, 67, 70, 73, 76, 79, 82, 85, 88, 91, 94, 97, 100, 103, 106 and 109, or the complement of such sequences.
"Polypeptide" as used herein, is a single linear chain of amino acids bonded together by peptide bonds, and having usally a sequence greater than 100 amino acids in length.
"Isolated" means altered "by the hand of man" from the natural state. If a composition or substance occurs in nature, it has been "isolated" if it has been changed or removed from its original environment, or both. For example, a polynucleotide or a polypeptide naturally present in a living plant or animal is not "isolated," but the same polynucleotide or polypeptide separated from the coexisting materials of its natural state is "isolated", as the term is employed herein.
The term "gene", as used herein, is defined as the genomic sequences of the fungi *M*. *phaseolina,* particularly polynucleotide sequence encoding polypeptide of the series of enzymes involved in the lignin degradation pathway.
A "coding sequence" or "coding region" refers to a nucleic acid molecule having sequence information necessary to produce a gene product, such as an amino acid or polypeptide, when the sequence is expressed. The coding sequence may comprise untranslated sequences (e.g., introns or 5' or 3' untranslated regions) within translated regions, or may lack such intervening untranslated sequences (e.g., as in cDNA).
The term "primer" as used herein, is an oligonucleotide capable of binding to a target nucleic acid sequence and priming the nucleic acid synthesis. An amplification oligonucleotide as defined herein will preferably be 10 to 50, most preferably 15 to 25 nucleotides in length. While the amplification oligonucleotides of the present disclosure may be chemically synthesized and such oligonucleotides are not naturally occurring nucleic acids.
The abbreviation used throughout the specification to refer to nucleic acids comprising nucleotide sequences are the conventional one-letter abbreviations. Thus when included in a nucleic acid, the naturally occurring encoding nucleotides are abbreviated as follows: adenine (A), guanine (G), cytosine (C), thymine (T) and uracil (U). Also, unless otherwise specified, the nucleic acid sequences presented herein is the 5' →3'direction.

As used herein, the term "complementary" and derivatives thereof are used in reference to pairing of nucleic acids by the well-known rules that A pairs with T or U and C pairs with G. Complement can be "partial" or "complete". In partial complement, only some of the nucleic acid bases are matched according to the base pairing rules; while in complete or total complement, all the bases are matched according to the pairing rule. The degree of complement between the nucleic acid strands may have significant effects on the efficiency and strength of hybridization between nucleic acid strands as well known in the art. The efficiency and strength of said hybridization is depends upon in detection method.

The term "host cell", as used herein, includes any cell type which is susceptible to transformation, transfection, transduction, expression and the like with a nucleic acid construct or expression vector comprising a polynucleotide of the present invention.

The term "operably linked" denotes herein a configuration in which a control sequence is placed at an appropriate position relative to the coding sequence of the polynucleotide sequence such that the control sequence directs the expression of the coding sequence of a polypeptide.

A "vector" generally refers to a replicon, such as plasmid, phage, cosmid, yeast or virus to which another nucleic acid segment may be operably inserted so as to bring about the replication or expression of the segment. The term "vector" is also intended to refer to a nucleic acid molecule capable of transporting another nucleic acid to which it has been linked. One type of vector is a "plasmid," which refers to a circular double-stranded DNA loop into which additional DNA segments may be ligated. Another type of vector is a viral vector, where additional DNA segments may be ligated into the viral genome. Certain vectors are capable of autonomous replication in a host cell into which they are introduced (e.g., bacterial vectors having a bacterial origin of replication and episomal mammalian vectors). Other vectors can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The term "nucleic acid construct" or "DNA construct" is sometimes used to refer to a coding sequence or sequences operably linked to appropriate regulatory sequences and inserted into a vector for transforming a cell. This term may be used interchangeably with the term "transforming DNA" or "transgene."

The term "promoter" as used herein, refers to a nucleic acid sequence that functions to direct transcription of a downstream gene. The promoter will generally be appropriate to the host cell in which the target gene is being expressed. The promoter together with other transcriptional and translational regulatory nucleic acid sequences (also termed "control sequences") is necessary to express a given gene. In general, the transcriptional and translational regulatory sequences include, but are not limited to, promoter sequences, ribosomal binding sites, transcriptional start and stop sequences, translational start and stop sequences, and enhancer or activator sequences.

The term *"in vitro"* as used herein, refers to a biological reaction occurs in an artificial environment outside a living organism, which is usually conducted in a laboratory using components of an organism that have been isolated from their usual biological context in order to permit a more detailed or more convenient analysis to be performed.

The term "% homology" is used interchangeably herein with the term "% identity" herein and refers to the level of nucleic acid or amino acid sequence identity between the nucleic acid sequence that encodes any one of the inventive polypeptides or the inventive polypeptide's amino acid sequence, when aligned using a sequence alignment program.

For example, as used herein, 80% homology means the same thing as 80% sequence identity determined by a defined algorithm, and accordingly a homologue of a given sequence has greater than 80% sequence identity over a length of the given sequence. Exemplary levels of sequence identity include, but are not limited to, 80, 85, 90, 95, 98% or more sequence identity to a given sequence, *e.g*., the coding sequence for any one of the inventive polypeptides, as described herein.

Exemplary computer programs which can be used to determine identity between two sequences include, but are not limited to, the suite of BLAST programs, *e.g*., BLASTN, BLASTX, and TBLASTX, BLASTP and TBLASTN, publicly accessible at www.ncbi.nlm.nih.gov/BLAST.
Sequence searches are typically carried out using the BLASTN program, when evaluating a given nucleic acid sequence relative to nucleic acid sequences in the GenBank DNA Sequences and other public databases. The BLASTX program is preferred for searching nucleic acid sequences that have been translated in all reading frames against amino acid sequences in the GenBank Protein Sequences and other public databases.
A preferred alignment of selected sequences in order to determine "% identity" between two or more sequences is performed using for example, the CLUSTAL-W program.
As in setting forth, one embodiment of the present disclosure is an isolated polynucleotides encoding for lignin degrading polypeptide found in the fungi *M*. *phaseolina* comprising nucleotide sequence as setting forth in SEQ ID No. 2, 5, 8, 11, 14, 17, 20, 23, 26, 29, 32, 35, 38, 41, 44, 47, 50, 53, 56, 59, 62, 65, 68, 71, 74, 77, 80, 83, 86, 89, 92, 95, 98, 101, 104, 107, 110, 1, 4, 7, 10, 13, 16, 19, 22, 25, 28, 31, 34, 37, 40, 43, 46, 49, 52, 55, 58, 61, 64, 67, 70, 73, 76, 79, 82, 85, 88, 91, 94, 97, 100, 103, 106 and 109. Correspondingly, the respective lignin degrading polypeptide encoded by these nucleotide sequences shall possess amino acid sequence as setting forth in SEQ ID Nos. 3, 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96, 99, 102, 105, 108 and 111.
In one embodiment, the 939 bp long polynucleotide illustrated in ID No. 2 is the full length cDNA clone encoded Lignin peroxidase protein exhibiting an open reading frame encoding a 312 amino acid polypeptide, as in SEQ ID No. 3, with a calculated molecular mass of about 32 kD. Through SMART analysis of SEQ ID No. 2, it reveals presence of Pfam peroxidase domain in the sequence. The protein family with the Pfam domain consists and/or comprises of secretory fungal peroxidases. These are monomeric glycoproteins involved in the degradation of lignin.
Preferably, the 1116 bp long polynucleotide illustrated in SEQ ID No. 5 is the full length cDNA clone encoded Lignin peroxidase protein exhibiting an open reading frame encoding a 371 amino acid polypeptide, as in SEQ ID No. 6, with a calculated molecular mass of about 40 kD. Through SMART analysis of SEQ ID No. 5, it reveals presence of Pfam peroxidase domain in the sequence. The protein family with the Pfam domain consists and/or comprises of secretory fungal peroxidases. These are monomeric glycoproteins involved in the degradation of lignin.

Similarly, another aspect, the 1023 bp long polynucleotide illustrated in SEQ ID No. 8 is the full length cDNA clone encoded Lignin peroxidase protein exhibiting an open reading frame encoding a 340 amino acid polypeptide, as in SEQ ID No. 9, with a calculated molecular mass of about 35 kD. Through SMART analysis of SEQ ID No. 8, it reveals presence of Pfam peroxidase domain in the sequence. The protein family with the Pfam domain consists and/or comprises of secretory fungal peroxidases. These are monomeric glycoproteins involved in the degradation of lignin.

In another aspect, polynucleotides encoding for chloroperoxidase has 1260 bp as illustrated in SEQ ID No. 11 while the encoded protein is a 419 amino acid polypeptide with a calculated molecular mass of about 45 kD as illustrated in SEQ ID No. 12. The SEQ ID No. 11 contains a Pfam domain, namely peroxidase_2. The said domain involved in the lignin degradation.

In another aspect, polynucleotides encoding for chloroperoxidase has 690 bp as illustrated in SEQ ID No. 14 while the encoded protein is a 229 amino acid polypeptide with a calculated molecular mass of about 25 kD as illustrated in SEQ ID No. 15. The SEQ ID No. 14 contains a Pfam domain, namely peroxidase_2. The said domain involved in the lignin degradation.

In another aspect, polynucleotides encoding for chloroperoxidase has 1194 bp as illustrated in SEQ ID No. 17 while the encoded protein is a 397 amino acid polypeptide with a calculated molecular mass of about 43 kD as illustrated in SEQ ID No. 18. The SEQ ID No. 17 contains a Pfam domain, namely peroxidase_2. The said domain involved in the lignin degradation.

In another aspect, polynucleotides encoding for chloroperoxidase has 1317 bp as illustrated in SEQ ID No. 20 while the encoded protein is a 438 amino acid polypeptide with a calculated molecular mass of about 47 kD as illustrated in SEQ ID No. 21. The SEQ ID No. 20 contains a Pfam domain, namely peroxidase_2. The said domain involved in the lignin degradation.

In another aspect, polynucleotides encoding for chloroperoxidase has 780 bp as illustrated in SEQ ID No. 23 while the encoded protein is a 259 amino acid polypeptide with a calculated molecular mass of about 29 kD as illustrated in SEQ ID No. 24. The SEQ ID No. 23 contains a Pfam domain, namely peroxidase_2. The said domain involved in the lignin degradation.

In another aspect, polynucleotides encoding for chloroperoxidase has 951 bp as illustrated in SEQ ID No. 26 while the encoded protein is a 316 amino acid polypeptide with a calculated molecular mass of about 35 kD as illustrated in SEQ ID No. 27. The SEQ ID No. 26 contains a Pfam domain, namely peroxidase_2. The said domain involved in the lignin degradation.

In another aspect, polynucleotides encoding for haemperoxidase has 1116 bp as illustrated in SEQ ID No. 29 while the encoded protein is a 371 amino acid polypeptide with a calculated molecular mass of about 41 kD as illustrated in SEQ ID No. 30. The SEQ ID No. 29 contains a Pfam domain, namely peroxidase. Haemperoxidases oxidize lignin subunits using extracellular hydrogen peroxide generated by unrelated oxidases as a co-substrate. The said domain involved in the lignin degradation.

In another aspect, polynucleotides encoding for haemperoxidase has 1623 bp as illustrated in SEQ ID No. 32 while the encoded protein is a 540 amino acid polypeptide with a calculated molecular mass of about 57 kD as illustrated in SEQ ID No. 33. The SEQ ID No. 32 contains a Pfam domain, namely peroxidase. Haemperoxidases oxidize lignin subunits using extracellular hydrogen peroxide generated by unrelated oxidases as a co-substrate. The said domain involved in the lignin degradation.

In another aspect, polynucleotides encoding for haemperoxidase has 1599 bp as illustrated in SEQ ID No. 35 while the encoded protein is a 532 amino acid polypeptide with a calculated molecular mass of about 57 kD as illustrated in SEQ ID No. 36. The SEQ ID No. 35 contains a Pfam domain, namely peroxidase. Haemperoxidases oxidize lignin subunits using extracellular hydrogen peroxide generated by unrelated oxidases as a co-substrate. The said domain involved in the lignin degradation.

In another aspect, polynucleotides encoding for haemperoxidase has 2049 bp as illustrated in SEQ ID No. 38 while the encoded protein is a 682 amino acid polypeptide with a calculated molecular mass of about 70 kD as illustrated in SEQ ID No. 39. The SEQ ID No. 38 contains a Pfam domain, namely peroxidase. Haemperoxidases oxidize lignin subunits using extracellular hydrogen peroxide generated by unrelated oxidases as a co-substrate. The said domain involved in the lignin degradation.

In another aspect, polynucleotides encoding for haemperoxidase has 1605 bp as illustrated in SEQ ID No. 41 while the encoded protein is a 534 amino acid polypeptide with a calculated molecular mass of about 57 kD as illustrated in SEQ ID No. 42. The SEQ ID No. 41 contains a Pfam domain, namely peroxidase. Haemperoxidases oxidize lignin subunits using extracellular hydrogen peroxide generated by unrelated oxidases as a co-substrate. The said domain involved in the lignin degradation.

In another aspect, polynucleotides encoding for haemperoxidase has 960 bp as illustrated in SEQ ID No. 44 while the encoded protein is a 319 amino acid polypeptide with a calculated molecular mass of about 35 kD as illustrated in SEQ ID No. 45. The SEQ ID No. 44 contains a Pfam domain, namely peroxidase. Haemperoxidases oxidize lignin subunits using extracellular hydrogen peroxide generated by unrelated oxidases as a co-substrate. The said domain involved in the lignin degradation.

In another aspect, polynucleotides encoding for multicopper oxidases (laccases) has 1713 bp as illustrated in SEQ ID No. 47 while the encoded protein is a 570 amino acid polypeptide with a calculated molecular mass of about 64 kD as illustrated in SEQ ID No. 48. The SEQ ID No. 47 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1860 bp as illustrated in SEQ ID No. 50 while the encoded protein is a 619 amino acid polypeptide with a calculated molecular mass of about 69 kD as illustrated in SEQ ID No. 51. The SEQ ID No. 50 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1824 bp as illustrated in SEQ ID No. 53 while the encoded protein is a 607 amino acid polypeptide with a calculated molecular mass of about 67 kD as illustrated in SEQ ID No. 54. The ID No. 53 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1737 bp as illustrated in SEQ ID No. 56 while the encoded protein is a 578 amino acid polypeptide with a calculated molecular mass of about 63 kD as illustrated in SEQ ID No. 57. The SEQ ID No. 56 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1665 bp as illustrated in SEQ ID No. 59 while the encoded protein is a 554 amino acid polypeptide with a calculated molecular mass of about 61 kD as illustrated in SEQ ID No. 60. The SEQ ID No. 59 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1803 bp as illustrated in SEQ ID No. 62 while the encoded protein is an 600 amino acid polypeptide with a calculated molecular mass of about 65 kD as illustrated in SEQ ID No. 63. The SEQ ID No. 62 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1635 bp as illustrated in SEQ ID No. 65 while the encoded protein is a 544 amino acid polypeptide with a calculated molecular mass of about 61 kD as illustrated in SEQ ID No. 66. The SEQ ID No. 65 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1743 bp as illustrated in SEQ ID No. 68 while the encoded protein is a 580 amino acid polypeptide with a calculated molecular mass of about 63 kD as illustrated in SEQ ID No. 69. The ID No. 68 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1836 bp as illustrated in SEQ ID No. 71 while the encoded protein is a 611 amino acid polypeptide with a calculated molecular mass of about 67 kD as illustrated in SEQ ID No. 72. The SEQ ID No. 71 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 702 bp as illustrated in SEQ ID No. 74 while the encoded protein is a 233 amino acid polypeptide with a calculated molecular mass of about 26 kD as illustrated in SEQ ID No. 75. The SEQ ID No. 74 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1854 bp as illustrated in SEQ ID No. 77 while the encoded protein is a 617 amino acid polypeptide with a calculated molecular mass of about 68 kD as illustrated in SEQ ID No. 78. The SEQ ID No. 77 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 2073 bp as illustrated in SEQ ID No. 80 while the encoded protein is a 690 amino acid polypeptide with a calculated molecular mass of about 77 kD as illustrated in SEQ ID No. 81. The SEQ ID No. 80 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1824 bp as illustrated in SEQ ID No. 83 while the encoded protein is a 607 amino acid polypeptide with a calculated molecular mass of about 67 kD as illustrated in SEQ ID No. 84. The ID No. 83 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1932 bp as illustrated in SEQ ID No. 86 while the encoded protein is a 643 amino acid polypeptide with a calculated molecular mass of about 72 kD as illustrated in SEQ ID No. 87. The SEQ ID No. 86 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1950 bp as illustrated in SEQ ID No. 89 while the encoded protein is a 649 amino acid polypeptide with a calculated molecular mass of about 72 kD as illustrated in SEQ ID No. 90. The SEQ ID No. 89 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 2016 bp as illustrated in SEQ ID No. 92 while the encoded protein is a 671 amino acid polypeptide with a calculated molecular mass of about 74 kD as illustrated in SEQ ID No. 93. The SEQ ID No. 92 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 2085 bp as illustrated in SEQ ID No. 95 while the encoded protein is a 694 amino acid polypeptide with a calculated molecular mass of about 78 kD as illustrated in SEQ ID No. 96. The SEQ ID No. 95 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1821 bp as illustrated in SEQ ID No. 98 while the encoded protein is a 606 amino acid polypeptide with a calculated molecular mass of about 68 kD as illustrated in SEQ ID No. 99. The ID No. 98 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1854 bp as illustrated in SEQ ID No. 101 while the encoded protein is a 617 amino acid polypeptide with a calculated molecular mass of about 68 kD as illustrated in SEQ ID No. 102. The SEQ ID No. 101 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1815 bp as illustrated in SEQ ID No. 104 while the encoded protein is a 604 amino acid polypeptide with a calculated molecular mass of about 67 kD as illustrated in SEQ ID No. 105. The SEQ ID No. 104 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1632 bp as illustrated in SEQ ID No. 107 while the encoded protein is a 543 amino acid polypeptide with a calculated molecular mass of about 61 kD as illustrated in SEQ ID No. 108. The SEQ ID No. 107 contains a Pfam domain, namely multicopper oxidase. This multicopper oxidase (laccase) is extracellular, non-haem, copper containing proteins that catalyze the one-electron oxidation of phenols to phenoxy radicals.

In another aspect, polynucleotides encoding for multicopper oxidases (laccases) has 1752 bp as illustrated in SEQ ID No. 110 while the encoded protein is a 583 amino acid polypeptide with a calculated molecular mass of about 64 kD as illustrated in SEQ ID No. 111. The SEQ ID No. 110 contains a Pfam domain, namely multicopper oxidase. This multicopper oxidase (laccase) is extracellular, non-haem, copper containing proteins that catalyze the one-electron oxidation of phenols to phenoxy radicals.

The sequences provided by the present invention can also be used as preparatory materials for the rational modification or design of novel enzymes with characteristics that enable the enzymes to perform better in demanding processes.

The present disclosure includes as contained in the appended claims, as well as that of the foregoing description. Although this invention has been described in its preferred form with a degree of particularity, it is understood that the present disclosure of the preferred form has been made only by way of example and that numerous changes in the details of construction and the combination and arrangements of parts may be resorted to without departing from the scope of the invention and claims.

Various references are cited herein, the disclosures of which are incorporated by reference in their entireties.

### EXAMPLE

The following example is intended to further illustrate the invention, without any intent for the invention to be limited to the specific embodiments described therein.

### Example 1 Designing and synthesis of primers

The primers used in the study were either designed from the manually curated transcriptome and the "gene models" predicted from the genomic sequences of *M*. *phaseolina* ms6, by choosing the sequences manually with complete ORFs or using databases where similar genes have been successfully isolated from other plants. Comparative bioinformatic analysis of the nucleotide sequences obtained from transcriptome were carried out using NCBI BLAST, BLASTP, RPS-BLAST, BLASTX and PSI-BLAST to identify homologues of the related genes and for the proper identification of gene. Nucleotide sequence alignments were performed through clustalW version 1.82 whenever multiple sequences were found from the "gene pool". The alignment was then edited. Gene specific primers (both forward and reverse) were selected manually or through Primer 3 plus tool and the primers were custom synthesized.

All oligonucleotides used in this study were synthesized and HPLC purified by the supplier and procured from Integrated DNA Technologies (IDT). Stock solution of 100 pmol were prepared in autoclaved ddH₂O and stored at -20°C, in aliquots for use.

**Oligonucleotides Sequences used as primers for PCR**

| **Gene name** | **SEQ ID No.** | **Primer Sequence** | **Amplified product (bp)** |
|---|---|---|---|
| lignin peroxidase | 1 | Forward GAGACCGCTACACCCACCCCT | 1155 |
| | | Reverse CACCGCACTACGACCTCGCT | |
| lignin peroxidase | 4 | Forward GTCGTCGCGTGGCTGCTAGA | 1276 |
| | | Reverse CCAGGCATCGGGGAACTTCGG | |
| lignin peroxidase | 7 | Forward GGCGGCTCTCTCGCAGACGTA | 1234 |
| | | Reverse GCCCTGCCCCAACCGATTCA | |
| Chloroperoxidase | 10 | Forward TGCTGCCTCCGCTCTGTCGC | 1769 |
| | | Reverse CGCACCATGTCGCCTCTGCC | |
| Chloroperoxidase | 13 | Forward AACCGCTTTACCTGCCAGCCA | 964 |
| | | Reverse ATTGGGGTCGGTGCTCAGGAGT | |
| Chloroperoxidase | 16 | Forward ACGGAGCACATGAACACCGTCC | 1777 |
| | | Reverse CTTCGCACCGCGAGCAGAGG | |
| Chloroperoxidase | 19 | Forward TCCCGCGAGCCCTTGGTCTG | 1644 |
| | | Reverse GCCCTCGCTGGTTCCTTTGCT | |
| Chloroperoxidase | 22 | Forward ATGTTTTGTTTCGCGCCGCT | 1265 |
| | | Reverse AATCTACCACTCCCGTCCCGC | |
| Chloroperoxidase | 25 | Forward ACCGCCGCCTTCGTTTACGTC | 475 |
| | | Reverse GCCCGCTTACTTTGCCGGTC | |
| Haem peroxidase | 28 | Forward TCGCCTGTGCTCACACCACG | 1235 |
| | | Reverse TTAACGTCCGCCAAGCACGC | |
| Haem peroxidase | 31 | Forward CGTTCCCAAGCCCGACGACTC | 1177 |
| | | Reverse GGGCAAGTCCCCAAGCCCATC | |
| Haem peroxidase | 34 | Forward AATGCGGTTTCTCGGGGGCT | 1762 |
| | | Reverse TGTTGCTGGCCCTATGAAGGCAT | |
| Haem peroxidase | 37 | Forward CCATGGCAAGGCATCCCGGC | 1172 |
| | | Reverse TGTGGCATCCCAACAGGGGC | |
| Haem peroxidase | 40 | Forward TGGACCTGGCCGTCAAACCG | 1488 |
| | | Reverse GCCTCACAGAGCCGCACACTC | |
| Haem peroxidase | 43 | Forward CAGGCATGCGCTACGAGGCT | 800 |
| | | Reverse ACCAGCTTACAGACGTGCCCTGA | |
| Multicopper oxidase | 46 | Forward TGGGCGGGCAGGTACGTGAAT | 1935 |
| | | Reverse TCCGTGCTCTGGCCTCGCAT | |
| Multicopper oxidase | 49 | Forward GCTGGCGACGACAAGTGGCT | 2033 |
| | | Reverse CCACAGAGTTCGCGAGGCCC | |
| Multicopper oxidase | 52 | Forward CATCCCACCGCGGGAAGCCT | 2172 |
| | | Reverse ATCCCCGCCGTCACGGTTTT | |
| Multicopper oxidase | 55 | Forward TCGTTGAAGTCGCTGTCCCGT | 1944 |
| | | Reverse GCCCCGCACACCTGCCATAG | |
| Multicopper oxidase | 58 | Forward TGCTTGCTCAAGGGCGCTCA | 2177 |
| | | Reverse TCAACTCAGACTACTGTCGAAGTGC | |
| MuLticopper oxidase | 61 | Forward CACTGGCACGGCTTCACGCA | 4930 |
| | | Reverse CCGCTACGCGGTCGACTCCT | |
| Multicopper oxidase | 64 | Forward CACCCTCCGGTCGGGTAAGT | 2116 |
| | | Reverse TACAGGTGCTATGCCAGCGTGC | |
| Multicopper oxidase | 67 | Forward TAGCATCGGCACAACGCCCAT | 1246 |
| | | Reverse GAACCGGTGGCCGTGAAGGTG | |
| MuLticopper oxidase | 70 | Forward ACCCACCGCTCGCTCTCACA | 2083 |
| | | Reverse TGGAAATGCGCAGAAGGACCGT | |
| MuLticopper oxidase | 73 | Forward GCACGACATGTGGATTGCGGC | 788 |
| | | Reverse TTGCCAGCCGTGCTCCGTCA | |
| MuLticopper oxidase | 76 | Forward ACGACGTTGCAAGCTCCGCC | 2022 |
| | | Reverse CCATCGGGCATAGAAGTCGCCG | |
| Multicopper oxidase | 79 | Forward TTCACCGGAGTCGCCTTCCCA | 2249 |
| | | Reverse CGACGGGCTGCAGTACGAGA | |
| MuLticopper oxidase | 82 | Forward AATCCCCTCTCACCTCGCCGC | 2103 |
| | | Reverse GGCCACCCCTCAGAGACCGGA | |
| Multicopper oxidase | 85 | Forward CGCCGAACCAAAGCCTCCTCC | 2858 |
| | | Reverse GCACAGGAGAAAGAGCTCACCCC | |
| MuLticopper oxidase | 88 | Forward TCGCCCGTCCAGGAGAGATA | 2208 |
| | | Reverse CCCCCATCTACCGGCCATTC | |
| Multicopper oxidase | 91 | Forward ATGAGGGGTAATCGCGACGG | 2382 |
| | | Reverse CCCTCTCACAACTGACCCTGT | |
| Multicopper oxidase | 94 | Forward TCACTCAGTGCCCTACCGCTCC | 1299 |
| | | Reverse CCGCAACGACTCCGTCCGGT | |
| Multicopper oxidase | 97 | Forward TGGGCTGATCCCGTTGCAGGA | 1405 |
| | | Reverse GTAGCCGTGGCTGAGCGTGTT | |
| Multicopper oxidase | 100 | Forward TGCCGTTGCTGTAACATGCCGT | 2099 |
| | | Reverse GACGGCGCTTTGCTCTTGCG | |
| Multicopper oxidase | 103 | Forward AGCATGCAATACTCGGTCGGTCT | 633 |
| | | Reverse CGGGCAGCAGGATGTTTGCCAT | |
| Multicopper oxidase | 106 | Forward GGATACTCTCCGGGCACGTTCG | 1703 |
| | | Reverse ATGGCAGTGGACTGCGCGAC | |
| Multicopper oxidase | 109 | Forward GGAGGGAGCACTGATGCGCT | 1873 |
| | | Reverse GGCTGCTCCTCCGCTCATGG | |

### Example 2 Amplification, cloning and sequencing of lignin peroxidase, chloroperoxidase, haemperoxidase and multicopper peroxidase from M. phaseolina ms6

Total RNA was isolated from three days old mycelium grown on liquid medium as previously described by Chomezynski P and Sacchi N, Single-step method of RNA isolation by acid guanidinium thlocyanate-phenol-chloroform extraction. (Anal Biochem 1987, 162: 156-159). The quality or the integrity of the RNA was checked by agarose gel electrophoresis and was quantified using Thermo Scientific Nano Drop 2000 as per standard procedures, cDNA first strand was synthesised using SuperScript III reverse transcriptase (Invitrogen) following the manufacturer's instructions. The gene was amplified from the cDNA by PCR using the gene specific primers. The PCR reaction (50µL) contained 1 µL of cDNA, 20 pmoles of each primers, 5 µL of 10X PCR Buffer, 5 µL of 2.5 mM dNTP mix and 1.0 unit of PfuTaq DNA polymerase. PCR was carried out in Thermal Cycler (Applied Biosystems) using the following conditions: initial denaturation for 5 min at 95°C followed by 35 cycles of denaturation at 95°C for 30 sec, annealing at 59-61°C for 30 sec and extension at 72°C for 1 to 2.0 min depending on the length of the targeted gene, with a final extension at 72°C for 7 min. The PCR product was analyzed by 1% agarose gel using 1X TAE buffer and the amplicon was eluted from the gel using QIAGEN gel extraction kit following the manufacturer's instructions. The purified PCR product was ligated into pCR®8/GW/TOPO® TA cloning kit (Invitrogen) and transformed into competent *E. coli* cells (Invitrogen). Plasmids were isolated from putative colonies using QIAprip Spin Miniprep Kit (QIAGEN) following the manufacturer's instructions. The presence of the insert was checked by using the gene specific primers and positive plasmids were subjected to Sequencing.

### Example 3 Analysis of the sequence

The nucleotide sequence and the amino acid sequence were analyzed by BLASTN and BLASTP programs respectively. The sequences reported from other plants were aligned with ClustalW. Phylogenetic analysis was carried out using the Neighbour Joining (NJ).
SEQ ID NO : 1
   LENGTH : 1379 (including 150 bp 5' UTR and 150 bp 3' UTR)
   TYPE : DNA
   ORGANISM : *M. phaseolina* 
CTGCTCAGCAGATCTTATCCCTCCAGAGCCAGAGCATCTTTAACTGACCAGCTTGCCTTCGTCCCAT
CGTTTGCATTTTTTACACTTGACCAGTCAAGGCCCCTCTTCCTTCTTTGAGACCGCTACACCCACC
CCTTCGTACTGTCAAAATGTTATTCTCAAAGTCTTCCATCTTTCTCCTCTCCACTGCGGCCAGTGTG
CAAGCACTCAGCCTCTCCGATGTCTCCTCTGCCGCCTCGGTCCTGAAGCGTGAAGCTTCCGGCTTGG
GGAACAACCTTCTATCTCTCGTTCACCGTCGGGACTCTTGCCCTGATGTTTGGCAGAAAGTCGCATC
CGAGCTGAAGGGCTGGTTCTTGGATGGTTCCGTGTGCAGTGACGACGCACGCGCTGCCATCCGCCTC
TCTTTCCACGACTGCTTTTCCGGCGGCTGCGATGGGTCCATCATCCTTGCCCACGAGTACACCCGCT
CCGACAACGCTGGCTTAGCAGACTTTGCTATGAAGCTAGCGCCTCTCGCGGACCAGTACGAGGTCGG
AACAGCTGACCTGATCCAATTCGCTGGCGGTAAGCCTTATCTATCCACCTTAAAACAAAAAAAAAAA
aGGAAAAAAaGACTATCTCTAACACCTACCTCGACCAGCCCTCGCCACGGCCACCTGTCCCCTCGGC
CCCCGCATAGCCGTCAAAGTCGGCCGCCAGGACTCGTCAACGCCCTCGGCAGAGGGACAGCTCCCCT
CGTCGCGATCCTCGGCCTCCGTCCTGATCGACCAGTTCGCGGCGAAGGGGTTCTCTGAGATAGACCT
CGTCGCCCTCGTCGGTGCCCACAGCACTGCCAAGCAGTTCTTCGATCAGCCCGACAAGGCCGGCCAA
AGCCTCGACTCCACCCCCGGCACTTGGGACACCAACTTTTACCGCCAGACGACGCTCGGCACTGCGC
CCGTTACCCTCGAGAGCGATAAGAACCTCGCCACGGATCTGAGGACCGCGGTGCAGTGGACGGCGTT
TAATGCCCAGGGCGTGTGGGCCGCGGCGTATGTGAGCGCGTAAGTAGTGCATCATATGTTTCTTCTC
 
TGCTCTCAGATTGTTGAGAACTAATGGTCAATGTAGGATGAACAAGATGACCGTTTTGGGCAATGAT
GTCAGCAGCTTGACGGATTGCACGAGTGTTATCAGTGCGGCAACGAGCAAGCGCGACATCAAGGCCG
CACCTATTGCGGATAGGATTTGAGCTATGCAAGCAGTTCTGGGTGAGCGAGGTCGTAGTGCGGTGAC
TGGAGTTCTGTTTTTTTTTTTTTttCTTAATTGGTAATGTCTGCTCTTGTTTGATACCTGCCAGTATT
CGCGATTTATTCATCGGGAAGAGTTTAGAGAAGGGGGCA
SEQ ID NO : 2
   LENGTH : 939
   TYPE : DNA
   ORGANISM : *M. phaseolina*
   FEATURE NAME/KEY: CDS
   LOCATION : (1) ...... (939) 
atgttattctcaaagtcttccatctttctcctctccactgcggccagtgtgcaagcactc
 M  L  F  S  K  S  S  I  F  L  L  S  T  A  A  S  V  Q  A  L
agcctctccgatgtctcctctgccgcctcggtcctgaagcgtgaagcttccggcttgggg
 S  L  S  D  V  S  S  A  A  S  V  L  K  R  E  A  S  G  L  G
aacaaccttctatctctcgttcaccgtcgggactcttgccctgatgtttggcagaaagtc
 N  N  L  L  S  L  V  H  R  R  D  S  C  P  D  V  W  Q  K  V
gcatccgagctgaagggctggttcttggatggttccgtgtgcagtgacgacgcacgcgct
 A  S  E  L  K  G  W  F  L  D  G  S  V  C  S  D  D  A  R  A
gccatccgcctctctttccacgactgcttttccggcggctgcgatgggtccatcatcctt
 A  I  R  L  S  F  H  D  C  F  S  G  G  C  D  G  S  I  I  L
gcccacgagtacacccgctccgacaacgctggcttagcagactttgctatgaagctagcg
 A  H  E  Y  T  R  S  D  N  A  G  L  A  D  F  A  M  K  L  A
cctctcgcggaccagtacgaggtcggaacagctgacctgatccaattcgctggcgccctc
 P  L  A  D  Q  Y  E  V  G  T  A  D  L  I  Q  F  A  G  A  L
gccacggccacctgtcccctcggcccccgcatagccgtcaaagtcggccgccaggactcg
 A  T  A  T  C  P  L  G  P  R  I  A  V  K  V  G  R  Q  D  S
tcaacgccctcggcagagggacagctcccctcgtcgcgatcctcggcctccgtcctgatc
 S  T  P  S  A  E  G  Q  L  P  S  S  R  S  S  A  S  V  L  I
gaccagttcgcggcgaaggggttctctgagatagacctcgtcgccctcgtcggtgcccac
 D  Q  F  A  A  K  G  F  S  E  I  D  L  V  A  L  V  G  A  H
agcactgccaagcagttcttcgatcagcccgacaaggccggccaaagcctcgactccacc
 S  T  A  K  Q  F  F  D  Q  P  D  K  A  G  Q  S  L  D  S  T
cccggcacttgggacaccaacttttaccgccagacgacgctcggcactgcgcccgttacc
 P  G  T  W  D  T  N  F  Y  R  Q  T  T  L  G  T  A  P  V  T
ctcgagagcgataagaacctcgccacggatctgaggaccgcggtgcagtggacggcgttt
 L  E  S  D  K  N  L  A  T  D  L  R  T  A  V  Q  W  T  A  F
aatgcccagggcgtgtgggccgcggcgtatgtgagcgcgatgaacaagatgaccgttttg
 N  A  Q  G  V  W  A  A  A  Y  V  S  A  M  N  K  M  T  V  L
ggcaatgatgtcagcagcttgacggattgcacgagtgttatcagtgcggcaacgagcaag
 G  N  D  V  S  S  L  T  D  C  T  S  V  I  S  A  A  T  S  K
cgcgacatcaaggccgcacctattgcggataggatttga
 R  D  I  K  A  A  P  I  A  D  R  I  -
SEQ ID NO : 3
   LENGTH : 312
   TYPE : PRT
   ORGANISM : *M. phaseolina* 
MLFSKSSIFLLSTAASVQALSLSDVSSAASVLKREASGLGNNLLSLVHRRDSCPDVWQKVASELKGW
FLDGSVCSDDARAAIRLSFHDCFSGGCDGSIILAHEYTRSDNAGLADFAMKLAPLADQYEVGTADLI
QFAGALATATCPLGPRIAVKVGRQDSSTPSAEGQLPSSRSSASVLIDQFAAKGFSEIDLVALVGAHS
TAKQFFDQPDKAGQSLDSTPGTWDTNFYRQTTLGTAPVTLESDKNLATDLRTAVQWTAFNAQGVWAA
AYVSAMNKMTVLGNDVSSLTDCTSVISAATSKRDIKAAPIADRI*
SEQ ID NO : 4
   LENGTH : 1416 (including 150 bp 5' UTR and 150 bp 3' UTR)
   TYPE : DNA
   ORGANISM : *M. phaseolina* 
TCTATATCTTGCCCTTCGCCTGTTCGCTTCAGGAGAACCCAACGCCAACATTCATTCTCAACCTCCT
CTTTGAGTTACAATCGCTACTCTGGAAATTTGTCCCAGCCTTTCATTCAACAACTGTTTCTGTTTC
AAACCAATCCGACGCCATGAAGTTCTCCACAGTCATCTCGAGCGTTGCTCTCACTTCTCTACTCCAG
CCCGCCCTTGCCTACCCTGGCATGGCAAATGTCGTCTCGGAGATCAAGGCCCGTCAAAACACCAATA
ATGATGGTGACTCAAATCCCGAGATGATTGGCGATCTCGCCACCACCGGCCCAACCACCCCTGTGGG
CCAAAGCATATACAACATCCTGATGGGGACCGAGTCGGCCGAGACCAAGCAGGCTGGCTACATCCCC
CCTCTTATCGGCACCAACGCCTGCAAGAGGGACACCTGCTGCATCTGGGCCTACATCGCCGCCGAAC
TGACCCTCAATTTCAAGGGCATCACGGGCCGCTGCAACAAGAACGCGCGTGCCGCCATTCGGCTCGG
CTTCCACGACGCGGGACTTGGTCCAAGAGCAGCAACGGCGGCGGCGCGGACGGCTCGATCGCGCTG
TCGGGCACGGAGATCAACAAGGCCGAGAACAACGGGCTGCAGGACATCATCGGCAAGATGATCACGT
GGCAGAAGCGGTACGGGGTGGGCATGGCGGATCTGATCCAGTTCGCGGCCATCCACGCCGTGGTGAC
GTGCCCGCTGGGGCCGCGCATCCGCTTCTTCGTCGGGCGCAAGGACAGCAAAACGGCCAACGACGTC
AGCCTGCTGCCGGGCGTCAACGATTCGGCCGACAAGCTGATCGCGCTCTTCCAGGACAAGACCATCA
CGCCGCACGAGCTCGCCGCCCTGCTCGGCGCCCACACCACCTCACAGCAGTTCTTCGTCGACACCAC
CCGCGCCGGCGCCCCCCAGGACAGCACCCCCGGCGTCTGGGACACCCGCTTCTACAACCAAACCACC
TCCGACCAAGTTCCCAAGAAGGTCTTCCGCTTCGCCAGCGACGTTGTGCTGGCCAAGGACCCGCGTA
TGAGTGATGAGTGGGCCGCCTTCGCCGACCCCGTCAAGGGCCAGAACCACTGGAATGAGGATTACGC
CACCGCCTATACCCGCCTCAGCCTGCTCGGCGTCAACAACATCAATAACTTGACTGAGTGCAGCAAG
GTGCTGCCGTGGGCGCAACCGAAGTTCCCCGATGCCTGGAACCTGTTCTTGGACCAGTAGAGTGTAG
TTTCTTCTTAATATCTGGCGTTCTTGGGGGTTTGGTCAAAAAGGAGAAAGATATTATTGTCGATATT
TGGTATACCCTAATGTTGGGAACTATACATACGTATACAGGCTTCTTGTAATTACTCGGGCATCTTT
TGCACAGAG
SEQ ID NO : 5
   LENGTH : 1116
   TYPE : DNA
   ORGANISM : *M. phaseolina*
   FEATURE NAME/KEY: CDS
   LOCATION : (1) ...... (1116) 
atgaagttctccacagtcatctcgagcgttgctctcacttctctactccagcccgccctt
 M  K  F  S  T  V  I  S  S  V  A  L  T  S  L  L  Q  P  A  L
gcctaccctggcatggcaaatgtcgtctcggagatcaaggcccgtcaaaacaccaataat
 A  Y  P  G  M  A  N  V  V  S  E  I  K  A  R  Q  N  T  N  N
gatggtgactcaaatcccgagatgattggcgatctcgccaccaccggcccaaccacccct
 D  G  D  S  N  P  E  M  I  G  D  L  A  T  T  G  P  T  T  P
gtgggccaaagcatatacaacatcctgatgggaccgagtcggccgagaccaagcaggct
 V  G  Q  S  I  Y  N  I  L  M  G  T  E  S  A  E  T  K  Q  A
ggctacatcccccctcttatcggcaccaacgcctgcaagagggacacctgctgcatctgg
 G  Y  I  P  P  L  I  G  T  N  A  C  K  R  D  T  C  C  I  W
gcctacatcgccgccgaactgaccctcaatttcaagggcatcacgggccgctgcaacaag
 A  Y  I  A  A  E  L  T  L  N  F  K  G  I  T  G  R  C  N  K
aacgcgcgtgccgccattcggctcggcttccacgacgcggggacttggtccaagagcagc
 N  A  R  A  A  I  R  L  G  F  H  D  A  G  T  W  S  K  S  S
aacggcggcggcgcggacggctcgatcgcgctgtcgggcacggagatcaacaaggccgag
 N  G  G  G  A  D  G  S  I  A  L  S  G  T  E  I  N  K  A  E
aacaacggcctgcaggacatcatcggcaagatgatcacgtggcagaagcggtacggggtg
 N  N  G  L  Q  D  I  I  G  K  M  I  T  W  Q  K  R  Y  G  V
ggcatggcggatctgatccagttcgcggccatccacgccgtggtgacgtgcccgctgggg
 G  M  A  D  L  I  Q  F  A  A  I  H  A  V  V  T  C  P  L  G
ccgcgcatccgcttcttcgtcggcgcaaggacagcaaaacggccaacgacgtcagcctg
 P  R  I  R  F  F  V  G  R  K  D  S  K  T  A  N  D  V  S  L
 
ctgccgggcgtcaacgattcggccgacaagctgatcgcgctcttccaggacaagaccatc
 L  P  G  V  N  D  S  A  D  K  L  I  A  L  F  Q  D  K  T  I
acgccgcacgagctcgccgccctgctcggcgcccacaccacctcacagcagttcttcgtc
 T  P  H  E  L  A  A  L  L  G  A  H  T  T  S  Q  Q  F  F  V
gacaccacccgcgccggcgcccccaggacagcacccccggcgtctgggacacccgcttc
 D  T  T  R  A  G  A  P  Q  D  S  T  P  G  V  W  D  T  R  F
tacaaccaaaccacctccgaccaagttcccaagaaggtcttccgcttcgccagcgacgtt
 Y  N  Q  T  T  S  D  Q  V  P  K  K  V  F  R  F  A  S  D  V
gtgctggccaaggacccgcgtatgagtgatgagtgggccgccttcgccgaccccgtcaag
 V  L  A  K  D  P  R  M  S  D  E  W  A  A  F  A  D  P  V  K
ggccagaaccactggaatgaggattacgccaccgcctatacccgcctcagcctgctcggc
 G  Q  N  H  W  N  E  D  Y  A  T  A  Y  T  R  L  S  L  L  G
gtcaacaacatcaataacttgactgagtgcagcaaggtgctgccgtgggcgcaaccgaag
 V  N  N  I  N  N  L  T  E  C  S  K  V  L  P  W  A  Q  P  K
ttccccgatgcctggaacctgttcttggaccagtag
 F  P  D  A  W  N  L  F  L  D  Q  -
SEQ ID NO : 6
   LENGTH : 371
   TYPE : PRT
   ORGANISM : *M. phaseolina* 
MKFSTVISSVALTSLLQPALAYPGMANVVSEIKARQNTNNDGSNPEMIGDLATTGPTTPVGQSIYN
ILMGTESAETKQAGYIPPLIGTNACKRDTCCIWAYIAAELTLNFKGITGRCNKNARAAIRLGFHDAG
TWSKSSNGGGADGSIALSGTEINKAENNGLQDIIGKMITWQKRYGVGMADLIQFAAIHAVVTCPLGP
RIRFFVGRKDSKTANDVSLLPGVNDSADKLIALFQDKTITPHELAALLGAHTTSQQFFVDTTRAGAP
QDSTPGVWDTRFYNQTTSDQVPKKVFRFASDVVLAKDPRMSDEWAAFADPVKGQNHWNEDYATAYTR
LSLLGVNNINNLTECSKVLPWAQPKFPDAWNLFLDQ*
SEQ ID NO : 7
   LENGTH : 1323 (including 150 bp 5' UTR and 150 bp 3' UTR)
   TYPE : DNA
   ORGANISM : *M. phaseolina* 
TCGAGATGGCGGCTCTCTCGCAGACGTAAATCTATAAAGCCTTGCCGCGACTTTCTTTTGTGTATCT
CATCAGCATCGAGTTCCACAAGAGCTTGCTTCCTCTCCTCACTGAGCCTAGCGAGGAGCATCACTCA
GAACCCTCCAATCACAATGCGGACCTCATCCCTGTTCCTTGCTTCCGCATGCGGAACATCTGCTTAC
ACTCTCGTCTCGCTCGACTCTCTGCCAAGCACTCTTCATGACATAACCTCCCGCACCATCTCCAACC
TCGACCCCCGCAACCTCCTCTCCGCGCGCAAGACGCCCGACTGCCCGGCCATCTGGAGGACCATCTC
AGCCGACCTGACCAAGAGCTTCCTCGCCAACGGCGAGTGCACCGACCTCGCCCGCGCCGCCATCCGC
TACGCCTTCCACGACGCGGGCACCTTCTCGCTCAAGCTGCCCACCTACGCGCCGGCCTCCGGCGGCG
CCGACGGCTCGCTGCTGCTCGTCGATTCGGAGATCCAGCGGCCCGAGAACAACGGGCTGCAGGCGTA
CAACGACTTCATCAAGGCCAAGTACAGCACGTACAAGTCCTCGGGCGTCGGCGCCGCCGACCTGATC
CAGTTCGCCGGCAACCACGCCGTGGTGACGTGCCCGGGCGGGCCCACGGTCAAGACGCTCGTCGGCC
GCGGCGACAGCACGACCGCGTCGCCGCTGAACGTGATGCCGCCGGGGTTCGGCGCGGGCAGCGACCA
CGACTCGCTGCTCCAGCTCTTCCAGGACAAGGGGTTCAGCGCCGTCGACCTGGCCGCGCTGATCGGC
GCCCACACCACCTCCACGAACATCGCGGAGGCGCAGATCCCCGTCGGCGCGCCGCAGGACAGCACGC
CGGGCAGGTGGGACGTCAAGTACTACGCCGAGACGTACGCCCCGCCCGCGGGCGTCTCCCGCTTCGC
CTCCGACATCAACCTCTCCGACCCGACGAAAGCGGTCGGCAAAGAGTTCCAGGGATTCGTCAACAAC
CAGGGTAAGTGGACGGGCAAGTTTGCCGACGCCATGTTCCGTCTGAGTGTGTTGGGCATCCCGCCGG
CGACGTACAAGAATTTCGCGGACTGCACCGCTGCGCTGCCCAAGGGCACGAGCGCCAAGCGGGACAT
CCGCAGCGCCCCGATCAACGACCGCGCAAGGTAGAGGAGGGGAAAAGAAAGGAAGAAAAGAAAATAA
AAAGCGCAGCGAGGATGAATCGGTTGGGGCAGGGCGTTTCGGTTGAGGTTGTTTGTTTGCTCGCCTG
CCTTTTTTTTTTTTTtAATCCCTCTCATGATCCATCGAATGAGAACACTT
SEQ ID NO : 8
   LENGTH : 1023
   TYPE : DNA
   ORGANISM : *M. phaseolina*
   FEATURE NAME/KEY : CDS
   LOCATION : (1) ...... (1023) 
atgcggacctcatccctgttccttgcttccgcatgcggaacatctgcttacactctcgtc
 M  R  T  S  L  F  L  A  S  A  C  G  T  S  A  Y  T  L  V
tcgctcgactctctgccaagcactcttcatgacataacctcccgcaccatctccaacctc
 S  L  D  S  L  P  S  T  L  H  D  I  T  S  R  T  I  S  N  L
gacccccgcaacctcctctccgcgcgcaagacgcccgactgcccggccatctggaggacc
 D  P  R  N  L  L  S  A  R  K  T  P  D  C  P  A  I  W  R  T
atctcagccgacctgaccaagagcttcctcgccaacggcgagtgcaccgacctcgcccgc
 I  S  A  D  L  T  K  S  F  L  A  N  G  E  C  T  D  L  A  R
gccgccatccgctacgccttccacgacgcgggcaccttctcgctcaagctgcccacctac
 A  A  I  R  Y  A  F  H  D  A  G  T  F  S  L  K  L  P  T  Y
gcgccggcctccggcggcgccgacggctcgctgctgctcgtcgattcggagatccagcgg
 A  P  A  S  G  G  A  D  G  S  L  L  L  V  D  S  E  I  Q  R
cccgagaacaacggcctgcaggcgtacaacgacttcatcaaggccaagtacagcacgtac
 P  E  N  N  G  L  Q  A  Y  N  D  F  I  K  A  K  Y  S  T  Y
aagtcctcgggcgtcggcgccgccgacctgatccagttcgccggcaaccacgccgtggtg
 K  S  S  G  V  G  A  A  D  L  I  Q  F  A  G  N  H  A  V  V
acgtgcccgggcggcccacggtcaagacgctcgtcggccgcggcgacagcacgaccgcg
 T  C  P  G  G  P  T  V  K  T  L  V  G  R  G  D  S  T  T  A
tcgccgctgaacgtgatgccgccggggttcggcgcgggcagcgaccacgactcgctgctc
 S  P  L  N  V  M  P  P  G  F  G  A  G  S  D  H  D  S  L  L
cagctcttccaggacaaggggttcagcgccgtcgacctggccgcgctgatcggcgcccac
 Q  L  F  Q  D  K  G  F  S  A  V  D  L  A  A  L  I  G  A  H
accacctccacgaacatcgcggaggcgcagatccccgtcggcgcgccgcaggacagcacg
 T  T  S  T  N  I  A  E  A  Q  I  P  V  G  A  P  Q  D  S  T
ccgggcaggtgggacgtcaagtactacgccgagacgtacgccccgcccgcgggcgtctcc
 P  G  R  W  D  V  K  Y  Y  A  E  T  Y  A  P  P  A  G  V  S
cgcttcgcctccgacatcaacctctccgacccgacgaaagcggtcggcaaagagttccag
 R  F  A  S  D  I  N  L  S  D  P  T  K  A  V  G  K  E  F  Q
ggattcgtcaacaaccagggtaagtggacgggcaagtttgccgacgccatgttccgtctg
 G  F  V  N  N  Q  G  K  W  T  G  K  F  A  D  A  M  F  R  L
agtgtgttgggcatcccgccggcgacgtacaagaatttcgcggactgcaccgctgcgctg
 S  V  L  G  I  P  P  A  T  Y  K  N  F  A  D  C  T  A  A  L
cccaagggcacgagcgccaagcgggacatccgcagcgccccgatcaacgaccgcgcaagg
 P  K  G  T  S  A  K  R  D  I  R  S  A  P  I  N  D  R  A  R
tag
 -
SEQ ID NO : 9
   LENGTH : 340
   TYPE : PRT
   ORGANISM : *M. phaseolina* 
MRTSSLFLASACGTSAYTLVSLDSLPSTLHDITSRTISNLDPRNLLSARKTPDCPAIWRTISADLTK
SFLANGECTDLARAAIRYAFHDAGTFSLKLPTYAPASGGADGSLLLVDSEIQRPENNGLQAYNDFIK
AKYSTYKSSGVGAADLIQFAGNHAVVTCPGGPTVKTLVGRGDSTTASPLNVMPPGFGAGSDHDSLLQ
LFQDKGFSAVDLAALIGAHTTSTNIAEAQIPVGAPQDSTPGRWDVKYYAETYAPPAGVSRFASDINL
SDPTKAVGKEFQGFVNNQGKWTGKFADAMFRLSVLGIPPATYKNFADCTAALPKGTSAKRDIRSAPI
NDRAR*
SEQ ID NO : 10
   LENGTH : 1930 (including 150 bp 5' UTR and 150 bp 3' UTR)
   TYPE : DNA
   ORGANISM : *M. phaseolina* 
GAGCACGGCAGGTAGGTTGCATCCGAGAGGTCGTGGTAGGTAGGTATATATTGCTGCCTCCGCTCTG
TCGCTCTATAGAGGCAGGTCTTGGACATCTGTGAACAGGTTACCTTCTCATAATTTGATCCATTGGC
GAAGATATACTCAAGAATGCCGTCTACCTGGATGATTGCATTGGGCGCACTCACCcTTGCCGGCCAG
TCCGCAGCCTTTCCTGCTGTGGCAGAACAATACGCTGCTCAGACATCTCACAAGGAGAAAAGAGTAA
ATGCAATTAGCCCCGGGTTCAATGCGGCGGCACAGAGGATTGACGTCTCGGGAGCTCACACTTTCGT
GCCCCCTGGCCCCGGTGACCAGAGAGGGCCCTGCCCGGGTCTGAATGCCTTAGCGAACCAGTAAGTG
CTCTGCAAGTCTCACAGGTCTTCTTGTACTGACAAAGTGTAGCAACTATTTGCCCCAGTGAGTTCTC
CACAACTTCATTCCCGAAACCTTGTCACTGATTGTCTTCAGCAATGGCGTCGCAACAATCACGCAGT
TCGTCCAGGCCACAAACCAGGGTTAGTACAGCATCACCGCCACGGAAGCTACTACGTCAGCTGAACC
ACATACGCAGTATACGGCATGGGTCTCGACCTTGGCACGTTCCTGTCCGTCTACGGCGCGGTAATGG
ATGGCGACGGCCTCAGCTGGTCCATCGGCGGCGCGCCCAGTACCGCAAACCTACTCAACCTCCTTAC
TCAGCCGCAAGGCCTCTCAGGTTCGCACAACAAGTACGAGACAGATGCATCGCCTACGCGCGGAGAC
CTGTACCAATAGTGCGTCTATCCCCTTCTTCTGCCTCCCGCCACTCCGCTGACCTGCACCGCAGTGG
CGACAACTCCCGGGTCGTCATTTCGCAATGGGACGCCCTCTTCGCGAAGCAAGCGGCACTGCCCAAT
GACCAGTCCAATTATGGCCTAGGTGTGCTGACCGACTTCCGAGTCGAGCGTTTCCAGCAGAGCGTCG
ATGAAAACCCCTACTTCTTCAACGCGCCCTTTTCCGGTGTGCTCGTGCAGCCTGCCGCGTACACATT
CATCTACCGCTTCATGGGCAACAAGAGCGCCGAGAAACCCGAGGGTGTGCTGACGAAAGAGGTGCTG
AAGAGCTTCTTCGGCTTCACTGGGCCTGACGACGACATGACATACGTGAGTCCCATCCCGCCGCCTT
TCTCATTTCTTCCCACTCGAGTCTCCCACATCCCCCCCCCCCCCCCCCCCCCCCCccACcATTTGTTG
TCGCCCTTCCCTCCAGCCACGCCACTGCCCACAACGCCACCGTCTGACCCACCGCAGAACCCCGGCC
ACGAGCGCATCCCCGAAAACTGGTACAAGCGCGCCCCCGGCGACGAATACACGATCCCCTTCTACGC
ACTTGACCTCAACGCCGCGGCGCTGCAGCACCCGCAATTCCTGTCCGTCGGCGGCAACACGGGCACC
ACCAACTCCTTCGCCGGCGTCGACCTGCAGGACCTGAGCGGCGGCCTGTACAACGCCGCCAGCCTGC
TCGAGGGCAACAACCTCGCCTGCTTCGGTTTCCAGGCGGCCGTCCAATTCGCGCCGGACCTGTTGAA
AGGGCTGGTGAGCGATTTGACCAAGCCGTTGGGTGTCCTGGGAGATGCGTTGGCGAGTGCGTTGAAT
GGATTAGGGTGTCCGCAGTTAGGCGGCCAGGCGTGGGATGATAGCGCGCTGGCGCAGTTTCCGGGGT
ATGCTAGGCTGAGGGCGGATGGACGTACGGGAAGTAATGGACGCGGAGAAGTAGTGTGGCAGAGGC
GACATGGTGCGCATGGTGGACTGCGGCTTCAACGACAATGCAGAGATGATGGTATGAGGAAACTGCA
CAGCAGTCTGCATGGATTTGCTAGACTAGACGAATTTTATGATTTTGAATGAAC
SEQ ID NO : 11
   LENGTH : 1260
   TYPE : DNA
   ORGANISM : *M. phaseolina*
   FEATURE NAME/KEY: CDS
   LOCATION : (1) ...... (1260) 
atgccgtctacctggatgattgcattgggcgcactcacccttgccggccagtccgcagcc
 M  P  S  T  W  M  I  A  L  G  A  L  T  L  A  G  Q  S  A  A
tttcctgctgtggcagaacaatacgctgctcagacatctcacaaggagaaaagagtaaat
 F  P  A  V  A  E  Q  Y  A  A  Q  T  S  H  K  E  K  R  V  N
gcaattagccccgggttcaatgcggcggcacagaggattgacgtctcgggagctcacact
 A  I  S  P  G  F  N  A  A  A  Q  R  I  D  V  S  G  A  H  T
ttcgtgccccctggccccggtgaccagagagggccctgcccgggtctgaatgccttagcg
 F  V  P  P  G  P  G  D  Q  R  G  P  C  P  G  L  N  A  L  A
aaccacaatggcgtcgcaacaatcacgcagttcgtccaggccacaaaccaggtatacggc
 N  H  N  G  V  A  T  I  T  Q  F  V  Q  A  T  N  Q  V  Y  G
atgggtctcgaccttggcacgttcctgtccgtctacggcgcggtaatggatggcgacggc
 M  G  L  D  L  G  T  F  L  S  V  Y  G  A  V  M  D  G  D  G
ctcagctggtccatcggcggcgcgcccagtaccgcaaacctactcaacctccttactcag
 L  S  W  S  I  G  G  A  P  S  T  A  N  L  L  N  L  L  T  Q
ccgcaaggcctctcaggttcgcacaacaagtacgagacagatgcatcgcctacgcgcgga
 P  Q  G  L  S  G  S  H  N  K  Y  E  T  D  A  S  P  T  R  G
gacctgtaccaatatggcgacaactcccgggtcgtcatttcgcaatggacgccctcttc
 
 D  L  Y  Q  Y  G  D  N  S  R  V  V  I  S  Q  W  D  A  L  F
gcgaagcaagcggcactgcccaatgaccagtccaattatggcctaggtgtgctgaccgac
 A  K  Q  A  A  L  P  N  D  Q  S  N  Y  G  L  G  V  L  T  D
ttccgagtcgagcgtttccagcagagcgtcgatgaaaacccctacttcttcaacgcgccc
 F  R  V  E  R  F  Q  Q  S  V  D  E  N  P  Y  F  F  N  A  P
ttttccggtgtgctcgtgcagcctgccgcgtacacattcatctaccgcttcatgggcaac
 F  S  G  V  L  V  Q  P  A  A  Y  T  F  I  Y  R  F  M  G  N
aagagcgccgagaaacccgagggtgtgctgacgaaagaggtgctgaagagcttcttcggc
 K  S  A  E  K  P  E  G  V  L  T  K  E  V  L  K  S  F  F  G
ttcactggcctgacgacgacatgacatacaaccccggccacgagcgcatccccgaaaac
 F  T  G  P  D  D  D  M  T  Y  N  P  G  H  E  R  I  P  E  N
tggtacaagcgcgcccccggcgacgaatacacgatccccttctacgcacttgacctcaac
 W  Y  K  R  A  P  G  D  E  Y  T  I  P  F  Y  A  L  D  L  N
gccgcggcgctgcagcacccgcaattcctgtccgtcggcggcaacacgggcaccaccaac
 A  A  A  L  Q  H  P  Q  F  L  S  V  G  G  N  T  G  T  T  N
tccttcgccggcgtcgacctgcaggacctgagcggcggcctgtacaacgccgccagcctg
 S  F  A  G  V  D  L  Q  D  L  S  G  G  L  Y  N  A  A  S  L
ctcgagggcaacaacctcgcctgcttcggtttccaggcggccgtccaattcgcgccggac
 L  E  G  N  N  L  A  C  F  G  F  Q  A  A  V  Q  F  A  P  D
ctgttgaaaggcctggtgagcgatttgaccaagccgttgggtgtcctgggagatgcgttg
 L  L  K  G  L  V  S  D  L  T  K  P  L  G  V  L  G  D  A  L
gcgagtgcgttgaatggattagggtgtccgcagttaggcggccaggcgtgggatgatagc
 A  S  A  L  N  G  L  G  C  P  Q  L  G  G  Q  A  W  D  D  S
gcgctggcgcagtttccggggtatgctaggctgagggcggatggacgtacggaagtaa
 A  L  A  Q  F  P  G  Y  A  R  L  R  A  D  G  T  Y  G  K  -
SEQ ID NO : 12
   LENGTH : 419
   TYPE : PRT
   ORGANISM : *M. phaseolina* 
MPSTWMIALGALTLAGQSAAFPAVAEQYAAQTSHKEKRVNAISPGFNAAAQRIDVSGAHTFVPPGPG
DQRGPCPGLNALANHNGVATITQFVQATNQVYGMGLDLGTFLSVYGAVMDGDGLSWSIGGAPSTANL
LNLLTQPQGLSGSHNKYETDASPTRGDLYQYGDNSRVVISQWDALFAKQAALPNDQSNYGLGVLTDF
RVERFQQSVDENPYFFNAPFSGVLVQPAAYTFIYRFMGNKSAEKPEGVLTKEVLKSFFGFTGPDDDM
TYNPGHERIPENWYKRAPGDEYTIPFYALDLNAAALQHPQFLSVGGNTGTTNSFAGVDLQDLSGGLY
NAASLLEGNNLACFGFQAAVQFAPDLLKGLVSDLTKPLGVLGDALASALNGLGCPQLGGQAWDDSAL
AQFPGYARLRADGTYGK*
SEQ ID NO : 13
   LENGTH : 1108 (including 150 bp 5' UTR and 150 bp 3' UTR)
   TYPE : DNA
   ORGANISM : *M. phaseolina* 
CTGATGGTATGTACTTTGGTAAGCACGCCATTAACTACTCGCTCCATCGCCTCGAGTTACCCTGTTG
GAGCAGCAACCACAACTTGAGCGAACAAATTCGGCCATTAATACCATCGTACGAAGAAGGCTTAACC
GCTTTACCTGCCAGCCATGAATCCAATCTGCTTTTTATCCCTGCTCACGGCCATGCTAGGCATGGCC
ATGGGCGGTGGAAACCCTCTGAACCATGCTGAACCTTTTGATCCCACGAACAGCTTGTGGGTGTGAC
TGATGCGCATGAGCCCATTCTTCCTGATATCACCAACGCTCGCAGCCCCTGTCCCGGCCTTAACACG
TTGGCGAACCAGGAAGCTCATTCGCCCCCTTGCTATGAAGAAAACCTGGAATTTACGCTCCTCCCGG
TGGCTTCGACGGTGTCGCGAACTACGATGAACTTGTTAAGGCTCTGGTTGATAGTGTGTTGCTTTTG
ATCGGGGGTCACCACCTTTCGGTTGACGAGAACATCACGATTCCGCCTAATAATTTCACACGCAGCC
TCAGCGGCACACAAATCTCCCTAGATTCTGAGGCCTCGCCTACACGCCACGACGCGTACGACCCTCG
GGCCTACTCCGGCTCGAGCAGCATCGAAATGAAGTGGAACTTCTTCAAACAGCTGTACGAGAAGCAG
GCCGGGATCCCACGCGATGCGGTTAACTTTGCACTGGATGTTCTTGCTCAAAACATGTTGGAGCTGG
TGGTGGTCAGCATCAAGAACAACCCGAACTTCTTCCTGAGCCCAACGCACATCGCATTCGGACCATC
GACGGCCCACATGTGCATTCCCAACTTGTTCGCGAACCACAGCACTGAGCATTGGCTGACCTCTCTC
CTGAATAGAGAGGGAGGGTCTCGCCATTCATTCCATGCTCAAACTATGACGCTTTGTAGTCTTCAGC
 
TCAGATTGTCACCAATCTGAAGGCGAGATCCCACTTCTATACTCAGATTAAAGACCTAATAATACAA
TCGTATACTCAGCGAGAGAAGCACGAGAAGGACCGAGCCAACATTCACCTCATCCTTCACACAACAC
ACTCCTGAGCACCGACCCCAATTCTAAATCAGCCCC
SEQ ID NO : 14
   LENGTH : 690
   TYPE : DNA
   ORGANISM : *M. phaseolina*
   FEATURE NAME/KEY: CDS
   LOCATION : (1) ...... (690) 
atgaatccaatctgcttttatccctgctcacggccatgctaggcatggccatgggcggt
 M  N  P  I  C  F  L  S  L  L  T  A  M  L  G  M  A  M  G  G
ggaaaccctctgaaccatgctgaacctttgatcccacgaacagcttaaaacctggaatt
 G  N  P  L  N  H  A  E  P  F  D  P  T  N  S  L  K  P  G  I
tacgctcctcccggtggcttcgacggtgtcgcgaactacgatgaacttgttaaggctctg
 Y  A  P  P  G  G  F  D  G  V  A  N  Y  D  E  L  V  K  A  L
gttgatagtgtgttgcttttgatcgggggtcaccacctttcggttgacgagaacatcacg
 V  D  S  V  L  L  L  I  G  G  H  H  L  S  V  D  E  N  I  T
attccgcctaataatttcacacgcagcctcagcggcacacaaatctccctagattctgag
 I  P  P  N  N  F  T  R  S  L  S  G  T  Q  I  S  L  D  S  E
gcctcgcctacacgccacgacgcgtacgaccctcgggcctactccggctcgagcagcatc
 A  S  P  T  R  H  D  A  Y  D  P  R  A  Y  S  G  S  S  S  I
gaaatgaagtggaacttcttcaaacagctgtacgagaagcaggccgggatcccacgcgat
 E  M  K  W  N  F  F  K  Q  L  Y  E  K  Q  A  G  I  P  R  D
gcggttaactttgcactggatgttcttgctcaaaacatgttggagctggtggtggtcagc
 A  V  N  F  A  L  D  V  L  A  Q  N  M  L  E  L  V  V  V  S
atcaagaacaacccgaacttcttcctgagcccaacgcacatcgcattcggaccatcgacg
 I  K  N  N  P  N  F  F  L  S  P  T  H  I  A  F  G  P  S  T
gcccacatgtgcattcccaacttgttcgcgaaccacagcactgagcattggctgacctct
 A  H  M  C  I  P  N  L  F  A  N  H  S  T  E  H  W  L  T  S
ctcctgaatagagagggaggtctcgccattcattccatgctcaaactatgacgctttgt
 L  L  N  R  E  G  G  S  R  H  S  F  H  A  Q  T  M  T  L  C
agtcttcagctcagattgtcaccaatctga
 S  L  Q  L  R  L  S  P  I  -
SEQ ID NO : 15
   LENGTH : 229
   TYPE : PRT
   ORGANISM : *M. phaseolina* 
MNPICFLSLLTAMLGMAMGGGNPLNHAEPFDPTNSLKPGIYAPPGGFDGVANYDELVKALVDSVLLL
IGGHHLSVDENITIPPNNFTRSLSGTQISLDSEASPTRHDAYDPRAYSGSSSIEMKWNFFKQLYEKQ
AGIPRDAVNFALDVLAQNMLELVVVSIKNNPNFFLSPTHIAFGPSTAHMCIPNLFANHSTEHWLTSL
LNREGGSRHSFHAQTMTLCSLQLRLSPI*
SEQ ID NO : 16
   LENGTH : 1554 (including 150 bp 5' UTR and 150 bp 3' UTR)
   TYPE : DNA
   ORGANISM : *M. phaseolina* 
AAGCCTTGCTGGATCGACCTTTTTCAATTCCGGCAGATACACCCAAGTCATTGAACATCGAAACAGT
ACTTGCCTTCGTCAAATATGCGGTCACTCTTCCTTGCTTCCTTATTACTTTCTGCGGCGTCCGCTTT
CCCTTTCGTGGCAGATATGCCAGAGGTAGACTCTTCTCTCTTCCGTGAAGCACCCGTACGTAGGCAA
CAACCTGGCGGCAACCAACCTGGCGGAGCGGCGACTTGCCCTTTCAATGCCAACCACGTCCCCGCTG
CGCCAGTGACAGCTCGATTTCCCTATAACAACGCAAAGAACGGAGTTCCCGGCAACGGAAAGGGCGG
TTACCAGGTTCCAGCGCCTGGTGACACGGCTCATCAGTTCATTGCACCAACAGCGCACGATATCCGT
 
GGGCCTTGCCCGGGCCTGAACGCTGCGGCCAATCACGGCGTGAGTCTTTCCTGTAGTCCCCATTCGA
GTTTCAAGTCTCCTGACGGTGACCCTAAACAGTTCCTCGCGCGACGGCATAGTGACCTTCAACGA
ACTGGTCGACGCCCAGCAGAATGTCTACAATGTCGGCTACGACCTCTCTGTGCTGCTCGCCGTCCTC
GGCCTCACGCTCACCGACGGTGACCCCATCACCCAAAAACTGTCTATCGGCTGCGACGCAACGACAC
GCACATCTGTGGCCCCCCTGCTGACTGGCAGTCAGCCCGGTCTGGATGGCCACAACAAGTTCGAAGC
GGACACGTCGCTCACACGCAACGACTACTTCCTGGCGGGCGGCGACAACTTCAACTTCAACGGCACG
CTCTTCGGCATGATGGTGGATACGTGCCAGGGCAACTTCAACCGTGAGAACCTGGCGCTGTACCGCA
AGCAGCGCTACGACCAGAGCCTACGCGACAACGAGAACTTCTACTTCGGCCCGCTAAGCCTGCTGCT
GTTCGGCGCCGCCAGCTTCCTTTACGAGCTGATGCCCAGCGGCACGCACAACTACGCGCCCGATCTC
GACACCATCTCGTCCTTCTTCGGCGCCGAGCAGGCGCCCGATGGCTCCTGGCGCTTCACCGCCGAGC
GTATCCCGGACAACTGGACCAACCGTGTGCTGCCGTACACCAATGAGGACGTGACGCGCGAAATCCT
GGCTATGTACCTCCTCAACCCTGTGCTATTTGGCGGCGCCACAGGCGACGGCGGCTTCAACACGCTG
CCGAAGTTTGGCTCCATCCAGGACGGCAAGATCGTTGAGGCACCCAATACGCTGTGCCTGCTGTACC
AGCTGTCGACGCAGAGCGTGCCGAGCTCGCTGAATGGCATCATCACGCCGACTGTGGATGCGCTGAA
CCTGGTCGCGGATAAGCTAGCGCCGCAGTTCAAGAACTTGGGGTGCCCGAATCCGTTGACTTGAATG
AGTGATGGCCGTGTAGAGCCTCTGCTCGCGGTGCGAAGGAAAAGAAAAAAGTGAGGGGATTTTTGGC
GAGAAATGAAATTCACGAAGATACTGCCCGGATGGTAGAGGGAAACTTGGTCATGCATGTGCATGCG
AAGGCGTTGATAT
SEQ ID NO : 17
   LENGTH : 1194
   TYPE : DNA
   ORGANISM : *M. phaseolina*
   FEATURE NAME/KEY: CDS
   LOCATION : (1) ...... (1194) 
atgccagaggtagactcttctctcttccgtgaagcacccgtacgtaggcaacaacctggc
 M  P  E  V  D  S  S  L  F  R  E  A  P  V  R  R  Q  Q  P  G
ggcaaccaacctggcggagcggcgacttgccctttcaatgccaaccacgtccccgctgcg
 G  N  Q  P  G  G  A  A  T  C  P  F  N  A  N  H  V  P  A  A
ccagtgacagctcgatttccctataacaacgcaaagaacggagttcccggcaacggaaag
 P  V  T  A  R  F  P  Y  N  N  A  K  N  G  V  P  G  N  G  K
ggcggttaccaggttccagcgcctggtgacacggctcatcagttcattgcaccaacagcg
 G  G  Y  Q  V  P  A  P  G  D  T  A  H  Q  F  I  A  P  T  A
cacgatatccgtggcccttgcccgggcctgaacgctgcggccaatcacggcttcctcgcg
 H  D  I  R  G  P  C  P  G  L  N  A  A  A  N  H  G  F  L  A
cgcgacggcatagtgaccttcaacgaactggtcgacgcccagcagaatgtctacaatgtc
 R  D  G  I  V  T  F  N  E  L  V  D  A  Q  Q  N  V  Y  N  V
ggctacgacctctctgtgctgctcgccgtcctcggcctcacgctcaccgacggtgacccc
 G  Y  D  L  S  V  L  L  A  V  L  G  L  T  L  T  D  G  D  P
atcacccaaaaactgtctatcggctgcgacgcaacgacacgcacatctgtggcccccctg
 I  T  Q  K  L  S  I  G  C  D  A  T  T  R  T  S  V  A  P  L
ctgactggcagtcagcccggtctggatggccacaacaagttcgaagcggacacgtcgctc
 L  T  G  S  Q  P  G  L  D  G  H  N  K  F  E  A  D  T  S  L
acacgcaacgactacttcctggcgggcggcgacaacttcaacttcaacggcacgctcttc
 T  R  N  D  Y  F  L  A  G  G  D  N  F  N  F  N  G  T  L  F
ggcatgatggtggatacgtgccagggcaacttcaaccgtgagaacctggcgctgtaccgc
 G  M  M  V  D  T  C  Q  G  N  F  N  R  E  N  L  A  L  Y  R
aagcagcgctacgaccagagcctacgcgacaacgagaacttctacttcggcccgctaagc
 K  Q  R  Y  D  Q  S  L  R  D  N  E  N  F  Y  F  G  P  L  S
ctgctgctgttcggcgccgccagcttcctttacgagctgatgcccagcggcacgcacaac
 L  L  L  F  G  A  A  S  F  L  Y  E  L  M  P  S  G  T  H  N
tacgcgcccgatctcgacaccatctcgtccttcttcggcgccgagcaggcgcccgatggc
 Y  A  P  D  L  D  T  I  S  S  F  F  G  A  E  Q  A  P  D  G
tcctggcgcttcaccgccgagcgtatcccggacaactggaccaaccgtgtgctgccgtac
 S  W  R  F  T  A  E  R  I  P  D  N  W  T  N  R  V  L  P  Y
 
accaatgaggacgtgacgcgcgaaatcctggctatgtacctcctcaaccctgtgctattt
 T  N  E  D  V  T  R  E  I  L  A  M  Y  L  L  N  P  V  L  F
ggcggcgccacaggcgacggcggcttcaacacgctgccgaagtttggctccatccaggac
 G  G  A  T  G  D  G  G  F  N  T  L  P  K  F  G  S  I  Q  D
ggcaagatcgttgaggcacccaatacgctgtgcctgctgtaccagctgtcgacgcagagc
 G  K  I  V  E  A  P  N  T  L  C  L  L  Y  Q  L  S  T  Q  S
gtgccgagctcgctgaatggcatcatcacgccgactgtggatgcgctgaacctggtcgcg
 V  P  S  S  L  N  G  I  I  T  P  T  V  D  A  L  N  L  V  A
gataagctagcgccgcagttcaagaacttgggtgcccgaatccgttgacttga
 D  K  L  A  P  Q  F  K  N  L  G  C  P  N  P  L  T  -
SEQ ID NO : 18
   LENGTH : 397
   TYPE : PRT
   ORGANISM : *M. phaseolina* 
MPEVDSSLFREAPVRRQQPGGNQPGGAATCPFNANHVPAAPVTARFPYNNAKNGVPGNGKGGYQVPA
PGDTAHQFIAPTAHDIRGPCPGLNAAANHGFLARDGIVTFNELVDAQQNVYNVGYDLSVLLAVLGLT
LTDGDPITQKLSIGCDATTRTSVAPLLTGSQPGLDGHNKFEADTSLTRNDYFLAGGDNFNFNGTLFG
MMVDTCQGNFNRENLALYRKQRYDQSLRDNENFYFGPLSLLLFGAASFLYELMPSGTHNYAPDLDTI
SSFFGAEQAPDGSWRFTAERIPDNWTNRVLPYTNEDVTREILAMYLLNPVLFGGATGDGGFNTLPKF
GSIQDGKIVEAPNTLCLLYQLSTQSVPSSLNGIITPTVDALNLVADKLAPQFKNLGCPNPLT*
SEQ ID NO : 19
   LENGTH : 1792 (including 150 bp 5' UTR and 150 bp 3' UTR)
   TYPE : DNA
   ORGANISM : *M. phaseolina* 
ACCGACCACCATCCATTCGCTCTTTCGCTGGCGGCTGGCGTCCAAGGCTCACTCTCACTCTAGTCAC
TCTTTGCAATCTCTGAATTCCCGCGAGCCCTTGGTCTGCGTTGTTAAGGATTTGTTGTCCGGGCATC
CCCTCTCTTTTCCGCAATGAAGTTCTCGTCCGCCCTTCTCCTGCTCTCCTCGAGCTCCCTTGTCGTC
GATGCCTTCCCCGCCCTCGGTGCGCAGAACCTTGAGGGCCTCACTCCGGAAAGGTTGACCGCTGCCC
TCAAGACGGTCGAGAAGTACAGGAAGGAGAAGCGCCTTATCATCGACTCCAGCAAGCCTATCGATAC
CACCGGCGACCACGCTTTCCAGCCACCCAGCGAAACCGACCAGCGCGGCCCCTGTCCTGGCTTGAAC
GCCCTGGCCAACCATGGCTACATCTCTCGCGACGGCATCACCAGCTTCGCCGAGGTTGTCACCGCCA
TTAACCAAGGTTCGTCGTGGATTCGTCGAGAGAAGGCGTGGCTTGGCACTGACAAGGATGTGAAGTG
ATGGGCATGGGCATCGAGCTCTCTCTGATTCTCGGTGTTATGGGTACCGTGTGGACGGGTAACCCGC
TTTCGCTGGACCCTGGCTTCTCTATCGGTGGGACCGCCCCCGGTGATGGCTCCGACAACATTCTGGG
CAACCTTGTCGGCCTGCTCGGTACGTAACTGTCCCTCATCGGAGCCACGCCGCCGCAGCTAAGCTGA
GACGTCTGCAGGTGACCCTCGTGGTCTGCAAGGCTCCCACAACTGGATTGAGTCTGACTCCTCTCTG
ACGCGTGATGATCTGTACCTCACCGGAGATGCCTGGACGATGAACATGACGCTCTTCCGCGACATCT
ACGACCGCGCGGATGAGGATGGCGTCATCTCCATGGATCTGCTCGCCGACCAGGCCGCCCGTCGCTG
GGAGTACAGCATCGGCCACAACCCCAACTTCTACTACGGCCCTGTCACCGGCATGGTCAGCCGTAAT
GCCGGCTACTTTTTCCTCGGCCGCCTGCTGTCAAACCACACCGATGAACATCCGGACGGAATTCTCA
CTCAAGAAGTTTTCAAGAAGTTCTTCGCCGTCTACGAGGACGAGCAGGGCAACATGGAATACCGCAA
GGGCCACGAGACCTTCCCGGACAACTGGTACCGCAAGCCGGTCGAGTATGGCCTGGTCCCGCTCAAC
TTGGACCTCGTTGGCTGGGTCTTGAAGCACCCTGAGCTGGGAAGGTACGTCGTCCCTTCTCACCCCA
AGATGGGAAGGCATGTGAACTGACTCGGCTTCCCACAGCATCGGCGGTAACACTGGCACCGTCAACT
CCTTCTCCGGCCTCGATCTGCACAGCATCACCGGCGGCGTCCTCAATGCCACTTCGCTCCTCGAGAA
CAACAACCTGCTGTGTTTTGTCTTTGAAGTTCTCAAGACCTTCGCCCCCAACTCCCTCTCCCCGCTC
CTGTCGACGCTCGAAGTGCCGCTCAAGCTTATCGCCGACACCCTGGCCACCCCGCTCTTGAGCCTGG
CCTGCCCTGCCTGGAAGGATATGACCGACGGTGGCGAGCCGCTGTGGGATGGCATTCAGAACAGGTT
CCCTGGCGCGAGCAAGGCCGGATCGAGTTTGTAGAGTTGCTCGAGAGGACACAGGACGTCTGGAGCA
TGACGTCTGGGTGAACGTACTGCGTGGAAGAAGAGCAAAGGAACCAGCGAGGGCGAGAAATGATGTA
GTTAGCGTCTTGATTCTAATTCGATACCATTTACATTCTTCGCCTTATCC
SEQ ID NO : 20
   LENGTH : 1317
   TYPE : DNA
   ORGANISM : *M. phaseolina*
   FEATURE NAME/KEY: CDS
   LOCATION : (1) ...... (1317) 
atgaagttctcgtccgcccttctcctgctctcctcgagctcccttgtcgtcgatgccttc
 M  K  F  S  S  A  L  L  L  L  S  S  S  S  L  V  V  D  A  F
cccgccctcggtgcgcagaaccttgagggcctcactccggaaaggttgaccgctgccctc
 P  A  L  G  A  Q  N  L  E  G  L  T  P  E  R  L  T  A  A  L
aagacggtcgagaagtacaggaaggagaagcgccttatcatcgactccagcaagcctatc
 K  T  V  E  K  Y  R  K  E  K  R  L  I  I  D  S  S  K  P  I
gataccaccggcgaccacgctttccagccacccagcgaaaccgaccagcgcggcccctgt
 D  T  T  G  D  H  A  F  Q  P  P  S  E  T  D  Q  R  G  P  C
cctggcttgaacgccctggccaaccatggctacatctctcgcgacggcatcaccagcttc
 P  G  L  N  A  L  A  N  H  G  Y  I  S  R  D  G  I  T  S  F
gccgaggttgtcaccgccattaaccaagtgatgggcatgggcatcgagctctctctgatt
 A  E  V  V  T  A  I  N  Q  V  M  G  M  G  I  E  L  S  L  I
ctcggtgttatgggtaccgtgtggacgggtaacccgctttcgctggaccctggcttctct
 L  G  V  M  G  T  V  W  T  G  N  P  L  S  L  D  P  G  F  S
atcggtgggaccgcccccggtgatggctccgacaacattctgggcaaccttgtcggcctg
 I  G  G  T  A  P  G  D  G  S  D  N  I  L  G  N  L  V  G  L
ctcggtgaccctcgtggtctgcaaggctcccacaactggattgagtctgactcctctctg
 L  G  D  P  R  G  L  Q  G  S  H  N  W  I  E  S  D  S  S  L
acgcgtgatgatctgtacctcaccggagatgcctggacgatgaacatgacgctcttccgc
 T  R  D  D  L  Y  L  T  G  D  A  W  T  M  N  M  T  L  F  R
gacatctacgaccgcgcggatgaggatggcgtcatctccatggatctgctcgccgaccag
 D  I  Y  D  R  A  D  E  D  G  V  I  S  M  D  L  L  A  D  Q
gccgcccgtcgctgggagtacagcatcggccacaaccccaacttctactacggccctgtc
 A  A  R  R  W  E  Y  S  I  G  H  N  P  N  F  Y  Y  G  P  V
accggcatggtcagccgtaatgccggctactttttcctcggccgcctgctgtcaaaccac
 T  G  M  V  S  R  N  A  G  Y  F  F  L  G  R  L  L  S  N  H
accgatgaacatccggacggaattctcactcaagaagtttttcaagaagttcttcgccgtc
 T  D  E  H  P  D  G  I  L  T  Q  E  V  F  K  K  F  F  A  V
tacgaggacgagcagggcaacatggaataccgcaagggccacgagaccttcccggacaac
 Y  E  D  E  Q  G  N  M  E  Y  R  K  G  H  E  T  F  P  D  N
tggtaccgcaagccggtcgagtatggcctggtcccgctcaacttggacctcgttggctgg
 W  Y  R  K  P  V  E  Y  G  L  V  P  L  N  L  D  L  V  G  W
gtcttgaagcaccctgagctgggaagcatcggcggtaacactggcaccgtcaactccttc
 V  L  K  H  P  E  L  G  S  I  G  G  N  T  G  T  V  N  S  F
tccggcctcgatctgcacagcatcaccggcggcgtcctcaatgccacttcgctcctcgag
 S  G  L  D  L  H  S  I  T  G  G  V  L  N  A  T  S  L  L  E
aacaacaacctgctgtgtttgtctttgaagttctcaagaccttcgcccccaactccctc
 N  N  N  L  L  C  F  V  F  E  V  L  K  T  F  A  P  N  S  L
tccccgctcctgtcgacgctcgaagtgccgctcaagcttatcgccgacaccctggccacc
 S  P  L  L  S  T  L  E  V  P  L  K  L  I  A  D  T  L  A  T
ccgctcttgagcctggcctgccctgcctggaaggatatgaccgacggtggcgagccgctg
 P  L  L  S  L  A  C  P  A  W  K  D  M  T  D  G  G  E  P  L
tgggatggcattcagaacaggttccctggcgcgagcaaggccggatcgagtttgtag
 W  D  G  I  Q  N  R  F  P  G  A  S  K  A  G  S  S  L  -
SEQ ID NO : 21
   LENGTH : 438
   TYPE : PRT
   ORGANISM : *M. phaseolina* 
MKFSSALLLLSSSSLVVDAFPALGAQNLEGLTPERLTAALKTVEKYRKEKRLIIDSSKPIDTTGDHA
FQPPSETDQRGPCPGLNALANHGYISRDGITSFAEVVTAINQVMGMGIELSLILGVMGTVWTGNPLS
LDPGFSIGGTAPGDGSDNILGNLVGLLGDPRGLQGSHNWIESDSSLTRDDLYLTGDAWTMNMTLFRD
IYDRADEDGVISMDLLADQAARRWEYSIGHNPNFYYGPVTGMVSRNAGYFFLGRLLSNHTDEHPDGI
LTQEVFKKFFAVYEDEQGNMEYRKGHETFPDNWYRKPVEYGLVPLNLDLVGWVLKHPELGSIGGNTG
TVNSFSGLDLHSITGGVLNATSLLENNNLLCFVFEVLKTFAPNSLSPLLSTLEVPLKLIADTLATPL
LSLACPAWKDMTDGGEPLWDGIQNRFPGASKAGSSL*
SEQ ID NO : 22
   LENGTH : 1391 (including 150 bp 5' UTR and 150 bp 3' UTR)
   TYPE : DNA
   ORGANISM : *M. phaseolina* 
ATACTAACGCCGTGCAACCCCCGGGCAGCGCAGGTCTTGTTGATCGATATAAATGTTTTGTTTCGCG
CCGCTATTGAAATTGGACACTCCAACTGTTTTCCATACATTCATCTTTCCCTATTGATCAATTGCTG
CAAAAAACATTCCATCATGAAGCTCACCGTCTGTGCCACGACCCTTTGCTTCGCTTTGGTTTATGGA
CAAGGCTCCTACGAGGGGTGGAAGCCAGCTGGGCCAGACGACTGTATGTTCAGTGGAATTTCTACGT
TTCCTTTGATTTGCGCTGATGTACATCTACAGTTCGCGGCCCTTGCCCTATGATGAATACCTTGGCC
AACCATGGCTTTCTCCCCCACGATGGCAGGAATATCACGAAAGCCAACGCAATCCACGCTCTCAACA
CAGCTATCAACTTCAACACTTCCCTCGCTGCTATCATGTGGGAGCAGGCTATCATTGCAAACCCGGA
GCCCAATGCTACGTTCTTCACTCTGTACGTGACTTCAAGCAAGCGTCTTTTGAGCTACCCTGAGTAG
GGGGCTGCTCCGAAAGGCGACTTATCTAAACCTCCTCTTTCCACTATGTGAAGCTCTGACATTAACT
AATATTGAACAGTGACCATCTTAATCGTCACAACGTCTTGGAGCACGATGCCAGCTTGAGGTGAGTG
GAAGCCGCCTTTCTCCAGCCTATTCCAACAAATCTCCTGACCAACACTCCTCTTCAGCCGATCCGAC
GCCTTCTTCGGCAACAACCACGTCTTCAACCAAACTATCTTCGACGTCTCTCGCGCGTGGTGGACGG
AGGAAACCGTAGACGCCAAGATGCTGGCCAACAGCAAGTTGTTCCGGCAGATCGAGTCGCGAGCCGC
CAACCCGAATTACACCTTCACCCAAACTACCGAGGCCTTTAGCTTGGGCGAGGTGGCTGCTCCCATC
ATCGTCTTCGGCGACCACGCGGCCGGCACCGTCAACAGGAGTCTGGTCGAGTACTTCTTCGGTGAGC
CAAGACCTCAGAGACTTTGGTCTGCAAGGGTTCAAAATTACTAATTATGGGGTGGTCTTGCAGAGAA
CGAACGCCTCCCGACCGAGTTGGGCTGGACTAAGCAGGCTAATGATGTGTCTCTGGAGGTCATCCTG
GAGATCCAGGACCTCGTCCGCAACGCGACCAACCTGATCACCGATGCCCCGCTGCCGGCAGCGCCTC
ACAAGCGGGACCTGCACGCCCCTTACAGCCTCTAGATACGAATATAAAGCTGGCATGAAGATCACCA
GGGGTTTTTGAGAATTGTGTCTTCGGCGGGACGGGAGTGGTAGATTGAGCCTTGTTGGGCTTCAGGT
GTCTGGGGCATGGTTTGCTTTGGTCCTTTTACATGATTTCCCTAGCACGCC
SEQ ID NO : 23
   LENGTH : 780
   TYPE : DNA
   ORGANISM : *M. phaseolina*
   FEATURE NAME/KEY: CDS
   LOCATION : (1) ...... (780) 
atgaagctcaccgtctgtgccacgaccctttgcttcgctttggtttatggacaaggctcc
 M  K  L  T  V  C  A  T  T  L  C  F  A  L  V  Y  G  Q  G  S
tacgagggtggaagccagctggccagacgactttcgcggcccttgccctatgatgaat
 Y  E  G  W  K  P  A  G  P  D  D  F  R  G  P  C  P  M  M  N
accttggccaaccatggctttctcccccacgatggcaggaatatcacgaaagccaacgca
 T  L  A  N  H  G  F  L  P  H  D  G  R  N  I  T  K  A  N  A
atccacgctctcaacacagctatcaacttcaacacttccctcgctgctatcatgtgggag
 I  H  A  L  N  T  A  I  N  F  N  T  S  L  A  A  I  M  W  E
caggctatcattgcaaacccggagcccaatgctacgttcttcactcttgaccatcttaat
 Q  A  I  I  A  N  P  E  P  N  A  T  F  F  T  L  D  H  L  N
cgtcacaacgtcttggagcacgatgccagcttgagccgatccgacgccttcttcggcaac
 R  H  N  V  L  E  H  D  A  S  L  S  R  S  D  A  F  F  G  N
aaccacgtcttcaaccaaactatcttcgacgtctctcgcgcgtggtggacggaggaaacc
 N  H  V  F  N  Q  T  I  F  D  V  S  R  A  W  W  T  E  E  T
gtagacgccaagatgctggccaacagcaagttgttccggcagatcgagtcgcgagccgcc
 
V D A K M L A N S K L F R Q I E S R A A
aacccgaattacaccttcacccaaactaccgaggcctttagcttgggcgaggtggctgct
N P N Y T F T Q T T E A F S L G E V A A
cccatcatcgtcttcggcgaccacgcggccggcaccgtcaacaggagtctggtcgagtac
P I I V F G D H A A G T V N R S L V E Y
ttcttcgagaacgaacgcctcccgaccgagttgggctggactaagcaggctaatgatgtg
F F E N E R L P T E L G W T K Q A N D V
tctctggaggtcatcctggagatccaggacctcgtccgcaacgcgaccaacctgatcacc
S L E V I L E I Q D L V R N A T N L I T
gatgccccgctgccggcagcgcctcacaagcgggacctgcacgccccttacagcctctag
D A P L P A A P H K R D L H A P Y S L -
SEQ ID NO : 24
   LENGTH : 259
   TYPE : PRT
   ORGANISM : *M. phaseolina* 
MKLTVCATTLCFALVYGQGSYEGWKPAGPDDFRGPCPMMNTLANHGFLPHDGRNITKANAIHALNTA
INFNTSLAAIMWEQAIIANPEPNATFFTLDHLNRHNVLEHDASLSRSDAFFGNNHVFNQTIFDVSRA
WWTEETVDAKMLANSKLFRQIESRAANPNYTFTQTTEAFSLGEVAAPIIVFGDHAAGTVNRSLVEYF
FENERLPTELGWTKQANDVSLEVILEIQDLVRNATNLITDAPLPAAPHKRDLHAPYSL*
SEQ ID NO : 25
   LENGTH : 1314 (including 150 bp 5' UTR and 150 bp 3' UTR)
   TYPE : DNA
   ORGANISM : *M. phaseolina* 
GGCCCCCGAAAGGAGACAAAATACGAGATTCTCCTATTACGCGCCCAAGAATCGACAACACCGCCC
ATCAGCTACCGCCGCCTTCGTTTACGTCGTCCGCAGCAGCAACGCCCGACGCTACAGTCAGTCAACA
GCCTCCAGCCGCCACGATGCCCAACGCAGTGCATCTGTCAATGCTCGTCCTCACGCATGCGGTGCCG
ATCGCGGGTTATCCGGGCTGGGAAGCGCAGCAAGTTGTCGCAGAAGCGCCCTCCAAGCACCACAACG
ACCTCTACGTGCCCAACCCAAGCCATCCGGTGCCGGGGAAAGTGCCGTACATCCCGGACGAGGAAGA
GCACTACTTTGAGAAGCAGGTAAACGGCTCCGGCAATGGCTACTACCGGCGGTCGTCCTGCCCGGCA
GTCAACATCATGGCGAACAGGGGCTACATCAGCCGCTCGGGCCGGGACATCAGCTACGAGGAGATAG
CGATGGCATCGCGGGAGCTGTTCAACTTCGGCGACGACAACGTGAGCAGCGGCCCCTGCAGACCGGC
AAAGTAAGCGGGCTCACCAACAATTCCTTCTTGACAGATCATGATCGTGCTGGGGCCCAGCTTCGCG
GCGCACCCGGGCCGCGAGCGCATCGACCTCGACATGCTGGCCGACGACGCGGTGCAGCACATCACCA
ACTGCCCTGCGGCGCCGACGCGGACGGACCGCGCGCTGGGCGACAACGTGAACCTGAACACGACGCT
GCTGGAGCAGCTGCTGGCGACGTCCAAGGACGGCGTCACGCTGACGCTCGAAGACGCAGCCGAGCAC
CACCACCTGCGGCACAACCAGTCGCTGGCCGAGAACCCCGGCTTCCGCTTCAGCAACTCCGACGCCA
TCTGCTCGCTTGCGCAGTACGCCAACCTGTTCGGTATCCTGGGCCGGCAGGGCAAGCATGGGCTCAA
CACGCTGTATGTGGAAGACGTCAAGACCCTGTTCGTCGACGAAGACCTGCCGGACGGATACGGCCGG
AGGGAGCTGCCGTATTTCTCGACCGAGGCGAACAACTACATCGACCGTATGGCCCACCACATCGGCT
TCGAGATCGAGCGGCCGTTCCCGGCCAACGACGCCGACCTGAAGGACATCGAGCCGGTGCAAGCCAG
ATTTGAAGTGGTGGACGGATGCTGAGCGGTGCAAAATTAAAAAAAAATTTTTTTAGGCAGAAGTC
AAGCATTTAATCGGGCTATACACACATCATTCCGTGCAAACAAAATCATTTCTACAGCTGTACCGCC
TCGGAAAAGAATAACGGAGATTTAAAAAAAAAAAAAAAAAC
SEQ ID NO : 26
   LENGTH : 951
   TYPE : DNA
   ORGANISM : *M. phaseolina*
   FEATURE NAME/KEY: CDS
   LOCATION : (1) ...... (951) 
atgcccaacgcagtgcatctgtcaatgctcgtcctcacgcatgcggtgccgatcgcgggt
 M  P  N  A  V  H  L  S  M  L  V  L  T  H  A  V  P  I  A  G
tatccggctgggaagcgcagcaagttgtcgcagaagcgccctccaagcaccacaacgac
 
 Y  P  G  W  E  A  Q  Q  V  V  A  E  A  P  S  K  H  H  N  D
ctctacgtgcccaacccaagccatccggtgccggggaaagtgccgtacatcccggacgag
 L  Y  V  P  N  P  S  H  P  V  P  G  K  V  P  Y  I  P  D  E
gaagagcactactttgagaagcaggtaaacggctccggcaatggctactaccggcggtcg
 E  E  H  Y  F  E  K  Q  V  N  G  S  G  N  G  Y  Y  R  R  S
tcctgcccggcagtcaacatcatggcgaacaggggctacatcagccgctcgggccgggac
 S  C  P  A  V  N  I  M  A  N  R  G  Y  I  S  R  S  G  R  D
atcagctacgaggagatagcgatggcatcgcgggagctgttcaacttcggcgacgacaac
 I  S  Y  E  E  I  A  M  A  S  R  E  L  F  N  F  G  D  D  N
atcatgatcgtgctggggcccagcttcgcggcgcacccgggccgcgagcgcatcgacctc
 I  M  I  V  L  G  P  S  F  A  A  H  P  G  R  E  R  I  D  L
gacatgctggccgacgacgcggtgcagcacatcaccaactgccctgcggcgccgacgcgg
 D  M  L  A  D  D  A  V  Q  H  I  T  N  C  P  A  A  P  T  R
acggaccgcgcgctgggcgacaacgtgaacctgaacacgacgctgctggagcagctgctg
 T  D  R  A  L  G  D  N  V  N  L  N  T  T  L  L  E  Q  L  L
gcgacgtccaaggacggcgtcacgctgacgctcgaagacgcagccgagcaccaccacctg
 A  T  S  K  D  G  V  T  L  T  L  E  D  A  A  E  H  H  H  L
cggcacaaccagtcgctggccgagaaccccggcttccgcttcagcaactccgacgccatc
 R  H  N  Q  S  L  A  E  N  P  G  F  R  F  S  N  S  D  A  I
tgctcgcttgcgcagtacgccaacctgttcggtatcctgggccggcagggcaagcatggg
 C  S  L  A  Q  Y  A  N  L  F  G  I  L  G  R  Q  G  K  H  G
ctcaacacgctgtatgtggaagacgtcaagaccctgttcgtcgacgaagacctgccggac
 L  N  T  L  Y  V  E  D  V  K  T  L  F  V  D  E  D  L  P  D
ggatacggccggagggagctgccgtatttctcgaccgaggcgaacaactacatcgaccgt
 G  Y  G  R  R  E  L  P  Y  F  S  T  E  A  N  N  Y  I  D  R
atggcccaccacatcggcttcgagatcgagcggccgttcccggccaacgacgccgacctg
 M  A  H  H  I  G  F  E  I  E  R  P  F  P  A  N  D  A  D  L
aaggacatcgagccggtgcaagccagatttgaagtggtggacggatgctga
 K  D  I  E  P  V  Q  A  R  F  E  V  V  D  G  C  -
SEQ ID NO : 27
   LENGTH : 316
   TYPE : PRT
   ORGANISM : *M. phaseolina* 
MPNAVHLSMLVLTHAVPIAGYPGWEAQQVVAEAPSKHHNDLYVPNPSHPVPGKVPYIPDEEEHYFEK
QVNGSGNGYYRRSSCPAVNIMANRGYISRSGRDISYEEIAMASRELFNFGDDNIMIVLGPSFAAHPG
RERIDLDMLADDAVQHITNCPAAPTRTDRALGDNVNLNTTLLEQLLATSKDGVTLTLEDAAEHHHLR
HNQSLAENPGFRFSNSDAICSLAQYANLFGILGRQGKHGLNTLYVEDVKTLFVDEDLPDGYGRRELP
YFSTEANNYIDRMAHHIGFEIERPFPANDADLKDIEPVQARFEVVDGC*
SEQ ID NO : 28
   LENGTH : 1480 (including 150 bp 5' UTR and 150 bp 3' UTR)
   TYPE : DNA
   ORGANISM : *M. phaseolina* 
ATCTGGCTTTTCCTCTCTTCTCTGTCGGGCGTCCCTTGCGCAACAGATACCCATTCATCCCTTCAG
TGCTTCTtCTTATCTCTTCGCGCGCTTTTCCCCAACCACAGCCTCTTATCGCCTGTGCTCACACCAC
GTTTCCCGCACACCCAATGGCTTCCACCGCCAGGTCCGTCTTCGCCCGCAGCGCGCTTCTGCGCTCC
GCCCCGGCCTCCATCAAGTCGAATGCCGCCCGCTCATCTCGCTTCGCCGTCCCTACCCAAGCATTCC
GCCAGCAGTCTCGCCGCGGCTACTCTTCCGAGGCCGGCTCCAAGTCTAACGGCCCCAACCCCGCCAT
CTGGATTGGTGCTCTCGCCGTCCTGGGTGGCGCCGGGTACTATGCCTACAGCTCGGGTGCCGGCGCC
CAGATCGCCTCCAAGGAACCCTTCACCCCCAAGCCCGAGGACTACCAGAAGGTCTACGACGCCATCG
CCAAGGCCCTCGAAGAGCACGACGACTACGACGACGGCAGCTACGGCCCTGTCCTGCTGAGACTGGC
TTGGCACGCCAGCGGAACGTGAGTGACTTCCCCAACACTTCCAGCCCACCATTGAACCACGCACTGA
CGCCCTCCCTCACAGCTACGACAAGGAAACCGGCACCGGCGGCTCCAACGGCGCCACGATGCGCTTC
GCGCCCGAGGCGGACCACGGCGCCAACGCCGGCCTCAAGGCGGCCCGCGACTTCCTCGAGCCCATCA
AGCAGCAGTTCCCGTGGATTACGTACTCGGACCTGTGGACGCTGGCAGGCGTCGCTGCGATCCAGGA
 
GATGCAGGGCCCCAAGGTGCCGTGGCGCCCCGGCCGCAGCGATCGCGACGTCTCCTTCTGCACGCCC
GACGGCCGCCTGCCCGACGCCTCCAAGGACCAGAACCACCTCCGCGCCATCTTCGGCCGCATGGGTT
GGAATGACCAGGAGATCGTGGCGCTGTCGGGCGCGCATGCGCTGGGGAGGTGCCATACGGATAGGAG
TGGATTCGATGGCCCGTGGACCTTCAGCCCGACGACGCTGACGAACGATTATTTCAAGTTGTTGATC
GACGAGAAGTGGCAGTGGCGGAAGTGGGATGGACCTAAGCAGTTGGAGGACAAGAAGACGAAGAGCC
TGATGATGCTGCCGACGGATTACGCGTTGGTGCAGGACAAGAAGTTTAAGCCCTGGGTCGAGAGGTA
CGCGAAGGATCAGGATGCCTTCTTCAAGGACTTCTCGAACGTGGTTATGAGGTTGTTCGAGCTGGGC
GTGCCGTTCCAGAGTGGTGAGGACTCGAGGATTGTGTTTAAGAGCAGCTTCGACTAGGCGTGCTTGG
CGGACGTTAATTCTGATCGACGGGGTTTGATGGGAAGTTGTAAAAGGTTCTATGACGATCAGTAAAG
AAGGGTTGTTTTTGCTTTTGAGTTTCGAGGACTAAAGACTAAGACAAGAGTAGCGCAAAGGTGGGAA
AGAATA
SEQ ID NO : 29
   LENGTH : 1116
   TYPE : DNA
   ORGANISM : *M. phaseolina*
   FEATURE NAME/KEY: CDS
   LOCATION : (1) ...... (1116) 
atggcttccaccgccaggtccgtcttcgcccgcagcgcgcttctgcgctccgccccggcc
 M  A  S  T  A  R  S  V  F  A  R  S  A  L  L  R  S  A  P  A
tccatcaagtcgaatgccgcccgctcatctcgcttcgccgtccctacccaagcattccgc
 S  I  K  S  N  A  A  R  S  S  R  F  A  V  P  T  Q  A  F  R
cagcagtctcgccgcggctactcttccgaggccggctccaagtctaacggccccaacccc
 Q  Q  S  R  R  G  Y  S  S  E  A  G  S  K  S  N  G  P  N  P
gccatctggattggtgctctcgccgtcctgggtggcgccgggtactatgcctacagctcg
 A  I  W  I  G  A  L  A  V  L  G  G  A  G  Y  Y  A  Y  S  S
ggtgccggcgcccagatcgcctccaaggaacccttcacccccaagcccgaggactaccag
 G  A  G  A  Q  I  A  S  K  E  P  F  T  P  K  P  E  D  Y  Q
aaggtctacgacgccatcgccaaggccctcgaagagcacgacgactacgacgacggcagc
 K  V  Y  D  A  I  A  K  A  L  E  E  H  D  D  Y  D  D  G  S
tacggccctgtcctgctgagactggcttggcacgccagcggaacctacgacaaggaaacc
 Y  G  P  V  L  L  R  L  A  W  H  A  S  G  T  Y  D  K  E  T
ggcaccggcggctccaacggcgccacgatgcgcttcgcgcccgaggcggaccacggcgcc
 G  T  G  G  S  N  G  A  T  M  R  F  A  P  E  A  D  H  G  A
aacgccggcctcaaggcggcccgcgacttcctcgagcccatcaagcagcagttcccgtgg
 N  A  G  L  K  A  A  R  D  F  L  E  P  I  K  Q  Q  F  P  W
attacgtactcggacctgtggacgctggcaggcgtcgctgcgatccaggagatgcagggc
 I  T  Y  S  D  L  W  T  L  A  G  V  A  A  I  Q  E  M  Q  G
cccaaggtgccgtggcgccccggccgcagcgatcgcgacgtctccttctgcacgcccgac
 P  K  V  P  W  R  P  G  R  S  D  R  D  V  S  F  C  T  P  D
ggccgcctgcccgacgcctccaaggaccagaaccacctccgcgccatcttcggccgcatg
 G  R  L  P  D  A  S  K  D  Q  N  H  L  R  A  I  F  G  R  M
ggttggaatgaccaggagatcgtggcgctgtcgggcgcgcatgcgctggggaggtgccat
 G  W  N  D  Q  E  I  V  A  L  S  G  A  H  A  L  G  R  C  H
acggataggagtggattcgatggcccgtggaccttcagcccgacgacgctgacgaacgat
 T  D  R  S  G  F  D  G  P  W  T  F  S  P  T  T  L  T  N  D
tatttcaagttgttgatcgacgagaagtggcagtggcggaagtgggatggacctaagcag
 Y  F  K  L  L  I  D  E  K  W  Q  W  R  K  W  D  G  P  K  Q
ttggaggacaagaagacgaagagcctgatgatgctgccgacggattacgcgttggtgcag
 L  E  D  K  K  T  K  S  L  M  M  L  P  T  D  Y  A  L  V  Q
gacaagaagtttaagccctgggtcgagaggtacgcgaaggatcaggatgccttcttcaag
 D  K  K  F  K  P  W  V  E  R  Y  A  K  D  Q  D  A  F  F  K
gacttctcgaacgtggttatgaggttgttcgagctgggcgtgccgttccagagtggtgag
 D  F  S  N  V  V  M  R  L  F  E  L  G  V  P  F  Q  S  G  E
gactcgaggattgtgtttaagagcagcttcgactag
 D S R I V F K S S F D -
SEQ ID NO : 30
   LENGTH : 371
   TYPE : PRT
   ORGANISM : *M. phaseolina* 
MASTARSVFARSALLRSAPASIKSNAARSSRFAVPTQAFRQQSRRGYSSEAGSKSNGPNPAIWIGAL
AVLGGAGYYAYSSGAGAQIASKEPFTPKPEDYQKVYDAIAKALEEHDDYDDGSYGPVLLRLAWHASG
TYDKETGTGGSNGATMRFAPEADHGANAGLKAARDFLEPIKQQFPWITYSDLWTLAGVAAIQEMQGP
KVPWRPGRSDRDVSFCTPDGRLPDASKDQNHLRAIFGRMGWNDQEIVALSGAHALGRCHTDRSGFDG
PWTFSPTTLTNDYFKLLIDEKWQWRKWDGPKQLEDKKTKSLMMLPTDYALVQDKKFKPWVERYAKDQ
DAFFKDFSNVVMRLFELGVPFQSGEDSRIVFKSSFD*
SEQ ID NO : 31
   LENGTH : 1981 (including 150 bp 5' UTR and 150 bp 3' UTR)
   TYPE : DNA
   ORGANISM : *M. phaseolina* 
TAGAAGGCGCATGGAGTCGCACGCAGGACCATGCGCAACACGGTGCCTGCAGAGAGGCGAAAGCCC
ATGCATATCACATTGATTTTAAAGAAGGAAGTGCAAGAAGCATTGAGACATCGCACTCACCCTTCA
ACACCGCTATCGCGCGATGAAAGCCATCAGCATCGCCGTGTTCGCCGCGTCAGTGGTACCTCACACT
CTGGCAGACTATGTTTGGCCCTCGCAACATGACTTGTTGGAGGATATGTTGGCCATCCAACAAGGCT
ATATTCGCATGGGCTTCACTGACTGTATGTCGACATTCACTTGAGCCTGAAGTAATGTCTCCCTACT
AAAGCATCGTCGAAGTGGTTGTTCCTTGCGGTCACGGCAGCAACAGGCCCGGAGTAAACAACGCGGC
ACAATGGATTCGGACGGGTTTCCACGACTTCGCCACCCACGACTCGGCCGCTGGACCGGCGGCCTC
GATGCCTCCCTCCTCTACGAGGTCGAACGGCCCGAGAACGAGGGCTCAGCCTTTAACGACACCTTCG
CCGACATGCACGACTTTATCAATCCCCGATCAAGCGCCTCGGATCTGATAGCGCTGGCCGTCGTCGC
ATCGGTTGCGGCTTGCGGCGGACCCAAGATCCCCCTGCGAGCGGGACGAATTGATGCTGTGGAGGCG
GGCCCGGCCGGCGTTCCCAAGCCCGACGACTCACTGGAGAGCACGATCGATGCATTCGCGCGGACGG
GGTTCAACACCAGCGACATGATCGCCCTCGTTGCCTGTGGCCATACCGTCGGCGGCGTGCACAGCGT
AGATTTCCCCGAGATCACCGGCGGCGAGAAAGACGTCCTGGACGTGCCGCAATTTGACAGCAGCGGC
ACCATCTTCGACACCGCAGTCGTGGACGAATACCTCGACAGCAACGGCGCCAACCCCCTCGTCTTCG
GAGCCAACGACACCACGAACTCGGACAAGCGCGTCTTCAGCGCCGACGGCAACAGCACCATGGCCAA
GCTCAAAGACCCCGCCACATTCAAAGCCACCTGCGCCGCCCTCTTCGAGCGTATGATCAACACCGTC
CCCTCATCCGTCACCCTCAGCGAACCCATCGAGCTGGCCGACATCAAGCCCTACATCGACAAGCTCG
AGCTCACACCCAACGCCTCCGCCCTCGCCTTCGAAGGCCGCATCCGGCTGCGCACCTCCCCCGTCAC
CGGCCGCGACGCCGAGGGCACCAGCATCGCCCTCAATGTCACCGACCGCGCTGGCGGGCGCAAGCTG
GTGCCGGCGCCGCGCGCCGTGTTGCGCGGCGGCACCTCGTACGGCTTCTTCGACGAGCAGTTCAGCT
GGTTCGAATTTGCGACGCAGCTCGACGTCGCAGCCGGCATCCAGGCCTTTGATATCCAGCTCACGAC
GGAGGCGACCGGACACGTGGAGACGTTCGACAACGCTGGCACCGGCGGCTATCCTAGTCTCGACGAC
TTGCTATACTTGCAGTCGCAATCGTGCATGGACACGACTGCCACGGAGGGGAATATAACGGTGACGG
TTGCGGCGGCGGTGCGCGAGGATGCGGCGAAGGCGGGTGCGGCGCCAGTGGTGAGGATGGCGCATAA
GGTTCAGCAGATGGGTGTGATGTTGCCGAAGCTTGTCGTAGAGGCAGTGCCAATGGAGAGGTCAAAC
GTTTCGCAGGGCGGGTATGTATTGTACGAGGTGGACATCCCAATCGATGCCGCTGGCTGGAGCACGA
AGTTCGATGTTGTGTTGACGGCTGGGGGAGACGAGATTGTGTCTGGCCTGCACGGGACCAGCGATCT
TACCACATGTTCGGGAAACTGACCGGTTGATGGGCTTGGGGACTTGCCCTGAGTAGCATAGCATCGG
CGTGTTTTGGGTTTGTTTACAGGTGTGGAAGATCAGATGAAGGAAGAACCAACAGGTGATTGAACCT
ATGTCGCAAGTCAGCAATGCAGTGATGGTCTCGGCGTC
SEQ ID NO : 32
   LENGTH : 1623
   TYPE : DNA
   ORGANISM : *M. phaseolina*
   FEATURE NAME/KEY: CDS
   LOCATION : (1) ...... (1623) atgaaagccatcagcatcgccgtgttcgccgcgtcagtggtacctcacactctggcagac 
 M  K  A  I  S  I  A  V  F  A  A  S  V  V  P  H  T  L  A  D
tatgtttggccctcgcaacatgacttgttggaggatatgttggccatccaacaaggctat
 Y  V  W  P  S  Q  H  D  L  L  E  D  M  L  A  I  Q  Q  G  Y
attcgcatgggcttcactgacttggttgttccttgcggtcacggcagcaacaggcccgga
 I  R  M  G  F  T  D  L  V  V  P  C  G  H  G  S  N  R  P  G
gtaaacaacgcggcacaatggattcggacgggtttccacgacttcgccacccacgactcg
 V  N  N  A  A  Q  W  I  R  T  G  F  H  D  F  A  T  H  D  S
gccgctgggaccggcggcctcgatgcctccctcctctacgaggtcgaacggcccgagaac
 A  A  G  T  G  G  L  D  A  S  L  L  Y  E  V  E  R  P  E  N
gagggctcagcctttaacgacaccttcgccgacatgcacgactttatcaatccccgatca
 E  G  S  A  F  N  D  T  F  A  D  M  H  D  F  I  N  P  R  S
agcgcctcggatctgatagcgctggccgtcgtcgcatcggttgcggcttgcggcggaccc
 S  A  S  D  L  I  A  L  A  V  V  A  S  V  A  A  C  G  G  P
aagatcccccctgcgagcgggacgaattgatgctgtggaggcgggcccggccggcgttccc
 K  I  P  L  R  A  G  R  I  D  A  V  E  A  G  P  A  G  V  P
aagcccgacgactcactggagagcacgatcgatgcattcgcgcggacggggttcaacacc
 K  P  D  D  S  L  E  S  T  I  D  A  F  A  R  T  G  F  N  T
agcgacatgatcgccctcgttgcctgtggccataccgtcggcggcgtgcacagcgtagat
 S  D  M  I  A  L  V  A  C  G  H  T  V  G  G  V  H  S  V  D
ttccccgagatcaccggcggcgagaaagacgtcctggacgtgccgcaatttgacagcagc
 F  P  E  I  T  G  G  E  K  D  V  L  D  V  P  Q  F  D  S  S
ggcaccatcttcgacaccgcagtcgtggacgaatacctcgacagcaacggcgccaacccc
 G  T  I  F  D  T  A  V  V  D  E  Y  L  D  S  N  G  A  N  P
ctcgtcttcggagccaacgacaccacgaactcggacaagcgcgtcttcagcgccgacggc
 L  V  F  G  A  N  D  T  T  N  S  D  K  R  V  F  S  A  D  G
aacagcaccatggccaagctcaaagaccccgccacattcaaagccacctgcgccgccctc
 N  S  T  M  A  K  L  K  D  P  A  T  F  K  A  T  C  A  A  L
ttcgagcgtatgatcaacaccgtcccctcatccgtcaccctcagcgaacccatcgagctg
 F  E  R  M  I  N  T  V  P  S  S  V  T  L  S  E  P  I  E  L
gccgacatcaagccctacatcgacaagctcgagctcacacccaacgcctccgccctcgcc
 A  D  I  K  P  Y  I  D  K  L  E  L  T  P  N  A  S  A  L  A
ttcgaaggccgcatccggctgcgcacctcccccgtcaccggccgcgacgccgagggcacc
 F  E  G  R  I  R  L  R  T  S  P  V  T  G  R  D  A  E  G  T
agcatcgccctcaatgtcaccgaccgcgctggcggcgcaagctggtgccggcgccgcgc
 S  I  A  L  N  V  T  D  R  A  G  G  R  K  L  V  P  A  P  R
gccgtgttgcgcggcggcacctcgtacggcttcttcgacgagcagttcagctggttcgaa
 A  V  L  R  G  G  T  S  Y  G  F  F  D  E  Q  F  S  W  F  E
tttgcgacgcagctcgacgtcgcagccggcatccaggcctttgatatccagctcacgacg
 F  A  T  Q  L  D  V  A  A  G  I  Q  A  F  D  I  Q  L  T  T
gaggcgaccggacacgtggagacgttcgacaacgctggcaccggcggctatcctagtctc
 E  A  T  G  H  V  E  T  F  D  N  A  G  T  G  G  Y  P  S  L
gacgacttgctatacttgcagtcgcaatcgtgcatggacacgactgccacggaggggaat
 D  D  L  L  Y  L  Q  S  Q  S  C  M  D  T  T  A  T  E  G  N
ataacggtgacggttgcggcggcggtgcgcgaggatgcggcgaaggcgggtgcggcgcca
 I  T  V  T  V  A  A  A  V  R  E  D  A  A  K  A  G  A  A  P
gtggtgaggatggcgcataaggttcagcagatgggtgtgatgttgccgaagcttgtcgta
 V  V  R  M  A  H  K  V  Q  Q  M  G  V  M  L  P  K  L  V  V
gaggcagtgccaatggagaggtcaaacgtttcgcagggcgggtatgtattgtacgaggtg
 E  A  V  P  M  E  R  S  N  V  S  Q  G  G  Y  V  L  Y  E  V
gacatcccaatcgatgccgctggctggagcacgaagttcgatgttgtgttgacggctggg
 D  I  P  I  D  A  A  G  W  S  T  K  F  D  V  V  L  T  A  G
ggagacgagattgtgtctggcctgcacggaccagcgatcttaccacatgttcgggaaac
 G  D  E  I  V  S  G  L  H  G  T  S  D  L  T  T  C  S  G  N
tga
 -
SEQ ID NO : 33
   LENGTH : 540
   TYPE : PRT
   ORGANISM : *M. phaseolina* 
MKAISIAVFAASVVPHTLADYVWPSQHDLLEDMLAIQQGYIRMGFTDLVVPCGHGSNRPGVNNAAQW
IRTGFHDFATHDSAAGTGGLDASLLYEVERPENEGSAFNDTFADMHDFINPRSSASDLIALAVVASV
AACGGPKIPLRAGRIDAVEAGPAGVPKPDDSLESTIDAFARTGFNTSDMIALVACGHTVGGVHSVDF
PEITGGEKDVLDVPQFDSSGTIFDTAVVDEYLDSNGANPLVFGANDTTNSDKRVFSADGNSTMAKLK
DPATFKATCAALFERMINTVPSSVTLSEPIELADIKPYIDKLELTPNASALAFEGRIRLRTSPVTGR
DAEGTSIALNVTDRAGGRKLVPAPRAVLRGGTSYGFFDEQFSWFEFATQLDVAAGIQAFDIQLTTEA
TGHVETFDNAGTGGYPSLDDLLYLQSQSCMDTTATEGNITVTVAAAVREDAAKAGAAPVVRMAHKVQ
QMGVMLPKLVVEAVPMERSNVSQGGYVLYEVDIPIDAAGWSTKFDVVLTAGGDEIVSGLHGTSDLTT
CSGN*
SEQ ID NO : 34
   LENGTH : 2012 (including 150 bp 5' UTR and 150 bp 3' UTR)
   TYPE : DNA
   ORGANISM : *M. phaseolina* 
GGGGGAGGCCAATGCATGTCAGCAAAATACCTGTGGCTACCCCTGTTATGGCCACATCCAGCCCATT
TTGAATGGACGGTATAAAATCTCTTCTGAAACCTTCAGAAAATTAAGGCAGGGACTATTGAAGGTTT
TGCCGTATCTGCCAAAATGCGGTTTCTCGGGGGCTTCTATCTACTCCTTGCTGCCACTCGCCACACA
CCTACGGCCAGAGCTGCGCTGCACTACCCAAATGCCCTCATTTCTCGCATGGAACACCTGCTGGTTG
ATACGGACGGCTCATTCAGGTCTGGTTTCAAGGACGCCATCAACCCCTGCACGAACTACATTTCAGG
TGCCCAAACGCTGGGGCGGCAGACCTCTGCACAGTGGCTCCGAGTTGCCTTCCATGATTTCGTCACG
GCCCACGTCGACGAGGGCACTGGCGGGATCGACGCGTCCATCGGCTTCGAAACTCTCCGAGCAGAAG
ATTCCGGGTCCGCATTCAATGACAGTTTCGCCTTCTTTGCTCCGTTCGTGGATGCGCAAACTTCCAG
TATCACCCATCGAGCCTTCCCCGTCGAGGGCCGTGGCATGCGCTGACGTCCTTGCAGTGGCCGACCT
CGTAGCCCTCTCTGTGGTGACTTCTCTGGGCCACTGCGGTGGTCTGCATGTCCCATATCGAGCGGGC
CGTATCGATGCTACGGGCGGAGGGCCGTTCGGCGTTCCCGAACCCGAGACAAGCCTGGAAGAGACCC
TGGAAGAGTTTGCCAATGCTGGTTTCAATGCTGAAGATGCCATTGGATTAACGGCGTGCGGGCATTC
TCTCGGCCGCGTCCATCACGGCGGGTTCCCCAACGTCGTGCCCGAATCGGCCATAGCACCAAACAAC
ACCGCGGGCGGCGTGAACCTGGACTCCACACGGGATAAATTCGACATCAGCATTGTCAAAGAATACC
TCGGCAACTATGGGCAGCGCGGCGGACCTCTCGTTACCAGTGACAACGTGACTGTCCGCTCGGATCT
TCGGCTGTACGAAAGCGATCAGAACAGGACAATGCAAGCTCTCGGTCAGTCAAAAGAATACTTCTTT
AGCACCTGTGGAAATCTATTTGAGAGGATGATCAACACCGTTCCGCGCGAGGTCACTTTGTCAGATG
TCATCCATCCGATGACGGTGCAGCCGGTGAATTTCACGTTCGATATCATCAATGACCAGGCGCTGAG
GTTATCAGGAGTAGTGCGGGTGAGCTTCCAAATAGAGCTGTTTCGGTCTGCACGCAACTGACCGAGC
CCCGCAGTATTTGCCCTCAGATAATGCTGCACCGTCGACGCTCGAGGTCTCACTCGCCGACAAAGCC
GGAAAGACTATGGCATCCATTACAGCAAGGGTTATTGAAGAGAAAGGAATAGTTTCTGGGGGGCCA
CAGCTTACTACCCGGTCGTTTTTGACATAAACCTTGCTGGGATTGCTTCTCGCAACAATCTTCCTGG
GAAGCTCCAGGTTCGCACAGCATCTCCTCAGACATTTGAGCTGCAACCGGAGCTGTTTTTCATACCA
TCCCGTTCTAGCCCTGGCACGGGGATAAGTGTGGGGGCTGCCATAGGTGCTGCTGCGCGGTCCCGCA
ACTCGACTCTGAGTGTAGAGAGTGTGGAAGCCGTGGTCAGTGTGCCGGTATCCCAGACGGGAACACT
GGCACCGAAGGTCGAGAAGCATGAGTTGAATCTCGAAAGAGATCAAGATATTGGTTTGTACTCTATA
TTCAAGGGGAACCTAAGCGATGATTTGGCTTTCAACCTGCAGACGACCGTTGATATCACGGCAACTT
TTTCAGACGGCACTGTCTGGCAAGATAGTTACAACAGATTTGCAGTGCCGTAGAGCATCCTGAAAAT
GTCTTCGTCAAAATGCCTTCATAGGGCCAGCAACAAATTTTCAGGCAGATATGTAGCTAGAGAGTCA
TTCAAATACAAATGTCTTGGTTGTCGAACAAGTTCGTGAGACGCTGCTCATGCACAATACAAGTGAG
CC
SEQ ID NO : 35
   LENGTH : 1599
   TYPE : DNA
   ORGANISM : *M. phaseolina*
   FEATURE NAME/KEY : CDS
   LOCATION : (1) ...... (1599) 
atgcggtttctcgggggcttctatctactccttgctgccactcgccacacacctacggcc
 M  R  F  L  G  G  F  Y  L  L  L  A  A  T  R  H  T  P  T  A
agagctgcgctgcactacccaaatgccctcatttctcgcatggaacacctgctggttgat
 R  A  A  L  H  Y  P  N  A  L  I  S  R  M  E  H  L  L  V  D
acggacggctcattcaggtctggtttcaaggacgccatcaacccctgcacgaactacatt
 T  D  G  S  F  R  S  G  F  K  D  A  I  N  P  C  T  N  Y  I
tcaggtgcccaaacgctggggcggcagacctctgcacagtggctccgagttgccttccat
 S  G  A  Q  T  L  G  R  Q  T  S  A  Q  W  L  R  V  A  F  H
gatttcgtcacggcccacgtcgacgagggcactggcggatcgacgcgtccatcggcttc
 D  F  V  T  A  H  V  D  E  G  T  G  G  I  D  A  S  I  G  F
gaaactctccgagcagaagattccgggtccgcattcaatgacagtttcgccttctttgct
 E  T  L  R  A  E  D  S  G  S  A  F  N  D  S  F  A  F  F  A
ccgttcgtggatgcgcaaacttccatggccgacctcgtagccctctctgtggtgacttct
 P  F  V  D  A  Q  T  S  M  A  D  L  V  A  L  S  V  V  T  S
ctgggccactgcggtggtctgcatgtcccatatcgagcgggccgtatcgatgctacgggc
 L  G  H  C  G  G  L  H  V  P  Y  R  A  G  R  I  D  A  T  G
ggagggccgttcggcgttcccgaacccgagacaagcctggaagagaccctggaagagttt
 G  G  P  F  G  V  P  E  P  E  T  S  L  E  E  T  L  E  E  F
gccaatgctggtttcaatgctgaagatgccattggattaacggcgtgcggcattctctc
 A  N  A  G  F  N  A  E  D  A  I  G  L  T  A  C  G  H  S  L
ggccgcgtccatcacggcgggttccccaacgtcgtgcccgaatcggccatagcaccaaac
 G  R  V  H  H  G  G  F  P  N  V  V  P  E  S  A  I  A  P  N
aacaccgcgggcggcgtgaacctggactccacacgggataaattcgacatcagcattgtc
 N  T  A  G  G  V  N  L  D  S  T  R  D  K  F  D  I  S  I  V
aaagaatacctcggcaactatggcagcgcggcggacctctcgttaccagtgacaacgtg
 K  E  Y  L  G  N  Y  G  Q  R  G  G  P  L  V  T  S  D  N  V
actgtccgctcggatcttcggctgtacgaaagcgatcagaacaggacaatgcaagctctc
 T  V  R  S  D  L  R  L  Y  E  S  D  Q  N  R  T  M  Q  A  L
ggtcagtcaaaagaatacttctttagcacctgtggaaatctatttgagaggatgatcaac
 G  Q  S  K  E  Y  F  F  S  T  C  G  N  L  F  E  R  M  I  N
accgttccgcgcgaggtcactttgtcagatgtcatccatccgatgacggtgcagccggtg
 T  V  P  R  E  V  T  L  S  D  V  I  H  P  M  T  V  Q  P  V
aatttcacgttcgatatcatcaatgaccaggcgctgaggttatcaggagtagtgcggtat
 N  F  T  F  D  I  I  N  D  Q  A  L  R  L  S  G  V  V  R  Y
ttgccctcagataatgctgcaccgtcgacgctcgaggtctcactcgccgacaaagccgga
 L  P  S  D  N  A  A  P  S  T  L  E  V  S  L  A  D  K  A  G
aagactatggcatccattacagcaagggttattgaagagaaaggaatagtttctgggg
 K  T  M  A  S  I  T  A  R  V  I  E  E  K  G  N  S  F  W  G
gccacagcttactacccggtcgttttgacataaaccttgctgggattgcttctcgcaac
 A  T  A  Y  Y  P  V  V  F  D  I  N  L  A  G  I  A  S  R  N
aatcttcctggaagctccaggttcgcacagcatctcctcagacatttgagctgcaaccg
 N  L  P  G  K  L  Q  V  R  T  A  S  P  Q  T  F  E  L  Q  P
gagctgtttttcataccatcccgttctagccctggcacggggataagtgtgggggctgcc
 E  L  F  F  I  P  S  R  S  S  P  G  T  G  I  S  V  G  A  A
ataggtgctgctgcgcggtcccgcaactcgactctgagtgtagagagtgtggaagccgtg
 I  G  A  A  A  R  S  R  N  S  T  L  S  V  E  S  V  E  A  V
gtcagtgtgccggtatcccagacgggaacactggcaccgaaggtcgagaagcatgagttg
 V  S  V  P  V  S  Q  T  G  T  L  A  P  K  V  E  K  H  E  L
aatctcgaaagagatcaagatattggtttgtactctatattcaaggggaacctaagcgat
 N  L  E  R  D  Q  D  I  G  L  Y  S  I  F  K  G  N  L  S  D
gatttggctttcaacctgcagacgaccgttgatatcacggcaacttttcagacggcact
 D  L  A  F  N  L  Q  T  T  V  D  I  T  A  T  F  S  D  G  T
gtctggcaagatagttacaacagatttgcagtgccgtag
 V W Q D S Y N R F A V P -
SEQ ID NO : 36
   LENGTH : 532
   TYPE : PRT
   ORGANISM : *M. phaseolina* 
MRFLGGFYLLLAATRHTPTARAALHYPNALISRMEHLLVDTDGSFRSGFKDAINPCTNYISGAQTLG
RQTSAQWLRVAFHDFVTAHVDEGTGGIDASIGFETLRAEDSGSAFNDSFAFFAPFVDAQTSMADLVA
LSVVTSLGHCGGLHVPYRAGRIDATGGGPFGVPEPETSLEETLEEFANAGFNAEDAIGLTACGHSLG
RVHHGGFPNVVPESAIAPNNTAGGVNLDSTRDKFDISIVKEYLGNYGQRGGPLVTSDNVTVRSDLRL
YESDQNRTMQALGQSKEYFFSTCGNLFERMINTVPREVTLSDVIHPMTVQPVNFTFDIINDQALRLS
GVVRYLPSDNAAPSTLEVSLADKAGKTMASITARVIEEKGNSFWGATAYYPVVFDINLAGIASRNNL
PGKLQVRTASPQTFELQPELFFIPSRSSPGTGISVGAAIGAAARSRNSTLSVESVEAVVSVPVSQTG
TLAPKVEKHELNLERDQDIGLYSIFKGNLSDDLAFNLQTTVDITATFSDGTVWQDSYNRFAVP*
SEQ ID NO : 37
   LENGTH : 2401 (including 150 bp 5' UTR and 150 bp 3' UTR)
   TYPE : DNA
   ORGANISM : *M. phaseolina* 
TTTTAATATATTCTGCCGCTAAAACGTTCGCCATGTGCTCCTGATTTTCTCGACCGCCATTTCTCG
CCTCCGCATCGGCTCCCTGCTTCGGCGCTCGTCGCTAGCATCCAGTTTCGCCAGTCCTTCCGTTT
CTCCCGCTCTTGAACCATGGCAAGGCATCCCGGCCTGACCCTGGTGTGGGTCGCAGGCGTGGTCTAC
CACGCCATTGGCACCGCTGCTGCCCCGACATGGCCATCTTCCATGGATGAACTGGAGGACATCATGG
TCCTCAACACCGGCTACCGCGCCCGCAGCTTCGCAGTGCCCGTCACACCCTGCGGCTTCTCGTCGCA
AGGCCCCGGACGCGTCCAAGCTGCCGAATGGATACGAACCGCCTTCCACGACATGGCTCCCGGCAGC
GTGTATACCGGCGTCGGAGGACTGGACGCATCGATAGCTTACGAAACACGGAGCTTGGAAAATCTCG
GCCCCGCCTTCAACACCACACTAGCTACCTACGCGCCGTATCTGACAAGCAGATCTTCCATGGCTGA
CATCATTGCGCTAGGAGTCTACACGGCGGTGCGGTCATGCGGAGGTCCAATCGTGCCTATCCGGACA
GGCAGAGTAGACGCAAAGGCAGCAGGCCCGCAAGGTGTGCCTCTGCCGCAGAATTCTATCGGAACTT
TTCAAAACCAGTTTCTCCGTACTGGCTTCAACACGACGGAGATGATCCAAGTGGTGGCGTGTGGCCA
CACTCTGGGCGGCGTGCACGCATCTGCCAACCCGGAGATCGTGCCCGTGGGGTCGGCGGAGGACGGC
GTCGTCAAGTTCGACACGACGGACGCGTTTGACAACAAGGTTGTCACCGAGTACCTCTCGAATACAA
CCAAGAACTCGCTTGTTGTGGGCCCCTCTACTGCGAACGGCCGGAACTCGGACGCTCGTGTCTTTGC
GGCGGATGGAAATGCTACGGTCAGGGCTCTGGCTGACCCTGATACGTTCAACAGTGTTTGCGCTAGG
ATGCTGCAGAAGATGATTGATGTTGTTCCAACGGGCGTGGTGCTCACTGACCCGATTTCAATCTATG
ATGTCAAACCCAGTGGGCTGCAGCTGACATTGCTTGGTGGAGGAGAGTCGGTAAAGCTTACGGAGA
TATCCGTGTTAGGACTACGGAGCGCTCTGCGAGCCAGATTGAGAAGGTCGAGCTTGTCTACAAGGAC
CGCGAGGGGGCCGAATCCTCGACAGCTTTGAGCACCGAATCCTCAGGTTCGGCCTCTGGGTTCGATG
ACAGCTTCGAGGTATAACTCATCCTAAGCCCCTGTTGGGATGCCACACTAACGGCCGAGAAAGTTCT
ACGGGGTCTCCGCAAACATCCCCACCGACTCCGGCATCTCCTCATTCAACGTCCTCATCACCCTCAC
CAGCGGCGAAACCGAACTTCACGACAATAATGGCAGCGGTTTCCCCCTCCAAGACACCGTCATCTTT
CAAAGCCCGCAGAGCTGCCTCAGCGGCACCACCATGACCGTCGCCGCCGCCGTCCTCAATACCGCCT
CCTCCGCCCCCACCCTCAGCGTCACCCTCAAGGTCCCCAACTCCCGCTCCGTCCTCCCCGTCCTCCA
GGTCAGCACAGTCGCCATGACGAAGAGCTCGTCTGTCGGCCCGTACGACCTCTACTCAACCACATAC
ACGCTAACCTCCGCCCAACTCGCCGACACGCGCTTTGACGTCAAGCTCGGCGCTGCCGCCGCCGACG
CCTTCAAGTCCTCCGCCGACCTCGGTGATGCCTGCCAGGACCTGAGCCCCGAACCGCCCACTTCTTC
CAGCGCGCCTAGCTCCTCGAGCACGGCTGCGCCGCCGGCCTCGTCCTCCTCTGCCGCACCAAGCTCC
TCTGACCTCCCGGCCCCCTCCCCCAGCACAACACCCACGTCCTCCCACCCAATCTCCTCATCTACAA
CATTAGCATCATCGACCACCCCAACGCCCACAACACTCGCCTGCCCCGCCGCTGACGGCGCCACCTG
GACGCTATCCGGCGGCCAGAAGTTCGCCGTCAAGTGCGGCAAGGACTACCAGGCCGGCCAGATCGGC
GTCACGTGGACGGCCAGCTTCGAGGCCTGTCTGCAGGCGTGCGTGGATACGGACACGTGCCAGGCGG
TCGCATTCGTGGGGAGTGCAGAGGCGGGCGGGCAGTGCTACTTGAAGGACCAGAGCGCGGGCAGTGT
GGATGTTGAGGGGGTGTGGGGGGCGGTTCTGGAAAGCTGAGCGGTCGGGCGATGGAGTAGGGGATAG
 
AGGGTTTGGCTGGGGGGTAATGAATTACTTCTGATGAGCTTCTACTACTGCAGGTGGCACAAATCA
TGGCCTCTCATGGACCTCCGACGCCGCATGCTGCCCGCGTTTCAAGGTATTCTTGG
SEQ ID NO : 38
   LENGTH : 2049
   TYPE : DNA
   ORGANISM : *M. phaseolina*
   FEATURE NAME/KEY: CDS
   LOCATION : (1) ...... (2049) 
atggcaaggcatcccggcctgaccctggtgtgggtcgcaggcgtggtctaccacgccatt
 M  A  R  H  P  G  L  T  L  V  W  V  A  G  V  V  Y  H  A  I
ggcaccgctgctgccccgacatggccatcttccatggatgaactggaggacatcatggtc
 G  T  A  A  A  P  T  W  P  S  S  M  D  E  L  E  D  I  M  V
ctcaacaccggctaccgcgcccgcagcttcgcagtgcccgtcacaccctgcggcttctcg
 L  N  T  G  Y  R  A  R  S  F  A  V  P  V  T  P  C  G  F  S
tcgcaaggccccggacgcgtccaagctgccgaatggatacgaaccgccttccacgacatg
 S  Q  G  P  G  R  V  Q  A  A  E  W  I  R  T  A  F  H  D  M
gctcccggcagcgtgtataccggcgtcggaggactggacgcatcgatagcttacgaaaca
 A  P  G  S  V  Y  T  G  V  G  G  L  D  A  S  I  A  Y  E  T
cggagcttggaaaatctcggccccgccttcaacaccacactagctacctacgcgccgtat
 R  S  L  E  N  L  G  P  A  F  N  T  T  L  A  T  Y  A  P  Y
ctgacaagcagatcttccatggctgacatcattgcgctaggagtctacacggcggtgcgg
 L  T  S  R  S  S  M  A  D  I  I  A  L  G  V  Y  T  A  V  R
tcatgcggaggtccaatcgtgcctatccggacaggcagagtagacgcaaaggcagcaggc
 S  C  G  G  P  I  V  P  I  R  T  G  R  V  D  A  K  A  A  G
ccgcaaggtgtgcctctgccgcagaattctatcggaacttttcaaaaccagtttctccgt
 P  Q  G  V  P  L  P  Q  N  S  I  G  T  F  Q  N  Q  F  L  R
actggcttcaacacgacggagatgatccaagtggtggcgtgtggccacactctgggcggc
 T  G  F  N  T  T  E  M  I  Q  V  V  A  C  G  H  T  L  G  G
gtgcacgcatctgccaacccggagatcgtgcccgtggggtcggcggaggacggcgtcgtc
 V  H  A  S  A  N  P  E  I  V  P  V  G  S  A  E  D  G  V  V
aagttcgacacgacggacgcgtttgacaacaaggttgtcaccgagtacctctcgaataca
 K  F  D  T  T  D  A  F  D  N  K  V  V  T  E  Y  L  S  N  T
accaagaactcgcttgttgtgggcccctctactgcgaacggccggaactcggacgctcgt
 T  K  N  S  L  V  V  G  P  S  T  A  N  G  R  N  S  D  A  R
gtctttgcggcggatggaaatgctacggtcaggctctggctgaccctgatacgttcaac
 V  F  A  A  D  G  N  A  T  V  R  A  L  A  D  P  D  T  F  N
agtgtttgcgctaggatgctgcagaagatgattgatgttgttccaacgggcgtggtgctc
 S  V  C  A  R  M  L  Q  K  M  I  D  V  V  P  T  G  V  V  L
actgacccgatttcaatctatgatgtcaaacccagtgggctgcagctgacattgcttggt
 T  D  P  I  S  I  Y  D  V  K  P  S  G  L  Q  L  T  L  L  G
ggaggagagtcggtaaagcttacgggagatatccgtgttaggactacggagcgctctgcg
 G  G  E  S  V  K  L  T  G  D  I  R  V  R  T  T  E  R  S  A
agccagattgagaaggtcgagcttgtctacaaggaccgcgagggggccgaatcctcgaca
 S  Q  I  E  K  V  E  L  V  Y  K  D  R  E  G  A  E  S  S  T
gctttgagcaccgaatcctcaggttcggcctctgggttcgatgacagcttcgagttctac
 A  L  S  T  E  S  S  G  S  A  S  G  F  D  D  S  F  E  F  Y
ggggtctccgcaaacatccccaccgactccggcatctcctcattcaacgtcctcatcacc
 G  V  S  A  N  I  P  T  D  S  G  I  S  S  F  N  V  L  I  T
ctcaccagcggcgaaaccgaacttcacgacaataatggcagcggtttccccctccaagac
 L  T  S  G  E  T  E  L  H  D  N  N  G  S  G  F  P  L  Q  D
accgtcatctttcaaagcccgcagagctgcctcagcggcaccaccatgaccgtcgccgcc
 T  V  I  F  Q  S  P  Q  S  C  L  S  G  T  T  M  T  V  A  A
gccgtcctcaataccgcctcctccgcccccaccctcagcgtcaccctcaaggtccccaac
 A  V  L  N  T  A  S  S  A  P  T  L  S  V  T  L  K  V  P  N
 
tcccgctccgtcctccccgtcctccaggtcagcacagtcgccatgacgaagagctcgtct
 S  R  S  V  L  P  V  L  Q  V  S  T  V  A  M  T  K  S  S  S
gtcggcccgtacgacctctactcaaccacatacacgctaacctccgcccaactcgccgac
 V  G  P  Y  D  L  Y  S  T  T  Y  T  L  T  S  A  Q  L  A  D
acgcgctttgacgtcaagctcggcgctgccgccgccgacgccttcaagtcctccgccgac
 T  R  F  D  V  K  L  G  A  A  A  A  D  A  F  K  S  S  A  D
ctcggtgatgcctgccaggacctgagccccgaaccgcccacttcttccagcgcgcctagc
 L  G  D  A  C  Q  D  L  S  P  E  P  P  T  S  S  S  A  P  S
tcctcgagcacggctgcgccgccggcctcgtcctcctctgccgcaccaagctcctctgac
 S  S  S  T  A  A  P  P  A  S  S  S  S  A  A  P  S  S  S  D
ctcccggcccccctcccccagcacaacacccacgtcctcccacccaatctcctcatctaca
 L  P  A  P  S  P  S  T  T  P  T  S  S  H  P  I  S  S  S  T
acattagcatcatcgaccaccccaacgcccacaacactcgcctgccccgccgctgacggc
 T  L  A  S  S  T  T  P  T  P  T  T  L  A  C  P  A  A  D  G
gccacctggacgctatccggcggccagaagttcgccgtcaagtgcggcaaggactaccag
 A  T  W  T  L  S  G  G  Q  K  F  A  V  K  C  G  K  D  Y  Q
gccggccagatcggcgtcacgtggacggccagcttcgaggcctgtctgcaggcgtgcgtg
 A  G  Q  I  G  V  T  W  T  A  S  F  E  A  C  L  Q  A  C  V
gatacggacacgtgccaggcggtcgcattcgtgggggagtgcagaggcgggcggcagtgc
 D  T  D  T  C  Q  A  V  A  F  V  G  S  A  E  A  G  G  Q  C
tacttgaaggaccagagcgcgggcagtgtggatgttgagggggtgtgggggcggttctg
 Y  L  K  D  Q  S  A  G  S  V  D  V  E  G  V  W  G  A  V  L
gaaagctga
 E  S  -
SEQ ID NO : 39
   LENGTH : 682
   TYPE : PRT
   ORGANISM : *M. phaseolina* 
MARHPGLTLVWVAGVVYHAIGTAAAPTWPSSMDELEDIMVLNTGYRARSFAVPVTPCGFSSQGPGRV
QAAEWIRTAFHDMAPGSVYTGVGGLDASIAYETRSLENLGPAFNTTLATYAPYLTSRSSMADIIALG
VYTAVRSCGGPIVPIRTGRVDAKAAGPQGVPLPQNSIGTFQNQFLRTGFNTTEMIQVVACGHTLGGV
HASANPEIVPVGSAEDGVVKFDTTDAFDNKVVTEYLSNTTKNSLVVGPSTANGRNSDARVFAADGNA
TVRALADPDTFNSVCARMLQKMIDVVPTGVVLTDPISIYDVKPSGLQLTLLGGGESVKLTGDIRVRT
TERSASQIEKVELVYKDREGAESSTALSTESSGSASGFDDSFEFYGVSANIPTDSGISSFNVLITLT
SGETELHDNNGSGFPLQDTVIFQSPQSCLSGTTMTVAAAVLNTASSAPTLSVTLKVPNSRSVLPVLQ
VSTVAMTKSSSVGPYDLYSTTYTLTSAQLADTRFDVKLGAAAADAFKSSADLGDACQDLSPEPPTSS
SAPSSSSTAAPPASSSSAAPSSSDLPAPSPSTTPTSSHPISSSTTLASSTTPTPTTLACPAADGATW
TLSGGQKFAVKCGKDYQAGQIGVTWTASFEACLQACVDTDTCQAVAFVGSAEAGGQCYLKDQSAGSV
DVEGVWGAVLES*
SEQ ID NO : 40
   LENGTH : 2004 (including 150 bp 5' UTR and 150 bp 3' UTR)
   TYPE : DNA
   ORGANISM : *M. phaseolina* 
CCTTCCTGAAATTTCATGGCCAGCTTCTCGGTCTTTCTAATGAATTATAAGGCTATCTTCCTGCAGC
TTGTCGTCTGCACGTTCTAGGATTCAACCACCCGTCATTCCTTCTCAAAATCTCAGTCCAGTCGTTA
CAGCTAATCACTTAATATGAAGCCCGCCGCTCTTGCAGGCATTGGCCTGCTTAATGTCCTACCCATT
ACCGCCGAATACGTCTGGCCTTCTAAATACGACTATTTGGAAGACCTGCTTTACCTTCAATCCGGTT
ATCTGCGTGAGGGATTTGTCGACGGTATGCATATATGAACAACTGCTGGTGTCCCCCAACTAATGGC
TTTTAGGTGTGGCTCCTTGTTCGTTTTCCTCCGCTGGACCTGGCCGTCAAACCGCAGCGGAATGGGT
CCGCACTGCATACCATGATATGGCCACTCATGATGCCGATGCTGGCACTGGTGGCTTGGACGCCTCT
ATCATGTTTGAGACCGAGCGGACGAAAACGTGGGCGATGCGTTCAATGGTACCTTCGGCTTTACAA
ACAACTACTACAACATCAAAGCATCCGCTGCTGATCTTCTTGCACTCTCCACTGTCATCGCCGTTGG
AAACTGCGGTGGCCCGAAGATTCCTTTCCGTGTCGGTCGCGTGGACGCCACGGAGGCTGGCCCTCTG
 
GGTGTTCCCAAGCCGGATCAAGACGTTGATACGCACATTCAAATTTTTGCCAAGGCAGGCTTTAACA
CCAGTAGGCCTTGAATTACCTGAGGTCCACCTACCAGCCGCTGACTAGAATAGGCGACATGATCACC
ATGGTGGCCTGCGGCCACACCCTTGGTGGCGTCCATGGCAAGGACTTCCCCGAGATCACTTTCAACG
ACACGGAGACCAATTTCGTCAAGTTCGAAGGCAACAACTCCTTCTCCAACTTTGATAACACCGTCGT
GACCGAGTATCTTGGCGGAAACCCCCCCAACCCCCTCGTCACCGGCAAGAACGAGACCAACAACAGC
GATAAGCGCGTCTTTGGTGCCGACAACAACGCCACAATGCACTCCCTGTCGGACCCCTCCGTCTTCC
AGTCCTCCTGCCAAGACATCCTCGCCCGCATGATCGACACCGTCCCCTCCAACGTCGCTCTCACCGA
GCCTCTCGACCCAATCCTCATCAAGCCCTACATCCAAACCTTCTCCCTCGTCAACGCCACCCACCTC
ACCCTGACCGGCCGCATCCGCGTCCGTACCGACTGGGACAGCTACACCGACCAGTCAGTCCACCTCA
CCTACAACCCGCGCACAGCGCCCGCCCAGAATGCGACCCTCAACACCACCATCCCCACCACCCGCGC
CACCTTCCAGGGCGGCACCTCCAGCGGCATCTTTGGTGAAGTCTTCGCCTGGCACGAGTTCTCCGCC
ACCCTCCCCACATCCAGCTCGATTACCGGCTTCACTGTCACCGTCACGCGCGGCTCCACGGGCGAAT
CCACCACCTACGACAACGCCGGCAGCACGAACGGCTACGCGCTCGACGACACGCTGCTCTACCAGAG
CGCGCAGTCGTGCCGCGACGTCGGCACCACAACCATCACCGCTGCTGTGCGCAAGGACTTCCTCGCC
CGAGGTGCAAAGGTCGCCGTCGAGATGGTGAACAGGGTGCCGCGCCAGGGCGTGTACGTGCCGGCGC
TAGAGGTCGAACCGTGGGGGGCGGAGACGGTCAAGGAGGTCGGCGAGTGGGTCATCGTGCAGGCTAA
GGGTGAGTTGACGATGGAGAGCTTGAGCACAACGTTTGACGTCGTTGCGGGCGAGAAAAGGGTCGAG
TTCCAAAGGACGAATGTGCTGGGGGAGGAGTGTGCGGCTCTGTGAGGCGTAACTTAAAAAAAAAAAA
AAAGGAAGGATATATCAGTTTCTGGTACATACTTCGAATGAAAGTCTATGATTCGTATGTCCATAAC
ATTACTCTAGGCTTGAAACAACAATGCTGAATGTGCTTTATTGTGAGATAAAGAGTGTCCT
SEQ ID NO : 41
   LENGTH : 1605
   TYPE : DNA
   ORGANISM : *M. phaseolina*
   FEATURE NAME/KEY: CDS
   LOCATION : (1) ...... (1605) 
atgaagcccgccgctcttgcaggcattggcctgcttaatgtcctacccattaccgccgaa
 M  K  P  A  A  L  A  G  I  G  L  L  N  V  L  P  I  T  A  E
tacgtctggccttctaaatacgactatttggaagacctgctttaccttcaatccggttat
 Y  V  W  P  S  K  Y  D  Y  L  E  D  L  L  Y  L  Q  S  G  Y
ctgcgtgagggatttgtcgacggtgtggctccttgttcgttttcctccgctggacctggc
 L  R  E  G  F  V  D  G  V  A  P  C  S  F  S  S  A  G  P  G
cgtcaaaccgcagcggaatgggtccgcactgcataccatgatatggccactcatgatgcc
 R  Q  T  A  A  E  W  V  R  T  A  Y  H  D  M  A  T  H  D  A
gatgctggcactggtggcttggacgcctctatcatgtttgagaccgagcgggacgaaaac
 D  A  G  T  G  G  L  D  A  S  I  M  F  E  T  E  R  D  E  N
gtgggcgatgcgttcaatggtaccttcggctttacaaacaactactacaacatcaaagca
 V  G  D  A  F  N  G  T  F  G  F  T  N  N  Y  Y  N  I  K  A
tccgctgctgatcttcttgcactctccactgtcatcgccgttggaaactgcggtggcccg
 S  A  A  D  L  L  A  L  S  T  V  I  A  V  G  N  C  G  G  P
aagattcctttccgtgtcggtcgcgtggacgccacggaggctggccctctgggtgttccc
 K  I  P  F  R  V  G  R  V  D  A  T  E  A  G  P  L  G  V  P
aagccggatcaagacgttgatacgcacattcaaattttgccaaggcaggctttaacacc
 K  P  D  Q  D  V  D  T  H  I  Q  I  F  A  K  A  G  F  N  T
agcgacatgatcaccatggtggcctgcggccacacccttggtggcgtccatggcaaggac
 S  D  M  I  T  M  V  A  C  G  H  T  L  G  G  V  H  G  K  D
ttccccgagatcactttcaacgacacggagaccaatttcgtcaagttcgaaggcaacaac
 F  P  E  I  T  F  N  D  T  E  T  N  F  V  K  F  E  G  N  N
tccttctccaactttgataacaccgtcgtgaccgagtatcttggcggaaacccccccaac
 S  F  S  N  F  D  N  T  V  V  T  E  Y  L  G  G  N  P  P  N
cccctcgtcaccggcaagaacgagaccaacaacagcgataagcgcgtctttggtgccgac
 P  L  V  T  G  K  N  E  T  N  N  S  D  K  R  V  F  G  A  D
aacaacgccacaatgcactccctgtcggacccctccgtcttccagtcctcctgccaagac
 N  N  A  T  M  H  S  L  S  D  P  S  V  F  Q  S  S  C  Q  D
 
atcctcgcccgcatgatcgacaccgtcccctccaacgtcgctctcaccgagcctctcgac
 I  L  A  R  M  I  D  T  V  P  S  N  V  A  L  T  E  P  L  D
ccaatcctcatcaagccctacatccaaaccttctccctcgtcaacgccacccacctcacc
 P  I  L  I  K  P  Y  I  Q  T  F  S  L  V  N  A  T  H  L  T
ctgaccggccgcatccgcgtccgtaccgactgggacagctacaccgaccagtcagtccac
 L  T  G  R  I  R  V  R  T  D  W  D  S  Y  T  D  Q  S  V  H
ctcacctacaacccgcgcacagcgcccgcccagaatgcgaccctcaacaccaccatcccc
 L  T  Y  N  P  R  T  A  P  A  Q  N  A  T  L  N  T  T  I  P
accacccgcgccaccttccagggcggcacctccagcggcatctttggtgaagtcttcgcc
 T  T  R  A  T  F  Q  G  G  T  S  S  G  I  F  G  E  V  F  A
tggcacgagttctccgccaccctccccacatccagctcgattaccggcttcactgtcacc
 W  H  E  F  S  A  T  L  P  T  S  S  S  I  T  G  F  T  V  T
gtcacgcgcggctccacgggcgaatccaccacctacgacaacgccggcagcacgaacggc
 V  T  R  G  S  T  G  E  S  T  T  Y  D  N  A  G  S  T  N  G
tacgcgctcgacgacacgctgctctaccagagcgcgcagtcgtgccgcgacgtcggcacc
 Y  A  L  D  D  T  L  L  Y  Q  S  A  Q  S  C  R  D  V  G  T
acaaccatcaccgctgctgtgcgcaaggacttcctcgcccgaggtgcaaaggtcgccgtc
 T  T  I  T  A  A  V  R  K  D  F  L  A  R  G  A  K  V  A  V
gagatggtgaacagggtgccgcgccagggcgtgtacgtgccggcgctagaggtcgaaccg
 E  M  V  N  R  V  P  R  Q  G  V  Y  V  P  A  L  E  V  E  P
tgggggcggagacggtcaaggaggtcggcgagtgggtcatcgtgcaggctaagggtgag
 W  G  A  E  T  V  K  E  V  G  E  W  V  I  V  Q  A  K  G  E
ttgacgatggagagcttgagcacaacgtttgacgtcgttgcgggcgagaaaaggtcgag
 L  T  M  E  S  L  S  T  T  F  D  V  V  A  G  E  K  R  V  E
ttccaaaggacgaatgtgctgggggaggagtgtgcggctctgtga
 F  Q  R  T  N  V  L  G  E  E  C  A  A  L  -
SEQ ID NO : 42
   LENGTH : 534
   TYPE : PRT
   ORGANISM : *M. phaseolina* 
MKPAALAGIGLLNVLPITAEYVWPSKYDYLEDLLYLQSGYLREGFVDGVAPCSFSSAGPGRQTAAEWVRTAYH
DMATHDADAGTGGLDASIMFETERDENVGDAFNGTFGFTNNYYNIKASAADLLALSTVIAVGNCGGPKIPFRV
GRVDATEAGPLGVPKPDQDVDTHIQIFAKAGFNTSDMITMVACGHTLGGVHGKDFPEITFNDTETNFVKFEGN
NSFSNFDNTVVTEYLGGNPPNPLVTGKNETNNSDKRVFGADNNATMHSLSDPSVFQSSCQDILARMIDTVPSN
VALTEPLDPILIKPYIQTFSLVNATHLTLTGRIRVRTDWDSYTDQSVHLTYNPRTAPAQNATLNTTIPTTRAT
FQGGTSSGIFGEVFAWHEFSATLPTSSSITGFTVTVTRGSTGESTTYDNAGSTNGYALDDTLLYQSAQSCRDV
GTTTITAAVRKDFLARGAKVAVEMVNRVPRQGVYVPALEVEPWGAETVKEVGEWVIVQAKGELTMESLSTTFD
VVAGEKRVEFQRTNVLGEECAAL*
SEQ ID NO : 43
   LENGTH : 1414 (including 150 bp 5' UTR and 150 bp 3' UTR)
   TYPE : DNA
   ORGANISM : *M. phaseolina* 
AGCTTGCCTCTTCTTCTGTCTTTCTGCCCCCTTCTTCTCCAGACTCCTTTCGCCGTAGATTTCTGCC
TTTTGGACCTCGTTTTCTGCCCTCCACCAATAGCAAGCCAAGAGATTAGATTAGACATCGCCATCCC
ATCGCCGACTCCAACAATGCCCAGCAACCCAGGCGACTACGACGCCGTCCGCCACGACGTCAAGAAC
CTCCTGCACCAGCCCGAGTACGACGACGGTTCCGCCGGGCCCGTCCTCGTCCGTCTCGCATGGTATG
TTACGGCTACCCGCATCCTCCAGCCCAATCCGCCGTTTATCTGCTGTTCGTGGCTCTGCCATCTTCT
CTCCAGGGGCAGCAAGTTTCCCGAGCCTTTTTTTTTGTCTTACGAACGGCATCTCGAACAGCCCGC
TGACAACCACACCAGGCATTCCGCAGGGACCTACGACGCCCACTCTGACACAGGAGGCAGCAACGGT
GCAGGCATGCGCTACGAGGCTGAAGGCGGCGACCCCGCCAATGCCGGCCTGCAGCACGCCCGCGTCT
TCCTCGAGCCCATCAAGGCCGCGCACCCCTGGATAACCTACTCTGATCTGTGGACGCTGGCGGGCGT
GGTCGCCATCAAGGAGATGGGCGGCCCGGACATCCCGTGGCAGCCCGGCCGCACCGACTTCGTCGAC
GACAGCAAGCTGCCGCCGCGGGGCCGCCTGCCGGACGCCGCGCAGGGCGCTGACCACATCCGCTGGA
 
TTTTCTACCGCATGGGCTTCAACGATCAGGAAATTGTCGCCCTCAGCGGCGCCCACAACCTCGGCCG
CTGCCACGCCGACCGCTCCGGCTTCGACGGCGCCTGGGTCAACAACCCCACCCGCTTCTCCAACCAG
TACTTTAAGCTCCTGACCTCGGTCGAGTGGAAAGAGAAGACCCTCCCCAGCGGCATCAAGCAGTTCG
CCTACTATGATGAGGACTCGGAGGAGGAGCTCATGATGCTGCCCACCGATATCGCTCTCTTGCACGA
CCCCTCCTTCCGGCCGTGGGTCGAGAAGTATGCCGAGGACAAGGATGCCTTTTTCGCAGACTTCTCA
AAGGTCTTTGCCAAGCTGATTGAGCTGGGCATAGTCAGAGATGAGAGCGGTGCGGTAATCAACACTG
ATAACGTCAAGGGCGGCTACATCTCTGCGCCCAAGAAGAGTGAGCTGCCTGGTGCTCCGGGTAAGGC
TAATGAGGAGGCTGAGCCGCTCATGAAAGAGAATGAGAGGTTCAGGGCACGTCTGTAAGCTGGTTGG
TTTAGATTTCCTTTTTTTTTTTTTTTCAAGACCGTTAGACTGCGCAAGCTAGAGGGGACGGATAGAT
GGACGATACCACCTAGATTCCTATGCCTTAAGAGGAGAAAGATAGTGCCTAGAGTTTTCATAGAGAA
TGCAAAC
SEQ ID NO : 44
   LENGTH : 960
   TYPE : DNA
   ORGANISM : *M. phaseolina*
   FEATURE NAME/KEY: CDS
   LOCATION : (1) ...... (960) 
atgcccagcaacccaggcgactacgacgccgtccgccacgacgtcaagaacctcctgcac
 M  P  S  N  P  G  D  Y  D  A  V  R  H  D  V  K  N  L  L  H
cagcccgagtacgacgacggttccgccggcccgtcctcgtccgtctcgcatggcattcc
 Q  P  E  Y  D  D  G  S  A  G  P  V  L  V  R  L  A  W  H  S
gcagggacctacgacgcccactctgacacaggaggcagcaacggtgcaggcatgcgctac
 A  G  T  Y  D  A  H  S  D  T  G  G  S  N  G  A  G  M  R  Y
gaggctgaaggcggcgaccccgccaatgccggcctgcagcacgcccgcgtcttcctcgag
 E  A  E  G  G  D  P  A  N  A  G  L  Q  H  A  R  V  F  L  E
cccatcaaggccgcgcacccctggataacctactctgatctgtggacgctggcgggcgtg
 P  I  K  A  A  H  P  W  I  T  Y  S  D  L  W  T  L  A  G  V
gtcgccatcaaggagatgggcggcccggacatcccgtggcagcccggccgcaccgacttc
 V  A  I  K  E  M  G  G  P  D  I  P  W  Q  P  G  R  T  D  F
gtcgacgacagcaagctgccgccgcggggccgcctgccggacgccgcgcagggcgctgac
 V  D  D  S  K  L  P  P  R  G  R  L  P  D  A  A  Q  G  A  D
cacatccgctggatttctaccgcatgggcttcaacgatcaggaaattgtcgccctcagc
 H  I  R  W  I  F  Y  R  M  G  F  N  D  Q  E  I  V  A  L  S
ggcgcccacaacctcggccgctgccacgccgaccgctccggcttcgacggcgcctgggtc
 G  A  H  N  L  G  R  C  H  A  D  R  S  G  F  D  G  A  W  V
aacaaccccacccgcttctccaaccagtactttaagctcctgacctcggtcgagtggaaa
 N  N  P  T  R  F  S  N  Q  Y  F  K  L  L  T  S  V  E  W  K
gagaagaccctccccagcggcatcaagcagttcgcctactatgatgaggactcggaggag
 E  K  T  L  P  S  G  I  K  Q  F  A  Y  Y  D  E  D  S  E  E
gagctcatgatgctgcccaccgatatcgctctcttgcacgacccctccttccggccgtgg
 E  L  M  M  L  P  T  D  I  A  L  L  H  D  P  S  F  R  P  W
gtcgagaagtatgccgaggacaaggatgcctttttcgcagacttctcaaaggtctttgcc
 V  E  K  Y  A  E  D  K  D  A  F  F  A  D  F  S  K  V  F  A
aagctgattgagctgggcatagtcagagatgagagcggtgcggtaatcaacactgataac
 K  L  I  E  L  G  I  V  R  D  E  S  G  A  V  I  N  T  D  N
gtcaagggcggctacatctctgcgcccaagaagagtgagctgcctggtgctccgggtaag
 V  K  G  G  Y  I  S  A  P  K  K  S  E  L  P  G  A  P  G  K
gctaatgaggaggctgagccgctcatgaaagagaatgagaggttcagggcacgtctgtaa
 A  N  E  E  A  E  P  L  M  K  E  N  E  R  F  R  A  R  L  -
SEQ ID NO : 45
   LENGTH : 319
   TYPE : PRT
   ORGANISM : *M. phaseolina* 
MPSNPGDYDAVRHDVKNLLHQPEYDDGSAGPVLVRLAWHSAGTYDAHSDTGGSNGAGMRYEAEGGDPANAGLQ
HARVFLEPIKAAHPWITYSDLWTLAGVVAIKEMGGPDIPWQPGRTDFVDDSKLPPRGRLPDAAQGADHIRWIF
YRMGFNDQEIVALSGAHNLGRCHADRSGFDGAWVNNPTRFSNQYFKLLTSVEWKEKTLPSGIKQFAYYDEDSE
EELMMLPTDIALLHDPSFRPWVEKYAEDKDAFFADFSKVFAKLIELGIVRDESGAVINTDNVKGGYISAPKKS
ELPGAPGKANEEAEPLMKENERFRARL*
SEQ ID NO : 46
   LENGTH : 2160 (including 150 bp 5' UTR and 150 bp 3' UTR)
   TYPE : DNA
   ORGANISM : *M. phaseolina* 
CAACTCTTTGGATCATTCGCAGTTCTGCAGATTAGACTACTCTTTTCTCGCTCCACAATGAAGCTTG
CATCATGGCTGCTCCTCCCTCAAGTACTGGCAGCGCTGGCGGCAGGTACGTGAATATAACTTGAC
TCTTGAGGCAAGCTGGATGGCTCAAGGTAGATGTCCCCGAAGAAACAAAGTGATAGTATCTCTGCTA
ACATGGACAGACGGAACCCTCGCGGTGTCTTAACTATCAACGGCCAGACACCTGGTCCATTGATCT
GGGGATATGAAGGAGACACACTTCGCGTCACTGTGACCAATAAAATGTTTATTGAGGCTACTATGCA
TTGGTCAGAGTGAAGTCTGTCTGGACTGGGAGAATCAACTAACGGAAAGCAGGCACGGTGTCTATCA
GGTCGACAAGTACTGGAACGACGGAGTACCTGGCGTGACTCAATGGCCCATTGAATCCAGGATTCG
TATACTTACGAGTTTACTCTCACCAACCAAACTGGAAGCTACTTCTACCATGGCCACTTTGGACCCG
CATTCGCGGACGGCCAACGAGGCCCGCTGTGGATTGCACCGGCCCCCTGGAGACCCCGTCCGTATGA
GCTTGCGTCTGATGACCCGGCAGAGGTTGCAGCAATGCGCGCGGCCGAAGACAATCCGAGACACCTC
ATGGTTTCCGACTGGAACTATGAGGGAATGGAAGTGCTGATTGTGGGCTTCAGAGATGCAGGCATTG
CTCCGGCATGTTCTGCGTCCCTCGTGACAAATGGAAAGGGCAGGACAACTTGCCTCGGCCCAGATGA
TATCAAGAAATACGATCCCGAGGGTCGGAGGAATTCACTTGGGTGCCTTCCTCCTCCAGTCGGCGCT
GAGTTCACCAACAAGAGAGAATGCCGCGAGACTACCACCGACTTCGAGATCATTCAGGCCGAAGAAG
GGGAGAAGTATATCTACATGAACTTTATCCACCCTGGAGCCCACCATGAACTGCGAATCGCGGTGGA
CGAGCACGACATGATCATCGTGGCAGCTGACGGGGATTTTGTCATGCCGAAAAAAGTCCAGGTACGT
TAATGCGAGAACTTGCACTGAGCGGTGCTGACCTATGGCTGCAGGCAATAAACCTCAACATGGGCGA
CAGGATCAGTGTCCTGGTACCGCTAGACAAGAAGCCGGGGGAATACGCCATCCGCCTGTCGTCCATT
TCCGAGGAGCAATTGATTACGGGCTTGAGCATCTTGCGCTACCCCGGTGTGCAGGAGCGCCGCAAAG
ACGGTATTATGCTGGCACCGGAAACAAAACCCCATATTGATCTGTTGGGGCGGATGGTCACTGAAGG
AGGTGTCATGATGGATGAAATGACCGATTTGGCCCCCTTTCCGCCGCGCTCACCCCCAGCGACGTCT
GATCACACGTTTCGGTTTTTATCAAACCGCACCGGCCCGAGCACATGGATGCTGTCGAGCGAGCCAC
ACCAAGGCTTCCGCCAGCAGATGCCTCCTATTATGTGGAACGAAGAGTCTCGTGGCCCTACAACCAT
TCAGGGGATGAAGAATGGATCCACCGTAGACATCATCTTCGAGAGCCGCGCATACGCCATGCACCCT
TTCCACAAACACAATCACAAGGCCTGGATTATTGGTAGAGGAAAGGCTACTTCCGTTGGCCAGATG
TTGCTACTGCCATCTCGGAAGCTCCAGAGAATTTCAACCTGATCAACCCGCCGTTGCGAGATGGTGC
CCGGCTCGAAGCGGAAGAGGGATCCTGGACGGTGATCCGTTACACCATCACCTTTCCTGCCATGAGC
ATGCTGCACTGCCATCGTATTCAGCACTTTGCGGTAAGTGTGTGATCTAGCCACCAAACGAATGGTC
ACTGATTGAAACAGGCTGGACAACAGATAGTCCTTTTGGAGGGGCAGGATGTGATGCAAAGCCCTCC
TGAGTACATCAAAAAAATGACGCATGCGAGCTTTGTGCCGCCACTCCGATACGGCCCCCTTGACTGA
GAGTTCCCATGCGAGGCCAGAGCACGGAATTGGGTAGTTGAAGTTAAAACCTGCCACATAAAAAATA
GCTCGAATAACAGGATTTTGCTAGCTCATGGAACTAGCCACTTGTTCCTTTGCGTTCAGTCTAATGG
CTGCGGTGAAAGCGAA
SEQ ID NO : 47
   LENGTH : 1713
   TYPE : DNA
   ORGANISM : *M. phaseolina*
   FEATURE NAME/KEY: CDS
   LOCATION : (1) ...... (1713) 
atggctcaaggtagatgtccccgaagaaacaaagtgatagtatctctgctaacatggaca
M A Q G R C P R R N K V I V S L L T W T
 
gacgggaaccctcgcggtgtcttaactatcaacggccagacacctggtccattgatctgg
 D  G  N  P  R  G  V  L  T  I  N  G  Q  T  P  G  P  L  I  W
ggatatgaaggagacacacttcgcgtcactgtgaccaataaaatgtttattgaggctact
 G  Y  E  G  D  T  L  R  V  T  V  T  N  K  M  F  I  E  A  T
atgcattggcacggtgtctatcaggtcgacaagtactggaacgacggagtacctggcgtg
 M  H  W  H  G  V  Y  Q  V  D  K  Y  W  N  D  G  V  P  G  V
actcaatggcccattgaatccagggattcgtatacttacgagtttactctcaccaaccaa
 T  Q  W  P  I  E  S  R  D  S  Y  T  Y  E  F  T  L  T  N  Q
actggaagctacttctaccatggccactttggacccgcattcgcggacggccaacgaggc
 T  G  S  Y  F  Y  H  G  H  F  G  P  A  F  A  D  G  Q  R  G
ccgctgtggattgcaccggcccccctggagaccccgtccgtatgagcttgcgtctgatgac
 P  L  W  I  A  P  A  P  W  R  P  R  P  Y  E  L  A  S  D  D
ccggcagaggttgcagcaatgcgcgcggccgaagacaatccgagacacctcatggtttcc
 P  A  E  V  A  A  M  R  A  A  E  D  N  P  R  H  L  M  V  S
gactggaactatgagggaatggaagtgctgattgtgggcttcagagatgcaggcattgct
 D  W  N  Y  E  G  M  E  V  L  I  V  G  F  R  D  A  G  I  A
ccggcatgttctgcgtccctcgtgacaaatggaaagggcaggacaacttgcctcggccca
 P  A  C  S  A  S  L  V  T  N  G  K  G  R  T  T  C  L  G  P
gatgatatcaagaaatacgatcccgagggtcggaggaattcacttgggtgccttcctcct
 D  D  I  K  K  Y  D  P  E  G  R  R  N  S  L  G  C  L  P  P
ccagtcggcgctgagttcaccaacaagagagaatgccgcgagactaccaccgacttcgag
 P  V  G  A  E  F  T  N  K  R  E  C  R  E  T  T  T  D  F  E
atcattcaggccgaagaaggggagaagtatatctacatgaactttatccaccctggagcc
 I  I  Q  A  E  E  G  E  K  Y  I  Y  M  N  F  I  H  P  G  A
caccatgaactgcgaatcgcggtggacgagcacgacatgatcatcgtggcagctgacggg
 H  H  E  L  R  I  A  V  D  E  H  D  M  I  I  V  A  A  D  G
gattttgtcatgccgaaaaaagtccaggcaataaacctcaacatgggcgacaggatcagt
 D  F  V  M  P  K  K  V  Q  A  I  N  L  N  M  G  D  R  I  S
gtcctggtaccgctagacaagaagccgggggaatacgccatccgcctgtcgtccatttcc
 V  L  V  P  L  D  K  K  P  G  E  Y  A  I  R  L  S  S  I  S
gaggagcaattgattacgggcttgagcatcttgcgctaccccggtgtgcaggagcgccgc
 E  E  Q  L  I  T  G  L  S  I  L  R  Y  P  G  V  Q  E  R  R
aaagacggtattatgctggcaccggaaacaaaaccccatattgatctgttggggcggatg
 K  D  G  I  M  L  A  P  E  T  K  P  H  I  D  L  L  G  R  M
gtcactgaaggaggtgtcatgatggatgaaatgaccgatttggcccccttttccgccgcgc
 V  T  E  G  G  V  M  M  D  E  M  T  D  L  A  P  F  P  P  R
tcacccccagcgacgtctgatcacacgtttcggtttttatcaaaccgcaccggcccgagc
 S  P  P  A  T  S  D  H  T  F  R  F  L  S  N  R  T  G  P  S
acatggatgctgtcgagcgagccacaccaaggcttccgccagcagatgcctcctattatg
 T  W  M  L  S  S  E  P  H  Q  G  F  R  Q  Q  M  P  P  I  M
tggaacgaagagtctcgtggccctacaaccattcaggggatgaagaatggatccaccgta
 W  N  E  E  S  R  G  P  T  T  I  Q  G  M  K  N  G  S  T  V
gacatcatcttcgagagccgcgcatacgccatgcaccctttccacaaacacaatcacaag
 D  I  I  F  E  S  R  A  Y  A  M  H  P  F  H  K  H  N  H  K
gcctggattattggtagaggaaaggctacttccgttggccagatgttgctactgccatc
 A  W  I  I  G  R  G  K  G  Y  F  R  W  P  D  V  A  T  A  I
tcggaagctccagagaatttcaacctgatcaacccgccgttgcgagatggtgcccggctc
 S  E  A  P  E  N  F  N  L  I  N  P  P  L  R  D  G  A  R  L
gaagcggaagagggatcctggacggtgatccgttacaccatcacctttcctgccatgagc
 E  A  E  E  G  S  W  T  V  I  R  Y  T  I  T  F  P  A  M  S
atgctgcactgccatcgtattcagcactttgcggctggacaacagatagtccttttggag
 M  L  H  C  H  R  I  Q  H  F  A  A  G  Q  Q  I  V  L  L  E
gggcaggatgtgatgcaaagccctcctgagtacatcaaaaaaatgacgcatgcgagcttt
 G  Q  D  V  M  Q  S  P  P  E  Y  I  K  K  M  T  H  A  S  F
gtgccgccactccgatacggccccccttgactga
 V P P L R Y G P L D -
SEQ ID NO : 48
   LENGTH : 570
   TYPE : PRT
   ORGANISM : *M. phaseolina* 
MAQGRCPRRNKVIVSLLTWTDGNPRGVLTINGQTPGPLIWGYEGDTLRVTVTNKMFIEATMHWHGVY
QVDKYWNDGVPGVTQWPIESRDSYTYEFTLTNQTGSYFYHGHFGPAFADGQRGPLWIAPAPWRPRPY
ELASDDPAEVAAMRAAEDNPRHLMVSDWNYEGMEVLIVGFRDAGIAPACSASLVTNGKGRTTCLGPD
DIKKYDPEGRRNSLGCLPPPVGAEFTNKRECRETTTDFEIIQAEEGEKYIYMNFIHPGAHHELRIAV
DEHDMIIVAADGDFVMPKKVQAINLNMGDRISVLVPLDKKPGEYAIRLSSISEEQLITGLSILRYPG
VQERRKDGIMLAPETKPHIDLLGRMVTEGGVMMDEMTDLAPFPPRSPPATSDHTFRFLSNRTGPSTW
MLSSEPHQGFRQQMPPIMWNEESRGPTTIQGMKNGSTVDIIFESRAYAMHPFHKHNHKAWIIGRGKG
YFRWPDVATAISEAPENFNLINPPLRDGARLEAEEGSWTVIRYTITFPAMSMLHCHRIQHFAAGQQI
VLLEGQDVMQSPPEYIKKMTHASFVPPLRYGPLD*
SEQ ID NO : 49
   LENGTH : 2269 (including 150 bp 5' UTR and 150 bp 3' UTR)
   TYPE : DNA
   ORGANISM : *M. phaseolina* 
AGTATGTGCGTTGATCCCGCTGTCTATTCGAGCACCAGAGCACTGTCCAGCCACCTTGAGCTATCTC
AAGGGGCTATCTCATCTGGTCATCTTCGCTGGGGCATCGGTTGCCTTCTCAACCCTTCTCAATTCCC
CTCACGCTTCTGCAACATGTTCTTCGGCTCCCTCCACTTGGGCATCGGCGCCCTATTGGTTGCCGGC
ACTCTTGCTGGCGACGACAAGTGGCTTAGCCCCGTCTACAAGAACTTTTACGAGTTCCCCCTACCTA
AGCCACCAATCAAGGAAGCGAAAGCGTAAGTCAACTGATATTTCTCTCGCTCGTCATTTGCATGCCT
AATCTTCGCAGGAAATATACCAACCCGACTACCGGTGCTGTGATCAACTACTACGAAATCACCATCT
CACCCCTGCAGCAACAGGTTTATCCTGGCCTTGGCAAGGCAAACCTCGTTGGCTACGATGGTATCTC
TCCCGGTCCCACTTTTAAGATGGAGAGGGGAGAAGAGGCTGTCGTTCGTTTCATCAACAAGGCCTCC
ATTCCCAATTCCGTCCATCTTCACGGCTCCTACTCCTTTGCCCCCTTCGATGGCTGGGCGGAGGATA
CGACCAGCCCAGGCCAATACAAGGACTACTACTACCCCAATGCCCAGTCTGCCCGTACCCTCTGGTA
CCACGACCATGCCGTCTTCCACACTGCCGAGAACGCCTACTACGGTCAGGCAGGTTTCTACATCCTG
CACGACTCGGCTGAGGATAGTCTGGGTCTCCCGTCTGGAGACTACGACATCCCGCTCGGTCTGAGCT
CGAAGCAATACCAGTCCAACGGTGACCTTTTCAGCCCGAATGGCGAGACGGATAGCCTTTTTGGCGA
TGTTATCCATGTCAACGGCCAGCCCTGGCCGTACCTCAAGGTCGAGCCCAGGAAGTACCGCTTCCGC
CTGCTTGATACAAGCATTTCCCGTGCCTTCCAGCTGTCACTCCAAGACGATAAGAGCAAGAAGATTG
ACTTTAACGTCATCGCCTCCGATGCCGGCCTCCTGTCCAGCCCTGTTCCGACCAACCTGCTACACAT
TTCCATGGCCGAACGCTGGGAAATTGTCGTCGACTTCTCCCAGTACGCTGGCAAGAACATCACCATG
AAGAACGAGCGTGACGTGCAGGCCGATGAAGACTACAACAGCACTGACAAGGTCATGCGCTTCGTAG
TAGGCAACAAGGTTACCTCGACTGCCAACAACAACCTGCCTGGCAGCCTCCGCAGCGTGCCTTTCCC
GCCGAATAAGTCTGGTGTTGACAGGAGCTTCAAGTTCGAGCGCAAAGGCGGTGAATGGACTATCAAC
GGCGTTACCTTTGCCGACGTCGAGAACCGTATTCTAGGCAAGCCCCAACGCGGACAGGTTGAGGTCT
GGGAGCTCGAGAACTCTTCCGGCGGCTGGTCTCACCCCGTTCACATCCATCTCATCGACTTCCAGGT
TATTTCTCGCACTGGTGGCAAGCGTGATGTCCTGCCTTACGAGAAGAACGGTCTCAAAGATGTCGTC
TTGCTTGGCGTGAACGAGAAGGTCAGAGTTGTCGCTCGCTTCCAGCCCTGGAAGGTGTCTACATGT
TCCATTGCCACAACCTGATCCACGAGGACCACGATATGGTGCGCTCCCAGCATTCCTCTGATTACTC
GCAACAAAGACTGACAAATAATTACAGATGGCCGCTTTCAATGTATCCACCCTTTCAGACTACGGCT
ACGACCAGAAGGAGACCCTTTTCATCGACCCGATGGAGTCCCGCTGGCGCGCAAAGGATGTCAAGAC
CGAAGACTTCACAACCGATGCCATCCAGTCGAAGCTTGCCGCTTTCGCTGAAATAAATCGCTACAAG
GACGTTGCGAAGATCGAAAGTGCTCTTGAAAATTATTGGAAGACCGCTCCCACCGGGTTCAAGATTA
GCACCACCTCCTCAACTCCGAGCTCAACCGCCGCCACCTCCACCGCTTCCAGCAGTGGCTCAAACAC
CTCTATTACCGCCCCCATTCAGTCCCCGGCCACTACTTCACCCGCGACCACTTCGACCAAGGCGGAT
GACAAGGGCAAGGCTAAGACTTCTACGACCAAAACGAAGTAACGAAATGGCTTTAGATCACGCTCA
TTATAGATAAAACTTGGGAGAATTTGGGTCGGTGTTTTTGGATAGTTTGGTTCGGAGGAGCGCTCTC
TGTAAATATGGGCCTCGCGAACTCTGTGGATATTTGTTTCTCGGTGTCTGTTAATATC
SEQ ID NO : 50
   LENGTH : 1860
   TYPE : DNA
   ORGANISM : *M. phaseolina*
   FEATURE NAME/KEY: CDS
   LOCATION : (1)......(1860) 
atgttcttcggctccctccacttgggcatcggcgccctattggttgccggcactcttgct
 M  F  F  G  S  L  H  L  G  I  G  A  L  L  V  A  G  T  L  A
ggcgacgacaagtggcttagccccgtctacaagaacttttacgagttccccctacctaag
 G  D  D  K  W  L  S  P  V  Y  K  N  F  Y  E  F  P  L  P  K
ccaccaatcaaggaagcgaaagcgaaatataccaacccgactaccggtgctgtgatcaac
 P  P  I  K  E  A  K  A  K  Y  T  N  P  T  T  G  A  V  I  N
tactacgaaatcaccatctcaccccctgcagcaacaggtttatcctggccttggcaaggca
 Y  Y  E  I  T  I  S  P  L  Q  Q  Q  V  Y  P  G  L  G  K  A
aacctcgttggctacgatggtatctctcccggtcccactttaagatggagaggggagaa
 N  L  V  G  Y  D  G  I  S  P  G  P  T  F  K  M  E  R  G  E
gaggctgtcgttcgtttcatcaacaaggcctccattcccaattccgtccatcttcacggc
 E  A  V  V  R  F  I  N  K  A  S  I  P  N  S  V  H  L  H  G
tcctactcctttgccccttcgatggctgggcggaggatacgaccagcccaggccaatac
 S  Y  S  F  A  P  F  D  G  W  A  E  D  T  T  S  P  G  Q  Y
aaggactactactaccccaatgcccagtctgcccgtaccctctggtaccacgaccatgcc
 K  D  Y  Y  Y  P  N  A  Q  S  A  R  T  L  W  Y  H  D  H  A
gtcttccacactgccgagaacgcctactacggtcaggcaggtttctacatcctgcacgac
 V  F  H  T  A  E  N  A  Y  Y  G  Q  A  G  F  Y  I  L  H  D
tcggctgaggatagtctgggtctcccgtctggagactacgacatcccgctcggtctgagc
 S  A  E  D  S  L  G  L  P  S  G  D  Y  D  I  P  L  G  L  S
tcgaagcaataccagtccaacggtgaccttttcagcccgaatggcgagacggatagcctt
 S  K  Q  Y  Q  S  N  G  D  L  F  S  P  N  G  E  T  D  S  L
tttggcgatgttatccatgtcaacggccagccctggccgtacctcaaggtcgagcccagg
 F  G  D  V  I  H  V  N  G  Q  P  W  P  Y  L  K  V  E  P  R
aagtaccgcttccgcctgcttgatacaagcatttcccgtgccttccagctgtcactccaa
 K  Y  R  F  R  L  L  D  T  S  I  S  R  A  F  Q  L  S  L  Q
gacgataagagcaagaagattgactttaacgtcatcgcctccgatgccggcctcctgtcc
 D  D  K  S  K  K  I  D  F  N  V  I  A  S  D  A  G  L  L  S
agccctgttccgaccaacctgctacacatttccatggccgaacgctgggaaattgtcgtc
 S  P  V  P  T  N  L  L  H  I  S  M  A  E  R  W  E  I  V  V
gacttctcccagtacgctggcaagaacatcaccatgaagaacgagcgtgacgtgcaggcc
 D  F  S  Q  Y  A  G  K  N  I  T  M  K  N  E  R  D  V  Q  A
gatgaagactacaacagcactgacaaggtcatgcgcttcgtagtaggcaacaaggttacc
 D  E  D  Y  N  S  T  D  K  V  M  R  F  V  V  G  N  K  V  T
tcgactgccaacaacaacctgcctggcagcctccgcagcgtgcctttcccgccgaataag
 S  T  A  N  N  N  L  P  G  S  L  R  S  V  P  F  P  P  N  K
tctggtgttgacaggagcttcaagttcgagcgcaaaggcggtgaatggactatcaacggc
 S  G  V  D  R  S  F  K  F  E  R  K  G  G  E  W  T  I  N  G
gttacctttgccgacgtcgagaaccgtattctaggcaagccccaacgcggacaggttgag
 V  T  F  A  D  V  E  N  R  I  L  G  K  P  Q  R  G  Q  V  E
gtctgggagctcgagaactcttccggcggctggtctcaccccgttcacatccatctcatc
 V  W  E  L  E  N  S  S  G  G  W  S  H  P  V  H  I  H  L  I
gacttccaggttatttctcgcactggtggcaagcgtgatgtcctgccttacgagaagaac
 D  F  Q  V  I  S  R  T  G  G  K  R  D  V  L  P  Y  E  K  N
ggtctcaaagatgtcgtcttgcttggcgtgaacgagaaggtcagagttgtcgctcgcttc
 G  L  K  D  V  V  L  L  G  V  N  E  K  V  R  V  V  A  R  F
cagccctgggaaggtgtctacatgttccattgccacaacctgatccacgaggaccacgat
 Q  P  W  E  G  V  Y  M  F  H  C  H  N  L  I  H  E  D  H  D
atgatggccgctttcaatgtatccaccctttcagactacggctacgaccagaaggagacc
 
M M A A F N V S T L S D Y G Y D Q K E T
cttttcatcgacccgatggagtcccgctggcgcgcaaaggatgtcaagaccgaagacttc
L F I D P M E S R W R A K D V K T E D F
acaaccgatgccatccagtcgaagcttgccgctttcgctgaaataaatcgctacaaggac
T T D A I Q S K L A A F A E I N R Y K D
gttgcgaagatcgaaagtgctcttgaaaattattggaagaccgctcccaccgggttcaag
V A K I E S A L E N Y W K T A P T G F K
attagcaccacctcctcaactccgagctcaaccgccgccacctccaccgcttccagcagt
I S T T S S T P S S T A A T S T A S S S
ggctcaaacacctctattaccgcccccattcagtccccggccactacttcacccgcgacc
G S N T S I T A P I Q S P A T T S P A T
acttcgaccaaggcggatgacaagggcaaggctaagacttctacgaccaaaacgaagtaa
T S T K A D D K G K A K T S T T K T K -
SEQ ID NO : 51
   LENGTH : 619
   TYPE : PRT
   ORGANISM : *M. phaseolina* 
MFFGSLHLGIGALLVAGTLAGDDKWLSPVYKNFYEFPLPKPPIKEAKAKYTNPTTGAVINYYEITIS
PLQQQVYPGLGKANLVGYDGISPGPTFKMERGEEAVVRFINKASIPNSVHLHGSYSFAPFDGWAEDT
TSPGQYKDYYYPNAQSARTLWYHDHAVFHTAENAYYGQAGFYILHDSAEDSLGLPSGDYDIPLGLSS
KQYQSNGDLFSPNGETDSLFGDVIHVNGQPWPYLKVEPRKYRFRLLDTSISRAFQLSLQDDKSKKID
FNVIASDAGLLSSPVPTNLLHISMAERWEIVVDFSQYAGKNITMKNERDVQADEDYNSTDKVMRFVV
GNKVTSTANNNLPGSLRSVPFPPNKSGVDRSFKFERKGGEWTINGVTFADVENRILGKPQRGQVEVW
ELENSSGGWSHPVHIHLIDFQVISRTGGKRDVLPYEKNGLKDVVLLGVNEKVRVVARFQPWEGVYMF
HCHNLIHEDHDMMAAFNVSTLSDYGYDQKETLFIDPMESRWRAKDVKTEDFTTDAIQSKLAAFAEIN
RYKDVAKIESALENYWKTAPTGFKISTTSSTPSSTAATSTASSSGSNTSITAPIQSPATTSPATTST
KADDKGKAKTSTTKTK*
SEQ ID NO : 52
   LENGTH : 2423 (including 150 bp 5' UTR and 150 bp 3' UTR)
   TYPE : DNA
   ORGANISM : *M. phaseolina* 
AAAGGCCTTTGGCTCCCGCCAAAATTGTTCATCCTCACGCTCACTCCTGCGCTCTTCCCTACAGACC
TTTTGCCTGTAGCTTTAGCTTCACGGTCTTTTCATTCCCCTTCTCTGGCATTCATCCCACCGCGGA
AGCCTCAATTACCACAATGGTGTCCCTTAAGCAAATCGGTGCCACTCTGTTGGCACTGACTGCTCAA
ACCTTCGCTGCTGCCATCCCTGAGGCCGAACCGGTGGACCTCGTCGCCCGTCAAGCCACGACCACTA
CGTCCACCACCTCCTCAACCACCTCGAGGGTTCCCGATTCCCGATGTACTTGGGGCCCTTCGAGCAG
GGGTTGCTGGAAGAACGGCTTCAGCATTGCCACTGACTTTGACACGAAGTGGCCATCTACTGGCAAG
ACTGTCTCATATCGCTTGGAAGTTACGAATGTCACCAACTGCGAGGACTATCAAAGCAAGGGAATTG
GAGATGGCTTCTGCAGGCCGATGCTGCTCATCAACAATCAGTTCCCAGGGCCTACCAGTCAGTTTTT
CCCTCCTCGATGCCGGGTGACAAGGGCGCTAATGACTCGTACTTTCCAGTCAATGCGGAATGGGGAG
ACAACCTTGAGATTACTGTTGTCAATAGTATGCAAGACAACGGCACCTCATTCCACTGGCACGGCAT
TCGTCAGCTGAACTCATGCCAAAACGATGGTGCCAACGGCGTCACCGAGTGCCCTATTCCTCCCGGC
GGAAGCTTCACTTATAAGTTCAAGGCTACACAGTATGGTGAGTCTTTGCCTTCTCTTCTACCTTTTC
CATTTCATGCAAGCATCTTCATGTGCGTTCTGTTGAGTTTTGAGGCGGAAATACTCTTGAGCTTGCC
CCGTTCCGTTTGTGAGCCACGTGCAAGGCCACCTGCTCCCACCCTTGTCATAATACTGACCGGCCCT
TcCAGGAACAACATGGTACCATAGCCATCACTCTGCTCAGTACGGCGATGGGATCCAAGGTGCCATC
GTGATCAATGGCCCGGCAACCGCCAATTACGACGAGGATTTAGGCCCCGTTGCCCTCACCGAAACCT
ACGATGAGACGGCATGGACGAAGAACTGGCTGGCGCTGCACGTGGCATTCCCTCCTCAGCCCCTCAA
CATTCTCTTCAATGGCTCCATGGTCAACAGCACCGGCGGCGGCCGCTACAACACCATCTCAGTCAAG
CAAGGCAAGACTTACCGGCTGCGCCTGATTAACATGAGCGTCGACACTTTCTTCGTATTCTCCATGG
ACGGGCACGAGTTCCAGATCATCACGGCCGACCTCGTCCCCGTGCACCCTTACAATGCGACCTCGAT
CATGATCGGCATCGGCCAGCGCTACGACATCGTCTTCAAGGCCAACCAGCCCGCTGCCAACTACTGG
CTGCGTACCGAGATCGCCAGCTGCAGTGCCAACGCCATCACGGCCGAAGCCGACATCGTCCCCGGCG
 
GCATCCTGAACTACGACACCATCGACAAGACGGATCTGCCAGTCTCCACCAAGTCCGTTATCGAGAC
GACCGACTGCGCCGCCGAGCCCTACGACAAGCTGGTCCCCTGGTGGAGACGCAGGTCCCCAAGGAC
CAGTTCCTGACCCAGCTCGAGGGCATCGACCTGACGTTCGCGGCGGGCGCCACGGTCGGCAGCGAGA
CTGGTCTTGTGCAGTGGTACCTGAACGACAGCGCCATGGTCGTCGACTGGGCCAAACCGACTCTGGA
GTACTTCTCCGAGGGAGACACTAACTATACGTCTTCAATGAACGTTTTCCAGATGCCCGCGGAGGGG
AAGTGGTCGTTCTGGATCATCCACAACAACGCGGCCGCTCTGCTCGACCACCCGATCCATCTCCACG
GCCACGACTTTTTCCACCTGGGCGCCGGCACCGGCACCTGGGACGGCAACGTGGACTCCTTGATCTT
CGACAATCCTATGCGCAGGGACGTGATGATCTTGCCCACAGGATGGCTTATTATCGCCTTTCCAGCG
GACAAcCCCGGCGCGTGGTTGATGCATTGCCACATCGTAAGTACCCAACAAGCCCCATCTCTCTCTC
TCTCTCTCATCAAAGATGACGGCAACTGACAAGCAACCAGGCATGGCACGTTACCGACGGGCTTTCC
TTGCAGTTCGTTGAAAACCCAGGCTCATTCACGCAGGACCTCTCGGGCATGAAGAGCAACTGCGCCG
CCTGGAAAGAGTACGAGGAGAAGGCTTACTATGAGAAGGAGGTTGGCGATTCCGGCTTGTAAAACCG
TGACGGCGGGGATGAGGAGTTTATGACGAGATGTTCACTTTATACCTTCTACCACCACCACCACCCT
TTTCCTCTTTCTTTTACCGACGTAGGGCTGCACTATCTGGCGCGTCTGTGGTCTGCTTTGCATATGC
ATAAATACCTT
SEQ ID NO : 53
   LENGTH : 1824
   TYPE : DNA
   ORGANISM : *M. phaseolina*
   FEATURE NAME/KEY: CDS
   LOCATION : (1)......(1824) 
atggtgtcccttaagcaaatcggtgccactctgttggcactgactgctcaaaccttcgct
 M  V  S  L  K  Q  I  G  A  T  L  L  A  L  T  A  Q  T  F  A
gctgccatccctgaggccgaaccggtggacctcgtcgcccgtcaagccacgaccactacg
 A  A  I  P  E  A  E  P  V  D  L  V  A  R  Q  A  T  T  T  T
tccaccacctcctcaaccacctcgagggttcccgattcccgatgtacttggggcccttcg
 S  T  T  S  S  T  T  S  R  V  P  D  S  R  C  T  W  G  P  S
agcaggggttgctggaagaacggcttcagcattgccactgactttgacacgaagtggcca
 S  R  G  C  W  K  N  G  F  S  I  A  T  D  F  D  T  K  W  P
tctactggcaagactgtctcatatcgcttggaagttacgaatgtcaccaactgcgaggac
 S  T  G  K  T  V  S  Y  R  L  E  V  T  N  V  T  N  C  E  D
tatcaaagcaagggaattggagatggcttctgcaggccgatgctgctcatcaacaatcag
 Y  Q  S  K  G  I  G  D  G  F  C  R  P  M  L  L  I  N  N  Q
ttcccagggcctaccatcaatgcggaatggggagacaaccttgagattactgttgtcaat
 F  P  G  P  T  I  N  A  E  W  G  D  N  L  E  I  T  V  V  N
agtatgcaagacaacggcacctcattccactggcacggcattcgtcagctgaactcatgc
 S  M  Q  D  N  G  T  S  F  H  W  H  G  I  R  Q  L  N  S  C
caaaacgatggtgccaacggcgtcaccgagtgccctattcctcccggcggaagcttcact
 Q  N  D  G  A  N  G  V  T  E  C  P  I  P  P  G  G  S  F  T
tataagttcaaggctacacagtatggaacaacatggtaccatagccatcactctgctcag
 Y  K  F  K  A  T  Q  Y  G  T  T  W  Y  H  S  H  H  S  A  Q
tacggcgatggatccaaggtgccatcgtgatcaatggcccggcaaccgccaattacgac
 Y  G  D  G  I  Q  G  A  I  V  I  N  G  P  A  T  A  N  Y  D
gaggatttaggccccgttgccctcaccgaaacctacgatgagacggcatggacgaagaac
 E  D  L  G  P  V  A  L  T  E  T  Y  D  E  T  A  W  T  K  N
tggctggcgctgcacgtggcattccctcctcagcccctcaacattctcttcaatggctcc
 W  L  A  L  H  V  A  F  P  P  Q  P  L  N  I  L  F  N  G  S
atggtcaacagcaccggcggcggccgctacaacaccatctcagtcaagcaaggcaagact
 M  V  N  S  T  G  G  G  R  Y  N  T  I  S  V  K  Q  G  K  T
taccggctgcgcctgattaacatgagcgtcgacactttcttcgtattctccatggacggg
 Y  R  L  R  L  I  N  M  S  V  D  T  F  F  V  F  S  M  D  G
cacgagttccagatcatcacggccgacctcgtccccgtgcacccttacaatgcgacctcg
 H  E  F  Q  I  I  T  A  D  L  V  P  V  H  P  Y  N  A  T  S
atcatgatcggcatcggccagcgctacgacatcgtcttcaaggccaaccagcccgctgcc
 
I M I G I G Q R Y D I V F K A N Q P A A
aactactggctgcgtaccgagatcgccagctgcagtgccaacgccatcacggccgaagcc
N Y W L R T E I A S C S A N A I T A E A
gacatcgtccccggcggcatcctgaactacgacaccatcgacaagacggatctgccagtc
D I V P G G I L N Y D T I D K T D L P V
tccaccaagtccgttatcgagacgaccgactgcgccgccgagccctacgacaagctggtc
S T K S V I E T T D C A A E P Y D K L V
ccctggtgggagacgcaggtccccaaggaccagttcctgacccagctcgagggcatcgac
P W W E T Q V P K D Q F L T Q L E G I D
ctgacgttcgcggcgggcgccacggtcggcagcgagactggtcttgtgcagtggtacctg
L T F A A G A T V G S E T G L V Q W Y L
aacgacagcgccatggtcgtcgactgggccaaaccgactctggagtacttctccgaggga
N D S A M V V D W A K P T L E Y F S E G
gacactaactatacgtcttcaatgaacgttttccagatgcccgcggaggggaagtggtcg
D T N Y T S S M N V F Q M P A E G K W S
ttctggatcatccacaacaacgcggccgctctgctcgaccacccgatccatctccacggc
F W I I H N N A A A L L D H P I H L H G
cacgacttttttccacctgggcgccggcaccggcacctgggacggcaacgtggactccttg
H D F F H L G A G T G T W D G N V D S L
atcttcgacaatcctatgcgcagggacgtgatgatcttgcccacaggatggcttattatc
I F D N P M R R D V M I L P T G W L I I
gcctttccagcggacaaccccggcgcgtggttgatgcattgccacatcgcatggcacgtt
A F P A D N P G A W L M H C H I A W H V
accgacgggctttccttgcagttcgttgaaaacccaggctcattcacgcaggacctctcg
T D G L S L Q F V E N P G S F T Q D L S
ggcatgaagagcaactgcgccgcctggaaagagtacgaggagaaggcttactatgagaag
G M K S N C A A W K E Y E E K A Y Y E K
gaggttggcgattccggcttgtaa
E V G D S G L -
SEQ ID NO : 54
   LENGTH : 607
   TYPE : PRT
   ORGANISM : *M. phaseolina* 
MVSLKQIGATLLALTAQTFAAAIPEAEPVDLVARQATTTTSTTSSTTSRVPDSRCTWGPSSRGCWKN
GFSIATDFDTKWPSTGKTVSYRLEVTNVTNCEDYQSKGIGDGFCRPMLLINNQFPGPTINAEWGDNL
EITVVNSMQDNGTSFHWHGIRQLNSCQNDGANGVTECPIPPGGSFTYKFKATQYGTTWYHSHHSAQY
GDGIQGAIVINGPATANYDEDLGPVALTETYDETAWTKNWLALHVAFPPQPLNILFNGSMVNSTGGG
RYNTISVKQGKTYRLRLINMSVDTFFVFSMDGHEFQIITADLVPVHPYNATSIMIGIGQRYDIVFKA
NQPAANYWLRTEIASCSANAITAEADIVPGGILNYDTIDKTDLPVSTKSVIETTDCAAEPYDKLVPW
WETQVPKDQFLTQLEGIDLTFAAGATVGSETGLVQWYLNDSAMVVDWAKPTLEYFSEGDTNYTSSMN
VFQMPAEGKWSFWIIHNNAAALLDHPIHLHGHDFFHLGAGTGTWDGNVDSLIFDNPMRRDVMILPTG
WLIIAFPADNPGAWLMHCHIAWHVTDGLSLQFVENPGSFTQDLSGMKSNCAAWKEYEEKAYYEKEVG
DSGL*
SEQ ID NO : 55
   LENGTH : 2147 (including 150 bp 5' UTR and 150 bp 3' UTR)
   TYPE : DNA
   ORGANISM : *M. phaseolina* 
GCCACGGCTCCATTTGGATCGTCACTCACCACCACCACCACAACCACTCTCTACTCACCTCTTCACC
AGCCCCCTCTTTCTCCTCACTGGCATCCATTCGTTGAAGTCGCTGTCCCGTTTTCTTGTCTTTCGTC
TTCTTCCACCGTCAGAATGAGGGCTTCTTACCTCTCTGCGGCTGCCTTCCTGGGCCTCTCGGCTGCC
GCGCCGCAAGCCGCCAGCACTTCTTCTTCCTCTGCTTCAGCCAACGCCAGTTCCACCAGCTCAGCAG
CTACTTCCACTTGCACTGGCAACACTGCCGACGACCGCACTGTGTGGTGCGACTACGACATCAGCAC
 
CGACTACTACAACGACGGACCCGACACGGGGTCACCCGCGAGTACTACTTCGTCGTCAGCGACGTG
ACCGTCTCGCCCGATGGCATCTCGCGCTCCGCTATGGCGGTGAACGGCAGCATCCCCGGCCCCACCA
TTTTCGCCGACTGGGGTGACACAGTGAAAGTTACTGTCTACAACGACCTCACCACGAGCGGCAACGG
CTCTTCCATCCATTGGCACGGTATCCGGCAGAACTACACGAACCAGAATGATGGTGTGGTGTCTATT
ACGCAATGCCCGATTGCGGTCGGCGAGACCTACACCTACGAGTGGAAGGCCACGCAGTACGGCTCTT
CCTGGTACCACTCTCACATTGGCCTGCAGGCCTGGGAGGGTGTTTTCGGTGGTATCATTATCAACGG
TCCCGCTACTGCAAATTACGACGAGGACCTCGGCATCATGTTTCTCAATGATTGGGATCACTCGACT
GTTGACGAGCTCTACGATTCAGCTCAGAGCAGCGGTCCTCCTACGCTTGACACCGGTCTCATTAACG
GAACCAACATCTACAATGACTCCGGAACAGTTACTGGATCTCGCTGGGAGGCCAGCCTGACCGAGGG
TACCAGCTACCGGCTCCGTCTTGTCAACGCTGCTGTAGACTCGCACTTCAAATTTTCGATCGATAAC
CACACCCTCCAGGTTATCGCCATGGACCTGGTCCCCATTGAGCCCTACGAGACTACTGTTCTGGACA
TTGGCATGGGTCAGCGCTACGACGTCATTGTTACGGCAGACCAGGCTTCTGTTGCTTCTGATTTCTG
GCTTCGCGCAATTCCCCAGACCGCCTGCTCGGACAACGATAACGCAGATGATATCAAGGGCATAATC
CACTACGGATCATCAACTGGTACTCCGGAAACCACTGCTTAGTAAGTTCCGATGTCCACATCTTCCG
AGCTCTCCGCTAATGATTCAACAGTGATTACACTGATGCCTGTGTCGATGAGGACAGCTCTGATCTC
GTAAGTGCTCGAGATTCACGGTCGTCTGGAAAGAGTAGGCGCGCTGACTGTGATCTTTAGGTCCCGT
ATGTCTCTAAGACTGCCACCTCCGGTACCTCCCTGGCCGAGGCTGTTTCCGTCGGCTACAACTCGGA
CAACCTCTTCCGCTGGTACATGAACGAGACCTCTATGGAGGTCGAGTGGGAGAATCCAACCCTTCTG
CAGGTCTACAACGACAATCTGACGTTCACTGACACATCGGGTGTTGTTCAACTTGACACCGCAGACC
AATGGTACTTCTTCGTCATCGAGACCGACAACGCTGTGCCACACCCAATCCATCTTCACGGCCACGA
CTTCTTCGTCCTGGCTGCGGGCACCGGCTCTTACAGTTCAGACGTTACTCTGACTCTGGATAACCCT
CCCCGCCGCGACACGGCTATGCTTGACTCCTCTGGCTACTTGGTCCTGGCTTTCGAGACCGACAACC
CAGGTGCGTGGTTGATGCACTGCCACATCGGCTGGCACACCAGCGAGGGCTTTGCCCTTCAGATCTT
GGAGCGCTACACCGAGATCCAGGATAGCCTGATCGACTACGACGTCCTCAATGACACCTGCTCGACT
TGGTCTACTTACTCCGAGGCAAACTCGATCGAGGAGGAGGACTCTGGTGTGTAAGGAAAACTCTGAG
TGACAATCCGTCTATGGCAGGTGTGCGGGGCGCTTTGGAGCTCTCTCTATCTCTGTGAAAGATCTTG
TATATAACGTGATGCCAGCCTCTCCCTGACATTTGGTGCTTCGGTTGATCTACATATATTTCCTTAT
TTA
SEQ ID NO : 56
   LENGTH : 1737
   TYPE : DNA
   ORGANISM : *M. phaseolina*
   FEATURE NAME/KEY: CDS
   LOCATION : (1)......(1737) 
atgaggcttcttacctctctgcggctgccttcctgggcctctcggctgccgcgccgcaa
 M  R  A  S  Y  L  S  A  A  A  F  L  G  L  S  A  A  A  P  Q
gccgccagcacttcttcttcctctgcttcagccaacgccagttccaccagctcagcagct
 A  A  S  T  S  S  S  S  A  S  A  N  A  S  S  T  S  S  A  A
acttccacttgcactggcaacactgccgacgaccgcactgtgtggtgcgactacgacatc
 T  S  T  C  T  G  N  T  A  D  D  R  T  V  W  C  D  Y  D  I
agcaccgactactacaacgacggacccgacacgggggtcacccgcgagtactacttcgtc
 S  T  D  Y  Y  N  D  G  P  D  T  G  V  T  R  E  Y  Y  F  V
gtcagcgacgtgaccgtctcgcccgatggcatctcgcgctccgctatggcggtgaacggc
 V  S  D  V  T  V  S  P  D  G  I  S  R  S  A  M  A  V  N  G
agcatccccggccccaccatttcgccgactggggtgacacagtgaaagttactgtctac
 S  I  P  G  P  T  I  F  A  D  W  G  D  T  V  K  V  T  V  Y
aacgacctcaccacgagcggcaacggctcttccatccattggcacggtatccggcagaac
 N  D  L  T  T  S  G  N  G  S  S  I  H  W  H  G  I  R  Q  N
tacacgaaccagaatgatggtgtggtgtctattacgcaatgcccgattgcggtcggcgag
 Y  T  N  Q  N  D  G  V  V  S  I  T  Q  C  P  I  A  V  G  E
acctacacctacgagtggaaggccacgcagtacggctcttcctggtaccactctcacatt
 T  Y  T  Y  E  W  K  A  T  Q  Y  G  S  S  W  Y  H  S  H  I
ggcctgcaggcctgggaggtgttttcggtggtatcattatcaacggtcccgctactgca
 
G L Q A W E G V F G G I I I N G P A T A
aattacgacgaggacctcggcatcatgtttctcaatgattgggatcactcgactgttgac
N Y D E D L G I M F L N D W D H S T V D
gagctctacgattcagctcagagcagcggtcctcctacgcttgacaccggtctcattaac
E L Y D S A Q S S G P P T L D T G L I N
ggaaccaacatctacaatgactccggaacagttactggatctcgctgggaggccagcctg
G T N I Y N D S G T V T G S R W E A S L
accgagggtaccagctaccggctccgtcttgtcaacgctgctgtagactcgcacttcaaa
T E G T S Y R L R L V N A A V D S H F K
ttttcgatcgataaccacaccctccaggttatcgccatggacctggtccccattgagccc
F S I D N H T L Q V I A M D L V P I E P
tacgagactactgttctggacattggcatgggtcagcgctacgacgtcattgttacggca
Y E T T V L D I G M G Q R Y D V I V T A
gaccaggcttctgttgcttctgatttctggcttcgcgcaattccccagaccgcctgctcg
D Q A S V A S D F W L R A I P Q T A C S
gacaacgataacgcagatgatatcaagggcataatccactacggatcatcaactggtact
D N D N A D D I K G I I H Y G S S T G T
ccggaaaccactgcttatgattacactgatgcctgtgtcgatgaggacagctctgatctc
P E T T A Y D Y T D A C V D E D S S D L
gtcccgtatgtctctaagactgccacctccggtacctccctggccgaggctgtttccgtc
V P Y V S K T A T S G T S L A E A V S V
ggctacaactcggacaacctcttccgctggtacatgaacgagacctctatggaggtcgag
G Y N S D N L F R W Y M N E T S M E V E
tgggagaatccaacccttctgcaggtctacaacgacaatctgacgttcactgacacatcg
W E N P T L L Q V Y N D N L T F T D T S
ggtgttgttcaacttgacaccgcagaccaatggtacttcttcgtcatcgagaccgacaac
G V V Q L D T A D Q W Y F F V I E T D N
gctgtgccacacccaatccatcttcacggccacgacttcttcgtcctggctgcgggcacc
A V P H P I H L H G H D F F V L A A G T
ggctcttacagttcagacgttactctgactctggataaccctccccgccgcgacacggct
G S Y S S D V T L T L D N P P R R D T A
atgcttgactcctctggctacttggtcctggctttcgagaccgacaacccaggtgcgtgg
M L D S S G Y L V L A F E T D N P G A W
ttgatgcactgccacatcggctggcacaccagcgagggctttgcccttcagatcttggag
L M H C H I G W H T S E G F A L Q I L E
cgctacaccgagatccaggatagcctgatcgactacgacgtcctcaatgacacctgctcg
R Y T E I Q D S L I D Y D V L N D T C S
acttggtctacttactccgaggcaaactcgatcgaggaggaggactctggtgtgtaa
T W S T Y S E A N S I E E E D S G V -
SEQ ID NO : 57
   LENGTH : 578
   TYPE : PRT
   ORGANISM : *M. phaseolina* 
MRASYLSAAAFLGLSAAAPQAASTSSSSASANASSTSSAATSTCTGNTADDRTVWCDYDISTDYYND
GPDTGVTREYYFVVSDVTVSPDGISRSAMAVNGSIPGPTIFADWGDTVKVTVYNDLTTSGNGSSIHW
HGIRQNYTNQNDGVVSITQCPIAVGETYTYEWKATQYGSSWYHSHIGLQAWEGVFGGIIINGPATAN
YDEDLGIMFLNDWDHSTVDELYDSAQSSGPPTLDTGLINGTNIYNDSGTVTGSRWEASLTEGTSYRL
RLVNAAVDSHFKFSIDNHTLQVIAMDLVPIEPYETTVLDIGMGQRYDVIVTADQASVASDFWLRAIP
QTACSDNDNADDIKGIIHYGSSTGTPETTAYDYTDACVDEDSSDLVPYVSKTATSGTSLAEAVSVGY
NSDNLFRWYMNETSMEVEWENPTLLQVYNDNLTFTDTSGVVQLDTADQWYFFVIETDNAVPHPIHLH
GHDFFVLAAGTGSYSSDVTLTLDNPPRRDTAMLDSSGYLVLAFETDNPGAWLMHCHIGWHTSEGFAL
QILERYTEIQDSLIDYDVLNDTCSTWSTYSEANSIEEEDSGV*
SEQ ID NO : 58
   LENGTH : 2302 (including 150 bp 5' UTR and 150 bp 3' UTR)
   TYPE : DNA
   ORGANISM : *M. phaseolina* 
TGCGCCCCTCCCGCGTGCTGGGCACTCTTTTATAAAGGCAGCTCTCCACTCCTGCCCTGTGAATGCT
TGCTCAAGGGCGCTCAGGCATAGGTAAGTAGGGTAAGTATTGAGGCGTCTGGCTTTGCGGCGAATCT
TGGGACGATTTCAGCCATGACTCGCCTTCCGTTCGTCCTCGGGCTCGTGGCTACGGCTTTGGCCAAG
ACGGTCACTGTTGACTGGGACATTGGCTGGGTTTCCAGGGCTCCCGATGGATTCGAGCGGCCTGTCA
TCGCAATCAACGGCCAATGGCCGCTCCCTGTGCTGGAGGCCGACGTCAACGACACCATCATCGCCAC
TGTCCACAATTCTCTTGGCAACGAAACGACCAGCATCCACTGGCACGGCATGTGGCAGAGAGGCACG
CCCGAGCAAGACGGCGGGCTGGCGTCACGCAGTGTCCGATCCCGCCTGGCGAGACTTTCACGTACG
AGTTCAAAGCATACCCGGCCGGTACTTTCTGGTACCACTCGCATGACATGGGCCAGTATCCCGATGG
CCTGCGCGCACCCATGATCATCCATGACCCCGACTCCGAAACCCAGAAGAGCAGTGATGGTGAAGTC
GTGCTCTATGTGTCCGACTGGTACCACGACCAGATGCCGCCGCTTATCCACAGCTTCCTGACCACCC
CCAATTTCAACGGCGCGATGCCCAACCCGAACTCCAGCTTGATCAACGATCAGCAGTCCACGTCCAT
CAACATCCGTCCCGGCGAGAAGAAATACGTGCGCATCATCAACACGTCCGCCCTCGCCACGTACTAC
CTGCAGTTTGGTGGGTTCCGCTTCAGCCACCCCTCCCCTGCGGAAGTGACTGACGATTTCAGACCAA
CACAACATCACCGTTGTCGCAATTGACGGTGTTGACGGTCCGTACTACCCCTGTCGCTTATGCTGTG
GCTTCGTGCATACCCGTCGGCAGCTTCCCGCCGTTGCATGCCCGAGGCGATACAAGGTTTTAGTGCT
GACCGTAGTCGTGCAGTCGAACCGCAGAGCTGGAAGGCCCTGGAGATCGTCCCCGGCCAGCGGTACG
ACTTCATCATCGAAGGTCTCGAGAACCCCACAAGGAACTACGCATTCATCAACAAGATGGCTGTTCT
CGGTCTGCAGAACGTCAACAGCCTGGTCTACGACGAGTCCTTCGGCGAGCCGGAGTCGTTCAGCCTG
AGCTCTGGCGATCTCGGAAGTGATTTCACCCTGGTGCCTCTAGATCACGAGCCTCTCTTGGAATCCG
TGGACCACACCATCACGATGGAGGTCAATAATTTGAACATTGATGGCGTTGGCTTTCGGTACGGCTG
ATCCCGTGGCCGGGAGAAATCTTGCTCACATGTGCCACAGCATCACTCAAGGCCCGGACCCGTACAT
TTCACCCCGCACGCCTACCCTGTACACAGCCCTCAGCACCGGCTTCAACGCCACCGACCCAGAAATC
TACGGCCAGGTGAACCCCTACGTCGTCAACGCCGGTGAAGTCGTCCGGCTCGTCGTCAACAGCAACG
ATCTCGTCACCGCCAACAACTCTGGCCGCGGGCACCCCATGCATCTGCACGGCCACGTTTTCCAGGT
GGTCGGTCAGTTCTCCGAGCACTGGGACGGCAACACCTCGTCCTTCCCCGCCACGCCCATGAAGCGT
GACACCACCGTCCTATTCGCTGGCGGCAGCCTGGTGCTCCAGTTCCGTGCCGACAACCCCGGTGTTT
GGCTGTGTAAGTATTTTTTTTTCCGATGCCCATGGTGCCTGGACGTGAGCTGATGAGGATGGGCCTT
GCAGTTCACTGCCATATTGAGTGGCACCTAGACGCCGGCATGTCCGCTACAATCATCGAGGCGCCGC
TCGACTTGCAGCGCGAGGGCATCAAGATCCCCCAGCAGCATCTTGAATCGTGCAGAGCCTTGAACTT
GACCACTCAAGGCAATTGCGCCGGCAACACCGCCAACCTGGATGACACTGCCGCCTGCAGGGTCTAC
GACACCGATCCATGGGGGTAGGTCATATCGGAGTTTAATTGAGCTTGAAGGACATGTGCTGACCGTT
GCGTTTAGTGCGCTTATCACGGATGACGGTGGTGGAAATAGTACCCTGAACGGAACCACTTATAAGA
GAATTTAGTGTGTAATCTTAATACGTCTTCACATGTACACAACCTATGCATTGTATTCAAATTCTAC
ATAAGCACTTCGACAGTAGTCTGAGTTGACTGACTAGGCTCGTGGACGGAGGACGCCGTTTCCTCGT
CTTTGCCTGCTAAATTTTCGCGAT
SEQ ID NO : 59
   LENGTH : 1665
   TYPE : DNA
   ORGANISM : *M. phaseolina*
   FEATURE NAME/KEY: CDS
   LOCATION : (1)......(1665) 
atgactcgccttccgttcgtcctcgggctcgtggctacggctttggccaagacggtcact
 M  T  R  L  P  F  V  L  G  L  V  A  T  A  L  A  K  T  V  T
gttgactgggacattggctgggtttccagggctcccgatggattcgagcggcctgtcatc
 V  D  W  D  I  G  W  V  S  R  A  P  D  G  F  E  R  P  V  I
gcaatcaacggccaatggccgctccctgtgctggaggccgacgtcaacgacaccatcatc
 A  I  N  G  Q  W  P  L  P  V  L  E  A  D  V  N  D  T  I  I
gccactgtccacaattctcttggcaacgaaacgaccagcatccactggcacggcatgtgg
 A  T  V  H  N  S  L  G  N  E  T  T  S  I  H  W  H  G  M  W
cagagaggcacgcccgagcaagacggcggggctggcgtcacgcagtgtccgatcccgcct
 Q  R  G  T  P  E  Q  D  G  G  A  G  V  T  Q  C  P  I  P  P
 
ggcgagactttcacgtacgagttcaaagcatacccggccggtactttctggtaccactcg
 G  E  T  F  T  Y  E  F  K  A  Y  P  A  G  T  F  W  Y  H  S
catgacatgggccagtatcccgatggcctgcgcgcacccatgatcatccatgaccccgac
 H  D  M  G  Q  Y  P  D  G  L  R  A  P  M  I  I  H  D  P  D
tccgaaacccagaagagcagtgatggtgaagtcgtgctctatgtgtccgactggtaccac
 S  E  T  Q  K  S  S  D  G  E  V  V  L  Y  V  S  D  W  Y  H
gaccagatgccgccgcttatccacagcttcctgaccacccccaatttcaacggcgcgatg
 D  Q  M  P  P  L  I  H  S  F  L  T  T  P  N  F  N  G  A  M
cccaacccgaactccagcttgatcaacgatcagcagtccacgtccatcaacatccgtccc
 P  N  P  N  S  S  L  I  N  D  Q  Q  S  T  S  I  N  I  R  P
ggcgagaagaaatacgtgcgcatcatcaacacgtccgccctcgccacgtactacctgcag
 G  E  K  K  Y  V  R  I  I  N  T  S  A  L  A  T  Y  Y  L  Q
tttgaccaacacaacatcaccgttgtcgcaattgacggtgttgacgtcgaaccgcagagc
 F  D  Q  H  N  I  T  V  V  A  I  D  G  V  D  V  E  P  Q  S
tggaaggccctggagatcgtccccggccagcggtacgacttcatcatcgaaggtctcgag
 W  K  A  L  E  I  V  P  G  Q  R  Y  D  F  I  I  E  G  L  E
aaccccacaaggaactacgcattcatcaacaagatggctgttctcggtctgcagaacgtc
 N  P  T  R  N  Y  A  F  I  N  K  M  A  V  L  G  L  Q  N  V
aacagcctggtctacgacgagtccttcggcgagccggagtcgttcagcctgagctctggc
 N  S  L  V  Y  D  E  S  F  G  E  P  E  S  F  S  L  S  S  G
gatctcggaagtgatttcaccctggtgcctctagatcacgagcctctcttggaatccgtg
 D  L  G  S  D  F  T  L  V  P  L  D  H  E  P  L  L  E  S  V
gaccacaccatcacgatggaggtcaataatttgaacattgatggcgttggctttcgcatc
 D  H  T  I  T  M  E  V  N  N  L  N  I  D  G  V  G  F  R  I
actcaaggcccggacccgtacatttcaccccgcacgcctaccctgtacacagccctcagc
 T  Q  G  P  D  P  Y  I  S  P  R  T  P  T  L  Y  T  A  L  S
accggcttcaacgccaccgacccagaaatctacggccaggtgaacccctacgtcgtcaac
 T  G  F  N  A  T  D  P  E  I  Y  G  Q  V  N  P  Y  V  V  N
gccggtgaagtcgtccggctcgtcgtcaacagcaacgatctcgtcaccgccaacaactct
 A  G  E  V  V  R  L  V  V  N  S  N  D  L  V  T  A  N  N  S
ggccgcgggcaccccatgcatctgcacggccacgttttccaggtggtcggtcagttctcc
 G  R  G  H  P  M  H  L  H  G  H  V  F  Q  V  V  G  Q  F  S
gagcactgggacggcaacacctcgtccttccccgccacgcccatgaagcgtgacaccacc
 E  H  W  D  G  N  T  S  S  F  P  A  T  P  M  K  R  D  T  T
gtcctattcgctggcggcagcctggtgctccagttccgtgccgacaaccccggtgtttgg
 V  L  F  A  G  G  S  L  V  L  Q  F  R  A  D  N  P  G  V  W
ctgtttcactgccatattgagtggcacctagacgccggcatgtccgctacaatcatcgag
 L  F  H  C  H  I  E  W  H  L  D  A  G  M  S  A  T  I  I  E
gcgccgctcgacttgcagcgcgagggcatcaagatccccagcagcatcttgaatcgtgc
 A  P  L  D  L  Q  R  E  G  I  K  I  P  Q  Q  H  L  E  S  C
agagccttgaacttgaccactcaaggcaattgcgccggcaacaccgccaacctggatgac
 R  A  L  N  L  T  T  Q  G  N  C  A  G  N  T  A  N  L  D  D
actgccgcctgcagggtctacgacaccgatccatggggtgcgcttatcacggatgacggt
 T  A  A  C  R  V  Y  D  T  D  P  W  G  A  L  I  T  D  D  G
ggtggaaatagtaccctgaacggaaccacttataagagaatttag
 G  G  N  S  T  L  N  G  T  T  Y  K  R  I  -
SEQ ID NO : 60
   LENGTH : 554
   TYPE : PRT
   ORGANISM : *M. phaseolina* 
MTRLPFVLGLVATALAKTVTVDWDIGWVSRAPDGFERPVIAINGQWPLPVLEADVNDTIIATVHNSL
GNETTSIHWHGMWQRGTPEQDGGAGVTQCPIPPGETFTYEFKAYPAGTFWYHSHDMGQYPDGLRAPM
IIHDPDSETQKSSDGEVVLYVSDWYHDQMPPLIHSFLTTPNFNGAMPNPNSSLINDQQSTSINIRPG
EKKYVRIINTSALATYYLQFDQHNITVVAIDGVDVEPQSWKALEIVPGQRYDFIIEGLENPTRNYAF
 
INKMAVLGLQNVNSLVYDESFGEPESFSLSSGDLGSDFTLVPLDHEPLLESVDHTITMEVNNLNIDG
VGFRITQGPDPYISPRTPTLYTALSTGFNATDPEIYGQVNPYVVNAGEVVRLVVNSNDLVTANNSGR
GHPMHLHGHVFQVVGQFSEHWDGNTSSFPATPMKRDTTVLFAGGSLVLQFRADNPGVWLFHCHIEWH
LDAGMSATIIEAPLDLQREGIKIPQQHLESCRALNLTTQGNCAGNTANLDDTAACRVYDTDPWGALI
TDDGGGNSTLNGTTYKRI*
SEQ ID NO : 61
   LENGTH : 2460 (including 150 bp 5' UTR and 150 bp 3' UTR)
   TYPE : DNA
   ORGANISM : M. phaseolina 
CTCCGTATCTACTGCTGACTTCACATCCACAGCATGCAAGGACTCGAGGGCTTCTGATTTAACGAGC
ACTCACTTGGCTTTTGTCTGCGTTTTCTTTTCGTTTTCCTACTATTTCATCTTGATCCATTCGTTCT
CGCCGTCGCTTTCCAGATGTCTTTCGTTTCGAAAATTTCACAGGCCTCATCGCTTTTACCGCTGGC
TTAGGCCAAGATGAGACTAATGGGTGAGTATGCTGAACCTTGCGCAGGATATCTGAATAATAGTTGT
AGGGTTTCGAAATGGGGCACGTTCGATGCACCGAGGCTCCAGAAGTTTCTGGATCGGGTGAGGTTAT
TCTGAAACATTACTGAGCTCGGCACTGATTGGTCGGCAGGGTCATGGCATTCCCTGGGGCGGTATGA
CTTGTACCAACGCCAACCCGTATACTGAGTAGGACTCGGCCCGTGGTCCACAATCTTCTCTGCGCTG
ACGTTCGGAAAGAGCTCCAGACACAGGACAGACTGTGCGGTATGATTTCACAGTCCAGCGGCATCCG
GTGTCTCCGGACGGCTACAAGAAGAACGTTTTGCTTGTGAACGGGCAATTTCCAGGCCCGCTCATGG
AAGCTAACTGGGGAGACACAATTGAAGGTAGGATGAGAGTAAATACGGGCAGCAACAACTGCTGACA
GATACAGTGACTGTGCACAACAACATAGCCGGACCTGAGGAAGGCACACAAATCCACTGGCACGGCT
TCACGCAGAGAGGGACGCCGTTCATGGATGGTATCCCTTCCGTATCAAGCTGCCCCATTGCGCCCAA
CAATACCTTTGTGTATACCTTCAAGGCAGACCTTTACGGCACTGGCTGGTACCACTCTCATTACTCT
GGGCAATCCACCGGCGGCCTCCTCGGCCCAATCGTCGTCCATGGTCCCAGTGCGCTTGACTATGATA
TTGACCTTGGCCCTGTGTTTTTGAATGACTGGTACCACAAGGACTACTTGCAGCTTATTGACGGTGG
TGAGACTTTCCCGTAGGTCAAAGGGCTCGATAGCTGAAACCTCCAACAGTCGTCGGAACGGACCCCA
GCCTATGGCATCCCAAGGCGGACAACAACATGATAAACGGGAAGATGGACTACGATTGCTCCCTTGT
CACTGACGGCACGCCTTGCGTCTCTAATGCCGGCTTGGCCACGTTCAGCTTCACCAAGGGCGCCACG
CACCGTCTCAGGCTCATAAACGGAGGATCCGCCTCCCTGCAGCACTTCAGTATCGATGGACACGAGA
TGACAGTCATTTCCAATGACTTCGTAGCCGTAGAGCCATACCAGACGAAGACAGTGACTCTTGCAGT
AAGCCGCCCGATTTTTCCAGATATCCCACATTACTAACACCCACCAGGTCGGCCAGCGGACAGACGT
CCTCGTCACGGCCAACGGCGACGCCACCGGCGCCTACTGGATGCGCAGCACCGTCGCGGACGATGAA
TCCTGCAACTGGTCCAACCAGCCCGCCGCGCTCGCCGCAGTCTACTACGACGCGGCAAACCCCACCG
TCAAGCCCAACAGCACCGGCTGGCCCCCCGTCGCCAACCAGCAAGGCAGCTGCGACAACGACCCGCT
TACCCAAACCATCCCCCTCTTCCCCATCCCCGCGGACCCCAGCCCCTCAACGACGCTTGAGCTCGAC
TTCGGCTGGACGCAAAACGCAACCGGACACCAAGTCTGGACGGTCAACGACCGCGGCTTCCGCGGCA
ACTACAACCGCCCCGTGCTGCAGCTCGCCGCGGGCTCGGACACCGCGTCGTCCGCGTACGCGTGGGA
GCCCGAATGGAACGTCTACGACACGGGCCGCAACCGCACCGTCCGCATCGTCATGCACAACAACTCC
TCCATGTACCACGTACGTGAGCCCTTGTCTCCCCCCCCCCCCCCCTCCCCTCTCGCTAATGGAAAG
ACACATGCAGCCCATGCACCTCCACGGGCACAACGCGCAGATCCTCGCCGCGGGCGCCAACGGCCCC
TGGGACGGCCGCACCGTCGCGCGCCCCGCCAACCCGGCCCGCCGCGACGTCTACCAGCTCCCGCCGA
ACGGGCACCTGGTGATCCAGTACGCGCAGGACAACCCGGGCGTGTGGCCGCTGCACTGCCACATCGC
GTGGCACGCGAGCGCGGGCATGTTTGCGAGCGTGCTGGAGCGGGCGGGGGACATTGTGGGGAGCAGC
GGGGCCGGCGGGTGGAGGGAGGAGATGGCGGGCGTGTGTGCCGGGTGGAGGCGTACACGCGGATGA
ACGTGGTGGAGCAGGTGGACAGTGGGGTGTGAGTTGGGAGCAAGGAGGGGGGTTTGAGATGCGCGTA
TATGGTAAGCGGTTGGGGTTCTTGTTTGACTCGATTTGCTCTGCAGGCGGTGTCTCTTTTCGACTGT
GGCGCGTGCAATCTCGTTTCGTGCATCCTGGGTGGATAGGGAGCGGCG
SEQ ID NO : 62
   LENGTH : 1803
   TYPE : DNA
   ORGANISM : M. phaseolina
   FEATURE NAME/KEY : CDS
   LOCATION : (1)......(1803) atgtctttcgtttcgaaaattttcacaggcctcatcgcttttaccgctggcttaggccaa 
M S F V S K I F T G L I A F T A G L G Q
gatgagactaatggggtttcgaaatggggcacgttcgatgcaccgaggctccagaagttt
 D E T N G V S K W G T F D A P R L Q K F
ctggatcggggtcatggcattccctgggcggtatgacttgtaccaacgccaacccgtat
 L D R G H G I P W G G M T C T N A N P Y
actgaagctccagacacaggacagactgtgcggtatgatttcacagtccagcggcatccg
 T E A P D T G Q T V R Y D F T V Q R H P
gtgtctccggacggctacaagaagaacgttttgcttgtgaacggcaatttccaggcccg
 V S P D G Y K K N V L L V N G Q F P G P
ctcatggaagctaactggggagacacaattgaagtgactgtgcacaacaacatagccgga
 L M E A N W G D T I E V T V H N N I A G
cctgaggaaggcacacaaatccactggcacggcttcacgcagagaggacgccgttcatg
 P E E G T Q I H W H G F T Q R G T P F M
gatggtatcccttccgtatcaagctgccccattgcgcccaacaatacctttgtgtatacc
 D G I P S V S S C P I A P N N T F V Y T
ttcaaggcagacctttacggcactggctggtaccactctcattactctgggcaatccacc
 F K A D L Y G T G W Y H S H Y S G Q S T
ggcggcctcctcggcccaatcgtcgtccatggtcccagtgcgcttgactatgatattgac
 G G L L G P I V V H G P S A L D Y D I D
cttggccctgtgttttgaatgactggtaccacaaggactacttgcagcttattgacggt
 L G P V F L N D W Y H K D Y L Q L I D G
gtcgtcggaacggaccccagcctatggcatcccaaggcggacaacaacatgataaacggg
 V V G T D P S L W H P K A D N N M I N G
aagatggactacgattgctcccttgtcactgacggcacgccttgcgtctctaatgccggc
 K M D Y D C S L V T D G T P C V S N A G
ttggccacgttcagcttcaccaaggcgccacgcaccgtctcaggctcataaacggagga
 L A T F S F T K G A T H R L R L I N G G
tccgcctccctgcagcacttcagtatcgatggacacgagatgacagtcatttccaatgac
 S A S L Q H F S I D G H E M T V I S N D
ttcgtagccgtagagccataccagacgaagacagtgactcttgcagtcggccagcggaca
 F V A V E P Y Q T K T V T L A V G Q R T
gacgtcctcgtcacggccaacggcgacgccaccggcgcctactggatgcgcagcaccgtc
 D V L V T A N G D A T G A Y W M R S T V
gcggacgatgaatcctgcaactggtccaaccagcccgccgcgctcgccgcagtctactac
 A D D E S C N W S N Q P A A L A A V Y Y
gacgcggcaaaccccaccgtcaagcccaacagcaccggctggcccccgtcgccaaccag
 D A A N P T V K P N S T G W P P V A N Q
caaggcagctgcgacaacgacccgcttacccaaaccatccccctcttccccatccccgcg
 Q G S C D N D P L T Q T I P L F P I P A
gaccccagcccctcaacgacgcttgagctcgacttcggctggacgcaaaacgcaaccgga
 D P S P S T T L E L D F G W T Q N A T G
caccaagtctggacggtcaacgaccgcggcttccgcggcaactacaaccgccccgtgctg
 H Q V W T V N D R G F R G N Y N R P V L
cagctcgccgcgggctcggacaccgcgtcgtccgcgtacgcgtgggagcccgaatggaac
 Q L A A G S D T A S S A Y A W E P E W N
gtctacgacacgggccgcaaccgcaccgtccgcatcgtcatgcacaacaactcctccatg
 V Y D T G R N R T V R I V M H N N S S M
taccaccccatgcacctccacggcacaacgcgcagatcctcgccgcgggcgccaacggc
 Y H P M H L H G H N A Q I L A A G A N G
ccctgggacggccgcaccgtcgcgcgccccgccaacccggcccgccgcgacgtctaccag
 P W D G R T V A R P A N P A R R D V Y Q
ctcccgccgaacggcacctggtgatccagtacgcgcaggacaacccgggcgtgtggccg
 L P P N G H L V I Q Y A Q D N P G V W P
ctgcactgccacatcgcgtggcacgcgagcgcgggcatgtttgcgagcgtgctggagcgg
 L H C H I A W H A S A G M F A S V L E R
 
gcgggggacattgtggggagcagcggggccggcgggtggagggaggagatggcgggcgtg
 A  G  D  I  V  G  S  S  G  A  G  G  W  R  E  E  M  A  G  V
tgtgccgggtgggaggcgtacacgcggatgaacgtggtggagcaggtggacagtggggtg
 C  A  G  W  E  A  Y  T  R  M  N  V  V  E  Q  V  D  S  G  V
tga
 -
SEQ ID NO : 63
   LENGTH : 600
   TYPE : PRT
   ORGANISM : M. phaseolina 
MSFVSKIFTGLIAFTAGLGQDETNGVSKWGTFDAPRLQKFLDRGHGIPWGGMTCTNANPYTEAPDTG
QTVRYDFTVQRHPVSPDGYKKNVLLVNGQFPGPLMEANWGDTIEVTVHNNIAGPEEGTQIHWHGFTQ
RGTPFMDGIPSVSSCPIAPNNTFVYTFKADLYGTGWYHSHYSGQSTGGLLGPIVVHGPSALDYDIDL
GPVFLNDWYHKDYLQLIDGVVGTDPSLWHPKADNNMINGKMDYDCSLVTDGTPCVSNAGLATFSFTK
GATHRLRLINGGSASLQHFSIDGHEMTVISNDFVAVEPYQTKTVTLAVGQRTDVLVTANGDATGAYW
MRSTVADDESCNWSNQPAALAAVYYDAANPTVKPNSTGWPPVANQQGSCDNDPLTQTIPLFPIPADP
SPSTTLELDFGWTQNATGHQVWTVNDRGFRGNYNRPVLQLAAGSDTASSAYAWEPEWNVYDTGRNRT
VRIVMHNNSSMYHPMHLHGHNAQILAAGANGPWDGRTVARPANPARRDVYQLPPNGHLVIQYAQDNP
GVWPLHCHIAWHASAGMFASVLERAGDIVGSSGAGGWREEMAGVCAGWEAYTRMNVVEQVDSGV*
SEQ ID NO : 64
   LENGTH : 2285 (including 150 bp 5' UTR and 150 bp 3' UTR)
   TYPE : DNA
   ORGANISM : *M. phaseolina* 
TGAAGCCCTCTCTGACGCAGGTATCTATTTATTAAACCAGTTTCACCCTCCGGTCGGGTAAGTATT
TGCTCAAACAAACTTGTCCAAGTGTCAGTTAGGTAAGCTAGGAGGCTCACGAATTCAAGAGACTCTC
CAGAGATATCTTCGCCATGATTCGCCTTTCTCTCGTGCTCGGCTTCATGGCCACGACACTGGCCAAG
ACCGTCACTCTTAACTGGGATATTGGGTGGGTTTCTGCGGCTCCAGATGGATTTACACGGCCCGTCA
TTGGCATCAACGGGGAGTGGCCGCCCCcGTTTTGGAAGCCGACGTCAACGACACTATTATAGTAAT
TACGCGGAACCTCCTGCGTAACGAGACGACGAGCTTGCACTGGCATGGTATGTGGCACTATAACTCG
ACCCACATGGACGGCGGAGCCAGGATTTCACAGTGTGAAATCCCTCCGGGGGGACATTCACGTACA
AGTTCAAGGCGTACCCGGCCGGCACCTTTTGGTATCATTCTCACGATATGGGCCAATATCCCGACGG
CCTGCGCGCCCCAATGATCATTCACGACCCTAAGGCTGCCGCGGAGCGGGACACTGATAAGGAGTAC
GTACTTACGGTCTCCGACTGGTACCGTGACCAGATGCCGTCACTTATCCACCGCTACTTGACAACTT
CCACTTATAATAGTACTATGCCAAATCCAAACTCGAGCTTGATCAACGATCAGCAGTCTACAACGCT
AAACATCCGCCCCGGGCAGAAGATATATGTTCGGATTATTAACATGTCAGCCCTCGCAACGTACTAT
CTACAGTTCGGTAGGTGTATCCATATGGCCCTGTTACACGGACGAAGCTAACGATTAAGATCAACAC
CACTTGACTGTTATTGCCATTGACGGTGTCGACGGTTGGTAACTCTATTACCTATTCTGCGCTAAGA
TGCATGTCGGCATATGTGCCGTTAGCTGCATGCAAACGCGTTACAAGGTTAAGTGCTAACAACGGGC
AGTCGATCCCCAGACTTGGGAGGCTCTGGAGATTATCCCTGGACAGCGGTACGACGTCATTATCACC
GGCCTAGAGAACCCCGAAAGGAACTATGCATTTATCAACAAGATGGCTACTCTTGGCTTCCAGAACA
ATAACGTCCTTAGTTACGATTCGTCCTGGCCTGTCCCGGAGCCGTTGAACGTGGGCAGCTTCAATCT
TAGAAGCGATTTCAACCTGACCCCGCTTGATGAGGAGCTACTACTGGAGCCCGTGGACCACACCTTC
ACCATGGAGGTCAATAACGTGAACGTCGACGGCGTAGGCTCCCGGTGAGATATTCCCTACCCCCCCG
GCCCCCGGGCGGGTCCCGAAGTCGGTATGAGGCTTACTCACTCCAATCCGCAGCATCACGCAGGGA
CCGGACCCTTACATCGCCCCGCGTACGCCCACCCTATACACCACCCTGAGCACCGGCTCTAATGCTA
TTAACCCCGCCATATACGGCCAGGCGAACGCTTACGTAGTGGAAGCCGGCGATATTGTCCAGCTTGT
CGTCAATAGTAACGAACCCGTCACTACCAACACTTCCGGTCGTGGGCACCCTATGCACTTGCACGGC
CACACCTTCCAAGTGGTTGGCCAATATGGCAGCCCTTGGGACGGCGATGCCTCTAAATTCCCTGCTG
TTCCTATGAAGCGGGATACCACTGTTCTGTTTACTGGCGGGAGCTTGGTGATCCGGTTCCGCGCGGA
CAATCCTGGAGTTTGGATGTGTACGGCTCCGCTTTGCTCGAAGACCCGCGTTTGAAAGCGTTGACTG
ACCTGTAACTCGCAGTCCATTGCCACAACGAATGGCACCTTGACGCCGGCATGGCTGGAACGATTAT
CGAAGCGCCACTCGAGCTTCAACAAAGCGGTCTGACGATTCCGCCGCAGCACCTCGCGTCGTGCAGG
GCGTTAAACTTAACGACCCGGGGCAATTGTGCCGGTAATACTGCGAACCTAGAGGATACGGCTGCAT
GCAGAGTCTACGACACTGAGCCTTGGGGGTGAGTTACTCCATTATTGTTTTGTTGTTATAACCCTTG
 
CTGATGACAATACTTCTCTAGTGCACTTATCAAGAGAGATGAGGAAACAGCATATTAAATAGCACGC
TGGCATAGCACCTGTAGATGTAGACTGATTTCTAGTATTTATTACCTGTGTACACTTGAAGAAAATT
GTTGTAAAATGATATGTCCCCATGCAACTGAACATGGCCTACGTGGCGAGAGTTTATCAGGCGCCTT
CTGCTCC
SEQ ID NO : 65
   LENGTH : 1635
   TYPE : DNA
   ORGANISM : *M. phaseolina*
   FEATURE NAME/KEY: CDS
   LOCATION : (1)......(1635) 
atgattcgcctttctctcgtgctcggcttcatggccacgacactggccaagaccgtcact
 M  I  R  L  S  L  V  L  G  F  M  A  T  T  L  A  K  T  V  T
cttaactgggatattgggtgggtttctgcggctccagatggatttacacggccgtcatt
 L  N  W  D  I  G  W  V  S  A  A  P  D  G  F  T  R  P  V  I
ggcatcaacggggagtggccgcccccgtttggaagccgacgtcaacgacactattata
 G  I  N  G  E  W  P  P  P  V  L  E  A  D  V  N  D  T  I  I
gtaattacgcggaacctcctgcgtaacgagacgacgagcttgcactggcatggtatgtgg
 V  I  T  R  N  L  L  R  N  E  T  T  S  L  H  W  H  G  M  W
cactataactcgacccacatggacggcggagccaggatttcacagtgtgaaatccctccg
 H  Y  N  S  T  H  M  D  G  G  A  R  I  S  Q  C  E  I  P  P
gggggacattcacgtacaagttcaaggcgtacccggccggcacctttggtatcattct
 G  G  T  F  T  Y  K  F  K  A  Y  P  A  G  T  F  W  Y  H  S
cacgatatgggccaatatcccgacggcctgcgcgccccaatgatcattcacgaccctaag
 H  D  M  G  Q  Y  P  D  G  L  R  A  P  M  I  I  H  D  P  K
gctgccgcggagcgggacactgataaggagtacgtacttacggtctccgactggtaccgt
 A  A  A  E  R  D  T  D  K  E  Y  V  L  T  V  S  D  W  Y  R
gaccagatgccgtcacttatccaccgctacttgacaacttccacttataatagtactatg
 D  Q  M  P  S  L  I  H  R  Y  L  T  T  S  T  Y  N  S  T  M
ccaaatccaaactcgagcttgatcaacgatcagcagtctacaacgctaaacatccgcccc
 P  N  P  N  S  S  L  I  N  D  Q  Q  S  T  T  L  N  I  R  P
gggcagaagatatatgttcggattattaacatgtcagccctcgcaacgtactatctacag
 G  Q  K  I  Y  V  R  I  I  N  M  S  A  L  A  T  Y  Y  L  Q
ttcgatcaacaccacttgactgttattgccattgacggtgtcgacgtcgatccccagact
 F  D  Q  H  H  L  T  V  I  A  I  D  G  V  D  V  D  P  Q  T
tgggaggctctggagattatccctggacagcggtacgacgtcattatcaccggcctagag
 W  E  A  L  E  I  I  P  G  Q  R  Y  D  V  I  I  T  G  L  E
aaccccgaaaggaactatgcatttatcaacaagatggctactcttggcttccagaacaat
 N  P  E  R  N  Y  A  F  I  N  K  M  A  T  L  G  F  Q  N  N
aacgtccttagttacgattcgtcctggcctgtcccggagccgttgaacgtgggcagcttc
 N  V  L  S  Y  D  S  S  W  P  V  P  E  P  L  N  V  G  S  F
aatcttagaagcgatttcaacctgaccccgcttgatgaggagctactactggagcccgtg
 N  L  R  S  D  F  N  L  T  P  L  D  E  E  L  L  L  E  P  V
gaccacaccttcaccatggaggtcaataacgtgaacgtcgacggcgtaggctcccgcatc
 D  H  T  F  T  M  E  V  N  N  V  N  V  D  G  V  G  S  R  I
acgcagggaccggacccttacatcgccccgcgtacgcccaccctatacaccaccctgagc
 T  Q  G  P  D  P  Y  I  A  P  R  T  P  T  L  Y  T  T  L  S
accggctctaatgctattaaccccgccatatacggccaggcgaacgcttacgtagtggaa
 T  G  S  N  A  I  N  P  A  I  Y  G  Q  A  N  A  Y  V  V  E
gccggcgatattgtccagcttgtcgtcaatagtaacgaacccgtcactaccaacacttcc
 A  G  D  I  V  Q  L  V  V  N  S  N  E  P  V  T  T  N  T  S
ggtcgtgggcaccctatgcacttgcacggccacaccttccaagtggttggccaatatggc
 G  R  G  H  P  M  H  L  H  G  H  T  F  Q  V  V  G  Q  Y  G
agcccttgggacggcgatgcctctaaattccctgctgttcctatgaagcgggataccact
 S  P  W  D  G  D  A  S  K  F  P  A  V  P  M  K  R  D  T  T
 
gttctgtttactggcgggagcttggtgatccggttccgcgcggacaatcctggagtttgg
 V  L  F  T  G  G  S  L  V  I  R  F  R  A  D  N  P  G  V  W
atgttccattgccacaacgaatggcaccttgacgccggcatggctggaacgattatcgaa
 M  F  H  C  H  N  E  W  H  L  D  A  G  M  A  G  T  I  I  E
gcgccactcgagcttcaacaaagcggtctgacgattccgccgcagcacctcgcgtcgtgc
 A  P  L  E  L  Q  Q  S  G  L  T  I  P  P  Q  H  L  A  S  C
agggcgttaaacttaacgacccggggcaattgtgccggtaatactgcgaacctagaggat
 R  A  L  N  L  T  T  R  G  N  C  A  G  N  T  A  N  L  E  D
acggctgcatgcagagtctacgacactgagccttggggtgcacttatcaagagagatgag
 T  A  A  C  R  V  Y  D  T  E  P  W  G  A  L  I  K  R  D  E
gaaacagcatattaa
 E  T  A  Y  -
SEQ ID NO : 66
   LENGTH : 544
   TYPE : PRT
   ORGANISM : *M. phaseolina* 
MIRLSLVLGFMATTLAKTVTLNWDIGWVSAAPDGFTRPVIGINGEWPPPVLEADVNDTIIVITRNLL
RNETTSLHWHGMWHYNSTHMDGGARISQCEIPPGGTFTYKFKAYPAGTFWYHSHDMGQYPDGLRAPM
IIHDPKAAAERDTDKEYVLTVSDWYRDQMPSLIHRYLTTSTYNSTMPNPNSSLINDQQSTTLNIRPG
QKIYVRIINMSALATYYLQFDQHHLTVIAIDGVDVDPQTWEALEIIPGQRYDVIITGLENPERNYAF
INKMATLGFQNNNVLSYDSSWPVPEPLNVGSFNLRSDFNLTPLDEELLLEPVDHTFTMEVNNVNVDG
VGSRITQGPDPYIAPRTPTLYTTLSTGSNAINPAIYGQANAYVVEAGDIVQLVVNSNEPVTTNTSGR
GHPMHLHGHTFQVVGQYGSPWDGDASKFPAVPMKRDTTVLFTGGSLVIRFRADNPGVWMFHCHNEWH
LDAGMAGTIIEAPLELQQSGLTIPPQHLASCRALNLTTRGNCAGNTANLEDTAACRVYDTEPWGALI
KRDEETAY*
SEQ ID NO : 67
   LENGTH : 2412 (including 150 bp 5' UTR and 150 bp 3' UTR)
   TYPE : DNA
   ORGANISM : *M. phaseolina* 
GGGAGACGATCTGGAAGTGTTTGTTCAGAATGATTTGGCCGTTGAAACCACCATTCATTGGCACGGT
CGGTATCCATGGTATACTTTTACGAAGGCTAGTCAGCTGAAGGAGAAGTTCGGCAGGCATCCTTCA
GCAAGGAACACCACACATGGACGGAGTTCCGGGAGTAACACAGGTTGGTTGATGACCTTCTATCGGG
ATGACTAGGGCCTAACTTTTGGCGCAGGAGCCGATCCCACCAGGAGGAAACTTCACGTACCGCTTCT
CGCTCAAAAACGAATACGGCTTCTACTGGTATCACTCGCATTTCCGAGCATACTCGGACGACGCCAT
CCGCGGACCACTAGTCATCCATCCATCCTCACAACGCCCCCGGCCATACGAGACTCTCGCGAGGAAC
CAGACTGAGCTTACTGCTTTGCAAGAAGCTGAACGCGAAGCGGTGCCTATCCTCCTATCTGACTGGT
ACCATCGCGTTTCGGACGACATCTTCAACGAATACCTAACAACAGGCGCGTTCCCCAGTTGCGTGGA
CAGTCTCCTCGCCAATGGATACGGAAGGGTGCGGTGTCTACCGGAATACATTCTGGCAGCCGGAGCT
GGGCTAGGCATGGAGCCTGCACCTGTCAATGCTACCGCGACTAGCATCGGCACAACGCCCATGTCTT
CCATGGCGATGGGTACAAAGTACATGGAGACCAATACCAAGGAGTCGATGCGTATGGAGTCTATGGC
ACTAGACACTACGTCCATGGAGCATCACATGCGACGCATGGATTCCATGTCAGCGGAGGATATGTCC
ATGAATACGATGACTGCTCATTCTACGCCTGCGGCTGCGCCCGACTCAGGCATGCCGATGGGCTCCA
TGTCAATGGCTTCTCTACCCGTATCAGGCATATCGGGCACGTCGGGCCTGTCGGGCATGTCAAATAT
GGCGAGCGGTCCCCTTGGTCCTCGCGGCTGCAGTGCTCCCATGATGTTCAGACCGGGCTACAACATC
AGCTCTCTCCCGCCAGAGACTTGCACAGACACGTCAGCGCCGCTGCTGACTGTCGATGCAAACTATA
CACGAGGCTGGCTTGCATTAAACCTAGTCAACTCCGGTTCAGTAACCAAGCTCAGCGTCTCACTAGA
CGCCCATTCGATGTTTGTCTATGCGGCCGACGGATTCTTTGTGAAACCCCAAGAAGTAGAAGTAAGT
TGCACCTCCCCCTAACTTGTCGTCGGAAGACTTACCGCGTCTAGGTATTACAAATCTCAATCGGGCA
ACGGTACTCAGTAATGATCAAACTTAATCAACGGCCCGGAAACTACACTCTACGGTTTGCGTCGTAT
CCCTACGGCGATATGCAACAGGTTATCGAAGGCCAAGCGACCGTCTCATATAAGGTAAGCAGCAAGC
TTTTCCCCCACTCGGTATAGAACTAACTTCGTTAGGTTGATGCCGCAGAGGACATTATGCCGGTGGA
CTTGACAAACGACCCTACTGCAACGTGGATGCTTGTCAACGGTTCGGCAAAGTCGAACGCTTCTGAA
TTGAAGACGGACATGCTCGCCCCGTTCGAGGCAATTGCGCCGCCATCCCAAGCGGATATAACTTACG
 
ACTTCACGATCAGTCAGACAGAGATCGTAACTTGGGTGTTAAACGGATATCCCTATTCGGAACCTTC
GACGCCTATTATCTACGGCAATGCGTCGGAAGCGTGGAACGCAAATACTACAATCCGCATTCCTTCC
AACTCGACCGTAGACATTATAATGCGGATTGCCAACGACTCAATGGATACGGCAAGTTGCAGTCCAG
GCCGCCGTCAAAACGCCTGCATAAGCTAACCCGATAATGCCGTAGATGGGTCATCCTATGCACCTTC
ACGGCCACCGGTTCTTCGCCCTCGGCTCTGGATCGGGCTCCTTCCCATATCAGAACGCCGTCGACGC
GCCTCCATCCCTCATTAACCTCGAAAACCCTCCGTACCGGGACACAACCGATTTACCACCTTCAGGC
TGGGCAGTCATTCGCTATGTAGCCAACAATCCAGGCGCATGGATGTTTCACTGCCACATCCAGTGGC
ACCTCGTGAGCGGCATGGCATTGGTGTTTGTCGAAGGAGAAGAGCAGCTGCCTGGTTTGGTGGGTGC
GGCTGCGAACGGAACAAGCAATGCGAATAGCGCGTCACCGGCGCGTAGCACTCGAGAGCATGCGGCC
TTTGCCGTTGTTGCTACCCTAAGCACTGTTTTCTTCGCGTATGGCTACTAATGCGCGAAGCGCCATG
TAAGAGCGCGCCTGAGGTAATAAAGGTGGCGACTGCAAAAAGCTCATGTCCCGCGAATGATGGGAGT
TCTTTGGAGATTCTGAATCAAAGACGTCGATATTCTGGGTCCAGTCGGTGCAGAACAGACCCCATGG
SEQ ID NO : 68
   LENGTH : 1743
   TYPE : DNA
   ORGANISM : *M. phaseolina*
   FEATURE NAME/KEY: CDS
   LOCATION : (1)......(1743) 
atggacggagttccgggagtaacacaggagccgatcccaccaggaggaaacttcacgtac
 M  D  G  V  P  G  V  T  Q  E  P  I  P  P  G  G  N  F  T  Y
cgcttctcgctcaaaaacgaatacggcttctactggtatcactcgcatttccgagcatac
 R  F  S  L  K  N  E  Y  G  F  Y  W  Y  H  S  H  F  R  A  Y
tcggacgacgccatccgcggaccactagtcatccatccatcctcacaacgcccccggcca
 S  D  D  A  I  R  G  P  L  V  I  H  P  S  S  Q  R  P  R  P
tacgagactctcgcgaggaaccagactgagcttactgctttgcaagaagctgaacgcgaa
 Y  E  T  L  A  R  N  Q  T  E  L  T  A  L  Q  E  A  E  R  E
gcggtgcctatcctcctatctgactggtaccatcgcgtttcggacgacatcttcaacgaa
 A  V  P  I  L  L  S  D  W  Y  H  R  V  S  D  D  I  F  N  E
tacctaacaacaggcgcgttccccagttgcgtggacagtctcctcgccaatggatacgga
 Y  L  T  T  G  A  F  P  S  C  V  D  S  L  L  A  N  G  Y  G
agggtgcggtgtctaccggaatacattctggcagccggagctgggctaggcatggagcct
 R  V  R  C  L  P  E  Y  I  L  A  A  G  A  G  L  G  M  E  P
gcacctgtcaatgctaccgcgactagcatcggcacaacgcccatgtcttccatggcgatg
 A  P  V  N  A  T  A  T  S  I  G  T  T  P  M  S  S  M  A  M
ggtacaaagtacatggagaccaataccaaggagtcgatgcgtatggagtctatggcacta
 G  T  K  Y  M  E  T  N  T  K  E  S  M  R  M  E  S  M  A  L
gacactacgtccatggagcatcacatgcgacgcatggattccatgtcagcggaggatatg
 D  T  T  S  M  E  H  H  M  R  R  M  D  S  M  S  A  E  D  M
tccatgaatacgatgactgctcattctacgcctgcggctgcgcccgactcaggcatgccg
 S  M  N  T  M  T  A  H  S  T  P  A  A  A  P  D  S  G  M  P
atgggctccatgtcaatggcttctctacccgtatcaggcatatcgggcacgtcgggcctg
 M  G  S  M  S  M  A  S  L  P  V  S  G  I  S  G  T  S  G  L
tcgggcatgtcaaatatggcgagcggtccccttggtcctcgcggctgcagtgctcccatg
 S  G  M  S  N  M  A  S  G  P  L  G  P  R  G  C  S  A  P  M
atgttcagaccgggctacaacatcagctctctcccgccagagacttgcacagacacgtca
 M  F  R  P  G  Y  N  I  S  S  L  P  P  E  T  C  T  D  T  S
gcgccgctgctgactgtcgatgcaaactatacacgaggctggcttgcattaaacctagtc
 A  P  L  L  T  V  D  A  N  Y  T  R  G  W  L  A  L  N  L  V
aactccggttcagtaaccaagctcagcgtctcactagacgcccattcgatgtttgtctat
 N  S  G  S  V  T  K  L  S  V  S  L  D  A  H  S  M  F  V  Y
gcggccgacggattctttgtgaaaccccaagaagtagaagtattacaaatctcaatcggg
 A  A  D  G  F  F  V  K  P  Q  E  V  E  V  L  Q  I  S  I  G
caacggtactcagtaatgatcaaacttaatcaacggcccggaaactacactctacggttt
 Q  R  Y  S  V  M  I  K  L  N  Q  R  P  G  N  Y  T  L  R  F
 
gcgtcgtatccctacggcgatatgcaacaggttatcgaaggccaagcgaccgtctcatat
 A  S  Y  P  Y  G  D  M  Q  Q  V  I  E  G  Q  A  T  V  S  Y
aaggttgatgccgcagaggacattatgccggtggacttgacaaacgaccctactgcaacg
 K  V  D  A  A  E  D  I  M  P  V  D  L  T  N  D  P  T  A  T
tggatgcttgtcaacggttcggcaaagtcgaacgcttctgaattgaagacggacatgctc
 W  M  L  V  N  G  S  A  K  S  N  A  S  E  L  K  T  D  M  L
gccccgttcgaggcaattgcgccgccatcccaagcggatataacttacgacttcacgatc
 A  P  F  E  A  I  A  P  P  S  Q  A  D  I  T  Y  D  F  T  I
agtcagacagagatcatgggtcatcctatgcaccttcacggccaccggttcttcgccctc
 S  Q  T  E  I  M  G  H  P  M  H  L  H  G  H  R  F  F  A  L
ggctctggatcgggctccttcccatatcagaacgccgtcgacgcgcctccatccctcatt
 G  S  G  S  G  S  F  P  Y  Q  N  A  V  D  A  P  P  S  L  I
aacctcgaaaaccctccgtaccgggacacaaccgatttaccaccttcaggctgggcagtc
 N  L  E  N  P  P  Y  R  D  T  T  D  L  P  P  S  G  W  A  V
attcgctatgtagccaacaatccaggcgcatggatgtttcactgccacatccagtggcac
 I  R  Y  V  A  N  N  P  G  A  W  M  F  H  C  H  I  Q  W  H
ctcgtgagcggcatggcattggtgtttgtcgaaggagaagagcagctgcctggtttggtg
 L  V  S  G  M  A  L  V  F  V  E  G  E  E  Q  L  P  G  L  V
ggtgcggctgcgaacggaacaagcaatgcgaatagcgcgtcaccggcgcgtagcactcga
 G  A  A  A  N  G  T  S  N  A  N  S  A  S  P  A  R  S  T  R
gagcatgcggcctttgccgttgttgctaccctaagcactgttttcttcgcgtatggctac
 E  H  A  A  F  A  V  V  A  T  L  S  T  V  F  F  A  Y  G  Y
taa
 -
SEQ ID NO : 69
   LENGTH : 580
   TYPE : PRT
   ORGANISM : *M. phaseolina* 
MDGVPGVTQEPIPPGGNFTYRFSLKNEYGFYWYHSHFRAYSDDAIRGPLVIHPSSQRPRPYETLARN
QTELTALQEAEREAVPILLSDWYHRVSDDIFNEYLTTGAFPSCVDSLLANGYGRVRCLPEYILAAGA
GLGMEPAPVNATATSIGTTPMSSMAMGTKYMETNTKESMRMESMALDTTSMEHHMRRMDSMSAEDMS
MNTMTAHSTPAAAPDSGMPMGSMSMASLPVSGISGTSGLSGMSNMASGPLGPRGCSAPMMFRPGYNI
SSLPPETCTDTSAPLLTVDANYTRGWLALNLVNSGSVTKLSVSLDAHSMFVYAADGFFVKPQEVEVL
QISIGQRYSVMIKLNQRPGNYTLRFASYPYGDMQQVIEGQATVSYKVDAAEDIMPVDLTNDPTATWM
LVNGSAKSNASELKTDMLAPFEAIAPPSQADITYDFTISQTEIMGHPMHLHGHRFFALGSGSGSFPY
QNAVDAPPSLINLENPPYRDTTDLPPSGWAVIRYVANNPGAWMFHCHIQWHLVSGMALVFVEGEEQL
PGLVGAAANGTSNANSASPARSTREHAAFAVVATLSTVFFAYGY*
SEQ ID NO : 70
   LENGTH : 2242 (including 150 bp 5' UTR and 150 bp 3' UTR)
   TYPE : DNA
   ORGANISM : *M. phaseolina* 
AGTCCACCCTTTCCTATTTCTTTCCTCACGTCACATTCAGATACCCGGGCTCCTTTCAACTGCCAAA
TTTCCTTTTTCCCGGACTCATTCCTCCCCCGACTCTCTTACTCTTCTCTCAATTTCCTACCACCC
ACCGCTCGCTCTCACAATGTCTTTCGTTGCCAGGTCCGTAGTAAGCGGCCTCGCCAGCAGAGCCGCC
TTTACTAATGGATCATGGACCGGTGCCTGGGAGACTCATGGCTCCACCCAAGGTTCCATCTCACCGC
AACTACGCAACGGTACGTACCAGGTTCAGTTGTTTAAATCCCAAATTTGACTGATCTTTGTTGCAGG
CGGCGGGCCTCTTGGTACCTTGGACGCTCCAAACTTGCCTGGCTGTATTGGTTCTCCACCGTGGGGC
AACCATGACTCTCATGACCCTTTCGGCATGCCTGATACTGGCATTACTCGGAGTATGACTTTACTC
TCACTTATCAAGACATCGCTCCCGATGGCGTCACTAAGAGGGGCGTCGTTGTCAATGGTCAATATCC
CGGGCCCACGATCGAGGCCAACTGGTGCGTACATCACTACATCACGCACATGACATCAGGCTAACAC
TTCGTTCAGGGGCGACTGGATCCAGGTCACGGTCCACAACGGTCTAGGCGAGGACGAAGGTGAGGGT
ACCGCGATGCACTGGCACGGCTTTTTGCAGAAGGAGAGCCAGTGGATGGATGGTGTCCCCGGTGTTC
AACAATGTCCTATTCCTCCCGGAGAGAGCTTCACCTACCGCTTCCGCGCTGAGCAGTATGGCACTTC
 
TTGGTACCACAGCCACTACAGCGCAGTACTCGGCGGTGCTGCTGGTCCTCTCATCGTATACGGT
CCGGACAGCCAGAGTTACGACGTTGACCTCGGTCCCGTCATGGTATCTGACTGGTACCACTCGCAAT
ACTACGATATTGTCAAGCAGACCATGCAGGCCGACCCCACGGCACCACCCCTCCGCCGCCGCCTCA
GTCCGACAACAACCTGATCCAGGGCTTTGGCGAATTCGACTGCTCGCTGACGACGAAGCCCTGCATT
CCTGACGCCGGTGTAGCCAAGTTCAAGTTCACTTCCGGCAAGAAGCATCGCCTCAGGCTCATCAACT
CCGGCTCCGAGGCGATGCAGAGGTTCTCTATCGACGGCCACACCATGAAGATCATCGCACACGACTT
CGTCCCGATCGAGCCCTACGAGGTCACGGCCCTGACCCTTGGCGTCGGACAGCGGGCCGACGTGGTT
GTCGAAGCCACGGGCAAGCCCTCGGACGCGTACTGGATGCGCTCAGAGATCGGGCTCAACAAGTGCA
ACGTCTTCAATGCGAACGCCTCCGAGGCCCTCGCCGTCATCCTCTACGAGGATGCAGACCTGCTGGC
CGTGCCCAGCTCCTCCGCCCAGCCCGATGCGGAGCTGACATCGTGCACCAACGACGACATCTCCGTC
GGCCTGCCGCTCCAGCACATCCTGCCCGACCCCAACCCGTCCGTCACGACCGAGCTGCACATCGAGA
ACAAGTACAACGGCACGCACTGGCTCTGGCACTTCGGCGGCCCCAGCTACCGCGCCGACTTCAACGA
CGCCCTCCTCTACCAGCTCCAACAGGGCACGCCCGACTTCGGCCCGCAGTCCAACGTGCACGACTTC
GGCGCCAACAAATCCGTCCGCTTCGTCGTCTACAACCACGTCCCGGCTCAGCATCCCATGCACTTGC
ATGGCCACAACTTCTGGGTCCTCGCCGACGGCGTCGGCACCTGGGACGGCGCCATCGCCAACCCGCA
GAACCCCCAGCGCCGCGACGTGCACGTCCTTCAGCCCGCCCAGGGCAGCACCCCGTCGTACATGGTC
GTACAGATTGAGCTGGACAACCCGGGCCTGTGGCCGTTCCACTGCCACATCGCGTGGCACGTCTCGG
CGGGGTTGTATTTGAACGTGCTGGAGCGCCCGGACGACATCAAGGCGCTGCAGATTCCGGCCGCGGT
TGGTGATCAGTGCAGGGCGTGGGCGGACTACACGGCAAAGAATACGGTGGATCAGATCGACTCGGGG
TTGAAGAAGGAGTAGAAGGCGTGATAGTTTGCTATTGTTCTTTCTCTCTCTCTCTTTTTTTTTTTTT
TTTTACACCTTCCCTTCTTGATACCAGCATGTTTTTGGGCGTTATTTACGGTCCTTCTGCGCATTTC
CAAGGCGTTATGTCTTTCCCTTGCGGATAAG
SEQ ID NO : 71
   LENGTH : 1836
   TYPE : DNA
   ORGANISM : *M. phaseolina*
   FEATURE NAME/KEY: CDS
   LOCATION : (1)......(1836) 
atgtctttcgttgccaggtccgtagtaagcggcctcgccagcagagccgcctttactaat
 M  S  F  V  A  R  S  V  V  S  G  L  A  S  R  A  A  F  T  N
ggatcatggaccggtgcctgggagactcatggctccacccaaggttccatctcaccgcaa
 G  S  W  T  G  A  W  E  T  H  G  S  T  Q  G  S  I  S  P  Q
ctacgcaacggcggcggcctcttggtaccttggacgctccaaacttgcctggctgtatt
 L  R  N  G  G  G  P  L  G  T  L  D  A  P  N  L  P  G  C  I
ggttctccaccgtggggcaaccatgactctcatgaccctttcggcatgcctgatactggc
 G  S  P  P  W  G  N  H  D  S  H  D  P  F  G  M  P  D  T  G
attactcgggagtatgactttactctcacttatcaagacatcgctcccgatggcgtcact
 I  T  R  E  Y  D  F  T  L  T  Y  Q  D  I  A  P  D  G  V  T
aagagggggcgtcgttgtcaatggtcaatatcccgggcccacgatcgaggccaactggggc
 K  R  G  V  V  V  N  G  Q  Y  P  G  P  T  I  E  A  N  W  G
gactggatccaggtcacggtccacaacggtctaggcgaggacgaaggtgagggtaccgcg
 D  W  I  Q  V  T  V  H  N  G  L  G  E  D  E  G  E  G  T  A
atgcactggcacggcttttttgcagaaggagagccagtggatggatggtgtccccggtgtt
 M  H  W  H  G  F  L  Q  K  E  S  Q  W  M  D  G  V  P  G  V
caacaatgtcctattcctcccggagagagcttcacctaccgcttccgcgctgagcagtat
 Q  Q  C  P  I  P  P  G  E  S  F  T  Y  R  F  R  A  E  Q  Y
ggcacttcttggtaccacagccactacagcgcgcagtactcgggcggtgctgctggtcct
 G  T  S  W  Y  H  S  H  Y  S  A  Q  Y  S  G  G  A  A  G  P
ctcatcgtatacggtccggacagccagagttacgacgttgacctcggtcccgtcatggta
 L  I  V  Y  G  P  D  S  Q  S  Y  D  V  D  L  G  P  V  M  V
tctgactggtaccactcgcaatactacgatattgtcaagcagaccatgcaggccgacccc
 S  D  W  Y  H  S  Q  Y  Y  D  I  V  K  Q  T  M  Q  A  D  P
acgggcaccacccctccgccgccgcctcagtccgacaacaacctgatccagggctttggc
 T  G  T  T  P  P  P  P  P  Q  S  D  N  N  L  I  Q  G  F  G
 
gaattcgactgctcgctgacgacgaagccctgcattcctgacgccggtgtagccaagttc
 E  F  D  C  S  L  T  T  K  P  C  I  P  D  A  G  V  A  K  F
aagttcacttccggcaagaagcatcgcctcaggctcatcaactccggctccgaggcgatg
 K  F  T  S  G  K  K  H  R  L  R  L  I  N  S  G  S  E  A  M
cagaggttctctatcgacggccacaccatgaagatcatcgcacacgacttcgtcccgatc
 Q  R  F  S  I  D  G  H  T  M  K  I  I  A  H  D  F  V  P  I
gagccctacgaggtcacggccctgacccttggcgtcggacagcgggccgacgtggttgtc
 E  P  Y  E  V  T  A  L  T  L  G  V  G  Q  R  A  D  V  V  V
gaagccacgggcaagccctcggacgcgtactggatgcgctcagagatcgggctcaacaag
 E  A  T  G  K  P  S  D  A  Y  W  M  R  S  E  I  G  L  N  K
tgcaacgtcttcaatgcgaacgcctccgaggccctcgccgtcatcctctacgaggatgca
 C  N  V  F  N  A  N  A  S  E  A  L  A  V  I  L  Y  E  D  A
gacctgctggccgtgcccagctcctccgcccagcccgatgcggagctgacatcgtgcacc
 D  L  L  A  V  P  S  S  S  A  Q  P  D  A  E  L  T  S  C  T
aacgacgacatctccgtcggcctgccgctccagcacatcctgcccgaccccaacccgtcc
 N  D  D  I  S  V  G  L  P  L  Q  H  I  L  P  D  P  N  P  S
gtcacgaccgagctgcacatcgagaacaagtacaacggcacgcactggctctggcacttc
 V  T  T  E  L  H  I  E  N  K  Y  N  G  T  H  W  L  W  H  F
ggcggccccagctaccgcgccgacttcaacgacgccctcctctaccagctccaacagggc
 G  G  P  S  Y  R  A  D  F  N  D  A  L  L  Y  Q  L  Q  Q  G
acgcccgacttcggcccgcagtccaacgtgcacgacttcggcgccaacaaatccgtccgc
 T  P  D  F  G  P  Q  S  N  V  H  D  F  G  A  N  K  S  V  R
ttcgtcgtctacaaccacgtcccggctcagcatcccatgcacttgcatggccacaacttc
 F  V  V  Y  N  H  V  P  A  Q  H  P  M  H  L  H  G  H  N  F
tgggtcctcgccgacggcgtcggcacctgggacggcgccatcgccaacccgcagaacccc
 W  V  L  A  D  G  V  G  T  W  D  G  A  I  A  N  P  Q  N  P
cagcgccgcgacgtgcacgtccttcagcccgcccagggcagcaccccgtcgtacatggtc
 Q  R  R  D  V  H  V  L  Q  P  A  Q  G  S  T  P  S  Y  M  V
gtacagattgagctggacaacccgggcctgtggccgttccactgccacatcgcgtggcac
 V  Q  I  E  L  D  N  P  G  L  W  P  F  H  C  H  I  A  W  H
gtctcggcggggttgtatttgaacgtgctggagcgcccggacgacatcaaggcgctgcag
 V  S  A  G  L  Y  L  N  V  L  E  R  P  D  D  I  K  A  L  Q
attccggccgcggttggtgatcagtgcagggcgtgggcggactacacggcaaagaatacg
 I  P  A  A  V  G  D  Q  C  R  A  W  A  D  Y  T  A  K  N  T
gtggatcagatcgactcggggttgaagaaggagtag
 V  D  Q  I  D  S  G  L  K  K  E  -
SEQ ID NO : 72
   LENGTH : 611
   TYPE : PRT
   ORGANISM : *M. phaseolina* 
MSFVARSVVSGLASRAAFTNGSWTGAWETHGSTQGSISPQLRNGGPLGTLDAPNLPGCIGSPPWGN
HDSHDPFGMPDTGITREYDFTLTYQDIAPDGVTKRGVVVNGQYPGPTIEANWGDWIQVTVHNGLGED
EGEGTAMHWHGFLQKESQWMDGVPGVQQCPIPPGESFTYRFRAEQYGTSWYHSHYSAQYSGGAAGPL
IVYGPDSQSYDVDLGPVMVSDWYHSQYYDIVKQTMQADPTGTTPPPPPQSDNNLIQGFGEFDCSLTT
KPCIPDAGVAKFKFTSGKKHRLRLINSGSEAMQRFSIDGHTMKIIAHDFVPIEPYEVTALTLGVGQR
ADVVVEATGKPSDAYWMRSEIGLNKCNVFNANASEALAVILYEDADLLAVPSSSAQPDAELTSCTND
DISVGLPLQHILPDPNPSVTTELHIENKYNGTHWLWHFGGPSYRADFNDALLYQLQQGTPDFGPQSN
VHDFGANKSVRFVVYNHVPAQHPMHLHGHNFWVLADGVGTWDGAIANPQNPQRRDVHVLQPAQGSTP
SYMVVQIELDNPGLWPFHCHIAWHVSAGLYLNVLERPDDIKALQIPAAVGDQCRAWADYTAKNTVDQ
IDSGLKKE*
SEQ ID NO : 73
   LENGTH : 1048 (including 150 bp 5' UTR and 150 bp 3' UTR)
   TYPE : DNA
   ORGANISM : *M. phaseolina* 
GGACCGTTGCGGCGTTACTGATCTTCGGGTGAACGTCTCTCTTCGTACCAGGAAGCCGCCAACGCCG
AAGAAAGGAGAAATACGTGTGGCTCAACTTTATCCACCCTGGCGCCCATCATGAGCTGCGGATCTC
CGTTGACGAGCACGACATGTGGATTGCGGCCGCAGATGGAGACTTCGTAAAACCaAAGAAAGTCCAA
GTAAGTTTCCACGTCTCGGTTTCGAGCACGCGCTGACAATCTCAAGGCCATCAATGTCAACATGGGC
GAGAGGATAAGCGTCCTTATTCCTCTTACCCAAAGTCCTGGAGAGTACGCCATCCGCATGGTCTCTC
TCGCGGAAGAGCAGCTCATCTGGGGCTTGGGAATTCTCCGGTACCCCGGTGTTCAAGAGAGACGAGA
TGAGAATGGCATAATGATCCTGCCAGAAAGCCAGCCACACATTGATGTTCAGGATAACCTGTTGACT
GACGGAATTGTGATGGACGAAATGACCGACCTGATCCCGTTTCCCGCGCGCCGTCCTCCAGCCAAGG
CCGACCACACCTTTCGCTTCGCCATCAAGCGGCCGAATCCAAGCACGTGGATTTTGGCATCGGAGCC
GCATCAAGGATTCAGACAACAGCTTCCGCCGGTGCTTTGGAACAAGGATTCCCGTGGCCCTACGACG
TTCGGCGGAATGAAGAACGGCTCGGTTGTGGACATCATTTATGAAAACGGAGCATTTGGGATGCATC
CGTTTCACCAGTGGATGAACGAATCGCATCACAGTATGGCAACTGCTGACATTTCATGCACAACCAC
AAAGCGTTCATCATCGGCATGGGGGATGGGTTCTTCCGGTGGCCAGACGTTGCCTCGGCCCTCAAGG
AGGCCCCTGAAAACTTTAACATGGTGAACCCTCCTCTCCGTGACGGAGCACGGCTGGCAAAGGGAGA
AGGATCGTGGACTGTAATCCGCTACCAGATCACCTCTCCCGCAATGTCCATGTTACACTGTAAGCAG
CTCTCTGCAAAGTGCCTGACGGTGTAAGCACTGACGACCTCTC
SEQ ID NO : 74
   LENGTH : 702
   TYPE : DNA
   ORGANISM : *M. phaseolina*
   FEATURE NAME/KEY: CDS
   LOCATION : (1)......(702) 
atgtggattgcggccgcagatggagacttcgtaaaaccaaagaaagtccaagccatcaat
 M  W  I  A  A  A  D  G  D  F  V  K  P  K  K  V  Q  A  I  N
gtcaacatgggcgagaggataagcgtccttattcctcttacccaaagtcctggagagtac
 V  N  M  G  E  R  I  S  V  L  I  P  L  T  Q  S  P  G  E  Y
gccatccgcatggtctctctcgcggaagagcagctcatctggggcttgggaattctccgg
 A  I  R  M  V  S  L  A  E  E  Q  L  I  W  G  L  G  I  L  R
taccccggtgttcaagagagacgagatgagaatggcataatgatcctgccagaaagccag
 Y  P  G  V  Q  E  R  R  D  E  N  G  I  M  I  L  P  E  S  Q
ccacacattgatgttcaggataacctgttgactgacggaattgtgatggacgaaatgacc
 P  H  I  D  V  Q  D  N  L  L  T  D  G  I  V  M  D  E  M  T
gacctgatcccgtttcccgcgcgccgtcctccagccaaggccgaccacacctttcgcttc
 D  L  I  P  F  P  A  R  R  P  P  A  K  A  D  H  T  F  R  F
gccatcaagcggccgaatccaagcacgtggatttggcatcggagccgcatcaaggattc
 A  I  K  R  P  N  P  S  T  W  I  L  A  S  E  P  H  Q  G  F
agacaacagcttccgccggtgctttggaacaaggattcccgtggccctacgacgttcggc
 R  Q  Q  L  P  P  V  L  W  N  K  D  S  R  G  P  T  T  F  G
ggaatgaagaacggctcggttgtggacatcatttatgaaaacggagcatttggatgcat
 G  M  K  N  G  S  V  V  D  I  I  Y  E  N  G  A  F  G  M  H
ccgtttcaccagtggatgaacgaatcgcatcacagtatggcaactgctgacatttcatgc
 P  F  H  Q  W  M  N  E  S  H  H  S  M  A  T  A  D  I  S  C
acaaccacaaagcgttcatcatcggcatgggggatgggttcttccggtggccagacgttg
 T  T  T  K  R  S  S  S  A  W  G  M  G  S  S  G  G  Q  T  L
cctcggccctcaaggaggcccctgaaaactttaacatggtga
 P  R  P  S  R  R  P  L  K  T  L  T  W  -
SEQ ID NO : 75
   LENGTH : 233
   TYPE : PRT
   ORGANISM : *M. phaseolina* 
MWIAAADGDFVKPKKVQAINVNMGERISVLIPLTQSPGEYAIRMVSLAEEQLIWGLGILRYPGVQER
RDENGIMILPESQPHIDVQDNLLTDGIVMDEMTDLIPFPARRPPAKADHTFRFAIKRPNPSTWILAS
EPHQGFRQQLPPVLWNKDSRGPTTFGGMKNGSVVDIIYENGAFGMHPFHQWMNESHHSMATADISCT
TTKRSSSAWGMGSSGGQTLPRPSRRPLKTLTW*
SEQ ID NO : 76
   LENGTH : 2269 (including 150 bp 5' UTR and 150 bp 3' UTR)
   TYPE : DNA
   ORGANISM : *M. phaseolina* 
GCTGCGAGGCATTCGAGAGGATTACCGAGTATTGGAGAGGCCGCGTGAAGGCAGCAGAGCATTAAAT
ACGTGCTTTGGAGCACGCGAATTACTTTCAAGTTGAACAAGATATCACCTGTCTTGCACGACGTTGC
AAGCTCCGCCGCAGCCATGAAGTGTGCAACTCTTTGGAGCTATCTTGCCTCTGTCCTGACCGTCGGC
GCTTCTGCGAGGTATTCCATTCAGCATAGACTTCAAACATTACCTAAATGCTGACAACTCATTTACA
GAAGCTTGACGCGGTCCTTGCCTCAGCACAAGACCAACGGAGGGTCCAATTGGGGAACTTTGGACTG
TCCAAAGTTACCCGATTTTTGACCTCGAACCCGCTGCCCGGCGGCTTCCCGTGGGGTGACAGAAGC
GGCCTAAGCAACGATCCCTACACTGATGTGCCGAACACCGGGGTGACGAGGTACTACGATTTCAGCG
TCGCACGTGGCTATCTCGCTCCAGATGGCTACAACAAAAGTGGCATCTTCATCAACGGCGAGTTCCC
TGGGCCTGCCATTGAGGCCAATTGGGGCGACATGATTGAAGTACGAGTGCACAACAACATCGTCGGC
CCTGAAGAAGGCACTGCGTTCCACTGGCATGGCATTACTCAGAAGGGCACGCAATGGTTTGACGGCG
TTCCCGGCGTGTCCCAATGCCCCATTGCCCCTGGATCCTCTTTACCTACCGCTTCCGTGCCGACGT
CTACGGCACTTCTTGGTGGCACTCGCACTTCTCTGCGCAATATACCGCTGGTGCTTTTGGGCCCCTC
ATTATCTACGGCCCCAAGCATGTTCCTTACGATGTTGATGTCGGCCCTGTAATTCTCGGTGATTACT
ACCACCGTGACTACTTTGATGTTCTGGAGGATGCTGCCAGCAACACCACTGACTTCAACATCTACGT
CCCTTGGTCCGACAACAATCTGATCAATGGCAAGAACAACTATAATTGCTCCATGGTAGCTGGAAAC
TCTACGAGCTTCGCCAACGCTACAAGCTCCTCGAACGCCACGTGCTTCTCCAACGCTGGCCTTGCCC
AGTTCCGTTTCGAGCCAGGCAAAGTGCACCGCCTGCGTCTGATGAACGTGGGCGCAGCAGCACTGCT
GCACTTCTCAATCGACGGGCACAAAATGCAAGTCATCGCCCACGACTTCGAACCTGTTGTCCCGTAC
GAGGCAGACGTCATCACGCTGGGCGCCGCCCAACGCACCGACATCCTCGTCACTGCAGATGCCAACC
CCAACGAGACATACTGGATCCGCTCCACCATCTCGCTCAACTGCTCTGTCTCGCACAACACCAACGC
GCTGGCCGTTCTCTCCTACGAAGGCAATGACCACATAGAAGAGCCACGCAGCCGCATTAGCGCCGCC
GCGGCCGCTGCTGACGAGAAGAGCTTCCTCTGCAAGAACGACGACCTGTCCCAGACGGTGCCCTTCT
TCCCCAAGCCCGTCGCCGAGCCCGATGTGACCGAGACGATCGAAGTCGACCTCTTCACCAATGCGAC
CGGCCACCATGTGTGGATCATGAACAACCGCACGCAGCGCACGAACTACAACGAGCCCGTCTTGCTG
CTCGCCAACCAGGGCAACAGCACCTTCCCGGACGAGTGGAACGTTTACGACTTTGGGCGCAACAAGA
CCATCCGCATCGTCCTCAACACCGTCTACCAGTCCGCCCACCCGATGCACTTGCACGGCCACTCTTT
CGTAAGACCCTTCATCCCTTCGCCACGCATGTGTGCCCACTCTGACCTCTTCCTCGCAGCAAGTCCT
CGCCGAAGGCCCCGGCGCCTGGACGGCACGACCATCACCAACCCATCCAATCCCGCCCGCCGCGAC
ACGCACATGCAGCGCCGGTACGGGCACCTGGTAATCCAGTTCGAGGCCGACAACCCGGGCGCGTGGA
GCTACCACTGCCACATCGCCTGGCACGCCAGCATGGGCTACAACATCGAGATCCTCGAGCGCGGCGA
CGAGCTGGCGGCCGCCGGCGCTATTCCTATGGTCATGCAGCAGACGTGTGATGATTGGAAGGAGTGG
AGCGGCAGGAATGTGGTCAATCAGATCGACGCGGGCATTTAGATTATCGGCGACTTCTATGCCCGAT
GGTATAATGTTTTTACCGAGCGTGGGATGATGTGGGTTTGGGGTCGGAATTTGTAGATATGGGCGGG
GGATTGCCTGCACAAAATAGACCGATGCACATGTTTATAAAGCAAAAACTCTTCCCAT
SEQ ID NO : 77
   LENGTH : 1854
   TYPE : DNA
   ORGANISM : *M. phaseolina*
   FEATURE NAME/KEY: CDS
   LOCATION : (1)......(1854) 
atgaagtgtgcaactctttggagctatcttgcctctgtcctgaccgtcggcgcttctgcg
 M  K  C  A  T  L  W  S  Y  L  A  S  V  L  T  V  G  A  S  A
agaagcttgacgcggtccttgcctcagcacaagaccaacggaggtccaattggggaact
 R  S  L  T  R  S  L  P  Q  H  K  T  N  G  G  S  N  W  G  T
ttggactgtccaaagttacccgatttttgacctcgaacccgctgcccggcggcttcccg
 
L D C P K L P D F L T S N P L P G G F P
tggggtgacagaagcggcctaagcaacgatccctacactgatgtgccgaacaccggggtg
W G D R S G L S N D P Y T D V P N T G V
acgaggtactacgatttcagcgtcgcacgtggctatctcgctccagatggctacaacaaa
T R Y Y D F S V A R G Y L A P D G Y N K
agtggcatcttcatcaacggcgagttccctggcctgccattgaggccaattggggcgac
S G I F I N G E F P G P A I E A N W G D
atgattgaagtacgagtgcacaacaacatcgtcggccctgaagaaggcactgcgttccac
M I E V R V H N N I V G P E E G T A F H
tggcatggcattactcagaagggcacgcaatggtttgacggcgttcccggcgtgtcccaa
W H G I T Q K G T Q W F D G V P G V S Q
tgccccattgcccctggatcctcttttacctaccgcttccgtgccgacgtctacggcact
C P I A P G S S F T Y R F R A D V Y G T
tcttggtggcactcgcacttctctgcgcaatataccgctggtgctttgggccccctcatt
S W W H S H F S A Q Y T A G A F G P L I
atctacggccccaagcatgttccttacgatgttgatgtcggccctgtaattctcggtgat
I Y G P K H V P Y D V D V G P V I L G D
tactaccaccgtgactactttgatgttctggaggatgctgccagcaacaccactgacttc
Y Y H R D Y F D V L E D A A S N T T D F
aacatctacgtcccttggtccgacaacaatctgatcaatggcaagaacaactataattgc
N I Y V P W S D N N L I N G K N N Y N C
tccatggtagctggaaactctacgagcttcgccaacgctacaagctcctcgaacgccacg
S M V A G N S T S F A N A T S S S N A T
tgcttctccaacgctggccttgcccagttccgtttcgagccaggcaaagtgcaccgcctg
C F S N A G L A Q F R F E P G K V H R L
cgtctgatgaacgtgggcgcagcagcactgctgcacttctcaatcgacggcacaaaatg
R L M N V G A A A L L H F S I D G H K M
caagtcatcgcccacgacttcgaacctgttgtcccgtacgaggcagacgtcatcacgctg
Q V I A H D F E P V V P Y E A D V I T L
ggcgccgcccaacgcaccgacatcctcgtcactgcagatgccaaccccaacgagacatac
G A A Q R T D I L V T A D A N P N E T Y
tggatccgctccaccatctcgctcaactgctctgtctcgcacaacaccaacgcgctggcc
W I R S T I S L N C S V S H N T N A L A
gttctctcctacgaaggcaatgaccacatagaagagccacgcagccgcattagcgccgcc
V L S Y E G N D H I E E P R S R I S A A
gcggccgctgctgacgagaagagcttcctctgcaagaacgacgacctgtcccagacggtg
A A A A D E K S F L C K N D D L S Q T V
cccttcttccccaagcccgtcgccgagcccgatgtgaccgagacgatcgaagtcgacctc
P F F P K P V A E P D V T E T I E V D L
ttcaccaatgcgaccggccaccatgtgtggatcatgaacaaccgcacgcagcgcacgaac
F T N A T G H H V W I M N N R T Q R T N
tacaacgagcccgtcttgctgctcgccaaccagggcaacagcaccttcccggacgagtgg
Y N E P V L L L A N Q G N S T F P D E W
aacgtttacgactttggcgcaacaagaccatccgcatcgtcctcaacaccgtctaccag
N V Y D F G R N K T I R I V L N T V Y Q
tccgcccacccgatgcacttgcacggccactctttccaagtcctcgccgaaggccccggc
S A H P M H L H G H S F Q V L A E G P G
gcctgggacggcacgaccatcaccaacccatccaatcccgcccgccgcgacacgcacatg
A W D G T T I T N P S N P A R R D T H M
cagcgccggtacgggcacctggtaatccagttcgaggccgacaacccgggcgcgtggagc
Q R R Y G H L V I Q F E A D N P G A W S
taccactgccacatcgcctggcacgccagcatgggctacaacatcgagatcctcgagcgc
Y H C H I A W H A S M G Y N I E I L E R
ggcgacgagctggcggccgccggcgctattcctatggtcatgcagcagacgtgtgatgat
G D E L A A A G A I P M V M Q Q T C D D
 
tggaaggagtggagcggcaggaatgtggtcaatcagatcgacgcgggcatttag
W K E W S G R N V V N Q I D A G I -
SEQ ID NO : 78
   LENGTH : 617
   TYPE : PRT
   ORGANISM : *M. phaseolina* 
MKCATLWSYLASVLTVGASARSLTRSLPQHKTNGGSNWGTLDCPKLPDFLTSNPLPGGFPWGDRSGL
SNDPYTDVPNTGVTRYYDFSVARGYLAPDGYNKSGIFINGEFPGPAIEANWGDMIEVRVHNNIVGPE
EGTAFHWHGITQKGTQWFDGVPGVSQCPIAPGSSFTYRFRADVYGTSWWHSHFSAQYTAGAFGPLII
YGPKHVPYDVDVGPVILGDYYHRDYFDVLEDAASNTTDFNIYVPWSDNNLINGKNNYNCSMVAGNST
SFANATSSSNATCFSNAGLAQFRFEPGKVHRLRLMNVGAAALLHFSIDGHKMQVIAHDFEPVVPYEA
DVITLGAAQRTDILVTADANPNETYWIRSTISLNCSVSHNTNALAVLSYEGNDHIEEPRSRISAAAA
AADEKSFLCKNDDLSQTVPFFPKPVAEPDVTETIEVDLFTNATGHHVWIMNNRTQRTNYNEPVLLLA
NQGNSTFPDEWNVYDFGRNKTIRIVLNTVYQSAHPMHLHGHSFQVLAEGPGAWDGTTITNPSNPARR
DTHMQRRYGHLVIQFEADNPGAWSYHCHIAWHASMGYNIEILERGDELAAAGAIPMVMQQTCDDWKE
WSGRNVVNQIDAGI*
SEQ ID NO : 79
   LENGTH : 2373 (including 150 bp 5' UTR and 150 bp 3' UTR)
   TYPE : DNA
   ORGANISM : *M. phaseolina* 
GACAATCCCTCCGCTAAATCCGACATCCGGTACTCTCGCCTCCCGGATCCCGAGCGGACACACAGCT
TCGCCTGTATACCACTTTCACCGGAGTCGCCTTCCCACCCCCTATAATTCTTTTCCTGTTGAGTCTA
CTCCGCGCGCGGTATCATGATAGATACCAAGAGCACAGGGTCCGGCGCAGACGGCGGTCGTTACGCC
GCCTTAAGGCAAGACGAGTCAGAGCTATACGAGCAGAAAGAACATACGCAGTGTACACAGCCCTCCG
GCGCCGCGTACCTCGGTGGAAATGGCAGAGAAGAAGCCCTGGGATTGATCGACGAAAATGTCGTCGC
TCGAACAAAAAGACGCTGTGGACCCACCCGGCGGTACTCTGTCTTCCTTGAATTTGCCATCCTCGGT
CTGGTACTCATAATCGCTCTACTCGGCGCTCTCGCTTGGTCCAGAGGCTCTCATCATCATACCGACC
CGGTATCTGGCAGTTCACAACCATCGTCCAAGGGTCGCCGCGGAAAGTATGTCCTGGACCCTGCCTG
GGATTTCGCTGCACCGCCGCAGGTCCGCAAATACCACTGGACGATCAGGGACATCGAGCTTCGCCCA
GACGGCGTGAAGCGGCCGCTGATCACCATCAATAACGAGTTCCCGGGGCCGACCATCGAATGCAACC
AGGGGGATACCGTGCGGGTTGAGGTTCATAACGAAGCCGTCAATTCAACTTCCTTTCACTGGCACGG
CATTTACCAGAACGGAACCACGTACATGGATGGCACGGTCGGCATCAGCCAGTGTCCTATCACGTCT
GGGTCTAGCATGACATATGAGTTCAAAGTCGACAGAGAATCCGGCACCTATTGGTATCATGCGCACA
TGGCTATGCAGGGCTCAGATGGTCTTTTTGGTCCTCTGATCGTCCATTCGAAAAATGAGCGGAAGCT
GCAGCAACTCGAATATGCCTCCGATCAAGTCATCATGGTCCACGATTACTACCACGATCTGACCAGC
GCGCTGATACCACACTACTTAGCGCCGGATAACGAGAACACAGAGCCTGTCCCTGACGGAGGTCTCA
TCAACGGAATGAATAAGAGAAATTGCGAGCTCCTCCGTGGTCGAGACTGTGATGCCACTGATGCACA
GCTTGCCACATTCGGCCTCGAACCGAACAAGAACCACCGTCTTCGAATTATCAATACTGGAGCATTT
GCTGAATTCCAGGTGAAGATTGACGAGCACACGTTCGCTGTGACGGAGGTGGATGGGACCGAAGTTG
CTCCCGCCTACTACCACAGGCTCAACATCAATCCCGGACAGCGCTACAGCATCGTAATAAACACCAA
TGTCACGGATCGTGACTCCTTCTGGCTGAGAGCTAAGATGATTGAGGCCTGTTTCGCTGAGGAGAAC
CCAAATCTGGATCCCGAAGTGCGCGCCATTATCCAATATACTCGCAAGGACGAGGATACCCAGCCCA
AGGAACCTTCGAGCAGAGACTGGGACGACATCGTGGACATGCAGTGTCTCGACATGAACGTGACAGA
GCTCCAGCCCGTAGAGAAGGCAACACCTCCACCTGCAGACACCACACTATACCTCCGCTCCAACTTC
GAGATCGGCAACTGGCGTCTGAGCCGCGGCTTCTTCAACAGCTCGTCCTGGCGTCCAACACTCTCAT
CCCCAAGCCTGCACCGCATGATCGACGGCCTCCACAGCCAAAACGCCAGCTTCCTCCCCGACCGAGC
GTACCCCTTCCAGATCAACTCGGCCGGCTTCGACACTGGGCCCGAGCTGGTCTACCAGACCAGCGGC
ATCCGCACCATCGACATCCTCGTTTCCAACTTCGACGACGGCAACCACCCGCTCCACCTGCACGGCT
ACAAGTACTTCGTCCTTGCGTCGGGCCACGGCTACCCGCCCGCCGACCTCTACGCGCATCTCGACAT
CTCGAACCCGCTGCGCCGCGACACCGCCTCGATCGAGGCGTTCGGCTGGATCCTACTGCGTCTCGTC
GCCGACAACCCGGGCGTCTGGGCCTTCCACTGCCACATCGGCTGGCACACCGAGGCCGGCATGCTGA
TGCAGTTCGCCACGCGCGTCGACGTGCTCGCATCCAGCCAAATCCCGGATACGCACCTCGCGCTCTG
CGCGGCCGACGGGCTCGACCGCGGCGCGTCGCCGCCAGACTCGACGTGGTTTGGGGATTTTGGGGAT
 
CTAGATCCTTGAAAAGTTCTGGAGAGGAGTGGGAGTTTCACCTGGCATGCAAGCGTCTCCGTTTCTT
CAGGCCAGGCTGACCCACTTCCCGCGCACGCCGATCTCGTACTGCAGCCCGTCGCCTTCCATGTTTA
GGTCCCACCTGCGGTACTCATCTTCGAC
SEQ ID NO : 80
   LENGTH : 2073
   TYPE : DNA
   ORGANISM : *M. phaseolina*
   FEATURE NAME/KEY: CDS
   LOCATION : (1)......(2073) 
atgatagataccaagagcacagggtccggcgcagacggcggtcgttacgccgccttaagg
 M  I  D  T  K  S  T  G  S  G  A  D  G  G  R  Y  A  A  L  R
caagacgagtcagagctatacgagcagaaagaacatacgcagtgtacacagccctccggc
 Q  D  E  S  E  L  Y  E  Q  K  E  H  T  Q  C  T  Q  P  S  G
gccgcgtacctcggtggaaatggcagagaagaagccctgggattgatcgacgaaaatgtc
 A  A  Y  L  G  G  N  G  R  E  E  A  L  G  L  I  D  E  N  V
gtcgctcgaacaaaaagacgctgtggacccacccggcggtactctgtcttccttgaattt
 V  A  R  T  K  R  R  C  G  P  T  R  R  Y  S  V  F  L  E  F
gccatcctcggtctggtactcataatcgctctactcggcgctctcgcttggtccagaggc
 A  I  L  G  L  V  L  I  I  A  L  L  G  A  L  A  W  S  R  G
tctcatcatcataccgacccggtatctggcagttcacaaccatcgtccaagggtcgccgc
 S  H  H  H  T  D  P  V  S  G  S  S  Q  P  S  S  K  G  R  R
ggaaagtatgtcctggaccctgcctgggatttcgctgcaccgccgcaggtccgcaaatac
 G  K  Y  V  L  D  P  A  W  D  F  A  A  P  P  Q  V  R  K  Y
cactggacgatcagggacatcgagcttcgcccagacggcgtgaagcggccgctgatcacc
 H  W  T  I  R  D  I  E  L  R  P  D  G  V  K  R  P  L  I  T
atcaataacgagttcccgggcccgaccatcgaatgcaaccagggggataccgtgcgggtt
 I  N  N  E  F  P  G  P  T  I  E  C  N  Q  G  D  T  V  R  V
gaggttcataacgaagccgtcaattcaacttcctttcactggcacggcatttaccagaac
 E  V  H  N  E  A  V  N  S  T  S  F  H  W  H  G  I  Y  Q  N
ggaaccacgtacatggatggcacggtcggcatcagccagtgtcctatcacgtctgggtct
 G  T  T  Y  M  D  G  T  V  G  I  S  Q  C  P  I  T  S  G  S
agcatgacatatgagttcaaagtcgacagagaatccggcacctattggtatcatgcgcac
 S  M  T  Y  E  F  K  V  D  R  E  S  G  T  Y  W  Y  H  A  H
atggctatgcagggctcagatggtctttttggtcctctgatcgtccattcgaaaaatgag
 M  A  M  Q  G  S  D  G  L  F  G  P  L  I  V  H  S  K  N  E
cggaagctgcagcaactcgaatatgcctccgatcaagtcatcatggtccacgattactac
 R  K  L  Q  Q  L  E  Y  A  S  D  Q  V  I  M  V  H  D  Y  Y
cacgatctgaccagcgcgctgataccacactacttagcgccggataacgagaacacagag
 H  D  L  T  S  A  L  I  P  H  Y  L  A  P  D  N  E  N  T  E
cctgtccctgacggaggtctcatcaacggaatgaataagagaaattgcgagctcctccgt
 P  V  P  D  G  G  L  I  N  G  M  N  K  R  N  C  E  L  L  R
ggtcgagactgtgatgccactgatgcacagcttgccacattcggcctcgaaccgaacaag
 G  R  D  C  D  A  T  D  A  Q  L  A  T  F  G  L  E  P  N  K
aaccaccgtcttcgaattatcaatactggagcatttgctgaattccaggtgaagattgac
 N  H  R  L  R  I  I  N  T  G  A  F  A  E  F  Q  V  K  I  D
gagcacacgttcgctgtgacggaggtggatgggaccgaagttgctcccgcctactaccac
 E  H  T  F  A  V  T  E  V  D  G  T  E  V  A  P  A  Y  Y  H
aggctcaacatcaatcccggacagcgctacagcatcgtaataaacaccaatgtcacggat
 R  L  N  I  N  P  G  Q  R  Y  S  I  V  I  N  T  N  V  T  D
cgtgactccttctggctgagagctaagatgattgaggcctgtttcgctgaggagaaccca
 R  D  S  F  W  L  R  A  K  M  I  E  A  C  F  A  E  E  N  P
aatctggatcccgaagtgcgcgccattatccaatatactcgcaaggacgaggatacccag
 N  L  D  P  E  V  R  A  I  I  Q  Y  T  R  K  D  E  D  T  Q
cccaaggaaccttcgagcagagactgggacgacatcgtggacatgcagtgtctcgacatg
 
P K E P S S R D W D D I V D M Q C L D M
aacgtgacagagctccagcccgtagagaaggcaacacctccacctgcagacaccacacta
N V T E L Q P V E K A T P P P A D T T L
tacctccgctccaacttcgagatcggcaactggcgtctgagccgcggcttcttcaacagc
Y L R S N F E I G N W R L S R G F F N S
tcgtcctggcgtccaacactctcatccccaagcctgcaccgcatgatcgacggcctccac
S S W R P T L S S P S L H R M I D G L H
agccaaaacgccagcttcctccccgaccgagcgtaccccttccagatcaactcggccggc
S Q N A S F L P D R A Y P F Q I N S A G
ttcgacactgggcccgagctggtctaccagaccagcggcatccgcaccatcgacatcctc
F D T G P E L V Y Q T S G I R T I D I L
gtttccaacttcgacgacggcaaccacccgctccacctgcacggctacaagtacttcgtc
V S N F D D G N H P L H L H G Y K Y F V
cttgcgtcgggccacggctacccgcccgccgacctctacgcgcatctcgacatctcgaac
L A S G H G Y P P A D L Y A H L D I S N
ccgctgcgccgcgacaccgcctcgatcgaggcgttcggctggatcctactgcgtctcgtc
P L R R D T A S I E A F G W I L L R L V
gccgacaacccgggcgtctgggccttccactgccacatcggctggcacaccgaggccggc
A D N P G V W A F H C H I G W H T E A G
atgctgatgcagttcgccacgcgcgtcgacgtgctcgcatccagccaaatcccggatacg
M L M Q F A T R V D V L A S S Q I P D T
cacctcgcgctctgcgcggccgacgggctcgaccgcggcgcgtcgccgccagactcgacg
H L A L C A A D G L D R G A S P P D S T
tggtttggggatttggggatctagatccttga
W F G D F G D L D P -
SEQ ID NO : 81
   LENGTH : 690
   TYPE : PRT
   ORGANISM : *M. phaseolina* 
MIDTKSTGSGADGGRYAALRQDESELYEQKEHTQCTQPSGAAYLGGNGREEALGLIDENVVARTKRR
CGPTRRYSVFLEFAILGLVLIIALLGALAWSRGSHHHTDPVSGSSQPSSKGRRGKYVLDPAWDFAAP
PQVRKYHWTIRDIELRPDGVKRPLITINNEFPGPTIECNQGDTVRVEVHNEAVNSTSFHWHGIYQNG
TTYMDGTVGISQCPITSGSSMTYEFKVDRESGTYWYHAHMAMQGSDGLFGPLIVHSKNERKLQQLEY
ASDQVIMVHDYYHDLTSALIPHYLAPDNENTEPVPDGGLINGMNKRNCELLRGRDCDATDAQLATFG
LEPNKNHRLRIINTGAFAEFQVKIDEHTFAVTEVDGTEVAPAYYHRLNINPGQRYSIVINTNVTDRD
SFWLRAKMIEACFAEENPNLDPEVRAIIQYTRKDEDTQPKEPSSRDWDDIVDMQCLDMNVTELQPVE
KATPPPADTTLYLRSNFEIGNWRLSRGFFNSSSWRPTLSSPSLHRMIDGLHSQNASFLPDRAYPFQI
NSAGFDTGPELVYQTSGIRTIDILVSNFDDGNHPLHLHGYKYFVLASGHGYPPADLYAHLDISNPLR
RDTASIEAFGWILLRLVADNPGVWAFHCHIGWHTEAGMLMQFATRVDVLASSQIPDTHLALCAADGL
DRGASPPDSTWFGDFGDLDP*
SEQ ID NO : 82
   LENGTH : 2372 (including 150 bp 5' UTR and 150 bp 3' UTR)
   TYPE : DNA
   ORGANISM : *M. phaseolina* 
TATATGTTCCTCCGTCCTCAACGGAAATCCGGCAGTGCGCAAGACGAGCCCCCTTTTCCTTCTGCTT
CGCCTGCTTTTCGTAGTCTCGTCCTCCGCCCTAGACGTCGTCTTCTCAGACAATTTGCTCAAATCCC
CTCTCACCTCGCCGCCATGCGCTTCGCGAGCCTCGCCGTGGCATGGCTCACAACGTGCGTTGTCCAA
TCACTTGCCAACGAGCCCATCCCGTTCGACAGGATCCTCTGGGGTGAGAATGGCCCCGCCGGTAACC
ATATGGTGAAGCGTCAGGCCAGCTCCAGCTCCCCTGCCTCATCCACCACAAGGGCCCCGGATTCCGC
CTGCACAAACGGCCCTTTGACGAGGAGCTGCTGGTCCAATGGCTTTTCCATAGCCACTGACTTTGAC
GCCAAGTGGCCCAATACCGGAAAGACCGTCCATGTAAGCGCGCCTGCAGATATGCATCCGTCCTGCT
GCTGACGTGCAGCCCATAGTATGACCTGACCATCAACAACGCCACCTGCAGCCCCGACGGCGGGCCC
AGCCGCCCGTGCCTGATGTTCAACAACAAGATCCCTGGGCCTACGCTTTACGCCAATTGGGGCGACA
 
TGATCTCCGTTACCATCACCAACAAGATGCCCAACAACGGCACCAGCGTGCACTGGCATGGTCTGCG
TCAGTACAATACAAACACCCAGGATGGCGTCAACGGAATCACGGAATGCCCTCTGGCTCCCGGCGAT
TCCAAGACCTACCTATTCCAGGCTACACAGTTCGGCACGACCTGGTTCCACAGCCATTTCTCTGCGC
AGTATGGCGACGGTGCCGTCGGCCAGCTCATCATCAATGGCCCCGCCTCGGCGAATTACGATTTCGA
TCTCGGCACCTACACTATGACCGACTGGTACTACAGCACTGCGTTCCAGGTCGAAGATCAATTTGAT
GCCGCCCTTCAGAGGAAGGCCCCCGGCCCGCCAGGTGACACCATCCTGGTGAACGGTACGATGAAGT
CTCCGGATGGCTCTGCTGGTAGCTACAGCCAAGTCAAGGGCCTTGTCAAGGGAAAGAAGTACCGCCT
GCGTCTCATCAACACCTCGGTGGACAACAACATCCGCGTCTCGCTGGACAACCACCCATTCACCGTC
GTCACTTCCGACTTCGTCCCGAGCAAGCCTTGGACTACTGACTGGCTTCTCTTAGCCATCGGCCAGC
GCTACGATGTCATCTTCACGGCCAATCAACCCGCGGCCAATTATTGGTTCCGTGCAGAAGTTGCCAC
CGCATGTGCTAGCGCCAACAAGTACCGCGGCCGCGGTATATTCAGCTACGTTGGTGCCGATGGCAGC
GCTCCCCCAGAGACCGCCGTGACTGTTCCAGGTGGCTGTACCGAGCCTCTGCCTGCGCCTTTCGTCG
CAAACCAGGTTCCAAGCCAAGTCTTCCTCGACCAAGTGAAGACCCTTAGCGTCGATGTTTATGCGGC
AAACGTCTCGACCAACCAGAAGAACATTGTCTTCTGGGGCATCAACATGACTGCCATTGACATTGAT
TGGGAGAAGCCGACGCTGGAATACGTCAGGACAAAAAATACCAGCTACCCCCACGTTTACAACTTGA
TCGAGTTGCCCACAGAGAACATTGTAAGCGGAGTTAAGCTTCAACCCCGAGAAATGTTATTAACGAT
CGCAACAGTGGACCTACTGGATCATCCAAGAAACTCCCGGCACTCCCCCAATTCCGCATCCAATTCA
CTTGCACGGTAAGCTGCAGGTCTCAACCTTGCAACCCCGTAGAGATACTGATGGTCGCCTCTGTCCA
GGCCACGACTTCTACATCCTCGGAACCGGCTCTGGTGCCTTCGACCGCAGCACCTCGCCGTCCTCCC
TCAATTTCAATAACCCCACCCGGCGCGACGTCGCACTGGTTCCCGGCGGCGGTTGGTTGGCCATTGC
CTTCCCGACCGACAACCCAGGCGCTTGGCTCATGCACTGCCATATCGTAAGTTTACCTATCTCTCCG
ACTACGATCAATTGTGTATATTCATGCGCACGGCGCTGACACGTGGATTTCCCTCGCCACAGGCTTG
GCACATTAGCGAAGGTCTCGGAGTTCAGTTCCTCGAGGGCAAGGATAAGATCAATCTTCCCGACGCT
GCGTGGGAGACGACCTGCTCGAATTGGGACAAGTACTGGACACCACTATCTACCCAAGCAGGATT
CCGGTCTCTGAGGGGTGGCCGGTTTTTTTCTTCTTTACGGGCCTGAGTTTAAGGGGGGTTTGATGT
TTTGCTGAAAAAAGACCCGTAGTGATTCTTGTATTATACTTTTCGTTTTATTCATATTGATAGGAAG
CAACCTGATCGCACTTCGTAAACCGCA
SEQ ID NO : 83
   LENGTH : 1824
   TYPE : DNA
   ORGANISM : *M. phaseolina*
   FEATURE NAME/KEY: CDS
   LOCATION : (1)......(1824) 
atgcgcttcgcgagcctcgccgtggcatggctcacaacgtgcgttgtccaatcacttgcc
 M  R  F  A  S  L  A  V  A  W  L  T  T  C  V  V  Q  S  L  A
aacgagcccatcccgttcgacaggatcctctggggtgagaatggccccgccggtaaccat
 N  E  P  I  P  F  D  R  I  L  W  G  E  N  G  P  A  G  N  H
atggtgaagcgtcaggccagctccagctcccctgcctcatccaccacaagggccccggat
 M  V  K  R  Q  A  S  S  S  S  P  A  S  S  T  T  R  A  P  D
tccgcctgcacaaacggccctttgacgaggagctgctggtccaatggcttttccatagcc
 S  A  C  T  N  G  P  L  T  R  S  C  W  S  N  G  F  S  I  A
actgactttgacgccaagtggcccaataccggaaagaccgtccattatgacctgaccatc
 T  D  F  D  A  K  W  P  N  T  G  K  T  V  H  Y  D  L  T  I
aacaacgccacctgcagccccgacggcggcccagccgcccgtgcctgatgttcaacaac
 N  N  A  T  C  S  P  D  G  G  P  S  R  P  C  L  M  F  N  N
aagatccctggccctacgctttacgccaattggggcgacatgatctccgttaccatcacc
 K  I  P  G  P  T  L  Y  A  N  W  G  D  M  I  S  V  T  I  T
aacaagatgcccaacaacggcaccagcgtgcactggcatggtctgcgtcagtacaataca
 N  K  M  P  N  N  G  T  S  V  H  W  H  G  L  R  Q  Y  N  T
aacacccaggatggcgtcaacggaatcacggaatgccctctggctcccggcgattccaag
 N  T  Q  D  G  V  N  G  I  T  E  C  P  L  A  P  G  D  S  K
acctacctattccaggctacacagttcggcacgacctggttccacagccatttctctgcg
 T  Y  L  F  Q  A  T  Q  F  G  T  T  W  F  H  S  H  F  S  A
cagtatggcgacggtgccgtcggccagctcatcatcaatggccccgcctcggcgaattac
 
Q Y G D G A V G Q L I I N G P A S A N Y
gatttcgatctcggcacctacactatgaccgactggtactacagcactgcgttccaggtc
D F D L G T Y T M T D W Y Y S T A F Q V
gaagatcaatttgatgccgcccttcagaggaaggcccccggcccgccaggtgacaccatc
E D Q F D A A L Q R K A P G P P G D T I
ctggtgaacggtacgatgaagtctccggatggctctgctggtagctacagccaagtcaag
L V N G T M K S P D G S A G S Y S Q V K
ggccttgtcaagggaaagaagtaccgcctgcgtctcatcaacacctcggtggacaacaac
G L V K G K K Y R L R L I N T S V D N N
atccgcgtctcgctggacaaccacccattcaccgtcgtcacttccgacttcgtcccgagc
I R V S L D N H P F T V V T S D F V P S
aagccttggactactgactggcttctcttagccatcggccagcgctacgatgtcatcttc
K P W T T D W L L L A I G Q R Y D V I F
acggccaatcaacccgcggccaattattggttccgtgcagaagttgccaccgcatgtgct
T A N Q P A A N Y W F R A E V A T A C A
agcgccaacaagtaccgcggccgcggtatattcagctacgttggtgccgatggcagcgct
S A N K Y R G R G I F S Y V G A D G S A
cccccagagaccgccgtgactgttccaggtggctgtaccgagcctctgcctgcgcctttc
P P E T A V T V P G G C T E P L P A P F
gtcgcaaaccaggttccaagccaagtcttcctcgaccaagtgaagacccttagcgtcgat
V A N Q V P S Q V F L D Q V K T L S V D
gtttatgcggcaaacgtctcgaccaaccagaagaacattgtcttctggggcatcaacatg
V Y A A N V S T N Q K N I V F W G I N M
actgccattgacattgattgggagaagccgacgctggaatacgtcaggacaaaaaatacc
T A I D I D W E K P T L E Y V R T K N T
agctacccccacgtttacaacttgatcgagttgcccacagagaacattggacctactgg
S Y P H V Y N L I E L P T E N I W T Y W
atcatccaagaaactcccggcactcccccaattccgcatccaattcacttgcacggccac
I I Q E T P G T P P I P H P I H L H G H
gacttctacatcctcggaaccggctctggtgccttcgaccgcagcacctcgccgtcctcc
D F Y I L G T G S G A F D R S T S P S S
ctcaatttcaataaccccacccggcgcgacgtcgcactggttcccggcggcggttggttg
L N F N N P T R R D V A L V P G G G W L
gccattgccttcccgaccgacaacccaggcgcttggctcatgcactgccatatcgcttgg
A I A F P T D N P G A W L M H C H I A W
cacattagcgaaggtctcggagttcagttcctcgagggcaaggataagatcaatcttccc
H I S E G L G V Q F L E G K D K I N L P
gacgctgcgtgggagacgacctgctcgaattgggacaagtactgggacaccactatctac
D A A W E T T C S N W D K Y W D T T I Y
cccaagcaggattccggtctctga
P K Q D S G L -
SEQ ID NO : 84
   LENGTH : 607
   TYPE : PRT
   ORGANISM : *M. phaseolina* 
MRFASLAVAWLTTCVVQSLANEPIPFDRILWGENGPAGNHMVKRQASSSSPASSTTRAPDSACTNGP
LTRSCWSNGFSIATDFDAKWPNTGKTVHYDLTINNATCSPDGGPSRPCLMFNNKIPGPTLYANWGDM
ISVTITNKMPNNGTSVHWHGLRQYNTNTQDGVNGITECPLAPGDSKTYLFQATQFGTTWFHSHFSAQ
YGDGAVGQLIINGPASANYDFDLGTYTMTDWYYSTAFQVEDQFDAALQRKAPGPPGDTILVNGTMKS
PDGSAGSYSQVKGLVKGKKYRLRLINTSVDNNIRVSLDNHPFTVVTSDFVPSKPWTTDWLLLAIGQR
YDVIFTANQPAANYWFRAEVATACASANKYRGRGIFSYVGADGSAPPETAVTVPGGCTEPLPAPFVA
NQVPSQVFLDQVKTLSVDVYAANVSTNQKNIVFWGINMTAIDIDWEKPTLEYVRTKNTSYPHVYNLI
ELPTENIWTYWIIQETPGTPPIPHPIHLHGHDFYILGTGSGAFDRSTSPSSLNFNNPTRRDVALVPG
 
GGWLAIAFPTDNPGAWLMHCHIAWHISEGLGVQFLEGKDKINLPDAAWETTCSNWDKYWDTTIYPKQ
DSGL*
SEQ ID NO : 85
   LENGTH : 3008 (including 150 bp 5' UTR and 150 bp 3' UTR)
   TYPE : DNA
   ORGANISM : *M. phaseolina* 
GAGAAGAGCTTTTCGAAGCCATGTCAGCAGGATTAGCTCCTGAGACAGCCCTGCCCCAAAGTCCCGT
ATTTCTATGGCCGCAGTTCTCTCTCCTCGCCGAACCAAAGCCTCCTCCAGTACTTTCTTCTCAAACC
TGCTCATACAAGTGCAATGGGGTTCTTCAATGCCTGCTGGGACTTGCTGCTCCACCTCCCTTTCTTT
GCTCCGCGGGGCGGCGGCTTCGAGCGGATCGCTCGCAGCTTCCGATCTCCTCCGGTCCGAGTGGCG
GCGTAGTCATCCACCCGGAGAATGCTTCCCCTGGCTTCACCTGCTCTTACCCGAGCATGGAAGGGTG
GGAAAGCTGCAACTCACCCGACGACAGGAGCTGCTGGCTTAAAGATGGGAGGGCGAGTCAGCCTTAC
TTCTCTCAATACGATATCCACACAGACTGTAAGTCCTCGATATGGTTGCCCGCATTGCATGCTTTCA
GCTTTACCAGGCCTTCGTGCTAAATTTATGAAGACGAGACCGTCTGGCCTCAGGGTGTAACCAGAGA
AGTAAGTGTGGAAAATCAAAGCCCTGGGGCGCGGGGAACCCGGCCTCTGAGCCTTCATCTATGGCAG
CCCGTACACATCGAGAGACGAAGTATTTATCGCGAAGAGAATGCCCCTGATCTCTTTAAGCACATAC
ATCTGCATGAATATTTCAATTCCCCTTGTCATCAGGGATGATCAAGGGGCCATGCCGTTACCAAAAT
GTCTGGGCAGAGTCCCTTCTTTTCACGCTGCAGCAGATTTGCTTACTCGGTGTTTACTGAACATTTG
CAGTACTGGATCAACCTCAAGGACCAAGTGGTAAGTGCTGCCATTCCCTTGAGTCCGCTGTAGCTGC
TCATACGCCCAGCTTTTCCCCGACGGTTATTCCAAGCCTTATGGCAAAGTAATCAACGACACCTACC
CAGGTCCTTTAATCGAGGCTTGCTGGGGCGATGAAGTAGTTGTTCATGTGACAAACTACCTGCAGAC
GAATGGCACTACGTATTCACACGTCTATATCAGTTGTGCCGCGGTATGAGCTGATGATGGGGCAGTA
TTCACTGGCACGGTGTGAGACAGCAGTTCAGCAACGAAATGGACGGAGTCAACGGTTTGTTCTGCAA
GATCCCACTATTTACCTCCTCGGCTCACGTCATGTAGGTATTACACAGTGTCCTATCGCTTATGGTG
ACACCTTCACCTACCGCTTTCGTGTTACTCAATATGGAACTACGTGTAAGTGTTTCCGACTCCTGAT
CTGGTCTCAGCCATTGACACGGAAGTAGGGTACCATTCACACTACTCGCTCCAATACCCAGATGGCG
TTGCTGGCCCTCTCGTATTTCACGGCCCTACAGCAGCCGATTGGGATGAAGAGTGGAGACCCCGCT
GATGATCACTGATTGGGTTCACGATTCGGCTTTTGGGGTCTTCTCCCAGGAACTCCTCGCGTCGGAT
CCTGCCAACCGGAACGTCACACCACCCGTGGGGGACAGCATTCTGCTTAACGGACACGGACATTACA
ACTGCAGCCTTTCTCAGGACCAAAACCGCTGCGCGCCGGGATATGGATCTTACTACACGCAAAGATT
CCAAAAGGGTAAAAGGTACTTGATCAGGCTAATTAACTCCTCAGCTGGAGCAGCATTTATCTTCTCC
ATAGACGGACACAAAATGAAGGTCATCTCCACGGATCTCGTTCCCATTGAGCCGTACGAGACAAATG
CAGTGCTCCTCAACATAGGTATATAGCGTTTGGTGCTCGTCCTAGCATGTCTCCCAGACACTAACTT
CCCGCAGGCCAACGCTACAACATCATCGTCGAAGCCAACGCCGAACCCGGCGACTACTGGATCCGTA
CCGAGATACCCGGCGGGCCAGGCGGCTGTGGCAGCGTGCACGACCGGGCCGGTAACGTGACGGGCAT
CCTGCGCTACGACGGACGCAGTACCGCGCTACCGACCTCATCGAAGAATGACTACCCGTCGGACTGC
CACGATGAGCCAGCGGAGCTGCTGCACCCAATCCTGCCGTGGACGGTGGATCCGCACCCGCAGAACG
ACGTACACAACAACACGTACGAGGTCGGCATTTCAGACGCCCAGTTCCACAAGGCCTTCCGCTGGGA
CCTGACCGACACGCCCATGTGGCTCGACTTCTCAAACCCGACCATCCTCAACCTGTACAACACCACC
TGGAACCCGGAGTACGCCGTCATCGACTGTGAGTTGTCCTTCTTACCTCCCGTTCTCCCCAAAAAAC
ACTAGCAACGAGTGGATGAAGACTGACAGGTACGAAAACAGACAACTATGACCGCGGCTTCGTCTAC
CTCGTCATCACGGCCAACCTGACACGGCTGGGCGACAACAAGCGCGAGATCCCCGCCGGCCACCCCA
TCCACCTGCACGGCCACGACTTCGCCGTCCTCGCCCAGTCCAACTCGACCTATGACGAGCGCTCCGA
CCCGCTCAATTTCACCCTCGCCAACCCGCCGCGCCGCGATGTCGTCTTCCTGCCGAGCAACGGCTAC
GTCGCGCTGGCGTTCAAGCCGGACAATCCCGGCATATGGTTGGTGCATTGCCATATCGCTTGGCATG
CCAGTTCTGGTGAGTGTTTTTCTTTTTTCTTTTTGGTAACTTTGTTTTCTGTGGCTTTTTAAAAGT
GTGTGAATGCATGCTGATGATTTTTATACGATGCACAGGCCTGGCACTGCAGATTCTGGAGAGGCAG
CCAGATATCCTGGATTCGATTGGCACGCTCGAGGCGACGAATAAGACGTGTGCTGGATGGGATACGT
ACGAGAGGGCGCATCCGATCGAGCAAGACGACAGCGGTATCTGATTAGACGTACCTCTGCGTCAGAT
CCGACGAGTTGGGAGTGAATGATGCCCTCTGGAAGGATTGTGAAGGATGGGGTGAGCTCTTTCTCCT
GTGCATTTTCTCTCATGTTGAGGAGCTCTGTTCGTAGCTGGAGTACGCTCATTTATTTCC
SEQ ID NO : 86
   LENGTH : 1932
   TYPE : DNA
   ORGANISM : *M. phaseolina*
   FEATURE NAME/KEY: CDS
   LOCATION : (1)......(1932) 
atggggttcttcaatgcctgctgggacttgctgctccacctccctttctttgctccgcgg
 M  G  F  F  N  A  C  W  D  L  L  L  H  L  P  F  F  A  P  R
ggcggcggcttcgagcgggatcgctcgcagcttccgatctcctccggtccgagtggcggc
 G  G  G  F  E  R  D  R  S  Q  L  P  I  S  S  G  P  S  G  G
gtagtcatccacccggagaatgcttccccctggcttcacctgctcttacccgagcatggaa
 V  V  I  H  P  E  N  A  S  P  G  F  T  C  S  Y  P  S  M  E
gggtgggaaagctgcaactcacccgacgacaggagctgctggcttaaagatggagggcg
 G  W  E  S  C  N  S  P  D  D  R  S  C  W  L  K  D  G  R  A
agtcagccttacttctctcaatacgatatccacacagactacgagaccgtctggcctcag
 S  Q  P  Y  F  S  Q  Y  D  I  H  T  D  Y  E  T  V  W  P  Q
ggtgtaaccagagaatactggatcaacctcaaggaccaagtgcttttccccgacggttat
 G  V  T  R  E  Y  W  I  N  L  K  D  Q  V  L  F  P  D  G  Y
tccaagccttatggcaaagtaatcaacgacacctacccaggtcctttaatcgaggcttgc
 S  K  P  Y  G  K  V  I  N  D  T  Y  P  G  P  L  I  E  A  C
tggggcgatgaagtagttgttcatgtgacaaactacctgcagacgaatggcactactatt
 W  G  D  E  V  V  V  H  V  T  N  Y  L  Q  T  N  G  T  T  I
cactggcacggtgtgagacagcagttcagcaacgaaatggacggagtcaacggtattaca
 H  W  H  G  V  R  Q  Q  F  S  N  E  M  D  G  V  N  G  I  T
cagtgtcctatcgcttatggtgacaccttcacctaccgctttcgtgttactcaatatgga
 Q  C  P  I  A  Y  G  D  T  F  T  Y  R  F  R  V  T  Q  Y  G
actacgtggtaccattcacactactcgctccaatacccagatggcgttgctggccctctc
 T  T  W  Y  H  S  H  Y  S  L  Q  Y  P  D  G  V  A  G  P  L
gtatttcacggccctacagcagccgattgggatgaagagtgggagacccgctgatgatc
 V  F  H  G  P  T  A  A  D  W  D  E  E  W  E  T  P  L  M  I
actgattgggttcacgattcggcttttggggtcttctcccaggaactcctcgcgtcggat
 T  D  W  V  H  D  S  A  F  G  V  F  S  Q  E  L  L  A  S  D
cctgccaaccggaacgtcacaccacccgtgggggacagcattctgcttaacggacacgga
 P  A  N  R  N  V  T  P  P  V  G  D  S  I  L  L  N  G  H  G
cattacaactgcagcctttctcaggaccaaaaccgctgcgcgccgggatatggatcttac
 H  Y  N  C  S  L  S  Q  D  Q  N  R  C  A  P  G  Y  G  S  Y
tacacgcaaagattccaaaagggtaaaaggtacttgatcaggctaattaactcctcagct
 Y  T  Q  R  F  Q  K  G  K  R  Y  L  I  R  L  I  N  S  S  A
ggagcagcatttatcttctccatagacggacacaaaatgaaggtcatctccacggatctc
 G  A  A  F  I  F  S  I  D  G  H  K  M  K  V  I  S  T  D  L
gttcccattgagccgtacgagacaaatgcagtgctcctcaacataggccaacgctacaac
 V  P  I  E  P  Y  E  T  N  A  V  L  L  N  I  G  Q  R  Y  N
atcatcgtcgaagccaacgccgaacccggcgactactggatccgtaccgagatacccggc
 I  I  V  E  A  N  A  E  P  G  D  Y  W  I  R  T  E  I  P  G
gggccaggcggctgtggcagcgtgcacgaccggccggtaacgtgacgggcatcctgcgc
 G  P  G  G  C  G  S  V  H  D  R  A  G  N  V  T  G  I  L  R
tacgacggacgcagtaccgcgctaccgacctcatcgaagaatgactacccgtcggactgc
 Y  D  G  R  S  T  A  L  P  T  S  S  K  N  D  Y  P  S  D  C
cacgatgagccagcggagctgctgcacccaatcctgccgtggacggtggatccgcacccg
 H  D  E  P  A  E  L  L  H  P  I  L  P  W  T  V  D  P  H  P
cagaacgacgtacacaacaacacgtacgaggtcggcatttcagacgcccagttccacaag
 Q  N  D  V  H  N  N  T  Y  E  V  G  I  S  D  A  Q  F  H  K
gccttccgctgggacctgaccgacacgcccatgtggctcgacttctcaaacccgaccatc
 A  F  R  W  D  L  T  D  T  P  M  W  L  D  F  S  N  P  T  I
ctcaacctgtacaacaccacctggaacccggagtacgccgtcatcgactacaactatgac
 
 L  N  L  Y  N  T  T  W  N  P  E  Y  A  V  I  D  Y  N  Y  D
cgcggcttcgtctacctcgtcatcacggccaacctgacacggctgggcgacaacaagcgc
 R  G  F  V  Y  L  V  I  T  A  N  L  T  R  L  G  D  N  K  R
gagatccccgccggccaccccatccacctgcacggccacgacttcgccgtcctcgcccag
 E  I  P  A  G  H  P  I  H  L  H  G  H  D  F  A  V  L  A  Q
tccaactcgacctatgacgagcgctccgacccgctcaatttcaccctcgccaacccgccg
 S  N  S  T  Y  D  E  R  S  D  P  L  N  F  T  L  A  N  P  P
cgccgcgatgtcgtcttcctgccgagcaacggctacgtcgcgctggcgttcaagccggac
 R  R  D  V  V  F  L  P  S  N  G  Y  V  A  L  A  F  K  P  D
aatcccggcatatggttggtgcattgccatatcgcttggcatgccagttctggcctggca
 N  P  G  I  W  L  V  H  C  H  I  A  W  H  A  S  S  G  L  A
ctgcagattctggagaggcagccagatatcctggattcgattggcacgctcgaggcgacg
 L  Q  I  L  E  R  Q  P  D  I  L  D  S  I  G  T  L  E  A  T
aataagacgtgtgctggatgggatacgtacgagagggcgcatccgatcgagcaagacgac
 N  K  T  C  A  G  W  D  T  Y  E  R  A  H  P  I  E  Q  D  D
agcggtatctga
 S  G  I  -
SEQ ID NO : 87
   LENGTH : 643
   TYPE : PRT
   ORGANISM : *M. phaseolina* 
MGFFNACWDLLLHLPFFAPRGGGFERDRSQLPISSGPSGGVVIHPENASPGFTCSYPSMEGWESCNS
PDDRSCWLKDGRASQPYFSQYDIHTDYETVWPQGVTREYWINLKDQVLFPDGYSKPYGKVINDTYPG
PLIEACWGDEVVVHVTNYLQTNGTTIHWHGVRQQFSNEMDGVNGITQCPIAYGDTFTYRFRVTQYGT
TWYHSHYSLQYPDGVAGPLVFHGPTAADWDEEWETPLMITDWVHDSAFGVFSQELLASDPANRNVTP
PVGDSILLNGHGHYNCSLSQDQNRCAPGYGSYYTQRFQKGKRYLIRLINSSAGAAFIFSIDGHKMKV
ISTDLVPIEPYETNAVLLNIGQRYNIIVEANAEPGDYWIRTEIPGGPGGCGSVHDRAGNVTGILRYD
GRSTALPTSSKNDYPSDCHDEPAELLHPILPWTVDPHPQNDVHNNTYEVGISDAQFHKAFRWDLTDT
PMWLDFSNPTILNLYNTTWNPEYAVIDYNYDRGFVYLVITANLTRLGDNKREIPAGHPIHLHGHDFA
VLAQSNSTYDERSDPLNFTLANPPRRDVVFLPSNGYVALAFKPDNPGIWLVHCHIAWHASSGLALQI
LERQPDILDSIGTLEATNKTCAGWDTYERAHPIEQDDSGI*
SEQ ID NO : 88
   LENGTH : 2404 (including 150 bp 5' UTR and 150 bp 3' UTR)
   TYPE : DNA
   ORGANISM : *M. phaseolina* 
AAATGCCATGGACAGCTCGCCCGGGATCGATGTCCTTTCTCCCCGAGCCCCCGTCGCCCGTCCAGGA
GAGATAAACCTTCTCATAGGGCCTCATTCCTACTCTCTCCTTCCCTTTTtCGTTACCCTCCCCGACC
GCGGTCCACCTTCGACATGTGGTGGTTGGCATTGTTCTCATTGCTTGTGGCCGCCACGGCCTGGGCC
AAGGAGCCCTACCTCAAGGTCCACGATGATACCTTTATTCCAGATGCCGTGTTGCGGGTAACAGAGG
AGAGTGCCTCCATTGGTTGCATCGAGAGGACCTCCGCCGTGGTCAACGGCACAGTTCCGGGGCCGAT
TTTGGAATTCCAATCCGGCAGCGTGGTATGGGTTCGTGTTTACAATGATATGGCAGACAAAAACCTC
ACCATGGTAAGCCCCGACGATATGCCTGCGGTGAATCAGACTAGTCAATTAACTTCTCGGTCCACAG
CATTGGCATGGCCTCACCATGGCAGCCGCTCCATTCGCCGACGGTTCCGTTGCAGCCAGCCAGTGGG
CGATCGAGCCGTTCAAATTCTTCGACTACGAGCTCAACCTATTCGACATCAAGCCTGGGACGTATTT
CTACCACTCCCATGTCGGATTCCAGGCAATTACTGCCACGGGTCCCCTGCTCATTACCAAAAAGCCG
GGAGAGGAGCCGCCGTATGAGTACGAGGAGGAACGCATCGTCCTCTTTTCCGACCTTTACAACACGA
CGGACCACGACATTGAAACCGGACTGGTAGCCAGCCCTTTCAAATGGAGCGGTGAGGTCGGAGACGT
CCTCGTCAACGGATATGGCATATCGCAGTACCCGGCCACGAACGACGCCGAGAGTTGCAACCTTGCA
CAGATTCCCGTGgAAGCTGGGAAGACGTACCGTTTGCGTTTCATTGGTGCCACTGCCCTTTCTTTCC
TCTCCGTTGGCTTTGAGAAGCACAATCTTACCATCATCGAGGCGGATGGCCACTACACGGAACCTGC
AGAAATCAGCTTCCTTCAAATCGGCGGCGGCCAACGGTATTCTGCTCTCCTAAAGACATGGACTTGC
GAGGAGCTCGCCGCCAAGACTGCTGGTCGAAACCAGTTCTACATCCAAATCGAGACTCGTGACAGGC
CCAAGAACCTCACCACCTACGCCATTCTGGACTACAGCGACAGCTGCGCCACCAATAGCATCAGCGT
 
CGAGGCCACCATCGGCAAGGACTCAACATCGCCCCCTTCCTACGCCCCTCCTTCGCTCAGCAAACCC
GGCAACAACAACAAAAACAACAACAACACCACGCTCTCCCGCAAGACGCCGCCCAAGACCCCACCGC
TGCACCTCCCGCCCACCGTGCAAGGCTGGCTCGACCATGACCTCCACCCGCTCGAAACCTACACGGA
CTTCCCGACGGCGGACGAGGTCACGCGCACCGTCTACATGGACATCTTCCAGCTAGGGCAGGACGGC
TACGTCAAGTGGGCGCAGAACAACCTGTCGTGGTACGAGCACACGCCCAAGGTTCCCTACCTCGTCG
CGCTCTACACCAACAGCACGCAGTACCTGCCCGACTACGACTATGCCGTCGCATCGGGGACGGCCA
CGACGACCGCGTCGGTGCCTGGCCCGCCAAGATGGGCGAGGTGCTCGAGATCATCGTCGTCAACACG
GGCAGCTACAGCGGCGGCATGGACGTGCATCCCATGCACCTGCACGGCGCGCACCCGTTCTATTTGG
GGAGTGGGAAGGGCACGTACAACAGAGAGGAGAACGAGAAGAAGTTGGCGGGGAGGGTGCCGGTCAC
GAGGGATTCGATGATGCTGTATCGGTATGGCGAGAAGGAGGAGCCGCACAAGGATAATAGCTGGATT
GCGCTGAGGATAAGGGTGACGCAGCCGGGGGTGTGGATGTTCCATTGCCATACGCTGGCGCATATGA
TTATGGGTGAGTTTGACCCTTTTtCCCTTTTTTTTTTTTTTTTTtCTtATTGCGCATGTTGGTTTGAT
GAAAACCATGGTGCTGATGTGATTTGGAACAGGTATGCAAACGGTCTGGGTGTTTGGCGATTCGAAG
GATATCCTCACGCTGCCGCTGCCGATGGTGGAAGGTTACTTGGTGCCTGGCGGAGATGTGTTCGGTG
ATGATGATCATGATCCGGTGGTGGTGCACTTCTTTGACCTGGACGACGACGACGATGATGATGA
TGCCAATAAGACCGACGGAACGGCGGGAAGAATGGCCGGTAGATGGGGGGAGGAGAGTTACTTGAT
TCGCAATGGAAGAGGTTACTGCGGAGGGGATTTGTAATGCATGCTACAGTTTTTATGTTATATGCCA
GGTATAACCAGAAGGAAAGCCGGATGATTAATCGTGGAAACAAGAGAAAGAAGCATTTA
SEQ ID NO : 89
   LENGTH : 1950
   TYPE : DNA
   ORGANISM : *M. phaseolina*
   FEATURE NAME/KEY: CDS
   LOCATION : (1)......(1950) 
atgtggtggttggcattgttctcattgcttgtggccgccacggcctgggccaaggagccc
 M  W  W  L  A  L  F  S  L  L  V  A  A  T  A  W  A  K  E  P
tacctcaaggtccacgatgatacctttattccagatgccgtgttgcgggtaacagaggag
 Y  L  K  V  H  D  D  T  F  I  P  D  A  V  L  R  V  T  E  E
agtgcctccattggttgcatcgagaggacctccgccgtggtcaacggcacagttccgggg
 S  A  S  I  G  C  I  E  R  T  S  A  V  V  N  G  T  V  P  G
ccgattttggaattccaatccggcagcgtggtatgggttcgtgtttacaatgatatggca
 P  I  L  E  F  Q  S  G  S  V  V  W  V  R  V  Y  N  D  M  A
gacaaaaacctcaccatgcattggcatggcctcaccatggcagccgctccattcgccgac
 D  K  N  L  T  M  H  W  H  G  L  T  M  A  A  A  P  F  A  D
ggttccgttgcagccagccagtgggcgatcgagccgttcaaattcttcgactacgagctc
 G  S  V  A  A  S  Q  W  A  I  E  P  F  K  F  F  D  Y  E  L
aacctattcgacatcaagcctgggacgtatttctaccactcccatgtcggattccaggca
 N  L  F  D  I  K  P  G  T  Y  F  Y  H  S  H  V  G  F  Q  A
attactgccacgggtcccctgctcattaccaaaaagccgggagaggagccgccgtatgag
 I  T  A  T  G  P  L  L  I  T  K  K  P  G  E  E  P  P  Y  E
tacgaggaggaacgcatcgtcctctttccgacctttacaacacgacggaccacgacatt
 Y  E  E  E  R  I  V  L  F  S  D  L  Y  N  T  T  D  H  D  I
gaaaccggactggtagccagccctttcaaatggagcggtgaggtcggagacgtcctcgtc
 E  T  G  L  V  A  S  P  F  K  W  S  G  E  V  G  D  V  L  V
aacggatatggcatatcgcagtacccggccacgaacgacgccgagagttgcaaccttgca
 N  G  Y  G  I  S  Q  Y  P  A  T  N  D  A  E  S  C  N  L  A
cagattcccgtggaagctggaagacgtaccgtttgcgtttcattggtgccactgccctt
 Q  I  P  V  E  A  G  K  T  Y  R  L  R  F  I  G  A  T  A  L
tctttcctctccgttggctttgagaagcacaatcttaccatcatcgaggcggatggccac
 S  F  L  S  V  G  F  E  K  H  N  L  T  I  I  E  A  D  G  H
tacacggaacctgcagaaatcagcttccttcaaatcggcggcggccaacggtattctgct
 Y  T  E  P  A  E  I  S  F  L  Q  I  G  G  G  Q  R  Y  S  A
ctcctaaagacatggacttgcgaggagctcgccgccaagactgctggtcgaaaccagttc
 L  L  K  T  W  T  C  E  E  L  A  A  K  T  A  G  R  N  Q  F
 
tacatccaaatcgagactcgtgacaggcccaagaacctcaccacctacgccattctggac
 Y  I  Q  I  E  T  R  D  R  P  K  N  L  T  T  Y  A  I  L  D
tacagcgacagctgcgccaccaatagcatcagcgtcgaggccaccatcggcaaggactca
 Y  S  D  S  C  A  T  N  S  I  S  V  E  A  T  I  G  K  D  S
acatcgcccccttcctacgcccctccttcgctcagcaaacccggcaacaacaacaaaaac
 T  S  P  P  S  Y  A  P  P  S  L  S  K  P  G  N  N  N  K  N
aacaacaacaccacgctctcccgcaagacgccgcccaagacccccaccgctgcacctcccg
 N  N  N  T  T  L  S  R  K  T  P  P  K  T  P  P  L  H  L  P
cccaccgtgcaaggctggctcgaccatgacctccacccgctcgaaacctacacggacttc
 P  T  V  Q  G  W  L  D  H  D  L  H  P  L  E  T  Y  T  D  F
ccgacggcggacgaggtcacgcgcaccgtctacatggacatcttccagctaggcaggac
 P  T  A  D  E  V  T  R  T  V  Y  M  D  I  F  Q  L  G  Q  D
ggctacgtcaagtgggcgcagaacaacctgtcgtggtacgagcacacgcccaaggttccc
 G  Y  V  K  W  A  Q  N  N  L  S  W  Y  E  H  T  P  K  V  P
tacctcgtcgcgctctacaccaacagcacgcagtacctgcccgactacgactatgccgtc
 Y  L  V  A  L  Y  T  N  S  T  Q  Y  L  P  D  Y  D  Y  A  V
gcatcggggacgggccacgacgaccgcgtcggtgcctggcccgccaagatgggcgaggtg
 A  S  G  T  G  H  D  D  R  V  G  A  W  P  A  K  M  G  E  V
ctcgagatcatcgtcgtcaacacgggcagctacagcggcggcatggacgtgcatcccatg
 L  E  I  I  V  V  N  T  G  S  Y  S  G  G  M  D  V  H  P  M
cacctgcacggcgcgcacccgttctatttggggagtgggaagggcacgtacaacagagag
 H  L  H  G  A  H  P  F  Y  L  G  S  G  K  G  T  Y  N  R  E
gagaacgagaagaagttggcgggggaggtgccggtcacgagggattcgatgatgctgtat
 E  N  E  K  K  L  A  G  R  V  P  V  T  R  D  S  M  M  L  Y
cggtatggcgagaaggaggagccgcacaaggataatagctggattgcgctgaggataagg
 R  Y  G  E  K  E  E  P  H  K  D  N  S  W  I  A  L  R  I  R
gtgacgcagccgggggtgtggatgttccattgccatacgctggcgcatatgattatgggt
 V  T  Q  P  G  V  W  M  F  H  C  H  T  L  A  H  M  I  M  G
atgcaaacggtctgggtgtttggcgattcgaaggatatcctcacgctgccgctgccgatg
 M  Q  T  V  W  V  F  G  D  S  K  D  I  L  T  L  P  L  P  M
gtggaaggttacttggtgcctggcggagatgtgttcggtgatgatgatcatgatccggtg
 V  E  G  Y  L  V  P  G  G  D  V  F  G  D  D  D  H  D  P  V
gtggtgcacttctttgacctggacgacgacgacgacgatgatgatgatgccaataagacc
 V  V  H  F  F  D  L  D  D  D  D  D  D  D  D  D  A  N  K  T
gacgggaacggcgggaagaatggccggtag
 D  G  N  G  G  K  N  G  R  -
SEQ ID NO : 90
   LENGTH : 649
   TYPE : PRT
   ORGANISM : *M. phaseolina* 
MWWLALFSLLVAATAWAKEPYLKVHDDTFIPDAVLRVTEESASIGCIERTSAVVNGTVPGPILEFQS
GSVVWVRVYNDMADKNLTMHWHGLTMAAAPFADGSVAASQWAIEPFKFFDYELNLFDIKPGTYFYHS
HVGFQAITATGPLLITKKPGEEPPYEYEEERIVLFSDLYNTTDHDIETGLVASPFKWSGEVGDVLVN
GYGISQYPATNDAESCNLAQIPVEAGKTYRLRFIGATALSFLSVGFEKHNLTIIEADGHYTEPAEIS
FLQIGGGQRYSALLKTWTCEELAAKTAGRNQFYIQIETRDRPKNLTTYAILDYSDSCATNSISVEAT
IGKDSTSPPSYAPPSLSKPGNNNKNNNNTTLSRKTPPKTPPLHLPPTVQGWLDHDLHPLETYTDFPT
ADEVTRTVYMDIFQLGQDGYVKWAQNNLSWYEHTPKVPYLVALYTNSTQYLPDYDYAVASGTGHDDR
VGAWPAKMGEVLEIIVVNTGSYSGGMDVHPMHLHGAHPFYLGSGKGTYNREENEKKLAGRVPVTRDS
MMLYRYGEKEEPHKDNSWIALRIRVTQPGVWMFHCHTLAHMIMGMQTVWVFGDSKDILTLPLPMVEG
YLVPGGDVFGDDDHDPVVVHFFDLDDDDDDDDDANKTDGNGGKNGR*
SEQ ID NO : 91
   LENGTH : 2569 (including 150 bp 5' UTR and 150 bp 3' UTR)
   TYPE : DNA
   ORGANISM : *M. phaseolina* 
CGCCCGATTCACACCATTCCTCTGACACTCTCCTCCGGAGCTCGCATCAGGGCGCTCTCTCGCCCTC
CTGACCTTCCGTCCGGCGCCGATATCGTCGTGTTCCGTATATGAGGGTAATCGCGACGGTGCTCCC
GCCCACACTCTCAGCCATGGCCCAGTCTCTGTTCCTCCTGCTTGCTGCTGCCCTGTGCAGCCGCGCT
GCCACAGTCACGTATGACTTTAACGTGACCTGGGTCACCGCCAACCCTGATGCTGCTTTTCGTCGCA
CTACCATTGGCATCAATGGCCAGTGGCCGCTCCCTGCAATCGACGTCACCAAGGGCGACCGCGTTGT
CATCAACGTCAACAACCAGCTGGAAACGGAGAGTACCAGTTTGCATTTCCATGGAATCTACATGAAT
GGCACCAACCACATGGATGGCCCGACTGGCGTGACCCAGTGCGAGATACCGCCCGGAGCTCATTCA
CATACAATTTTACGGTATAGCACGATCCCACCCCTTGTTTACTTATAGCTAACCGCACGCAGGTCGA
CCAACCGGGAACTTACTGGTGAGTAGCCAGCTGTTGGCGTCAAGACTGGAAAGCTGACGTAGCTTC
AGGTATCATTCTCACAACCGCGGCCAGTATCCCGATGGATTGCGAGGGCCTTTTATTGTCAGAGACC
CCGACAATCCGTTCAAGGATGACTATGATGAGGAAGTCGTCCTGACATTCTCCGACTGGTACCACGA
CCGGATCCCCACCCTCATGAAGAGTTTCATTAGTGTCACAAACCCCACCGGCGCGGAGCCTGTCCCG
AACGCGGCACTGATGAATGACACTCAGAACCTCACCTTCCAGATGACTCCCGGCGGAGATACATGT
TCCGACTAATAAACATTGGTGCATTTGCTGCTCAATACGTCTGGTTCGAAGGCCATACCATGCGTAT
CGTAGAAGTCGACGGCGTGTACACCGAAGCCGCAGATGCAGAGCGAATTTACATGACTGCCGCTCAA
CGCTACAGCGTAATCATCACCGCAAAGAACGAATCTACCTCAAACTTCGCCTTTGTTGGAAGCATGG
ATCAGGTCAGCTATCCTTTTACTGGCCGACATTTTCCTTCTTCTAACATAATGCAGGATCTCTTTG
ATACTATTCCAGCAGGCCTTAATAATAACGTGACCGGGTGGCTTGTCTACAATCAACAGAACGGCTT
GTTGCCACCTTTAGCTATCGGGGACTACGATCCGTTCGATGACTTCACACTTGTGCCTCAAGATGGT
ATGGAGCTCTACGATCACGTCGATCATTCCATCACCCTCGATATGAAGATGGACAATCTCGGGGACG
GAGCAAATTAGTATGTTGACTGTAGTTGACAACGGCCAAATGCCATGACTGACGAGCGCAGTGCCTT
TTTCAACGACGTGACCTACGTCGAGCCCAAGGTGCCGACTCTGTACACGGTGTTGTCTACTGGCAAT
AATGCCACAGACTCGAGAATCTACGGTAGCAACACCAACAGCTTCATATTGGCTAAGGACGAAGTCG
TCGAGATCATCCTCAACAACAATGATCCGGGAAAGCACCCTTTCCATCTGCATGGGCACGCATTCCA
AGCAATCGTTCGCTCCGAAGAAGAAGCCGGCGCGTACGTGGCAAACGAAACCTTCCCCCAGACGCCA
ATGCGCCGCGATACAATTCTTGTCCGGCCCAATGGCAATATAGTACTGAGATTCAAGGCTGACAATC
CTGGTGTCTGGCTGTTCCATTGTCATATTGAATGGTATGCTCCTTGGTATCTTTTATCCTAGCTCTA
CTGACCTCTTACTGTTAGGCATGTTGCGTCGGGCCTTATTGCCACCATGATAGAGGCACCGCTTGAC
CTGCAATCTTCTCTCGGCAACAGCATTCCTGCTGATCACTGGCGAGCTTGTGCCGCCGCCGGCACAC
CTACTGCAGGCAACGCAGCAGGCAACACGATTGATTATCTTGACCTGACTGGTGAAAACAAGAGCCC
CGGCCCGCTTCCCTCTGGCTTTGAAGCGAAAGGCATTGTCGCGCTTGTCTTCAGTTGTATTGCTGCT
GTCTTGGGCATGGCAGCCATCGTATGGTACGGATGGCGCCTCTGACAGATGGAACTGGTCAGCAAC
AACACGTTGTGGATCAACAGCCGCAAGGACGCTCCGCACAAGTTGGCGTCATGCCCCTAGCGGTCGC
GGCAAAACGTGAGCAAAGGATGGCCAGCCGGACTGATTTGGAGGCAGAGATGCGGCAAGAAAAGAGC
GGAGAAATTACCATGGTGGAGCAGAGGAGGAGCAGCCGTGGGTCATCGAACATGCTGGATGTCAGGA
GGAGTAGCCGCGGTTCGTCTAACATGCTAGAGACCAGAAGAAGCAGTCGTGGCAGTGAGCAGATGCT
GTCATGAGGACCAACATCATGGTAGCAAGGTAAGTGGAAATCGGCTGTGAAGAAATACAGGGTCAGT
TGTGAGAGGGAAAGCTATCACGAATGGCTGAAGAGCTTTGGGCCTTGTTACGATGTAATGTTGTCCG
GAAATGTTGTGGTAATTATTGTA
SEQ ID NO : 92
   LENGTH : 2016
   TYPE : DNA
   ORGANISM : *M. phaseolina*
   FEATURE NAME/KEY: CDS
   LOCATION : (1)......(2016) 
atggcccagtctctgttcctcctgcttgctgctgccctgtgcagccgcgctgccacagtc
 M  A  Q  S  L  F  L  L  L  A  A  A  L  C  S  R  A  A  T  V
acgtatgactttaacgtgacctgggtcaccgccaaccctgatgctgcttttcgtcgcact
 T  Y  D  F  N  V  T  W  V  T  A  N  P  D  A  A  F  R  R  T
accattggcatcaatggccagtggccgctccctgcaatcgacgtcaccaagggcgaccgc
 T  I  G  I  N  G  Q  W  P  L  P  A  I  D  V  T  K  G  D  R
gttgtcatcaacgtcaacaaccagctggaaacggagagtaccagtttgcatttccatgga
 V  V  I  N  V  N  N  Q  L  E  T  E  S  T  S  L  H  F  H  G
 
atctacatgaatggcaccaaccacatggatggcccgactggcgtgacccagtgcgagata
 I  Y  M  N  G  T  N  H  M  D  G  P  T  G  V  T  Q  C  E  I
ccgcccgggagctcattcacatacaatttacggtcgaccaaccgggaacttactggtat
 P  P  G  S  S  F  T  Y  N  F  T  V  D  Q  P  G  T  Y  W  Y
cattctcacaaccgcggccagtatcccgatggattgcgagggccttttattgtcagagac
 H  S  H  N  R  G  Q  Y  P  D  G  L  R  G  P  F  I  V  R  D
cccgacaatccgttcaaggatgactatgatgaggaagtcgtcctgacattctccgactgg
 P  D  N  P  F  K  D  D  Y  D  E  E  V  V  L  T  F  S  D  W
taccacgaccggatccccaccctcatgaagagtttcattagtgtcacaaccccaccggc
 Y  H  D  R  I  P  T  L  M  K  S  F  I  S  V  T  N  P  T  G
gcggagcctgtcccgaacgcggcactgatgaatgacactcagaacctcaccttccagatg
 A  E  P  V  P  N  A  A  L  M  N  D  T  Q  N  L  T  F  Q  M
actcccggcggagatacatgttccgactaataaacattggtgcatttgctgctcaatac
 T  P  G  R  R  Y  M  F  R  L  I  N  I  G  A  F  A  A  Q  Y
gtctggttcgaaggccataccatgcgtatcgtagaagtcgacggcgtgtacaccgaagcc
 V  W  F  E  G  H  T  M  R  I  V  E  V  D  G  V  Y  T  E  A
gcagatgcagagcgaatttacatgactgccgctcaacgctacagcgtaatcatcaccgca
 A  D  A  E  R  I  Y  M  T  A  A  Q  R  Y  S  V  I  I  T  A
aagaacgaatctacctcaaacttcgcctttgttggaagcatggatcaggatctctttgat
 K  N  E  S  T  S  N  F  A  F  V  G  S  M  D  Q  D  L  F  D
actattccagcaggccttaataataacgtgaccggtggcttgtctacaatcaacagaac
 T  I  P  A  G  L  N  N  N  V  T  G  W  L  V  Y  N  Q  Q  N
ggcttgttgccacctttagctatcggggactacgatccgttcgatgacttcacacttgtg
 G  L  L  P  P  L  A  I  G  D  Y  D  P  F  D  D  F  T  L  V
cctcaagatggtatggagctctacgatcacgtcgatcattccatcaccctcgatatgaag
 P  Q  D  G  M  E  L  Y  D  H  V  D  H  S  I  T  L  D  M  K
atggacaatctcggggacggagcaaattatgcctttttcaacgacgtgacctacgtcgag
 M  D  N  L  G  D  G  A  N  Y  A  F  F  N  D  V  T  Y  V  E
cccaaggtgccgactctgtacacggtgttgtctactggcaataatgccacagactcgaga
 P  K  V  P  T  L  Y  T  V  L  S  T  G  N  N  A  T  D  S  R
atctacggtagcaacaccaacagcttcatattggctaaggacgaagtcgtcgagatcatc
 I  Y  G  S  N  T  N  S  F  I  L  A  K  D  E  V  V  E  I  I
ctcaacaacaatgatccgggaaagcaccctttccatctgcatggcacgcattccaagca
 L  N  N  N  D  P  G  K  H  P  F  H  L  H  G  H  A  F  Q  A
atcgttcgctccgaagaagaagccggcgcgtacgtggcaaacgaaaccttcccccagacg
 I  V  R  S  E  E  E  A  G  A  Y  V  A  N  E  T  F  P  Q  T
ccaatgcgccgcgatacaattcttgtccggcccaatggcaatatagtactgagattcaag
 P  M  R  R  D  T  I  L  V  R  P  N  G  N  I  V  L  R  F  K
gctgacaatcctggtgtctggctgttccattgtcatattgaatggcatgttgcgtcgggc
 A  D  N  P  G  V  W  L  F  H  C  H  I  E  W  H  V  A  S  G
cttattgccaccatgatagaggcaccgcttgacctgcaatcttctctcggcaacagcatt
 L  I  A  T  M  I  E  A  P  L  D  L  Q  S  S  L  G  N  S  I
cctgctgatcactggcgagcttgtgccgccgccggcacacctactgcaggcaacgcagca
 P  A  D  H  W  R  A  C  A  A  A  G  T  P  T  A  G  N  A  A
ggcaacacgattgattatcttgacctgactggtgaaaacaagagccccggcccgcttccc
 G  N  T  I  D  Y  L  D  L  T  G  E  N  K  S  P  G  P  L  P
tctggctttgaagcgaaaggcattgtcgcgcttgtcttcagttgtattgctgctgtcttg
 S  G  F  E  A  K  G  I  V  A  L  V  F  S  C  I  A  A  V  L
ggcatggcagccatcgtatggtacggatggcgcctctgacagatggaactggtcagcaa
 G  M  A  A  I  V  W  Y  G  M  A  P  L  T  D  G  T  G  Q  Q
caacacgttgtggatcaacagccgcaaggacgctccgcacaagttggcgtcatgcccta
 Q  H  V  V  D  Q  Q  P  Q  G  R  S  A  Q  V  G  V  M  P  L
gcggtcgcggcaaaacgtgagcaaaggatggccagccggactgatttggaggcagagatg
 A  V  A  A  K  R  E  Q  R  M  A  S  R  T  D  L  E  A  E  M
cggcaagaaaagagcggagaaattaccatggtggagcagaggaggagcagccgtgggtca
 
 R  Q  E  K  S  G  E  I  T  M  V  E  Q  R  R  S  S  R  G  S
tcgaacatgctggatgtcaggaggagtagccgcggttcgtctaacatgctagagaccaga
 S  N  M  L  D  V  R  R  S  S  R  G  S  S  N  M  L  E  T  R
agaagcagtcgtggcagtgagcagatgctgtcatga
 R  S  S  R  G  S  E  Q  M  L  S  -
SEQ ID NO : 93
   LENGTH : 671
   TYPE : PRT
   ORGANISM : *M. phaseolina* 
MAQSLFLLLAAALCSRAATVTYDFNVTWVTANPDAAFRRTTIGINGQWPLPAIDVTKGDRVVINVNN
QLETESTSLHFHGIYMNGTNHMDGPTGVTQCEIPPGSSFTYNFTVDQPGTYWYHSHNRGQYPDGLRG
PFIVRDPDNPFKDDYDEEVVLTFSDWYHDRIPTLMKSFISVTNPTGAEPVPNAALMNDTQNLTFQMT
PGRRYMFRLINIGAFAAQYVWFEGHTMRIVEVDGVYTEAADAERIYMTAAQRYSVIITAKNESTSNF
AFVGSMDQDLFDTIPAGLNNNVTGWLVYNQQNGLLPPLAIGDYDPFDDFTLVPQDGMELYDHVDHSI
TLDMKMDNLGDGANYAFFNDVTYVEPKVPTLYTVLSTGNNATDSRIYGSNTNSFILAKDEVVEIILN
NNDPGKHPFHLHGHAFQAIVRSEEEAGAYVANETFPQTPMRRDTILVRPNGNIVLRFKADNPGVWLF
HCHIEWHVASGLIATMIEAPLDLQSSLGNSIPADHWRACAAAGTPTAGNAAGNTIDYLDLTGENKSP
GPLPSGFEAKGIVALVFSCIAAVLGMAAIVWYGMAPLTDGTGQQQHVVDQQPQGRSAQVGVMPLAVA
AKREQRMASRTDLEAEMRQEKSGEITMVEQRRSSRGSSNMLDVRRSSRGSSNMLETRRSSRGSEQML
S*
SEQ ID NO : 94
   LENGTH : 3063 (including 150 bp 5' UTR and 150 bp 3' UTR)
   TYPE : DNA
   ORGANISM : *M. phaseolina* 
ACGCCTCGCCAACCTCATCTTCGTCGAACAGGCTTAGGTATCAAGCCTCCTTTGCAATCACTTACGC
GGCCCTCTCTACCTCCTGCTCATGTTTGAACAAATCACACCTCTTTGAAACGCTAGAAACACACTGT
TTCTATACCGTCGAGAATGGCCTGGACCTCAGCTCTAGATCGATCCTCGTGGCTTGCCCCAGCTTCC
CCACCAAGGGGGTTTGGTAAGTCGTAAAAGTTTCCTCACAGTTCGCAAATTCAGAGTACCGGTTCAG
CCTCTGATCTTTGCCACTGTCACGGCGGCAGCTGAAGCCGCGTGTACGTCGAATTCTCCCCTGAATA
CGTATGTGCCGGACTTGTATTTCTTCATACCCAGCCGTCTAGGATCGTTCGGTATCGGTCTCTGCAA
CGACTACCACTACGACCACTGGGAAATCCTCCACGTTCTCTACGCTGACCAGCACTTCATCTTCGTC
TACAGCGAAGACTAGCTCGGCCAGCTCGGCATCGCTTTCACGCACTACTCCTTCACCTGCCAGCTCT
TCTTCCGTGAGCTCTTCGTCGATCAGATCCTCTTCTGGCGGATCTTCATCTGCTGGTACTTCTTCTG
CCCGCTCATCATATGTCAGCTCTTCTTTGAGCTCGCGTTCTGCTGTTTCTTCCCTAGTGACCAGCAT
ATCCTCTTCTCTCACCTCAACTGGCACGGTATCGTCCTCGCCCGCTTCGAAGAGCTCCAGCTCTTCA
TCTTCCACTCTTTCAGCCTCTTCAACCACAAAGGTTGCTACTGGTACCCCGTGTGCTGGAAACACTG
CGGCTTCCAGGACAGCCTGGTGCGATCACACCATTGACGATGACTACTATCCCATTATCCCAGATAC
CGGAGTCACACGCGAATACTGGTTCGACCTTGTCGAGGTGACTGTTGCCCCTGATGGCATTGAGAGA
GCTGCCATGGCGGTCAACGGCAGCATCCCTGGACCCACCATTGAGGCGGATTGGGGCGATATCGCCG
TCATGCATGTGACCAACAGCTTGATCTCGAGCAAGAACGGCACTAGCATTCATTTCCACGGTATCCA
GCAGAACTTCACAAATCAAACGGACGGTGTTATTTCCATCACTCAGTGCCCTACCGCTCCTGGCGAG
AACTACACGTATACCTGGAGGGCTGAGCAGTACGGAACTACATGGTGAGCTTGATCCGAGGATACAG
ATGTAAAGGAGATGCTAAGATATCACAGGTACCATTCTCACTTTGCGCTTCAAGCTTGGGAAGGTGT
CTTTGGAGGTACATACATCCCCGCTCCAGGACCTCCAACATTTATTAACGAAGCCCTGCAGGTATCA
AGATCAATGGTCCGGCGAGTGCCAACTACGATTACGATCTTGGCCATGTCTTCCTGTGAGTTTCGAC
GCCCCTTTTCCACTCATTGTATAAAGTAAGCTTATGCGACAAAAGCCAACGATTGGAGCCACGAGAC
TTCGAGCTCTCTCGAGATCGTTTCTGCAATTCGAGGTCCGCCAACACTTGAGAATGCCCTTATCAAC
GGTACCAACGTGTACAACAACTCGGGAACCATAACGGGCTCTCGTTTTGAGACAACATTTGAGGAAG
GCAAATCCTACAGGTTGAGGTTGGTCAGCGGCGCCATCGACACGCATTTCAAGGTGTCATTGGGTAA
GTTGAGCCAAAACATTGAGATTTCCTGAGATCTAAAGCTGATTGTGTTGCAGATAACCACAGCATGC
TTGTCATAGCCAACGACCTTGTACCCATCGTGCCGTACAACACGACCGTCCTGAACATCGGAATGGG
TCATCCACTTTCTATCAGGAACTGGCCATCAAACTGACACTGAACCCCAGGCCAACGCTACGATGTA
ATCATCACCGCCAACCAAGCCGTAGTCGCCACCGACTTCTGGCTGCGTGCCGTCCCGCAGACGGCCT
 
GCTCCAATAACGCCAACCCAGACAATATCAAAGGAATAATCCGGTACAGCACCTCCACCTCCGCCTA
CGACTGGACGAACGAATGCGTCGACGAGGCCCTCACCAACCTCGTGCCATGGGTGACTAAGAACGCG
GCCTCGGGCACGAGTCTATCTGAAGTGGTGACGCTGGGTCGCAACGTTGACAACCTCAACCGCTGGA
TGATGAACAGCACATCGATGGTTGTCGAGTGGAATGATCCCTCGTTGCTGCAGGTTTGGAATAATGA
TACCAATTTCACGGATACGAGCGGCGTGGTCAGGCTTGGTACGGCGGACGAGTGGGTCATGTTTGTT
ATCGAGATGACGCTGCCGATTCCGCGTCCCATCCATCTTCACGGGCATGATGTAGATTTCTTCCAGC
CGCCCCCCCCCCCCCCCTTTTTTTTGGGCTCTACTTCCTAGAATACCGGACGGAGTCGTTGCGGAGC
GCGCCTTCGCCGACTTTTGCGTTCCGATGTCTCAAGAAAGGTGTCCATGTTGTAATGTGTATCCCAG
AGGCTTCCCGGCTGACTTGTTTTGTCAACACAGTTCAACATCCTCGCCCAAGGACCGGCACGTACG
ATTCATCCGTGTCGCTCACCCTGTCAAACCCGCCGCGGAGGGACGTTGCGCTGCTGCCCGCAGCCGG
GTACTTGGTGATTGCATTCGCCACCGATAACCCTGGAGCCTGTGAGGACTTCTCTTTTTCTTGTGCA
TCCCTGGATGTCATCCTTGCTGTGAAATTTTTGGCTGACGCATTCGTCTCAATGCAGGGCTCATGCA
CTGCCACATCGGCCGGCGCACTACGGAGGGCTTCGCAATCCAGATCCTCGAGCGCTGGGACGAGATC
TCGCCGCTTATCGACTACGAGACGCTTGAAGGCAACTGCAACAGATGGGACGCGTACGTGTCGGTCA
GCGATGTCGTACAGGACGATTCTGGCGTATGAGCAATGAAGGTTAGAGTATTTGCGACGTGTTTTAT
CATATATACAACCTTTTTAATCGTAGTCGCTCCTTAACCATAATGTTAATCCATCACTTGGGCCATC
CAAGCACCAACAAAACATTTGAGCCTTCCCAATCAGGACTGGCACAAG
SEQ ID NO : 95
   LENGTH : 2085
   TYPE : DNA
   ORGANISM : *M. phaseolina*
   FEATURE NAME/KEY: CDS
   LOCATION : (1)......(2085) 
atggcctggacctcagctctagatcgatcctcgtggcttgccccagcttccccaccaagg
 M  A  W  T  S  A  L  D  R  S  S  W  L  A  P  A  S  P  P  R
gggtttgctgaagccgcgtgtacgtcgaattctcccctgaatacgtatgtgccggacttg
 G  F  A  E  A  A  C  T  S  N  S  P  L  N  T  Y  V  P  D  L
tatttcttcatacccagccgtctaggatcgttcggtatcggtctctgcaacgactaccac
 Y  F  F  I  P  S  R  L  G  S  F  G  I  G  L  C  N  D  Y  H
tacgaccactgggaaatcctccacgttctctacgctgaccagcacttcatcttcctcttc
 Y  D  H  W  E  I  L  H  V  L  Y  A  D  Q  H  F  I  F  L  F
ttccgtgagctcttcgtcgatcagatcctcttctggcggatcttcatctgctggtacttc
 F  R  E  L  F  V  D  Q  I  L  F  W  R  I  F  I  C  W  Y  F
ttctgcccgctcatcatatgtcagctcttctttgagctcgcgttctgctgtttcttccct
 F  C  P  L  I  I  C  Q  L  F  F  E  L  A  F  C  C  F  F  P
agtgaccagcatatcctcttctctcacctcaactggcacggtatcgtcctcgcccgcttc
 S  D  Q  H  I  L  F  S  H  L  N  W  H  G  I  V  L  A  R  F
gaagagctccagctcttcatcttccactctttcagcctcttcaaccacaaagcctggtgc
 E  E  L  Q  L  F  I  F  H  S  F  S  L  F  N  H  K  A  W  C
gatcacaccattgacgatgactactatcccattatcccagataccggagtcacacgcgaa
 D  H  T  I  D  D  D  Y  Y  P  I  I  P  D  T  G  V  T  R  E
tactggttcgaccttgtcgaggtgactgttgcccctgatggcattgagagagctgccatg
 Y  W  F  D  L  V  E  V  T  V  A  P  D  G  I  E  R  A  A  M
gcggtcaacggcagcatccctggacccaccattgaggcggattggggcgatatcgccgtc
 A  V  N  G  S  I  P  G  P  T  I  E  A  D  W  G  D  I  A  V
atgcatgtgaccaacagcttgatctcgagcaagaacggcactagcattcatttccacggt
 M  H  V  T  N  S  L  I  S  S  K  N  G  T  S  I  H  F  H  G
atccagcagaacttcacaaatcaaacggacggtgttatttccatcactcagtgccctacc
 I  Q  Q  N  F  T  N  Q  T  D  G  V  I  S  I  T  Q  C  P  T
gctcctggcgagaactacacgtatacctggagggctgagcagtacggaactacatggtac
 A  P  G  E  N  Y  T  Y  T  W  R  A  E  Q  Y  G  T  T  W  Y
cattctcactttgcgcttcaagcttgggaaggtgtctttggaggtacatacatccccgct
 H  S  H  F  A  L  Q  A  W  E  G  V  F  G  G  T  Y  I  P  A
ccaggacctccaacatttattaacgaagccctgcaggtatcaagatcaatggtccggcga
 
P G P P T F I N E A L Q V S R S M V R R
gtgccaactacgattacgatcttggccatgtcttcctccaacgattggagccacgagact
V P T T I T I L A M S S S N D W S H E T
tcgagctctctcgagatcgtttctgcaattcgaggtccgccaacacttgagaatgccctt
S S S L E I V S A I R G P P T L E N A L
atcaacggtaccaacgtgtacaacaactcgggaaccataacgggctctcgttttgagaca
I N G T N V Y N N S G T I T G S R F E T
acatttgaggaaggcaaatcctacaggttgaggttggtcagcggcgccatcgacacgcat
T F E E G K S Y R L R L V S G A I D T H
ttcaaggtgtcattggataaccacagcatgcttgtcatagccaacgaccttgtacccatc
F K V S L D N H S M L V I A N D L V P I
gtgccgtacaacacgaccgtcctgaacatcggaatgggccaacgctacgatgtaatcatc
V P Y N T T V L N I G M G Q R Y D V I I
accgccaaccaagccgtagtcgccaccgacttctggctgcgtgccgtcccgcagacggcc
T A N Q A V V A T D F W L R A V P Q T A
tgctccaataacgccaacccagacaatatcaaaggaataatccggtacagcacctccacc
C S N N A N P D N I K G I I R Y S T S T
tccgcctacgactggacgaacgaatgcgtcgacgaggccctcaccaacctcgtgccatgg
S A Y D W T N E C V D E A L T N L V P W
gtgactaagaacgcggcctcgggcacgagtctatctgaagtggtgacgctgggtcgcaac
V T K N A A S G T S L S E V V T L G R N
gttgacaacctcaaccgctggatgatgaacagcacatcgatggttgtcgagtggaatgat
V D N L N R W M M N S T S M V V E W N D
ccctcgttgctgcaggtttggaataatgataccaatttcacggatacgagcggcgtggtc
P S L L Q V W N N D T N F T D T S G V V
aggcttggtacggcggacgagtgggtcatgtttgttatcgagatgacgctgccgattccg
R L G T A D E W V M F V I E M T L P I P
cgtcccatccatcttcacggcatgatttcaacatcctcgcccaaggaccggcacgtac
R P I H L H G H D F N I L A Q G T G T Y
gattcatccgtgtcgctcaccctgtcaaacccgccgcggagggacgttgcgctgctgccc
D S S V S L T L S N P P R R D V A L L P
gcagccgggtacttggtgattgcattcgccaccgataaccctggagcctggctcatgcac
A A G Y L V I A F A T D N P G A W L M H
tgccacatcggccggcgcactacggagggcttcgcaatccagatcctcgagcgctgggac
C H I G R R T T E G F A I Q I L E R W D
gagatctcgccgcttatcgactacgagacgcttgaaggcaactgcaacagatgggacgcg
E I S P L I D Y E T L E G N C N R W D A
tacgtgtcggtcagcgatgtcgtacaggacgattctggcgtatga
Y V S V S D V V Q D D S G V -
SEQ ID NO : 96
   LENGTH : 694
   TYPE : PRT
   ORGANISM : *M. phaseolina* 
MAWTSALDRSSWLAPASPPRGFAEAACTSNSPLNTYVPDLYFFIPSRLGSFGIGLCNDYHYDHWEILHVLYAD
QHFIFLFFRELFVDQILFWRIFICWYFFCPLIICQLFFELAFCCFFPSDQHILFSHLNWHGIVLARFEELQLF
IFHSFSLFNHKAWCDHTIDDDYYPIIPDTGVTREYWFDLVEVTVAPDGIERAAMAVNGSIPGPTIEADWGDIA
VMHVTNSLISSKNGTSIHFHGIQQNFTNQTDGVISITQCPTAPGENYTYTWRAEQYGTTWYHSHFALQAWEGV
FGGTYIPAPGPPTFINEALQVSRSMVRRVPTTITILAMSSSNDWSHETSSSLEIVSAIRGPPTLENALINGTN
VYNNSGTITGSRFETTFEEGKSYRLRLVSGAIDTHFKVSLDNHSMLVIANDLVPIVPYNTTVLNIGMGQRYDV
IITANQAVVATDFWLRAVPQTACSNNANPDNIKGIIRYSTSTSAYDWTNECVDEALTNLVPWVTKNAASGTSL
SEVVTLGRNVDNLNRWMMNSTSMVVEWNDPSLLQVWNNDTNFTDTSGVVRLGTADEWVMFVIEMTLPIPRPIH
LHGHDFNILAQGTGTYDSSVSLTLSNPPRRDVALLPAAGYLVIAFATDNPGAWLMHCHIGRRTTEGFAIQILE
RWDEISPLIDYETLEGNCNRWDAYVSVSDVVQDDSGV*
SEQ ID NO : 97
   LENGTH : 2449 (including 150 bp 5' UTR and 150 bp 3' UTR)
   TYPE : DNA
   ORGANISM : *M. phaseolina* 
GCGCACCCCCGAATTTTCATGTGGCACTTTGCTGGTGGCGATGCATAAAGTCTCCCAAACTTGATCT
TCAGCGTCGAGAGCAGCTCAACGTCCGATCCGATTTTTCGTTCACCGCGCTTCTTGTCCCTCAAGCC
CCCAATACAATTCAACATGTTTTCTTTTGTCGTCCAGAAGCCTCAACTGCCGCTATGGCTGGACTTT
GGCCTTTCAATATGGTCAATGTTCGGCGGAAACAGTCAAGACTGCGTACACTCGCCCCAGAGCAGAC
ACTGCTGGCATGATGGGTTCGATATCAACACCGATTATGAAGCTAAGATACCGCCAGGGAAGCTCGT
GGAGGTAAGATAACCCAGCCACAGACGGCTCAGGCCATCGCAACTCACCTCCCTTAGTACGATTTCA
CTATCTCGGAAGCAGTCCTTGCTCCGGACGGATACTTGACGAATGTCACTCTGGTAAATGGAGTATT
CCCTGGTCCAACCCTCGAAGCTGAATGGGGAGATACGATCAGTATGCCTTCTTCATATGAAATCTTT
CTGTGTGCCTTTTGACGTGATTGCTGACATTTCCTGCCAGGGATAACGATCCACAACAACCTCACAA
ACCACAATGGCACATCCATACATTGGCATGGAATTCGCCAATTCGAGACCAATTGGCTTGATGGTGT
TCCCGGCGTCACTCAATGCCCGTCAAAGGTACTACGGCTGGTTTGCTTTGGGAGAGAAACAGGGGAG
CTAAGTGTCACCAGCCTGGAGACTCGCAAGTTGTCGAATTCCGAGCGATGCAATATGGACCGCATG
GTATCATTCACACTACAGTCTTCAGTGTCAGTATCAATCACAGAGCTCTTGAGCGGCACTTACGCTA
ACCATGATTAGATACAAACGGAGTTCTCGGTATTTGCTATCTCCCTTCCATACAGTTCACAACCTGT
GGGCTGATCCCGTTGCAGGACCCATTCACATCAAGGGACCCTCGAGCATGAACTACGACGTGGATCT
CGGGCCACTGTTGATCAGTGATTGGTATCACCACGATGCCTTCGGCCTATTCCATTATGAGATCGCT
TCACCGCACGCGCCGCTTCCGGTCACAACTATCTTGAATGGCAAGGGAGTCTTTGATTGCGATCCAG
CCAGCGATGCTCGTTGTACGGGAGAGCACCAACGGCACGAGATAGTGTTCGAAGAGGGTAAAAGATA
CAAAATTGGACTAATAAATACCGGCAGCCTTCTGACATACAAGTTCTGGATCGATGGCCATAATTTT
ACAGTTGTGCAGACGGATTTCGTTCCCATCAAACCATACGTCACCGACGTTCTGATCGTCGGGATAG
GTATGTTCAGTGCCTACAAAGAATTGGCGGTAAACTGATCATTCCCAGCTCAACGATACGAGATCAT
TATCGAAGCAAATGTGACATTCACACGTGGCTCCAACTTCTGGATTCACGCAACGTACTGTGACGAT
GATGACATGTTGGACTCGAGAGTTGGCATAGTCCGCTACGACGGCAGCGACGGTCGTGATCCGCACA
CGCCGCCCAAGAGTGAGCAACACCCCGGATACGGGTGTCGTGATCCAGCCACGGAGAATCTTGTTCC
CATCGTGAAGAGGGAAGTAGGCAAGAGAGTGAACGGGCTCAGCCCTGCTGATTACCTCAGGATCGGC
CTGCAGGGCTGGCCCAACATCTCGGACACAGATTCGCTCGTACACAAATGGACACTTACCAACAGAA
CCCAGTACATTGATTGGAGGGAGCCAACAATCAAGGCGCTCACTTCGGATGTCGGGGCTGATTTTGC
GGATGAGACATGCCCCATATACCTGGACTACGAGACTGGCGAGTGGGTGTACTTCGTCATCGAGAAC
AACTACACGCTGAGCGACGCCAACACGCCCCGCACCATCCCCCGCTCGGTCCATCCCATCCACTTAC
ACGGGCATGACTTCGTGATCCTCGCCCAGGGTGACGGCATGTTCGACCCCGTCGACGTGGTGCCGAA
CCTCCACAACCCAACCAGGAGGGACGTGGTCAATTGCCCGATTGGCGGCTACGTATGGATCGCATTC
CAGGTCAACAATCCAGGAGCGTGGCTGATGCATTGCCATATCGCCTGGCATGCCAGCGCCGGACTCT
CACTGCAGTTCATTGAGCAACCTGGCCTGATCAAGGGGTTAATGGAGCAGGCAGGAGCCTTGCCGGA
GCTTGCTGATCGATGCGAGGACTGGACTGAGTACTACAATACTGTAAACATACCAAAAGGCGCACTG
CAAGATGATTCGGGCATATGAGTTGGTATTGAACTTCCGGCTGAACACGCTCAGCCACGGCTACTTG
CTTGGCCAAGATTGAGGGTTGGCCGAGAAATGAGTAGTGGGGATTATCACTTTCTTAGAATGTGCAC
GAAAGGGTAGTGTTCGGTCATGTCTCTTAGCTTGAAA
SEQ ID NO : 98
   LENGTH : 1821
   TYPE : DNA
   ORGANISM : *M. phaseolina*
   FEATURE NAME/KEY: CDS
   LOCATION : (1)......(1821) 
atgttttcttttgtcgtccagaagcctcaactgccgctatggctggactttggcctttca
 M  F  S  F  V  V  Q  K  P  Q  L  P  L  W  L  D  F  G  L  S
atatggtcaatgttcggcggaaacagtcaagactgcgtacactcgccccagagcagacac
 I  W  S  M  F  G  G  N  S  Q  D  C  V  H  S  P  Q  S  R  H
tgctggcatgatgggttcgatatcaacaccgattatgaagctaagataccgccagggaag
 C  W  H  D  G  F  D  I  N  T  D  Y  E  A  K  I  P  P  G  K
ctcgtggagtacgatttcactatctcggaagcagtccttgctccggacggatacttgacg
 
L V E Y D F T I S E A V L A P D G Y L T
aatgtcactctggtaaatggagtattccctggtccaaccctcgaagctgaatggggagat
N V T L V N G V F P G P T L E A E W G D
acgatcaggataacgatccacaacaacctcacaaaccacaatggcacatccatacattgg
T I R I T I H N N L T N H N G T S I H W
catggaattcgccaattcgagaccaattggcttgatggtgttcccggcgtcactcaatgc
H G I R Q F E T N W L D G V P G V T Q C
ccgtcaaagcctggagactcgcaagttgtcgaattccgagcgatgcaatatggaccgca
P S K P G D S Q V V E F R A M Q Y G T A
tggtatcattcacactacagtcttcagtatacaaacggagttctcggacccattcacatc
W Y H S H Y S L Q Y T N G V L G P I H I
aagggaccctcgagcatgaactacgacgtggatctcggcccactgttgatcagtgattgg
K G P S S M N Y D V D L G P L L I S D W
tatcaccacgatgccttcggcctattccattatgagatcgcttcaccgcacgcgccgctt
Y H H D A F G L F H Y E I A S P H A P L
ccggtcacaactatcttgaatggcaagggagtctttgattgcgatccagccagcgatgct
P V T T I L N G K G V F D C D P A S D A
cgttgtacgggagagcaccaacggcacgagatagtgttcgaagagggtaaaagatacaaa
R C T G E H Q R H E I V F E E G K R Y K
attggactaataaataccggcagccttctgacatacaagttctggatcgatggccataat
I G L I N T G S L L T Y K F W I D G H N
tttacagttgtgcagacggatttcgttcccatcaaaccatacgtcaccgacgttctgatc
F T V V Q T D F V P I K P Y V T D V L I
gtcgggatagctcaacgatacgagatcattatcgaagcaaatgtgacattcacacgtggc
V G I A Q R Y E I I I E A N V T F T R G
tccaacttctggattcacgcaacgtactgtgacgatgatgacatgttggactcgagagtt
S N F W I H A T Y C D D D D M L D S R V
ggcatagtccgctacgacggcagcgacggtcgtgatccgcacacgccgcccaagagtgag
G I V R Y D G S D G R D P H T P P K S E
caacaccccggatacggtgtcgtgatccagccacggagaatcttgttcccatcgtgaag
Q H P G Y G C R D P A T E N L V P I V K
agggaagtaggcaagagagtgaacggctcagccctgctgattacctcaggatcggcctg
R E V G K R V N G L S P A D Y L R I G L
cagggctggcccaacatctcggacacagattcgctcgtacacaaatggacacttaccaac
Q G W P N I S D T D S L V H K W T L T N
agaacccagtacattgattggagggagccaacaatcaaggcgctcacttcggatgtcggg
R T Q Y I D W R E P T I K A L T S D V G
gctgattttgcggatgagacatgccccatatacctggactacgagactggcgagtgggtg
A D F A D E T C P I Y L D Y E T G E W V
tacttcgtcatcgagaacaactacacgctgagcgacgccaacacgccccgcaccatcccc
Y F V I E N N Y T L S D A N T P R T I P
cgctcggtccatcccatccacttacacggcatgacttcgtgatcctcgcccagggtgac
R S V H P I H L H G H D F V I L A Q G D
ggcatgttcgaccccgtcgacgtggtgccgaacctccacaacccaaccaggagggacgtg
G M F D P V D V V P N L H N P T R R D V
gtcaattgcccgattggcggctacgtatggatcgcattccaggtcaacaatccaggagcg
V N C P I G G Y V W I A F Q V N N P G A
tggctgatgcattgccatatcgcctggcatgccagcgccggactctcactgcagttcatt
W L M H C H I A W H A S A G L S L Q F I
gagcaacctggcctgatcaaggggttaatggagcaggcaggagccttgccggagcttgct
E Q P G L I K G L M E Q A G A L P E L A
gatcgatgcgaggactggactgagtactacaatactgtaaacataccaaaaggcgcactg
D R C E D W T E Y Y N T V N I P K G A L
caagatgattcgggcatatga
Q D D S G I -
SEQ ID NO : 99
   LENGTH : 606
   TYPE : PRT
   ORGANISM : *M. phaseolina* 
MFSFVVQKPQLPLWLDFGLSIWSMFGGNSQDCVHSPQSRHCWHDGFDINTDYEAKIPPGKLVEYDFT
ISEAVLAPDGYLTNVTLVNGVFPGPTLEAEWGDTIRITIHNNLTNHNGTSIHWHGIRQFETNWLDGV
PGVTQCPSKPGDSQVVEFRAMQYGTAWYHSHYSLQYTNGVLGPIHIKGPSSMNYDVDLGPLLISDWY
HHDAFGLFHYEIASPHAPLPVTTILNGKGVFDCDPASDARCTGEHQRHEIVFEEGKRYKIGLINTGS
LLTYKFWIDGHNFTVVQTDFVPIKPYVTDVLIVGIAQRYEIIIEANVTFTRGSNFWIHATYCDDDDM
LDSRVGIVRYDGSDGRDPHTPPKSEQHPGYGCRDPATENLVPIVKREVGKRVNGLSPADYLRIGLQG
WPNISDTDSLVHKWTLTNRTQYIDWREPTIKALTSDVGADFADETCPIYLDYETGEWVYFVIENNYT
LSDANTPRTIPRSVHPIHLHGHDFVILAQGDGMFDPVDVVPNLHNPTRRDVVNCPIGGYVWIAFQVN
NPGAWLMHCHIAWHASAGLSLQFIEQPGLIKGLMEQAGALPELADRCEDWTEYYNTVNIPKGALQDD
SGI*
SEQ ID NO : 100
   LENGTH : 2347 (including 150 bp 5' UTR and 150 bp 3' UTR)
   TYPE : DNA
   ORGANISM : M. phaseolina 
GATACAACTCTTCTCCCCGCCTCTCTTTCACAAACTGTCTATCGCTCCTTTGTTTTTCTGTCCCTCC
TTAAGGTCACATACTACTGAGACTATTCGTGTCTCGTGCCTCTCATTTAGTTTTAGTTGATTCATC
TTGCCGTTGCTGTAACATGCCGTTTTCTCCACGCCTAAGTATATGCCTTTTAGCATTTTGCTCTCAC
ATTGTGTTCGCACTTTCGATACCAAATGTAAAGGATAGGACCCCCCACTTAAGCCCGCGCAATTACG
GATTTCGTTTCGGCAACCTGTCTTGGGGCGGGGCGGAGCCTTCCGACCCACTCGCACAAGGTGCAGA
ACGTTTGGATGACATCAGTCTCCCGGCCCCTGAAGATGGCTGTCTTTTCTCCAAAGATGCTCGGAAC
TGCTGGCGCGATAACTTCAACATCGACACCGATTTCGACGAGCGCTTCCCCACGACCGGAAGACGG
TCACTGTAAGGAATCATCGCACCTCTTGGAACCCTTACGCTAATACCGAGGGCAGTATAATCTGGAG
ATCACAAACACTACCATGGCCCCTGACGGAATCGAACGCGTCGTCATGGCCGTGAATGGCCAATATC
CCGGCCCGACCCTTATTGCTGATTGGGGGGACACGATGGTTATCAACGTCAAGAATAGCCTGGACCA
CAACGGTACGGGTCTCCATTTCCATGGACTGCGCCAATACAAAAGCAACGGCGCCGATGGGGCGAAC
GGTATCACAGAATGCCCAATTGCCCCCGGCGAGACCAAGACCTACACCTTTCAATGTACCCAGCACG
GTAGCTCGTGGTACCACTCGCATTACTCTGTCCAGTACTCCGACGGCGTTCTCGGCGGCATTATCAT
CAACGGCCCCGCCGACGCGCACTACGACCACGATCTTGGTGTGTACATGCTTTCTGACTGGTACCAC
ACTCCAATGTTTGAACTAGCCGAAGCTGCCAGGCATTCGACAAGGGGCCCACCGAAGGCGGATAACG
GACTCATCAACGGGACGATGAAGAGCCCTGACGGTTCTCTTGGAGCCTATGGCCAGATCCATGTGAA
GAAGGGTCTGCGGTACAGGATTCGCGTAATGAATGTTGGCACTAACGACCACTACCTCTTCTCTGTT
GATGGGCACAACCTCACCGTGATCGCAAGTGATTTCGTGCCGGTCGTGCCCTTTTCGGCGTCCAGCA
TTTCCCTCGGTGTTGGTGAGATATCCGATCTTTATGTTGTTATTTCGCTGCTGACCGTCACTTTAGG
ACAGAGGTACGACGTGATCCTTATCGCCGACCAAGACATTGACAACTACTGGATCCGCTCCGACCCG
GACTCTGCCTGTAGCGTTAACGGCAACGCCGGCAACATAAAAGCCATCCTCTCGTATGACACGGCTC
CCGCGGACGCTCAGCCGAATAGCACGCGCCACAGCATCTCCTCGGGCTGCAAGGACATGGCAGTCGT
GCCAAGAGTTGCTAATACCGTGCCCTCTGATCGCTTCGCGGACGCCGTCCAGAGCCTGGCGATGAGC
GTCAATATCACGCAGCAGAACGGCCCGCTCGTCCAGTGGTATATCAACGGCTCGGCTATGGAGGTCG
ACTGGAGCTACCCGACGGTCCAATATGTCCTAGATGGGAACACGTCGTACCCGCGTGAGCTGAACCT
AGTCCAGCTGGATGAGGCGGACCAGTGGTACTACTTCGTCATCCAGACCGTGCAAGGCTTGCGTGTC
AACCTGCCGCACCCAATTCATCTTCATGGCCACGTACGTTCCCTCCTTTCCCTTCCTCCCTAAACGA
AACAGTAAAAGAGGGACAAGTGAATGCGACGTACTGATTTGTTGTCCAATTGGTCCAGGACTTCTAC
ATCCTCGGCGCCGGTCCCGGCGAGTGGGACGGCAACATCGATGGCCTGCAGTTCGACAACCCTCCGC
GGCGCGATACGGCAATGCTACCTGCGGGTGGCTACCTCATCCTCGCCTTTCCGGCCGACAACCCTGG
CGCCTGGCTCATGCATTGCCACATCCCGTTCCACGTCCAGCAGGGCTTCGGGCTGCAGTTCTTGGAG
CGGCCGGATGAGATTGAGGGCGTCATGGGCGATACGAGCCCGTTTTACAACGAGTGTGCGGCTTGGA
AGGACTACTATGGTGGGGGCCAGGCTTTTCAGCAGTCCGATTCGGGCTTGTAATTGGTCGTGGTGGC
TTGCGCAAGAGCAAAGCGCCGTCGGTGCTCTGGCGGTGATTTTTAGTGGCACGGTTTTTGTACTCAT
 
CAACTCCTAATCACTAAGTCAGCCACCCTCGTTTCCGCCCACCCTGTACAGTCAGCATCATAACGTA
AC
SEQ ID NO : 101
   LENGTH : 1854
   TYPE : DNA
   ORGANISM : M. phaseolina
   FEATURE NAME/KEY : CDS
   LOCATION : (1)......(1854) 
atgccgttttctccacgcctaagtatatgcctttagcatttgctctcacattgtgttc
 M  P  F  S  P  R  L  S  I  C  L  L  A  F  C  S  H  I  V  F
gcactttcgataccaaatgtaaaggataggacccccacttaagcccgcgcaattacgga
 A  L  S  I  P  N  V  K  D  R  T  P  H  L  S  P  R  N  Y  G
tttcgtttcggcaacctgtcttggggcggggcggagccttccgacccactcgcacaaggt
 F  R  F  G  N  L  S  W  G  G  A  E  P  S  D  P  L  A  Q  G
gcagaacgtttggatgacatcagtctcccggcccctgaagatggctgtctttctccaaa
 A  E  R  L  D  D  I  S  L  P  A  P  E  D  G  C  L  F  S  K
gatgctcggaactgctggcgcgataacttcaacatcgacaccgatttcgacgagcgcttc
 D  A  R  N  C  W  R  D  N  F  N  I  D  T  D  F  D  E  R  F
cccacgaccgggaagacggtcacttataatctggagatcacaaacactaccatggcccct
 P  T  T  G  K  T  V  T  Y  N  L  E  I  T  N  T  T  M  A  P
gacggaatcgaacgcgtcgtcatggccgtgaatggccaatatcccggcccgacccttatt
 D  G  I  E  R  V  V  M  A  V  N  G  Q  Y  P  G  P  T  L  I
gctgattgggggacacgatggttatcaacgtcaagaatagcctggaccacaacggtacg
 A  D  W  G  D  T  M  V  I  N  V  K  N  S  L  D  H  N  G  T
ggtctccatttccatggactgcgccaatacaaaagcaacggcgccgatgggcgaacggt
 G  L  H  F  H  G  L  R  Q  Y  K  S  N  G  A  D  G  A  N  G
atcacagaatgcccaattgcccccggcgagaccaagacctacacctttcaatgtacccag
 I  T  E  C  P  I  A  P  G  E  T  K  T  Y  T  F  Q  C  T  Q
cacggtagctcgtggtaccactcgcattactctgtccagtactccgacggcgttctcggc
 H  G  S  S  W  Y  H  S  H  Y  S  V  Q  Y  S  D  G  V  L  G
ggcattatcatcaacggccccgccgacgcgcactacgaccacgatcttggtgtgtacatg
 G  I  I  I  N  G  P  A  D  A  H  Y  D  H  D  L  G  V  Y  M
ctttctgactggtaccacactccaatgtttgaactagccgaagctgccaggcattcgaca
 L  S  D  W  Y  H  T  P  M  F  E  L  A  E  A  A  R  H  S  T
aggggcccaccgaaggcggataacggactcatcaacggacgatgaagagccctgacggt
 R  G  P  P  K  A  D  N  G  L  I  N  G  T  M  K  S  P  D  G
tctcttggagcctatggccagatccatgtgaagaagggtctgcggtacaggattcgcgta
 S  L  G  A  Y  G  Q  I  H  V  K  K  G  L  R  Y  R  I  R  V
atgaatgttggcactaacgaccactacctcttctctgttgatgggcacaacctcaccgtg
 M  N  V  G  T  N  D  H  Y  L  F  S  V  D  G  H  N  L  T  V
atcgcaagtgatttcgtgccggtcgtgccctttcggcgtccagcatttccctcggtgtt
 I  A  S  D  F  V  P  V  V  P  F  S  A  S  S  I  S  L  G  V
ggacagaggtacgacgtgatccttatcgccgaccaagacattgacaactactggatccgc
 G  Q  R  Y  D  V  I  L  I  A  D  Q  D  I  D  N  Y  W  I  R
tccgacccggactctgcctgtagcgttaacggcaacgccggcaacataaaagccatcctc
 S  D  P  D  S  A  C  S  V  N  G  N  A  G  N  I  K  A  I  L
tcgtatgacacggctcccgcggacgctcagccgaatagcacgcgccacagcatctcctcg
 S  Y  D  T  A  P  A  D  A  Q  P  N  S  T  R  H  S  I  S  S
ggctgcaaggacatggcagtcgtgccaagagttgctaataccgtgccctctgatcgcttc
 G  C  K  D  M  A  V  V  P  R  V  A  N  T  V  P  S  D  R  F
gcggacgccgtccagagcctggcgatgagcgtcaatatcacgcagcagaacggcccgctc
 A  D  A  V  Q  S  L  A  M  S  V  N  I  T  Q  Q  N  G  P  L
gtccagtggtatatcaacggctcggctatggaggtcgactggagctacccgacggtccaa
 V  Q  W  Y  I  N  G  S  A  M  E  V  D  W  S  Y  P  T  V  Q
 
tatgtcctagatgggaacacgtcgtacccgcgtgagctgaacctagtccagctggatgag
 Y  V  L  D  G  N  T  S  Y  P  R  E  L  N  L  V  Q  L  D  E
gcggaccagtggtactacttcgtcatccagaccgtgcaaggcttgcgtgtcaacctgccg
 A  D  Q  W  Y  Y  F  V  I  Q  T  V  Q  G  L  R  V  N  L  P
cacccaattcatcttcatggccacgacttctacatcctcggcgccggtcccggcgagtgg
 H  P  I  H  L  H  G  H  D  F  Y  I  L  G  A  G  P  G  E  W
gacggcaacatcgatggcctgcagttcgacaaccctccgcggcgcgatacggcaatgcta
 D  G  N  I  D  G  L  Q  F  D  N  P  P  R  R  D  T  A  M  L
cctgcgggtggctacctcatcctcgcctttccggccgacaaccctggcgcctggctcatg
 P  A  G  G  Y  L  I  L  A  F  P  A  D  N  P  G  A  W  L  M
cattgccacatcccgttccacgtccagcagggcttcggcctgcagttcttggagcggccg
 H  C  H  I  P  F  H  V  Q  Q  G  F  G  L  Q  F  L  E  R  P
gatgagattgagggcgtcatgggcgatacgagcccgttttacaacgagtgtgcggcttgg
 D  E  I  E  G  V  M  G  D  T  S  P  F  Y  N  E  C  A  A  W
aaggactactatggtgggggccaggctttcagcagtccgattcgggcttgtaa
 K  D  Y  Y  G  G  G  Q  A  F  Q  Q  S  D  S  G  L  -
SEQ ID NO : 102
   LENGTH : 617
   TYPE : PRT
   ORGANISM : M. phaseolina 
MPFSPRLSICLLAFCSHIVFALSIPNVKDRTPHLSPRNYGFRFGNLSWGGAEPSDPLAQGAERLDDI
SLPAPEDGCLFSKDARNCWRDNFNIDTDFDERFPTTGKTVTYNLEITNTTMAPDGIERVVMAVNGQY
PGPTLIADWGDTMVINVKNSLDHNGTGLHFHGLRQYKSNGADGANGITECPIAPGETKTYTFQCTQH
GSSWYHSHYSVQYSDGVLGGIIINGPADAHYDHDLGVYMLSDWYHTPMFELAEAARHSTRGPPKADN
GLINGTMKSPDGSLGAYGQIHVKKGLRYRIRVMNVGTNDHYLFSVDGHNLTVIASDFVPVVPFSASS
ISLGVGQRYDVILIADQDIDNYWIRSDPDSACSVNGNAGNIKAILSYDTAPADAQPNSTRHSISSGC
KDMAVVPRVANTVPSDRFADAVQSLAMSVNITQQNGPLVQWYINGSAMEVDWSYPTVQYVLDGNTSY
PRELNLVQLDEADQWYYFVIQTVQGLRVNLPHPIHLHGHDFYILGAGPGEWDGNIDGLQFDNPPRRD
TAMLPAGGYLILAFPADNPGAWLMHCHIPFHVQQGFGLQFLERPDEIEGVMGDTSPFYNECAAWKDY
YGGGQAFQQSDSGL*
SEQ ID NO : 103
   LENGTH : 2415 (including 150 bp 5' UTR and 150 bp 3' UTR)
   TYPE : DNA
   ORGANISM : *M. phaseolina* 
TCGAGCAAGATGAATGTATCCTCCGTGGAAAGTCTTTTTCTCAGGCGGCCGTCCGCTTTGACCTC
TGCCCTCTTGCTGGATTATAGAATACCCAGACGCCCATGTAGTGCATGGCCTTGTGAACTTACCCAC
CACCTCTCTCGCGAAGATGATTGGCCATTCTCTCGTAGCTGTAGCACTCGGCAGCGCAGCCATGGCA
CTGGCTCTTCCTCAGAGCCACGTCCCCGCTGCTTGCATGAACGGTCCGCACTCTCGGAAGTGCTGGG
GCAACTACTCCATCGACACGGACTGGTACACCGAAACTCCATACACGGGCGTGGTGAGAGAATACTG
GTTCCTGGTCGAGAATACCACCGTAGCACCAGATGTACACGCCCCTCTGGGTTGCGCCATTGGCAAC
GCATGGCAGGCTGACTTGGGGTGGCAGGGATATGAGACATGGGCTCTCACGGTAAACCGCTCGATTC
CTGGACCAACAATCGAGGCCAACTGGGGCGACGAAGGTAAAGTTGTTTCGACTGGGATTCGCCGGCC
GATAATGCTTACTGCTACGCAGTAATCGTTCATGTCACCAATGGCATGGAGCGGAATGGTACCGCAA
TCCATTTCCACGGCCTAAGGCAGCTGGGAGCCCACGAAATGGATGGTGTCCCTGGGGTTACGCAGTG
TCCTATAGTACGCCCGTACCCTCTCTCTCTCGCACTGCTGCGCGCAAGCTATTGATACTTCTCAGGC
TCCCGGACACTCCTACACATACAAGTGGCGAGCTACTCAATACGGAACGGTGAATCCCCCCTCGAGG
CAGAATCGGCCATACTTTAACTGACGGGTCTGAAAGAGCTGGTATCATTCCCACTTCAGCATGCAAT
ACTCGGTCGGTCTACAGGGCCCGATCGTCATTCACGGACCTGCTACGGCAGACTATGACGAGGACCT
GGGAACGGTCGTCTTGCAGGACTGGAGTCACACCTCTCCGTTCGCCATGTGGTGGTACGCACGTGTG
CCTTCCGGACCGCCCTCGCTCTCAAACTCGCTCATCAACGGCAAAAACGTCTTCTATTGCGACAGTA
CGACCGATTCGAGATGCTACGGTAACGGCACACGGTCGGAGTGGCGCTTTGAGCAGGGGAAAAAGTA
TCGAATGAGGCTCATCAATACAGGCCTCTACTCAAACTTCCGTTTTGCCATTGACAACCACAACCTC
ACCGTTATTGCGACCGACTTTGTTCCTATCAAACCCTACACCACAGATAACGTGAGTCACCAACCCC
 
TTGCTTTCGTGCACTCTAACGAGACATTTCTCCCAGGTGGCAATTTCAATGGGGCAACGCTACGACA
TCGTCGTTGAGGCAAATCAACCGGAAGGTGATTACTGGCTGCGGTAAGGCTCTAATGAATTGATCAG
ACATTGAAAAAAAAGGGGGGCGGGGGGAACCCCATCAACTGACATTCAACCCTACCTAGGGCTATAT
GGCAAACATCCTGCTGCCCGAACGACTACTCAAACAATACCCTTGGCATAATCCGCTACACAGCAAA
CTCCACTGCCGAACCAAACACAACAAGTCCTGCACTATCCTACCCGGACACATGCGGCGACGAGCCG
GCAGCGAGCCTCGTGCCGCACCTGGCCCTCAACGCGAGCACGCCGGCCGTCGTGCGCACCTACGACC
TGTCCAAAGTCACACTCGAGCTGCCAAAGGGCTTCCTCTGGACACTGAACGACACCTACCTCTGGAT
CAACTGGTCGTCGCCAACGAACTTGAGGCTGGCCGAGGGCGGCGCCGCCGCTGCGGCGAGCCTGCCC
GCCGAATATCTCGCCGTCGACAGCCGGGCCGGCAACGAGGGACGCTGGGCGTATCTCGTGTTCAACG
ACGTCTCCGCCCGCAATCGCTCGCACCCGATGCACCTGCACGGCCACGACTTCTTCCTGCTCGGCAC
CGGCCCTGGCTATTTTGAGTACGGCAGCAACAGCAGCAGCCTGGCGATGCTCAACCTGCACAACCCG
CCTCGTCGCGACACGGCGACCTGGCCCGAGTCTGGCTGGATGGTCGTCGCGTTCCTCATGGACAACC
CGGGGAGCTGGCTGATCCACTGCCACATCGCCTGGCACTCGAGCGAGTCGCTCGGCCTGCAGTTCCT
GGAGAGCCCTGAGACGTATGTCCCTCGATTGGAAGGCCAGAGATTGCGGGAGACGTGCGAGGCGTGG
GATGCATTCTGGAATCGCCACGACTCATACGAGCAAGAGGATGCGGGGATATAAGGGCCGCGAGGAC
TGCATGAACGCATGCTTTGGTCCTGCATGGAGGCTTCGTGAATTGGCGGGAAAGATGTGACTAACTT
CTTCCTCGCATGTTCTTGGCTCTCTCCTTCTCTACACTCTCTAAATAATCCGCAAATTTCCATGTGC
GTA
SEQ ID NO : 104
   LENGTH : 1815
   TYPE : DNA
   ORGANISM : *M. phaseolina*
   FEATURE NAME/KEY: CDS
   LOCATION : (1)......(1815) 
atgattggccattctctcgtagctgtagcactcggcagcgcagccatggcactggctctt
 M  I  G  H  S  L  V  A  V  A  L  G  S  A  A  M  A  L  A  L
cctcagagccacgtccccgctgcttgcatgaacggtccgcactctcggaagtgctggggc
 P  Q  S  H  V  P  A  A  C  M  N  G  P  H  S  R  K  C  W  G
aactactccatcgacacggactggtacaccgaaactccatacacgggcgtggtgagagaa
 N  Y  S  I  D  T  D  W  Y  T  E  T  P  Y  T  G  V  V  R  E
tactggttcctggtcgagaataccaccgtagcaccagatgtacacgcccctctgggttgc
 Y  W  F  L  V  E  N  T  T  V  A  P  D  V  H  A  P  L  G  C
gccattggcaacgcatggcaggctgacttggggtggcagggatatgagacatgggctctc
 A  I  G  N  A  W  Q  A  D  L  G  W  Q  G  Y  E  T  W  A  L
acggtaaaccgctcgattcctggaccaacaatcgaggccaactggggcgacgaagtaatc
 T  V  N  R  S  I  P  G  P  T  I  E  A  N  W  G  D  E  V  I
gttcatgtcaccaatggcatggagcggaatggtaccgcaatccatttccacggcctaagg
 V  H  V  T  N  G  M  E  R  N  G  T  A  I  H  F  H  G  L  R
cagctgggagcccacgaaatggatggtgtccctggggttacgcagtgtcctatagctccc
 Q  L  G  A  H  E  M  D  G  V  P  G  V  T  Q  C  P  I  A  P
ggacactcctacacatacaagtggcgagctactcaatacggaacgagctggtatcattcc
 G  H  S  Y  T  Y  K  W  R  A  T  Q  Y  G  T  S  W  Y  H  S
cacttcagcatgcaatactcggtcggtctacagggcccgatcgtcattcacggacctgct
 H  F  S  M  Q  Y  S  V  G  L  Q  G  P  I  V  I  H  G  P  A
acggcagactatgacgaggacctgggaacggtcgtcttgcaggactggagtcacacctct
 T  A  D  Y  D  E  D  L  G  T  V  V  L  Q  D  W  S  H  T  S
ccgttcgccatgtggtggtacgcacgtgtgccttccggaccgccctcgctctcaaactcg
 P  F  A  M  W  W  Y  A  R  V  P  S  G  P  P  S  L  S  N  S
ctcatcaacggcaaaaacgtcttctattgcgacagtacgaccgattcgagatgctacggt
 L  I  N  G  K  N  V  F  Y  C  D  S  T  T  D  S  R  C  Y  G
aacggcacacggtcggagtggcgctttgagcaggggaaaaagtatcgaatgaggctcatc
 N  G  T  R  S  E  W  R  F  E  Q  G  K  K  Y  R  M  R  L  I
aatacaggcctctactcaaacttccgttttgccattgacaaccacaacctcaccgttatt
 N  T  G  L  Y  S  N  F  R  F  A  I  D  N  H  N  L  T  V  I
 
gcgaccgactttgttcctatcaaaccctacaccacagataacgtggcaatttcaatgggg
 A  T  D  F  V  P  I  K  P  Y  T  T  D  N  V  A  I  S  M  G
caacgctacgacatcgtcgttgaggcaaatcaaccggaaggtgattactggctgcggct
 Q  R  Y  D  I  V  V  E  A  N  Q  P  E  G  D  Y  W  L  R  A
atatggcaaacatcctgctgcccgaacgactactcaaacaatacccttggcataatccgc
 I  W  Q  T  S  C  C  P  N  D  Y  S  N  N  T  L  G  I  I  R
tacacagcaaactccactgccgaaccaaacacaacaagtcctgcactatcctacccggac
 Y  T  A  N  S  T  A  E  P  N  T  T  S  P  A  L  S  Y  P  D
acatgcggcgacgagccggcagcgagcctcgtgccgcacctggccctcaacgcgagcacg
 T  C  G  D  E  P  A  A  S  L  V  P  H  L  A  L  N  A  S  T
ccggccgtcgtgcgcacctacgacctgtccaaagtcacactcgagctgccaaagggcttc
 P  A  V  V  R  T  Y  D  L  S  K  V  T  L  E  L  P  K  G  F
ctctggacactgaacgacacctacctctggatcaactggtcgtcgccaacgaacttgagg
 L  W  T  L  N  D  T  Y  L  W  I  N  W  S  S  P  T  N  L  R
ctggccgagggcggcgccgccgctgcggcgagcctgcccgccgaatatctcgccgtcgac
 L  A  E  G  G  A  A  A  A  A  S  L  P  A  E  Y  L  A  V  D
agccgggccggcaacgagggacgctgggcgtatctcgtgttcaacgacgtctccgcccgc
 S  R  A  G  N  E  G  R  W  A  Y  L  V  F  N  D  V  S  A  R
aatcgctcgcacccgatgcacctgcacggccacgacttcttcctgctcggcaccggccct
 N  R  S  H  P  M  H  L  H  G  H  D  F  F  L  L  G  T  G  P
ggctattttgagtacggcagcaacagcagcagcctggcgatgctcaacctgcacaacccg
 G  Y  F  E  Y  G  S  N  S  S  S  L  A  M  L  N  L  H  N  P
cctcgtcgcgacacggcgacctggcccgagtctggctggatggtcgtcgcgttcctcatg
 P  R  R  D  T  A  T  W  P  E  S  G  W  M  V  V  A  F  L  M
gacaacccgggggagctggctgatccactgccacatcgcctggcactcgagcgagtcgctc
 D  N  P  G  S  W  L  I  H  C  H  I  A  W  H  S  S  E  S  L
ggcctgcagttcctggagagccctgagacgtatgtccctcgattggaaggccagagattg
 G  L  Q  F  L  E  S  P  E  T  Y  V  P  R  L  E  G  Q  R  L
cgggagacgtgcgaggcgtgggatgcattctggaatcgccacgactcatacgagcaagag
 R  E  T  C  E  A  W  D  A  F  W  N  R  H  D  S  Y  E  Q  E
gatgcggggatataa
 D  A  G  I  -
SEQ ID NO : 105
   LENGTH : 604
   TYPE : PRT
   ORGANISM : *M. phaseolina* 
MIGHSLVAVALGSAAMALALPQSHVPAACMNGPHSRKCWGNYSIDTDWYTETPYTGVVREYWFLVEN
TTVAPDVHAPLGCAIGNAWQADLGWQGYETWALTVNRSIPGPTIEANWGDEVIVHVTNGMERNGTAI
HFHGLRQLGAHEMDGVPGVTQCPIAPGHSYTYKWRATQYGTSWYHSHFSMQYSVGLQGPIVIHGPAT
ADYDEDLGTVVLQDWSHTSPFAMWWYARVPSGPPSLSNSLINGKNVFYCDSTTDSRCYGNGTRSEWR
FEQGKKYRMRLINTGLYSNFRFAIDNHNLTVIATDFVPIKPYTTDNVAISMGQRYDIVVEANQPEGD
YWLRAIWQTSCCPNDYSNNTLGIIRYTANSTAEPNTTSPALSYPDTCGDEPAASLVPHLALNASTPA
VVRTYDLSKVTLELPKGFLWTLNDTYLWINWSSPTNLRLAEGGAAAAASLPAEYLAVDSRAGNEGRW
AYLVFNDVSARNRSHPMHLHGHDFFLLGTGPGYFEYGSNSSSLAMLNLHNPPRRDTATWPESGWMVV
AFLMDNPGSWLIHCHIAWHSSESLGLQFLESPETYVPRLEGQRLRETCEAWDAFWNRHDSYEQEDAG
I*
SEQ ID NO : 106
   LENGTH : 2275 (including 150 bp 5' UTR and 150 bp 3' UTR)
   TYPE : DNA
   ORGANISM : *M. phaseolina* 
TCGATGCATCCCTCTCTGACGCAGATCTCTTTTATAAAACCGACTGTCATCCTCGATTGAGTCAGTC
TTTGCTCAAACGGACTTATTCAAAGGTCAGTTGGGTGAGTTGGGAGGCTGATGGATTCAGGATACTC
TCCGGGCACGTTCGCCATGATTCGCCTTTTTCTCGTGCTCGGCTTTGTGGCCACGACATTGGCCAAG
 
ACTGTTACTTTTAACTGGAACATCGGCTGGGTTTCTGCGGCTCCAGATGGATTTACACGGCCCGTCA
TCGGCATCAACGGGCTGTGGCCGCCTCCCGTCTTAGAAGCCGACGTTAACGACACCATTATCGTAAC
CGCGCATAACTCGCTAGGCAATGAGACGACGAGCTTGCACTGGCATGGCATGTGGCAGAACAACTCG
ACTCATATAGACGGCGGAAGTAGAGTCTCGCAGTGCGAAATCCCTCTAGGGGGCACCTTTACGTACA
GGTTTAAGGCATACCCGGCAGGCACTTTTTGGTACCATTCTCACGCTATGGGCCAATATCCCGACGG
CCTACGGGCCCCGATGATTATTCACGACCCTGATTCTGCTGCGGAGCAGGACGCCGATGAGCAGCAT
ATACTTACGGTCTCGGACTGGTACCGTAACCAGATGCCGCCGCTTATCCACCGCTATCTAACTACTC
CTAACTATAACGGCGCCATGCCGAAACCCAACTCGAGCTTAGTTAACGACCAGCAGTCCAAGAGGCT
AAACATCCGCCCGGGCAGAAGAGCTATATCCGCATTATTAATATATCAGCGCTAGCAACGTTCTAT
CTACAGTTCGGTAGGTACATCCCCCTTGTTGTACAGACAAAGCTAACGATTTAGATCAACACAACAT
GACTGTCGTTTCTATCGACGATGTTAACGGTTGGTAATCCTGTTACTTATGCTACGCTAAGATGCAT
GTCGGCATATAAGCCGTAGTCGTATGCAAACACGTAAGATTAAAGTGCTAACGACAGGTAGTCGAGC
CCCAGAGTTGGGAGGCCCTAGAGATTACCCCCGGACAACGGTACGACGTTATCATCACCGGTCTAGA
GAACCCCCAAAGAAACTATGCATTTATCAATAAGATGGCCGTTCTCGGCTTGCAGAACAACAACATC
CTCAGTTATAATTCGTCCTGGCCTGACCCAGAGCCATTGGCCGTGAGTAGGTTCAACCTCGGAAGCG
ATATCAATCTAACTCCGCTTGACCACGAGCCACTGCTGGAGCCCGTGGACAAAACCTTCACCATGGA
GGTCAACAACCTCAACATCGACGGCGTAGGCTACCGGTGAGATTGCCCCGCGGCCCTTAATCCGCAT
GCCGCTTGCTAACTTCGATACACAGCATCACGCAAGGCCCGTACCCCTACATTACCCCGCGCACACC
CACTCTGTACACTGCCCTAACCACCGGCTTTAATGCTACCAACCCCGCCATCTACGGCCAGACCAAC
TCTTACATCGTAGAAGCCGGCGATATCGTCCAGCTCGTTGTCAACAGCAACGACCTTGTCACAACTA
ACACCTCCGGCCGCGGGCACCCCATGCACTTGCACGGCCACACCTTCCAAGTCGTGGGCCAATACGG
CACCCACTGGGACGGCGACACCGCAAAATTCCCTACCGTTCCAATGAAGCGGGACACGACCGTTCTC
TTCGCCGGCGGGAGCTTGGTCATTCGGTTCCAGGCGAACAATCCTGGTGTCTGGATGTGTACGGCTC
TCCTCCACTTTGCACGAAAACCCCAGTCCCCCGGCTGCTTGAAAAGCTTTTTGAACTGACCTTGTGA
GTCGCGCAGTCCACTGCCATATCGAATGGCATCTCGACGCCGGCATGGCCGCCACAATCATCGAAGC
GCCGCTCGAGTTCCAGCGAAGCGGTCTGCGGATCCCGCCACAGCACCTCGCGGCGTGCCGGGCATTA
AACTTAACGACCCGGGGCAATTGTGCCGGCAACACCGTTAACCTGGAGGATACGGCTGCGTGCAGAA
TCTACGACACTGATCCTTGGGGGTGAGTTGCTCTGATTTCGCGTTAACGCAGCCTTTGCTGACGACT
ACCGCTTTCTGCAGTGCGCTTATCGGGGAACGTGAGACAGCACGTTGAATAACACGCTGGCGTAGCA
CCGTATTTTGTATAGACTACTTTTCCATGTTAAATTTTTCTGTATACAGTTCGAAATAGATTCATTT
AGGGACAAATACCAGAAACAGGCTCATCCGCAACTCATGTGCACCCTGCGTAGATCGTTATGCT
SEQ ID NO : 107

   LENGTH : 1632
   TYPE : DNA
   ORGANISM : *M. phaseolina*
   FEATURE NAME/KEY: CDS
   LOCATION : (1)......(1632) 
atgattcgcctttttctcgtgctcggctttgtggccacgacattggccaagactgttact
 M  I  R  L  F  L  V  L  G  F  V  A  T  T  L  A  K  T  V  T
tttaactggaacatcggctgggtttctgcggctccagatggatttacacggcccgtcatc
 F  N  W  N  I  G  W  V  S  A  A  P  D  G  F  T  R  P  V  I
ggcatcaacgggctgtggccgcctcccgtcttagaagccgacgttaacgacaccattatc
 G  I  N  G  L  W  P  P  P  V  L  E  A  D  V  N  D  T  I  I
gtaaccgcgcataactcgctaggcaatgagacgacgagcttgcactggcatggcatgtgg
 V  T  A  H  N  S  L  G  N  E  T  T  S  L  H  W  H  G  M  W
cagaacaactcgactcatatagacggcggaagtagagtctcgcagtgcgaaatccctcta
 Q  N  N  S  T  H  I  D  G  G  S  R  V  S  Q  C  E  I  P  L
gggggcacctttacgtacaggtttaaggcatacccggcaggcacttttggtaccattct
 G  G  T  F  T  Y  R  F  K  A  Y  P  A  G  T  F  W  Y  H  S
cacgctatgggccaatatcccgacggcctacggccccgatgattattcacgaccctgat
 H  A  M  G  Q  Y  P  D  G  L  R  A  P  M  I  I  H  D  P  D
tctgctgcggagcaggacgccgatgagcagcatatacttacggtctcggactggtaccgt
 S  A  A  E  Q  D  A  D  E  Q  H  I  L  T  V  S  D  W  Y  R
aaccagatgccgccgcttatccaccgctatctaactactcctaactataacggcgccatg
 
N Q M P P L I H R Y L T T P N Y N G A M
ccgaaacccaactcgagcttagttaacgaccagcagtccaagaggctaaacatccgcccg
P K P N S S L V N D Q Q S K R L N I R P
gggcagaagagctatatccgcattattaatatatcagcgctagcaacgttctatctacag
G Q K S Y I R I I N I S A L A T F Y L Q
ttcgatcaacacaacatgactgtcgtttctatcgacgatgttaacgtcgagccccagagt
F D Q H N M T V V S I D D V N V E P Q S
tgggaggccctagagattaccccggacaacggtacgacgttatcatcaccggtctagag
W E A L E I T P G Q R Y D V I I T G L E
aacccccaaagaaactatgcatttatcaataagatggccgttctcggcttgcagaacaac
N P Q R N Y A F I N K M A V L G L Q N N
aacatcctcagttataattcgtcctggcctgacccagagccattggccgtgagtaggttc
N I L S Y N S S W P D P E P L A V S R F
aacctcggaagcgatatcaatctaactccgcttgaccacgagccactgctggagcccgtg
N L G S D I N L T P L D H E P L L E P V
gacaaaaccttcaccatggaggtcaacaacctcaacatcgacggcgtaggctaccgcatc
D K T F T M E V N N L N I D G V G Y R I
acgcaaggcccgtaccctacattaccccgcgcacacccactctgtacactgccctaacc
T Q G P Y P Y I T P R T P T L Y T A L T
accggctttaatgctaccaaccccgccatctacggccagaccaactcttacatcgtagaa
T G F N A T N P A I Y G Q T N S Y I V E
gccggcgatatcgtccagctcgttgtcaacagcaacgaccttgtcacaactaacacctcc
A G D I V Q L V V N S N D L V T T N T S
ggccgcgggcaccccatgcacttgcacggccacaccttccaagtcgtgggccaatacggc
G R G H P M H L H G H T F Q V V G Q Y G
acccactgggacggcgacaccgcaaaattccctaccgttccaatgaagcgggacacgacc
T H W D G D T A K F P T V P M K R D T T
gttctcttcgccggcgggagcttggtcattcggttccaggcgaacaatcctggtgtctgg
V L F A G G S L V I R F Q A N N P G V W
atgttccactgccatatcgaatggcatctcgacgccggcatggccgccacaatcatcgaa
M F H C H I E W H L D A G M A A T I I E
gcgccgctcgagttccagcgaagcggtctgcggatcccgccacagcacctcgcggcgtgc
A P L E F Q R S G L R I P P Q H L A A C
cgggcattaaacttaacgacccggggcaattgtgccggcaacaccgttaacctggaggat
R A L N L T T R G N C A G N T V N L E D
acggctgcgtgcagaatctacgacactgatccttggggtgcgcttatcggggaacgtgag
T A A C R I Y D T D P W G A L I G E R E
acagcacgttga
T A R -
SEQ ID NO : 108
   LENGTH : 543
   TYPE : PRT
   ORGANISM : *M. phaseolina* 
MIRLFLVLGFVATTLAKTVTFNWNIGWVSAAPDGFTRPVIGINGLWPPPVLEADVNDTIIVTAHNSL
GNETTSLHWHGMWQNNSTHIDGGSRVSQCEIPLGGTFTYRFKAYPAGTFWYHSHAMGQYPDGLRAPM
IIHDPDSAAEQDADEQHILTVSDWYRNQMPPLIHRYLTTPNYNGAMPKPNSSLVNDQQSKRLNIRPG
QKSYIRIINISALATFYLQFDQHNMTVVSIDDVNVEPQSWEALEITPGQRYDVIITGLENPQRNYAF
INKMAVLGLQNNNILSYNSSWPDPEPLAVSRFNLGSDINLTPLDHEPLLEPVDKTFTMEVNNLNIDG
VGYRITQGPYPYITPRTPTLYTALTTGFNATNPAIYGQTNSYIVEAGDIVQLVVNSNDLVTTNTSGR
GHPMHLHGHTFQVVGQYGTHWDGDTAKFPTVPMKRDTTVLFAGGSLVIRFQANNPGVWMFHCHIEWH
LDAGMAATIIEAPLEFQRSGLRIPPQHLAACRALNLTTRGNCAGNTVNLEDTAACRIYDTDPWGALI
GERETAR*
SEQ ID NO : 109
   LENGTH : 2052 (including 150 bp 5' UTR and 150 bp 3' UTR)
   TYPE : DNA
   ORGANISM : *M. phaseolina* 
GCAGCAACTACATCCGGGTAGGCATCAAACGCAAAGATGGTACGGAGGGAGCACTGATGCGCTTTTA
TAGCTCATATCAGCACAGCTGCGTGTAGTTGAATGGACCGACCGAGTTTTTTGTAGCCTCAATCT
TTCCACGCTCCGAACCATGTCCTCAGATTCTCAGAAGCATGGCGCTGTGCTCCGTAGGCGCAGCATA
CCACAGCAACCAGAAGAAGAAACCCCGCCTTCAACAACCCGGAAGTCATCGGAAAATCGACATAGCG
TTCTATCATGGTTTCTCTTTGTCCTTTCATGCGCCATCTTCGCGACGTTTATTTCCTATCTCAATAG
CGCAACGGCATACCAAACTGCGGGGTCTTATTACACAATAACTGGCCTCAAGGCTTTTCTGTCCCAT
GGGAACTCTGACAACAATTTCGACAGTCATCCTGGCAAGGGCCCATACGGCGGATCTCTGGGCCAGA
ACTTGCATCCTCGAGAGCACGTGGTCCGTGCTCCTAGTGTCAGGCACTACAGTTGGAAGGTTACCAA
GGCTTTTCGATACCCAGATGGGGTGAAAAAGGCTGTTTATCTGATTAACGACGGCTTTCTGGGACCC
ACAGTCGAAGCTCGTTCTGGCGATAGACTGGTGATTGAGGTCCAAAATGCGTTGGAAGACGAAGGTC
TCTCCTTCCACTGGCACGGTCTTTTAATGAGAGGTGCCAACTACATGGACGGTGCCGTCGGATTTAC
GCAAGACGCCATTCACCCGGGCGCCAACTTCACGTATGAGTTCGATATCGCGGATGACCAAGCCGGC
ACATTTTGGTATCACGCTCATGACCAAGTGCAGCGGGCGGATGGCCTGTTCGGAGGACTGATCATCC
ATCGCCCGGAAACCGCAACTGGAGTCGCCGATTTGGATAGATATGGGTACGATGAAGAGAGATTGCT
GCTTATCGGACACTGGTACCATCGTTCCGCACAAGATGTCCTAGCGTGGTACATCAGTGCTGGGTCC
TTTGGAAATGAGCCTGTGCCGGATTCACTCCTCATCAATGGAATGGGAGCATTCAATTGCTCAAAAG
CTATTCCTGCGAGACCTGTGGAATGCATTAACTTTGAAGGAACTGCCACACCTAATCTACAGTTCAA
CTTCACCAGACGTCACCGGCTGAGACTCGTCAACACTGGTACATTGGCTGGATTCACCTTGAGCATT
CCGGGTGCGGCCATGCAGGTCATCGAAGTTGACGGCGGCAATGCCGTTACCAGCGACTCTGAGAACG
ACACTTCAGTAGGGAGCTTATATCCAGGCCAACGTGCCGACCTAATCCTCTCTTGGCCAGAGGATAC
TCTAGAAGCCTCAAAAATTTCAATCACCCTTGACGGGGAGGACTTCAAGTACCCCAATCCAGCCCTG
ACTCGCACTCAGCACTTCTCCATATTTCGCTCTGCCCCTGTCCCGAAGGAAAAGAGCGAAGCCTCTG
CCTCTTCGGAACAAGGCAGGCCAGAAAGCCACGCACCACAAACCCTCATCGACCTCAATGCCCTTGT
CAGCGCAGATATCATCACCCATCACTGCCCCCTGCCACTGAACACACTCTCGTTCTATACGCCAAC
ACCCTCAAACTCTCCCACCGCGGCAACAAACCCCACGGCTACATGAACCAAACCAGCTGGTCGCCGC
AATCCTCTCCGCCCCGTCCGCTCATCGCGCTGCCGCGCTCCTCTTGGGACGCCAACCAGTTCGTCCC
GCGCATCCCTCTTCCCAACGGCACCTCAGACGCGCCCTGGGTCACCATCGTCCTCAACAACCTGGAC
GACGGCTCGCACCCCTTCCACCTACATGGCCACGCGTTCTGGGTGCTCCAAACCCACGCCGCAGGGT
GGGGCTGGGGGTCGTGGAATCCATGAGCGGAGGAGCAGCCGCCGGGCGGCCCACTGGAGCTGCAGCG
CGCGGTGACGAGGGATACGGTCATGGTGCCGCGGAGGGGGTATGCGGTGCTGCGATTTAGAGCGGAC
AATGAGGGGCTCTGGATGCTGCACTGCCACAATTTGTGGCAT
SEQ ID NO : 110
   LENGTH : 1752
   TYPE : DNA
   ORGANISM : *M. phaseolina*
   FEATURE NAME/KEY: CDS
   LOCATION : (1)......(1752) 
atgtcctcagattctcagaagcatggcgctgtgctccgtaggcgcagcataccacagcaa
 M  S  S  D  S  Q  K  H  G  A  V  L  R  R  R  S  I  P  Q  Q
ccagaagaagaaaccccgccttcaacaacccggaagtcatcggaaaatcgacatagcgtt
 P  E  E  E  T  P  P  S  T  T  R  K  S  S  E  N  R  H  S  V
ctatcatggtttctctttgtcctttcatgcgccatcttcgcgacgtttatttcctatctc
 L  S  W  F  L  F  V  L  S  C  A  I  F  A  T  F  I  S  Y  L
aatagcgcaacggcataccaaactgcggggtcttattacacaataactggcctcaaggct
 N  S  A  T  A  Y  Q  T  A  G  S  Y  Y  T  I  T  G  L  K  A
tttctgtcccatgggaactctgacaacaatttcgacagtcatcctggcaagggcccatac
 F  L  S  H  G  N  S  D  N  N  F  D  S  H  P  G  K  G  P  Y
ggcggatctctgggccagaacttgcatcctcgagagcacgtggtccgtgctcctagtgtc
 G  G  S  L  G  Q  N  L  H  P  R  E  H  V  V  R  A  P  S  V
aggcactacagttggaaggttaccaaggcttttcgatacccagatggggtgaaaaaggct
 
R H Y S W K V T K A F R Y P D G V K K A
gtttatctgattaacgacggctttctgggacccacagtcgaagctcgttctggcgataga
V Y L I N D G F L G P T V E A R S G D R
ctggtgattgaggtccaaaatgcgttggaagacgaaggtctctccttccactggcacggt
L V I E V Q N A L E D E G L S F H W H G
cttttaatgagaggtgccaactacatggacggtgccgtcggatttacgcaagacgccatt
L L M R G A N Y M D G A V G F T Q D A I
cacccgggcgccaacttcacgtatgagttcgatatcgcggatgaccaagccggcacattt
H P G A N F T Y E F D I A D D Q A G T F
tggtatcacgctcatgaccaagtgcagcgggcggatggcctgttcggaggactgatcatc
W Y H A H D Q V Q R A D G L F G G L I I
catcgcccggaaaccgcaactggagtcgccgatttggatagatatggtacgatgaagag
H R P E T A T G V A D L D R Y G Y D E E
agattgctgcttatcggacactggtaccatcgttccgcacaagatgtcctagcgtggtac
R L L L I G H W Y H R S A Q D V L A W Y
atcagtgctgggtcctttggaaatgagcctgtgccggattcactcctcatcaatggaatg
I S A G S F G N E P V P D S L L I N G M
ggagcattcaattgctcaaaagctattcctgcgagacctgtggaatgcattaactttgaa
G A F N C S K A I P A R P V E C I N F E
ggaactgccacacctaatctacagttcaacttcaccagacgtcaccggctgagactcgtc
G T A T P N L Q F N F T R R H R L R L V
aacactggtacattggctggattcaccttgagcattccgggtgcggccatgcaggtcatc
N T G T L A G F T L S I P G A A M Q V I
gaagttgacggcggcaatgccgttaccagcgactctgagaacgacacttcagtaggagc
E V D G G N A V T S D S E N D T S V G S
ttatatccaggccaacgtgccgacctaatcctctcttggccagaggatactctagaagcc
L Y P G Q R A D L I L S W P E D T L E A
tcaaaaatttcaatcacccttgacggggaggacttcaagtaccccaatccagccctgact
S K I S I T L D G E D F K Y P N P A L T
cgcactcagcacttctccatatttcgctctgcccctgtcccgaaggaaaagagcgaagcc
R T Q H F S I F R S A P V P K E K S E A
tctgcctcttcggaacaaggcaggccagaaagccacgcaccacaaaccctcatcgacctc
S A S S E Q G R P E S H A P Q T L I D L
aatgcccttgtcagcgcagatatcatcaccccatcactgccccctgccactgaacacact
N A L V S A D I I T P S L P P A T E H T
ctcgttctatacgccaacaccctcaaactctcccaccgcggcaacaaaccccacggctac
L V L Y A N T L K L S H R G N K P H G Y
atgaaccaaaccagctggtcgccgcaatcctctccgccccgtccgctcatcgcgctgccg
M N Q T S W S P Q S S P P R P L I A L P
cgctcctcttgggacgccaaccagttcgtcccgcgcatccctcttcccaacggcacctca
R S S W D A N Q F V P R I P L P N G T S
gacgcgccctgggtcaccatcgtcctcaacaacctggacgacggctcgcaccccttccac
D A P W V T I V L N N L D D G S H P F H
ctacatggccacgcgttctgggtgctccaaacccacgccgcagggtggggctgggggtcg
L H G H A F W V L Q T H A A G W G W G S
tggaatccatga
W N P -
SEQ ID NO : 111
   LENGTH : 583
   TYPE : PRT
   ORGANISM : *M. phaseolina*

MSSDSQKHGAVLRRRSIPQQPEEETPPSTTRKSSENRHSVLSWFLFVLSCAIFATFISYLNSATAYQTAGSYYTITGL
KAFLSHGNSDNNFDSHPGKGPYGGSLGQNLHPREHVVRAPSVRHYSWKVTKAFRYPDGVKKAVYLINDGFLGP
TVEARSGDRLVIEVQNALEDEGLSFHWHGLLMRGANYMDGAVGFTQDAIHPGANFTYEFDIADDQAGTFWY

HAHDQVQRADGLFGGLIIHRPETATGVADLDRYGYDEERLLLIGHWYHRSAQDVLAWYISAGSFGNEPVPDSLLI
NGMGAFNCSKAIPARPVECINFEGTATPNLQFNFTRRHRLRLVNTGTLAGFTLSIPGAAMQVIEVDGGNAVTSD
SENDTSVGSLYPGQRADLILSWPEDTLEASKISITLDGEDFKYPNPALTRTQHFSIFRSAPVPKEKSEASASSEQGRP
ESHAPQTLIDLNALVSADIITPSLPPATEHTLVLYANTLKLSHRGNKPHGYMNQTSWSPQSSPPRPLIALPRSSWD
ANQFVPRIPLPNGTSDAPWVTIVLNNLDDGSHPFHLHGHAFWVLQTHAAGWGWGSWNP*

### INCORPORATION BY REFERENCE

All of the U.S. patents, U.S. published patent applications, and published PCT applications that cited herein are hereby incorporated by reference.

### EQUIVALENTS

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto; the invention may be practiced otherwise than as specifically described and claimed.

## Claims

1. An isolated polynucleotide encoding for a lignin peroxidase comprising a nucleotide sequence which is at least 80% identical to the nucleotide sequence set forth in SEQ ID No. 1, 2, 4, 5, 7, or 8, or any mixtures thereof.

2. An isolated lignin peroxidase polypeptide comprising an amino acid sequence which is at least 70% identical to the amino acid sequence set forth in SEQ ID No. 3, 6, or 9, or any mixtures thereof.

3. An expression construct comprising the isolated polynucleotide of claim 1, wherein said polynucleotide is operably linked to at least one translational regulatory sequence, said translational regulatory sequence comprises a ribosomal binding site, translational start sequence, or translational stop sequence, or combination thereof, which enhances the expression of a polypeptide which is at least 70% identical to the amino acid sequence set forth in SEQ ID No. 3, 6, or 9, or any mixtures thereof.

4. A recombinant gene construct comprising the polynucleotide of claim 1, wherein the polynucleotide is expressible in a host cell to produce an enzyme which degrades lignin.

5. A recombinant gene construct according to claim 4 further comprising a promoter region operably-linked to enhance expression of the polynucleotide template.

6. A transformant comprising the expression construct of claim 3 or recombinant gene construct of claim 4, wherein said transformant produces an enzyme which accelerates lignin degradation.

7. A transgenic fungi of *M. phaseolina* with enhanced lignin degradation, comprising the expression construct of claim 3 or recombinant gene construct of claim 4.

## Patentansprüche

1. Isoliertes Polynukleotid, das für eine Lignin-Peroxidase codiert, umfassend eine Nukleotidsequenz, die zumindest zu 80% identisch ist mit der in SEQ ID No. 1, 2, 4, 5, 7 oder 8 ausgeführten Nukleotidsequenz, oder beliebige Gemische davon.

2. Isoliertes Lignin-Peroxidase-Polypeptid, umfassend eine Aminosäuresequenz, die zumindest zu 70% identisch ist mit der in SEQ ID No. 3, 6 oder 9 ausgeführten Aminosäuresequenz, oder beliebige Gemische davon.

3. Expressionskonstrukt, umfassend das isolierte Polynukleotid aus Anspruch 1, wobei das Polynukleotid funktionsfähig mit mindestens einer translationalen Regulationssequenz verknüpft ist, wobei die translationale Regulationssequenz eine ribosomale Bindungsstelle umfasst, eine translationale Startsequenz oder eine translationale Stopsequenz oder eine Kombination dieser, wodurch die Expression eines Polypeptids verbessert wird, das zumindest zu 70% identisch ist mit der in SEQ ID No. 3, 6 oder 9 ausgeführten Aminosäuresequenz, oder beliebige Gemische davon.

4. Rekombinantes Genkonstrukt, umfassend das Polynukleotid aus Anspruch 1, wobei das Polynukleotid in einer Wirtszelle exprimierbar ist, um ein Enzym zu erzeugen, das Lignin abbaut.

5. Rekombinantes Genkonstrukt nach Anspruch 4, ferner umfassend einer Promotor-Region, die funktionsfähig verknüpft ist, um die Expression der Polynukleotid-Matrize zu verbessern.

6. Transformante, umfassend das Expressionskonstrukt aus Anspruch 3 oder das rekombinante Genkonstrukt aus Anspruch 4, wobei die Transformante ein Enzym erzeugt, das den Ligninabbau beschleunigt.

7. Transgene Pilze von *M. phaseolina* mit verbessertem Ligninabbau, umfassend das Expressionskonstrukt aus Anspruch 3 oder das rekombinante Genkonstrukt aus Anspruch 4.

## Revendications

1. Polynucléotide isolé codant pour une peroxydase de la lignine comprenant une séquence nucléotidique qui est identique au moins à 80 % à la séquence nucléotidique indiquée dans SEQ ID No 1, 2, 4, 5, 7, ou 8, ou tout mélange associé.

2. Polypeptide de peroxydase de la lignine isolé comprenant une séquence d'acides aminés qui est identique au moins à 70 % à la séquence d'acides aminés indiquée dans SEQ ID No 3, 6, ou 9, ou tout mélange associé.

3. Construction d'expression comprenant le polynucléotide isolé selon la revendication 1, ledit polynucléotide étant lié de manière opérationnelle à au moins une séquence régulatrice translationnelle, ladite séquence régulatrice translationnelle comprenant un site de liaison ribosomale, une séquence de départ translationnelle ou une séquence d'arrêt translationnelle, ou une combinaison de celles-ci, qui améliore l'expression d'un polypeptide qui est identique à au moins 70 % à la séquence d'acides aminés indiquée dans SEQ ID No 3, 6 ou 9, ou tout mélange associé.

4. Construction génique de recombinaison comprenant le polynucléotide selon la revendication 1, le polynucléotide pouvant être exprimé dans une cellule hôte pour produire une enzyme qui altère la lignine.

5. Construction génique de recombinaison selon la revendication 4, comprenant en outre une région promotrice liée de manière opérationnelle pour améliorer l'expression de la matrice de polynucléotide.

6. Transformant comprenant la construction d'expression selon la revendication 3 ou construction génique de recombinaison selon la revendication 4, ledit transformant produisant une enzyme qui accélère l'altération de la lignine.

7. Champignons transgéniques de *M. phaseolina* ayant une altération accrue de la lignine, comprenant la construction d'expression selon la revendication 3 ou la construction génique de recombinaison selon la revendication 4.
